# EUROPEAN PATENT SPECIFICATION

(11) **EP 2 260 301 B1**
(45) Date of publication and mention of the grant of the patent: **30.11.2011**
(21) Application number: 09728011.9
(22) Date of filing: 02.04.2009
(51) Int. Cl.: G01N 33/50

(54) **GENE SIGNATURES**
GENSIGNATUREN
SIGNATURES GÉNIQUES

(30) Priority: 03.04.2008 US 72971; 19.05.2008 GB 0809066; 19.05.2008 US 128205; 02.12.2008 US 200705
(43) Date of publication of application: 15.12.2010
(73) Proprietor: Katholieke Universiteit Leuven, 3000 Leuven (BE)
(72) Inventor: HOLVOET, Paul, 3010 Leuven (BE); BENHABILES, Nora, 75016 Paris (FR); GEERAERT, Benjamin, 2950 Kapellen (BE)
(74) Representative: Laenen, Bart Roger Albert
(86) International application number: PCT/BE2009/000022
(87) International publication number: WO 2009/121152

(56) References cited:
- WO-A-00/09678
- WO-A-03/079748
- WO-A-2005/051314
- WO-A-2006/083798
- KIM J-A ET AL: "Phosphorylation of Ser<24> in the pleckstrin homology domain of insulin receptor substrate-1 by mouse Pelle-like kinase/interleukin-1 receptor-associated kinase: Cross-talk between inflammatory signaling and insulin signaling that may contribute to insulin resistance" JOURNAL OF BIOLOGICAL CHEMISTRY 20050617 AMERICAN SOCIETY FOR BIOCHEMISTRY AND MOLECULAR BIOLOGY INC. US, vol. 280, no. 24, 17 June 2005 (2005-06-17), pages 23173-23183, XP002551021

### SEQUENCE LISTING

<110> Katholieke Universiteit Leuven
<120> GENE SIGNATURES
<130> LRD-PCT-2008026
<140> NOT YET KNOWN
   <141> 2009-04-01
<150> US 61/072,971
   <151> 2008-04-03
<150> GB 0809069.8
   <151> 2008-05-19
<150> US 61/128,205
   <151> 2008-05-19
<150> US 61/200,705
   <151> 2008-12-02
<160> 25
<170> PatentIn version 3.4
<210> 1
   <211> 2288
   <212> DNA
   <213> Homo sapiens
<400> 1
   gcctgtcgca ggcgtgcagg gacctggact ccgcctcgtc cccggggctc gggcagccga     60
   gccatggcgg ggaactgtgg ggcccgcggc gcgctgtcgg cgcacacgct gctgttcgac    120
   ctgccgcccg cgctgctcgg agagctctgc gctgttctgg acagctgcga cggcgcgctg    180
   ggctggcgcg gcctggcaga gagactttca agcagctggc tggatgttcg tcatattgaa    240
   aagtatgtag accaaggtaa aagtggaaca agagaattac tttggtcctg ggcacagaaa    300
   aacaagacca tcggtgacct tttacaggtc ctccaggaga tgggacatcg tcgagctatt    360
   catttaatta caaactatgg agcagtgttg agtccttcag agaagagtta tcaggaaggt    420
   ggatttccaa atatattatt caaggaaaca gccaatgtca ccgtggataa tgttcttatt    480
   cctgaacata atgaaaaagg agtactgctt aaatcttcca tcagctttca aaatatcata    540
   gaaggaacta gaaatttcca caaagacttc ctaattggag aaggagagat ttttgaggta    600
   tacagagtgg agattcaaaa cctaacatat gctgtcaaat tatttaaaca ggagaaaaaa    660
   atgcagtgta agaagcattg gaagaggttt ttatctgagc ttgaagtttt actactgttt    720
   catcacccaa acatactaga gttggctgca tattttacag agactgagaa gttctgtctg    780
   atttatccat acatgagaaa tggaacactt tttgacagat tgcagtgtgt aggtgacacg    840
   gccccactcc cttggcacat tcgaatcggt atattaatag gaatatccaa agccattcac    900
   tacctgcaca acgttcaacc atgctcggtc atctgtggca gtatatcaag tgcaaacatc    960
   cttttggatg atcagtttca acccaaacta actgattttg ccatggcaca cttccggtcc   1020
   cacctagaac atcagagttg taccataaat atgaccagca gcagcagtaa acatctgtgg   1080
   tacatgccag aagagtacat cagacagggg aaactttcca ttaaaacaga tgtctacagc 1140
   tttggaattg taataatgga agttctaaca ggatgtagag tagtgttaga tgatccaaaa 1200
   catatccagc tgcgggatct ccttagagaa ttgatggaga agagaggcct ggattcatgt 1260
   ctctcatttc tagataagaa agtgcctccc tgccctcgga atttctctgc caagctcttc 1320
   tgtttggcag gccggtgtgc tgcaacgcgg gcaaagttaa gaccatcaat ggatgaagtt 1380
   ttaaatactc ttgaaagtac tcaagccagc ttgtattttg ctgaagatcc tcccacatca 1440
   ctaaagtcct tcaggtgtcc ttctcctcta ttcctggaga atgtaccaag tattccagtg 1500
   gaagatgatg aaagccagaa taacaattta ctaccttctg atgaaggcct gaggatagac 1560
   agaatgactc agaaaactcc ttttgaatgc agccagtctg aggttatgtt tctgagcttg 1620
   gacaaaaagc cagagagcaa gagaaatgag gaagcttgca acatgcccag ttcttcttgt 1680
   gaagaaagtt ggttcccaaa gtatatagtt ccatcccagg acttaaggcc ctataaggta 1740
   aatatagatc cttcttcaga agctccaggg cattcttgca ggagcaggcc agtggagagc 1800
   agctgttcct ccaaattttc ctgggatgaa tatgaacagt acaaaaaaga ataaattcta 1860
   ccagaagata aagaaaaaag caagtattgc ataggcacct gagcataggt atgaccttgg 1920
   gaagacattg gctccataag caatgccaag agaatgatca atagtgagtt tgggtgatgc 1980
   agataaacaa tctggataat tccatttctt ttttcccaaa ccctcaaaca gagtgcctta 2040
   aaaaattgtt ttatcaggat aattgtctca tgaccaaatc cacgctcaat tagagccatt 2100
   caaaattcct taagatcatg ggttctgact tcagccaaac aaaacaatca aaacctacca 2160
   aaaagggact ggattgtaat gtcctctcca tcatcctcag tgtgagtcct cagagcctcc 2220
   atctgccaag aacattcagt tggattccat cgtttggttt agcttgcttg cacgggttgt 2280
   aggaaatg 2288
<210> 2
   <211> 596
   <212> PRT
   <213> Homo sapiens
<400> 2
   Met Ala Gly Asn Cys Gly Ala Arg Gly Ala Leu Ser Ala His Thr Leu
   1               5                   10                  15
   Leu Phe Asp Leu Pro Pro Ala Leu Leu Gly Glu Leu Cys Ala Val Leu
               20                  25                  30
   Asp Ser Cys Asp Gly Ala Leu Gly Trp Arg Gly Leu Ala Glu Arg Leu
           35                  40                  45
   Ser Ser Ser Trp Leu Asp Val Arg His Ile Glu Lys Tyr Val Asp Gln
       50                  55                  60
   Gly Lys Ser Gly Thr Arg Glu Leu Leu Trp Ser Trp Ala Gln Lys Asn
   65                  70                  75                  80
   Lys Thr Ile Gly Asp Leu Leu Gln Val Leu Gln Glu Met Gly His Arg
                   85                  90                  95
   Arg Ala Ile His Leu Ile Thr Asn Tyr Gly Ala Val Leu Ser Pro Ser
               100                 105                 110
   Glu Lys Ser Tyr Gln Glu Gly Gly Phe Pro Asn Ile Leu Phe Lys Glu
           115                 120                 125
   Thr Ala Asn Val Thr Val Asp Asn Val Leu Ile Pro Glu His Asn Glu
       130                 135                 140
   Lys Gly Val Leu Leu Lys Ser Ser Ile Ser Phe Gln Asn Ile Ile Glu
   145                 150                 155                 160
   Gly Thr Arg Asn Phe His Lys Asp Phe Leu Ile Gly Glu Gly Glu Ile
                   165                 170                 175
   Phe Glu Val Tyr Arg Val Glu Ile Gln Asn Leu Thr Tyr Ala Val Lys
               180                 185                 190
   Leu Phe Lys Gln Glu Lys Lys Met Gln Cys Lys Lys His Trp Lys Arg
           195                 200                 205
   Phe Leu Ser Glu Leu Glu Val Leu Leu Leu Phe His His Pro Asn Ile
       210                 215                 220
   Leu Glu Leu Ala Ala Tyr Phe Thr Glu Thr Glu Lys Phe Cys Leu Ile
   225                 230                 235                 240
   Tyr Pro Tyr Met Arg Asn Gly Thr Leu Phe Asp Arg Leu Gln Cys Val
                   245                 250                 255
   Gly Asp Thr Ala Pro Leu Pro Trp His Ile Arg Ile Gly Ile Leu Ile
               260                 265                 270
   Gly Ile Ser Lys Ala Ile His Tyr Leu His Asn Val Gln Pro Cys Ser
           275                 280                 285
   Val Ile Cys Gly Ser Ile Ser Ser Ala Asn Ile Leu Leu Asp Asp Gln
       290                 295                 300
   Phe Gln Pro Lys Leu Thr Asp Phe Ala Met Ala His Phe Arg Ser His
   305                 310                 315                 320
   Leu Glu His Gln Ser Cys Thr Ile Asn Met Thr Ser Ser Ser Ser Lys
                   325                 330                 335
   His Leu Trp Tyr Met Pro Glu Glu Tyr Ile Arg Gln Gly Lys Leu Ser
   340 345 350
   Ile Lys Thr Asp Val Tyr Ser Phe Gly Ile Val Ile Met Glu Val Leu
   355 360 365
   Thr Gly Cys Arg Val Val Leu Asp Asp Pro Lys His Ile Gln Leu Arg
   370 375 380
   Asp Leu Leu Arg Glu Leu Met Glu Lys Arg Gly Leu Asp Ser Cys Leu
   385 390 395 400
   Ser Phe Leu Asp Lys Lys Val Pro Pro Cys Pro Arg Asn Phe Ser Ala
   405 410 415
   Lys Leu Phe Cys Leu Ala Gly Arg Cys Ala Ala Thr Arg Ala Lys Leu
   420 425 430
   Arg Pro Ser Met Asp Glu Val Leu Asn Thr Leu Glu Ser Thr Gln Ala
   435 440 445
   Ser Leu Tyr Phe Ala Glu Asp Pro Pro Thr Ser Leu Lys Ser Phe Arg
   450 455 460
   Cys Pro Ser Pro Leu Phe Leu Glu Asn Val Pro Ser Ile Pro Val Glu
   465 470 475 480
   Asp Asp Glu Ser Gln Asn Asn Asn Leu Leu Pro Ser Asp Glu Gly Leu
   485 490 495
   Arg Ile Asp Arg Met Thr Gln Lys Thr Pro Phe Glu Cys Ser Gln Ser
   500 505 510
   Glu Val Met Phe Leu Ser Leu Asp Lys Lys Pro Glu Ser Lys Arg Asn
   515 520 525
   Glu Glu Ala Cys Asn Met Pro Ser Ser Ser Cys Glu Glu Ser Trp Phe
   530 535 540
   Pro Lys Tyr Ile Val Pro Ser Gln Asp Leu Arg Pro Tyr Lys Val Asn
   545 550 555 560
   Ile Asp Pro Ser Ser Glu Ala Pro Gly His Ser Cys Arg Ser Arg Pro
   565 570 575
   Val Glu Ser Ser Cys Ser Ser Lys Phe Ser Trp Asp Glu Tyr Glu Gln
   580 585 590
   Tyr Lys Lys Glu
   595
<210> 3
   <211> 522
   <212> PRT
   <213> Homo sapiens
<400> 3
   Met Ser Leu Leu Asn Cys Glu Asn Ser Cys Gly Ser Ser Gln Ser Glu
   1               5                   10                  15
   Ser Asp Cys Cys Val Ala Met Ala Ser Ser Cys Ser Ala Val Thr Lys
               20                  25                  30
   Asp Asp Ser Val Gly Gly Thr Ala Ser Thr Gly Asn Leu Ser Ser Ser
           35                  40                  45
   Phe Met Glu Glu Ile Gln Gly Tyr Asp Val Glu Phe Asp Pro Pro Leu
       50                  55                  60
   Glu Ser Lys Tyr Glu Cys Pro Ile Cys Leu Met Ala Leu Arg Glu Ala
   65                  70                  75                  80
   Val Gln Thr Pro Cys Gly His Arg Phe Cys Lys Ala Cys Ile Ile Lys
                   85                  90                  95
   Ser Ile Arg Asp Ala Gly His Lys Cys Pro Val Asp Asn Glu Ile Leu
               100                 105                 110
   Leu Glu Asn Gln Leu Phe Pro Asp Asn Phe Ala Lys Arg Glu Ile Leu
           115                 120                 125
   Ser Leu Met Val Lys Cys Pro Asn Glu Gly Cys Leu His Lys Met Glu
       130                 135                 140
   Leu Arg His Leu Glu Asp His Gln Ala His Cys Glu Phe Ala Leu Met
   145                 150                 155                 160
   Asp Cys Pro Gln Cys Gln Arg Pro Phe Gln Lys Phe His Ile Asn Ile
                   165                 170                 175
   His Ile Leu Lys Asp Cys Pro Arg Arg Gln Val Ser Cys Asp Asn Cys
               180                 185                 190
   Ala Ala Ser Met Ala Phe Glu Asp Lys Glu Ile His Asp Gln Asn Cys
           195                 200                 205
   Pro Leu Ala Asn Val Ile Cys Glu Tyr Cys Asn Thr Ile Leu Ile Arg
       210                 215                 220
   Glu Gln Met Pro Asn His Tyr Asp Leu Asp Cys Pro Thr Ala Pro Ile
   225                 230                 235                 240
   Pro Cys Thr Phe Ser Thr Phe Gly Cys His Glu Lys Met Gln Arg Asn
                   245                 250                 255
   His Leu Ala Arg His Leu Gln Glu Asn Thr Gln Ser His Met Arg Met
   260 265 270
   Leu Ala Gln Ala Val His Ser Leu Ser Val Ile Pro Asp Ser Gly Tyr
   275 280 285
   Ile Ser Glu Val Arg Asn Phe Gln Glu Thr Ile His Gln Leu Glu Gly
   290 295 300
   Arg Leu Val Arg Gln Asp His Gln Ile Arg Glu Leu Thr Ala Lys Met
   305 310 315 320
   Glu Thr Gln Ser Met Tyr Val Ser Glu Leu Lys Arg Thr Ile Arg Thr
   325 330 335
   Leu Glu Asp Lys Val Ala Glu Ile Glu Ala Gln Gln Cys Asn Gly Ile
   340 345 350
   Tyr Ile Trp Lys Ile Gly Asn Phe Gly Met His Leu Lys Cys Gln Glu
   355 360 365
   Glu Glu Lys Pro Val Val Ile His Ser Pro Gly Phe Tyr Thr Gly Lys
   370 375 380
   Pro Gly Tyr Lys Leu Cys Met Arg Leu His Leu Gln Leu Pro Thr Ala
   385 390 395 400
   Gln Arg Cys Ala Asn Tyr Ile Ser Leu Phe Val His Thr Met Gln Gly
   405 410 415
   Glu Tyr Asp Ser His Leu Pro Trp Pro Phe Gln Gly Thr Ile Arg Leu
   420 425 430
   Thr Ile Leu Asp Gln Ser Glu Ala Pro Val Arg Gln Asn His Glu Glu
   435 440 445
   Ile Met Asp Ala Lys Pro Glu Leu Leu Ala Phe Gln Arg Pro Thr Ile
   450 455 460
   Pro Arg Asn Pro Lys Gly Phe Gly Tyr Val Thr Phe Met His Leu Glu
   465 470 475 480
   Ala Leu Arg Gln Arg Thr Phe Ile Lys Asp Asp Thr Leu Leu Val Arg
   485 490 495
   Cys Glu Val Ser Thr Arg Phe Asp Met Gly Ser Leu Arg Arg Glu Gly
   500 505 510
   Phe Gln Pro Arg Ser Thr Asp Ala Gly Val
   515 520
<210> 4
   <211> 2594
   <212> DNA
   <213> Homo sapiens
<400> 4
   gggcagtggc gtccgcagct ggggcttggc ctgcgggcgg ccagcgaagg tggcgaaggc     60
   tcccactgga tccagagttt gccgtccaag cagcctcgtc tcggcgcgca gtgtctgtgt    120
   ccgtcctcta ccggcgcctt ggctgagcgg agtcgtgcgg ttggtggggg agccctgccc    180
   tcctggttcg gcctccccgc gcactagaac gatcatgaac ttctgaaggg acccagcttt    240
   ctttgtgtgc tccaagtgat ttgcacaaat aataatatat atatttattg aaggagagaa    300
   tcagagcaag tgataatcaa gttactatga gtctgctaaa ctgtgaaaac agctgtggat    360
   tcagccagtc tgaaagtgac tgctgtgtgg ccatggccag ctcctgtagc gctgtaacaa    420
   aagatgatag tgtgggtgga actgccagca cggggaacct ctccagctca tttatggagg    480
   agatccaggg atatgatgta gagtttgacc cacccctgga aagcaagtat gaatgcccca    540
   tctgcttgat ggcattacga gaagcagtgc aaacgccatg cggccatagg ttctgcaaag    600
   cctgcatcat aaaatcaata agggatgcag gtcacaaatg tccagttgac aatgaaatac    660
   tgctggaaaa tcaactattt ccagacaatt ttgcaaaacg tgagattctt tctctgatgg    720
   tgaaatgtcc aaatgaaggt tgtttgcaca agatggaact gagacatctt gaggatcatc    780
   aagcacattg tgagtttgct cttatggatt gtccccaatg ccagcgtccc ttccaaaaat    840
   tccatattaa tattcacatt ctgaaggatt gtccaaggag acaggtttct tgtgacaact    900
   gtgctgcatc aatggcattt gaagataaag agatccatga ccagaactgt cctttggcaa    960
   atgtcatctg tgaatactgc aatactatac tcatcagaga acagatgcct aatcattatg   1020
   atctagactg ccctacagcc ccaattccat gcacattcag tacttttggt tgccatgaaa   1080
   agatgcagag gaatcacttg gcacgccacc tacaagagaa cacccagtca cacatgagaa   1140
   tgttggccca ggctgttcat agtttgagcg ttatacccga ctctgggtat atctcagagg   1200
   tccggaattt ccaggaaact attcaccagt tagagggtcg ccttgtaaga caagaccatc   1260
   aaatccggga gctgactgct aaaatggaaa ctcagagtat gtatgtaagt gagctcaaac   1320
   gaaccattcg aacccttgag gacaaagttg ctgaaatcga agcacagcag tgcaatggaa   1380
   tttatatttg gaagattggc aactttggaa tgcatttgaa atgtcaagaa gaggagaaac   1440
   ctgttgtgat tcatagccct ggattctaca ctggcaaacc cgggtacaaa ctgtgcatgc   1500
   gcttgcacct tcagttaccg actgctcagc gctgtgcaaa ctatatatcc ctttttgtcc   1560
   acacaatgca aggagaatat gacagccacc tcccttggcc cttccagggt acaatacgcc   1620
   ttacaattct tgatcagtct gaagcacctg taaggcaaaa ccacgaagag ataatggatg   1680
   ccaaaccaga gctgcttgct ttccagcgac ccacaatccc acggaaccca aaaggttttg   1740
   gctatgtaac ttttatgcat ctggaagccc taagacaaag aactttcatt aaggatgaca   1800
   cattattagt gcgctgtgag gtctccaccc gctttgacat gggtagcctt cggagggagg 1860
   gttttcagcc acgaagtact gatgcagggg tatagcttgc cctcacttgc tcaaaaacaa 1920
   ctacctggag aaaacagtgc ctttccttgc cctgttctca ataacatgca aacaaacaag 1980
   ccacgggaaa tatgtaatat ctactagtga gtgttgttag agaggtcact tactatttct 2040
   tcctgttaca aatgatctga ggcagttttt tcctgggaat ccacacgttc catgcttttt 2100
   cagaaatgtt aggcctgaag tgcctgtggc atgttgcagc agctattttg ccagttagta 2160
   tacctctttg ttgtactttc ttgggctttt gctctggtgt attttattgt cagaaagtcc 2220
   agactcaaga gtactaaact tttaataata atggattttc cttaaaactt cagtcttttt 2280
   gtagtattat atgtaatata ttaaaagtga aaatcactac cgccttgtgc tagtgccctc 2340
   gagaagagtt attgctctag aaagttgagt tctcattttt ttaacctgtt atagatttca 2400
   gaggatttga accataatcc ttggaaaact taagttctca ttcaccccag tttttcctcc 2460
   aggttgttac taaggatatt cagggatgag tttaaaccct aaatataacc ttaattattt 2520
   agtgtaaaca tgtctgttga ataatacttg tttaagtgtt aaaaaaaaaa aaaaaaagaa 2580
   aaaaaaaaaa aaaa 2594
<210> 5
   <211> 2594
   <212> DNA
   <213> Homo sapiens
<400> 5
   gggcagtggc gtccgcagct ggggcttggc ctgcgggcgg ccagcgaagg tggcgaaggc 60
   tcccactgga tccagagttt gccgtccaag cagcctcgtc tcggcgcgca gtgtctgtgt 120
   ccgtcctcta ccggcgcctt ggctgagcgg agtcgtgcgg ttggtggggg agccctgccc 180
   tcctggttcg gcctccccgc gcactagaac gatcatgaac ttctgaaggg acccagcttt 240
   ctttgtgtgc tccaagtgat ttgcacaaat aataatatat atatttattg aaggagagaa 300
   tcagagcaag tgataatcaa gttactatga gtctgctaaa ctgtgaaaac agctgtggat 360
   tcagccagtc tgaaagtgac tgctgtgtgg ccatggccag ctcctgtagc gctgtaacaa 420
   aagatgatag tgtgggtgga actgccagca cggggaacct ctccagctca tttatggagg 480
   agatccaggg atatgatgta gagtttgacc cacccctgga aagcaagtat gaatgcccca 540
   tctgcttgat ggcattacga gaagcagtgc aaacgccatg cggccatagg ttctgcaaag 600
   cctgcatcat aaaatcaata agggatgcag gtcacaaatg tccagttgac aatgaaatac 660
   tgctggaaaa tcaactattt ccagacaatt ttgcaaaacg tgagattctt tctctgatgg 720
   tgaaatgtcc aaatgaaggt tgtttgcaca agatggaact gagacatctt gaggatcatc 780
   aagcacattg tgagtttgct cttatggatt gtccccaatg ccagcgtccc ttccaaaaat 840
   tccatattaa tattcacatt ctgaaggatt gtccaaggag acaggtttct tgtgacaact 900
   gtgctgcatc aatggcattt gaagataaag agatccatga ccagaactgt cctttggcaa 960
   atgtcatctg tgaatactgc aatactatac tcatcagaga acagatgcct aatcattatg 1020
   atctagactg ccctacagcc ccaattccat gcacattcag tacttttggt tgccatgaaa 1080
   agatgcagag gaatcacttg gcacgccacc tacaagagaa cacccagtca cacatgagaa 1140
   tgttggccca ggctgttcat agtttgagcg ttatacccga ctctgggtat atctcagagg 1200
   tccggaattt ccaggaaact attcaccagt tagagggtcg ccttgtaaga caagaccatc 1260
   aaatccggga gctgactgct aaaatggaaa ctcagagtat gtatgtaagt gagctcaaac 1320
   gaaccattcg aacccttgag gacaaagttg ctgaaatcga agcacagcag tgcaatggaa 1380
   tttatatttg gaagattggc aactttggaa tgcatttgaa atgtcaagaa gaggagaaac 1440
   ctgttgtgat tcatagccct ggattctaca ctggcaaacc cgggtacaaa ctgtgcatgc 1500
   gcttgcacct tcagttaccg actgctcagc gctgtgcaaa ctatatatcc ctttttgtcc 1560
   acacaatgca aggagaatat gacagccacc tcccttggcc cttccagggt acaatacgcc 1620
   ttacaattct tgatcagtct gaagcacctg taaggcaaaa ccacgaagag ataatggatg 1680
   ccaaaccaga gctgcttgct ttccagcgac ccacaatccc acggaaccca aaaggttttg 1740
   gctatgtaac ttttatgcat ctggaagccc taagacaaag aactttcatt aaggatgaca 1800
   cattattagt gcgctgtgag gtctccaccc gctttgacat gggtagcctt cggagggagg 1860
   gttttcagcc acgaagtact gatgcagggg tatagcttgc cctcacttgc tcaaaaacaa 1920
   ctacctggag aaaacagtgc ctttccttgc cctgttctca ataacatgca aacaaacaag 1980
   ccacgggaaa tatgtaatat ctactagtga gtgttgttag agaggtcact tactatttct 2040
   tcctgttaca aatgatctga ggcagttttt tcctgggaat ccacacgttc catgcttttt 2100
   cagaaatgtt aggcctgaag tgcctgtggc atgttgcagc agctattttg ccagttagta 2160
   tacctctttg ttgtactttc ttgggctttt gctctggtgt attttattgt cagaaagtcc 2220
   agactcaaga gtactaaact tttaataata atggattttc cttaaaactt cagtcttttt 2280
   gtagtattat atgtaatata ttaaaagtga aaatcactac cgccttgtgc tagtgccctc 2340
   gagaagagtt attgctctag aaagttgagt tctcattttt ttaacctgtt atagatttca 2400
   gaggatttga accataatcc ttggaaaact taagttctca ttcaccccag tttttcctcc 2460
   aggttgttac taaggatatt cagggatgag tttaaaccct aaatataacc ttaattattt 2520
   agtgtaaaca tgtctgttga ataatacttg tttaagtgtt aaaaaaaaaa aaaaaaagaa 2580
   aaaaaaaaaa aaaa 2594
<210> 6
   <211> 2678
   <212> DNA
   <213> Homo sapiens
<400> 6
   atgcgacccg accgcgctga ggctccagga ccgcccgcca tggctgcagg aggtcccggc 60
   gcggggtctg cggccccggt ctcctccaca tcctcccttc ccctggctgc tctcaacatg 120
   cgagtgcggc gccgcctgtc tctgttcttg aacgtgcgga cacaggtggc ggccgactgg 180
   accgcgctgg cggaggagat ggactttgag tacttggaga tccggcaact ggagacacaa 240
   gcggacccca ctggcaggct gctggacgcc tggcagggac gccctggcgc ctctgtaggc 300
   cgactgctcg agctgcttac caagctgggc cgcgacgacg tgctgctgga gctgggaccc 360
   agcattgagg aggattgcca aaagtatatc ttgaagcagc agcaggagga ggctgagaag 420
   cctttacagg tggccgctgt agacagcagt gtcccacgga cagcagagct ggcgggcatc 480
   accacacttg atgaccccct ggggcatatg cctgagcgtt tcgatgcctt catctgctat 540
   tgccccagcg acatccagtt tgtgcaggag atgatccggc aactggaaca gacaaactat 600
   cgactgaagt tgtgtgtgtc tgaccgcgat gtcctgcctg gcacctgtgt ctggtctatt 660
   gctagtgagc tcatcgaaaa gaggtgccgc cggatggtgg tggttgtctc tgatgattac 720
   ctgcagagca aggaatgtga cttccagacc aaatttgcac tcagcctctc tccaggtgcc 780
   catcagaagc gactgatccc catcaagtac aaggcaatga agaaagagtt ccccagcatc 840
   ctgaggttca tcactgtctg cgactacacc aacccctgca ccaaatcttg gttctggact 900
   cgccttgcca aggccttgtc cctgccctga agactgttct gaggccctgg gtgtgtgtgt 960
   atctgtctgc ctgtccatgt acttctgccc tgcctcctcc tttcgttgta ggaggaatct 1020
   gtgctctact tacctctcaa ttcctggaga tgccaacttc acagacacgt ctgcagcagc 1080
   tggacatcac atttcatgtc ctgcatggaa ccagtggctg tgagtggcat gtccacttgc 1140
   tggattatca gccaggacac tatagaacag gaccagctga gactaagaag gaccagcaga 1200
   gccagctcag ctctgagcca ttcacacatc ttcaccctca gtttcctcac ttgaggagtg 1260
   ggatggggag aacagagagt agctgtgttt gaatccctgt aggaaatggt gaagcatagc 1320
   tctgggtctc ctgggggaga ccaggcttgg ctgcgggaga gctggctgtt gctggactac 1380
   atgctggcca ctgctgtgac cacgacactg ctggggcagc ttcttccaca gtgatgccta 1440
   ctgatgcttc agtgcctctg cacaccgccc attccacttc ctccttcccc acagggcagg 1500
   tggggaagca gtttggccca gcccaaggag accccatctt gagccttatt tcctaatggg 1560
   tccacctctc atctgcatct ttcacacctc ccagcttctg cccaaccttc agcagtgaca 1620
   agtccccaag agactcgcct gagcagcttg ggctgctttt catttccacc tgtcaggatg 1680
   cctgtggtca tgctctcagc tccacctggc atgagaaggg atcctggcct ctggcatatt 1740
   catcaagtat gagttctggg gatgagtcac tgtaatgatg tgagcaggga gccttcctcc 1800
   ctgggccacc tgcagagagc tttcccacca actttgtacc ttgattgcct tacaaagtta 1860
   tttgtttaca aacagcgacc atataaaagc ctcctgcccc aaagcttgtg ggcacatggg 1920
   cacatacaga ctcacataca gacacacaca tatatgtaca gacatgtact ctcacacaca 1980
   caggcaccag catacacacg tttttctagg tacagctccc aggaacagct aggtgggaaa 2040
   gtcccatcac tgagggagcc taaccatgtc cctgaacaaa aattgggcac tcatctattc 2100
   cttttctctt gtgtccctac tcattgaaac caaactctgg aaaggaccca atgtaccagt 2160
   atttatacct ctaatgaagc acagagagag gaagagagct gcttaaactc acacaacaat 2220
   gaactgcaga cacagctgtt ctctccctct ctccttccca gagcaattta tactttaccc 2280
   tcaggctgtc ctctggggag aaggtgccat ggtcttaggt gtctgtgccc caggacagac 2340
   cctaggaccc taaatccaat agaaaatgca tatctttgct ccactttcag ccaggctgga 2400
   gcaaggtacc ttttcttagg atcttgggag ggaatggatg cccctctctg catgatcttg 2460
   ttgaggcatt tagctgccat gcacctgtcc ccctttaata ctgggcattt taaagccatc 2520
   tcaagaggca tcttctacat gttttgtacg cattaaaata atttcaaaga tatctgagaa 2580
   aagccgatat ttgccattct tcctatatcc tggaatatat cttgcatcct gagtttataa 2640
   taataaataa tattctacct tggaaaaaaa aaaaaaaa 2678
<210> 7
   <211> 296
   <212> PRT
   <213> Homo sapiens
<400> 7
   Met Ala Ala Gly Gly Pro Gly Ala Gly Ser Ala Ala Pro Val Ser Ser
   1 5 10 15
   Thr Ser Ser Leu Pro Leu Ala Ala Leu Asn Met Arg Val Arg Arg Arg
   20 25 30
   Leu Ser Leu Phe Leu Asn Val Arg Thr Gln Val Ala Ala Asp Trp Thr
   35 40 45
   Ala Leu Ala Glu Glu Met Asp Phe Glu Tyr Leu Glu Ile Arg Gln Leu
   50 55 60
   Glu Thr Gln Ala Asp Pro Thr Gly Arg Leu Leu Asp Ala Trp Gln Gly
   65 70 75 80
   Arg Pro Gly Ala Ser Val Gly Arg Leu Leu Glu Leu Leu Thr Lys Leu
   85 90 95
   Gly Arg Asp Asp Val Leu Leu Glu Leu Gly Pro Ser Ile Glu Glu Asp
   100 105 110
   Cys Gln Lys Tyr Ile Leu Lys Gln Gln Gln Glu Glu Ala Glu Lys Pro
   115 120 125
   Leu Gln Val Ala Ala Val Asp Ser Ser Val Pro Arg Thr Ala Glu Leu
   130 135 140
   Ala Gly Ile Thr Thr Leu Asp Asp Pro Leu Gly His Met Pro Glu Arg
   145 150 155 160
   Phe Asp Ala Phe Ile Cys Tyr Cys Pro Ser Asp Ile Gln Phe Val Gln
   165 170 175
   Glu Met Ile Arg Gln Leu Glu Gln Thr Asn Tyr Arg Leu Lys Leu Cys
   180 185 190
   Val Ser Asp Arg Asp Val Leu Pro Gly Thr Cys Val Trp Ser Ile Ala
   195 200 205
   Ser Glu Leu Ile Glu Lys Arg Cys Arg Arg Met Val Val Val Val Ser
   210 215 220
   Asp Asp Tyr Leu Gln Ser Lys Glu Cys Asp Phe Gln Thr Lys Phe Ala
   225 230 235 240
   Leu Ser Leu Ser Pro Gly Ala His Gln Lys Arg Leu Ile Pro Ile Lys
   245 250 255
   Tyr Lys Ala Met Lys Lys Glu Phe Pro Ser Ile Leu Arg Phe Ile Thr
   260 265 270
   Val Cys Asp Tyr Thr Asn Pro Cys Thr Lys Ser Trp Phe Trp Thr Arg
   275 280 285
   Leu Ala Lys Ala Leu Ser Leu Pro
   290 295
<210> 8
   <211> 4426
   <212> DNA
   <213> Homo sapiens
<400> 8
   tgccttgacc aggacttggg actttgcgaa aggatcgcgg ggcccggaga ggtgttggag 60
   agcacaatgg ctgaacaagt ccttcctcag gctttgtatt tgagcaatat gcggaaagct 120
   gtgaagatac gggagagaac tccagaagac atttttaaac ctactaatgg gatcattcat 180
   cattttaaaa ccatgcaccg atacacactg gaaatgttca gaacttgcca gttttgtcct 240
   cagtttcggg agatcatcca caaagccctc atcgacagaa acatccaggc caccctggaa 300
   agccagaaga aactcaactg gtgtcgagaa gtccggaagc ttgtggcgct gaaaacgaac 360
   ggtgacggca attgcctcat gcatgccact tctcagtaca tgtggggcgt tcaggacaca 420
   gacttggtac tgaggaaggc gctgttcagc acgctcaagg aaacagacac acgcaacttt 480
   aaattccgct ggcaactgga gtctctcaaa tctcaggaat ttgttgaaac ggggctttgc 540
   tatgatactc ggaactggaa tgatgaatgg gacaatctta tcaaaatggc ttccacagac 600
   acacccatgg cccgaagtgg acttcagtac aactcactgg aagaaataca catatttgtc 660
   ctttgcaaca tcctcagaag gccaatcatt gtcatttcag acaaaatgct aagaagtttg 720
   gaatcaggtt ccaatttcgc ccctttgaaa gtgggtggaa tttacttgcc tctccactgg 780
   cctgcccagg aatgctacag ataccccatt gttctcggct atgacagcca tcattttgta 840
   cccttggtga ccctgaagga cagtgggcct gaaatccgag ctgttccact tgttaacaga 900
   gaccggggaa gatttgaaga cttaaaagtt cactttttga cagatcctga aaatgagatg 960
   aaggagaagc tcttaaaaga gtacttaatg gtgatagaaa tccccgtcca aggctgggac 1020
   catggcacaa ctcatctcat caatgccgca aagttggatg aagctaactt accaaaagaa 1080
   atcaatctgg tagatgatta ctttgaactt gttcagcatg agtacaagaa atggcaggaa 1140
   aacagcgagc aggggaggag agaggggcac gcccagaatc ccatggaacc ttccgtgccc 1200
   cagctttctc tcatggatgt aaaatgtgaa acgcccaact gccccttctt catgtctgtg 1260
   aacacccagc ctttatgcca tgagtgctca gagaggcggc aaaagaatca aaacaaactc 1320
   ccaaagctga actccaagcc gggccctgag gggctccctg gcatggcgct cggggcctct 1380
   cggggagaag cctatgagcc cttggcgtgg aaccctgagg agtccactgg ggggcctcat 1440
   tcggccccac cgacagcacc cagccctttt ctgttcagtg agaccactgc catgaagtgc 1500
   aggagccccg gctgcccctt cacactgaat gtgcagcaca acggattttg tgaacgttgc 1560
   cacaacgccc ggcaacttca cgccagccac gccccagacc acacaaggca cttggatccc 1620
   gggaagtgcc aagcctgcct ccaggatgtt accaggacat ttaatgggat ctgcagtact 1680
   tgcttcaaaa ggactacagc agaggcctcc tccagcctca gcaccagcct ccctccttcc 1740
   tgtcaccagc gttccaagtc agatccctcg cggctcgtcc ggagcccctc cccgcattct 1800
   tgccacagag ctggaaacga cgcccctgct ggctgcctgt ctcaagctgc acggactcct 1860
   ggggacagga cggggacgag caagtgcaga aaagccggct gcgtgtattt tgggactcca 1920
   gaaaacaagg gcttttgcac actgtgtttc atcgagtaca gagaaaacaa acattttgct 1980
   gctgcctcag ggaaagtcag tcccacagcg tccaggttcc agaacaccat tccgtgcctg 2040
   gggagggaat gcggcaccct tggaagcacc atgtttgaag gatactgcca gaagtgtttc 2100
   attgaagctc agaatcagag atttcatgag gccaaaagga cagaagagca actgagatcg 2160
   agccagcgca gagatgtgcc tcgaaccaca caaagcacct caaggcccaa gtgcgcccgg 2220
   gcctcctgca agaacatcct ggcctgccgc agcgaggagc tctgcatgga gtgtcagcat 2280
   cccaaccaga ggatgggccc tggggcccac cggggtgagc ctgcccccga agaccccccc 2340
   aagcagcgtt gccgggcccc cgcctgtgat cattttggca atgccaagtg caacggctac 2400
   tgcaacgaat gctttcagtt caagcagatg tatggctaac cggaaacagg tgggtcacct 2460
   cctgcaagaa gtggggcctc gagctgtcag tcatcatggt gctatcctct gaacccctca 2520
   gctgccactg caacagtggg cttaagggtg tctgagcagg agaggaaaga taagctcttc 2580
   gtggtgccca cgatgctcag gtttggtaac ccgggagtgt tcccaggtgg ccttagaaag 2640
   caaagcttgt aactggcaag ggatgatgtc agattcagcc caaggttcct cctctcctac 2700
   caagcaggag gccaggaact tctttggact tggaaggtgt gcggggactg gccgaggccc 2760
   ctgcaccctg cgcatcagga ctgcttcatc gtcttggctg agaaagggaa aagacacaca 2820
   agtcgcgtgg gttggagaag ccagagccat tccacctccc ctcccccagc atctctcaga 2880
   gatgtgaagc cagatcctca tggcagcgag gccctctgca agaagctcaa ggaagctcag 2940
   ggaaaatgga cgtattcaga gagtgtttgt agttcatggt ttttccctac ctgcccggtt 3000
   cctttcctga ggacccggca gaaatgcaga accatccatg gactgtgatt ctgaggctgc 3060
   tgagactgaa catgttcaca ttgacagaaa aacaagctgc tctttataat atgcaccttt 3120
   taaaaaatta gaatatttta ctgggaagac gtgtaactct ttgggttatt actgtcttta 3180
   cttctaaaga agttagcttg aactgaggag taaaagtgtg tacatatata atataccctt 3240
   acattatgta tgagggattt ttttaaatta tattgaaatg ctgccctaga agtacaatag 3300
   gaaggctaaa taataataac ctgttttctg gttgttgttg gggcatgagc ttgtgtatac 3360
   actgcttgca taaactcaac cagctgcctt tttaaaggga gctctagtcc tttttgtgta 3420
   attcacttta tttattttat tacaaacttc aagattattt aagtgaagat atttcttcag 3480
   ctctggggaa aatgccacag tgttctcctg agagaacatc cttgctttga gtcaggctgt 3540
   gggcaagttc ctgaccacag ggagtaaatt ggcctctttg atacactttt gcttgcctcc 3600
   ccaggaaaga aggaattgca tccaaggtat acatacatat tcatcgatgt ttcgtgcttc 3660
   tccttatgaa actccagcta tgtaataaaa aactatactc tgtgttctgt taatgcctct 3720
   gagtgtccta cctccttgga gatgagatag ggaaggagca gggatgagac tggcaatggt 3780
   cacagggaaa gatgtggcct tttgtgatgg ttttattttc tgttaacact gtgtcctggg 3840
   ggggctggga agtcccctgc atcccatggt accctggtat tgggacagca aaagccagta 3900
   accatgagta tgaggaaatc tctttctgtt gctggcttac agtttctctg tgtgctttgt 3960
   ggttgctgtc atatttgctc tagaagaaaa aaaaaaaagg aggggaaatg cattttcccc 4020
   agagataaag gctgccattt tgggggtctg tacttatggc ctgaaaatat ttgtgatcca 4080
   taactctaca cagcctttac tcatactatt aggcacactt tccccttaga gccccctaag 4140
   tttttcccag acgaatcttt ataatttcct ttccaaagat accaaataaa cttcagtgtt 4200
   ttcatctaat tctcttaaag ttgatatctt aatattttgt gttgatcatt atttccattc 4260
   ttaatgtgaa aaaaagtaat tatttatact tattataaaa agtatttgaa atttgcacat 4320
   ttaattgtcc ctaatagaaa gccacctatt ctttgttgga tttcttcaag tttttctaaa 4380
   taaatgtaac ttttcacaag agtcaacatt aaaaaataaa ttattt 4426
<210> 9
   <211> 790
   <212> PRT
   <213> Homo sapiens
<400> 9
<210> 10
   <211> 1440
   <212> DNA
   <213> Homo sapiens
<400> 10
   cagtcacatt tcagccactg ctctgagaat ttgtgagcag cccctaacag gctgttactt 60
   cactacaact gacgatatga tcatcttaat ttacttattt ctcttgctat gggaagacac 120
   tcaaggatgg ggattcaagg atggaatttt tcataactcc atatggcttg aacgagcagc 180
   cggtgtgtac cacagagaag cacggtctgg caaatacaag ctcacctacg cagaagctaa 240
   ggcggtgtgt gaatttgaag gcggccatct cgcaacttac aagcagctag aggcagccag    300
   aaaaattgga tttcatgtct gtgctgctgg atggatggct aagggcagag ttggataccc    360
   cattgtgaag ccagggccca actgtggatt tggaaaaact ggcattattg attatggaat    420
   ccgtctcaat aggagtgaaa gatgggatgc ctattgctac aacccacacg caaaggagtg    480
   tggtggcgtc tttacagatc caaagcaaat ttttaaatct ccaggcttcc caaatgagta    540
   cgaagataac caaatctgct actggcacat tagactcaag tatggtcagc gtattcacct    600
   gagtttttta gattttgacc ttgaagatga cccaggttgc ttggctgatt atgttgaaat    660
   atatgacagt tacgatgatg tccatggctt tgtgggaaga tactgtggag atgagcttcc    720
   agatgacatc atcagtacag gaaatgtcat gaccttgaag tttctaagtg atgcttcagt    780
   gacagctgga ggtttccaaa tcaaatatgt tgcaatggat cctgtatcca aatccagtca    840
   aggaaaaaat acaagtacta cttctactgg aaataaaaac tttttagctg gaagatttag    900
   ccacttataa aaaaaaaaaa aaggatgatc aaaacacaca gtgtttatgt tggaatcttt    960
   tggaactcct ttgatctcac tgttattatt aacatttatt tattattttt ctaaatgtga   1020
   aagcaataca taatttaggg aaaattggaa aatataggaa actttaaacg agaaaatgaa   1080
   acctctcata atcccactgc atagaaataa caagcgttaa cattttcata tttttttctt   1140
   tcagtcattt ttctatttgt ggtatatgta tatatgtacc tatatgtatt tgcatttgaa   1200
   attttggaat cctgctctat gtacagtttt gtattatact ttttaaatct tgaactttat   1260
   aaacattttc tgaaatcatt gattattcta caaaaacatg attttaaaca gctgtaaaat   1320
   attctatgat atgaatgttt tatgcattat ttaagcctgt ctctattgtt ggaatttcag   1380
   gtcattttca taaatattgt tgcaataaat atccttgaac acaaaaaaaa aaaaaaaaaa   1440
<210> 11
   <211> 277
   <212> PRT
   <213> Homo sapiens
<400> 11
   Met Ile Ile Leu Ile Tyr Leu Phe Leu Leu Leu Trp Glu Asp Thr Gln
   1               5                   10                  15
   Gly Trp Gly Phe Lys Asp Gly Ile Phe His Asn Ser Ile Trp Leu Glu
               20                  25                  30
   Arg Ala Ala Gly Val Tyr His Arg Glu Ala Arg Ser Gly Lys Tyr Lys
           35                  40                  45
   Leu Thr Tyr Ala Glu Ala Lys Ala Val Cys Glu Phe Glu Gly Gly His
       50                  55                  60
   Leu Ala Thr Tyr Lys Gln Leu Glu Ala Ala Arg Lys Ile Gly Phe His
   65                  70                  75                  80
   Val Cys Ala Ala Gly Trp Met Ala Lys Gly Arg Val Gly Tyr Pro Ile
   85 90 95
   Val Lys Pro Gly Pro Asn Cys Gly Phe Gly Lys Thr Gly Ile Ile Asp
   100 105 110
   Tyr Gly Ile Arg Leu Asn Arg Ser Glu Arg Trp Asp Ala Tyr Cys Tyr
   115 120 125
   Asn Pro His Ala Lys Glu Cys Gly Gly Val Phe Thr Asp Pro Lys Gln
   130 135 140
   Ile Phe Lys Ser Pro Gly Phe Pro Asn Glu Tyr Glu Asp Asn Gln Ile
   145 150 155 160
   Cys Tyr Trp His Ile Arg Leu Lys Tyr Gly Gln Arg Ile His Leu Ser
   165 170 175
   Phe Leu Asp Phe Asp Leu Glu Asp Asp Pro Gly Cys Leu Ala Asp Tyr
   180 185 190
   Val Glu Ile Tyr Asp Ser Tyr Asp Asp Val His Gly Phe Val Gly Arg
   195 200 205
   Tyr Cys Gly Asp Glu Leu Pro Asp Asp Ile Ile Ser Thr Gly Asn Val
   210 215 220
   Met Thr Leu Lys Phe Leu Ser Asp Ala Ser Val Thr Ala Gly Gly Phe
   225 230 235 240
   Gln Ile Lys Tyr Val Ala Met Asp Pro Val Ser Lys Ser Ser Gln Gly
   245 250 255
   Lys Asn Thr Ser Thr Thr Ser Thr Gly Asn Lys Asn Phe Leu Ala Gly
   260 265 270
   Arg Phe Ser His Leu
   275
<210> 12
   <211> 5828
   <212> DNA
   <213> Homo sapiens
<400> 12
   cggcggcgcg gtcggagggg gccggcgcgc agagccagac gccgccgctt gttttggttg 60
   gggctctcgg caactctccg aggaggagga ggaggaggga ggaggggaga agtaactgca 120
   gcggcagcgc cctcccgagg aacaggcgtc ttccccgaac ccttcccaaa cctcccccat 180
   cccctctcgc ccttgtcccc tcccctcctc cccagccgcc tggagcgagg ggcagggatg 240
   agtctgtccc tccggccggt ccccagctgc agtggctgcc cggtatcgtt tcgcatggaa 300
   aagccacttt ctccacccgc cgagatgggc ccggatgggg ctgcagagga cgcgcccgcg     360
   ggcggcggca gcagcagcag cagcagcagc agcaacagca acagccgcag cgccgcggtc     420
   tctgcgactg agctggtatt tgggcggctg gtggcggctg ggacggttgg ggggtgggag     480
   gaggcgaagg aggagggaga accccgtgca acgttgggac ttggcaaccc gcctccccct     540
   gcccaaggat atttaatttg cctcgggaat cgctgcttcc agaggggaac tcaggaggga     600
   aggcgcgcgc gcgcgcgcgc tcctggaggg gcaccgcagg gacccccgac tgtcgcctcc     660
   ctgtgccgga ctccagccgg ggcgacgaga gatgcatctt cgctccttcc tggtggcggc     720
   ggcggctgag aggagacttg gctctcggag gatcggggct gccctcaccc cggacgcact     780
   gcctccccgc cggcgtgaag cgcccgaaaa ctccggtcgg gctctctcct gggctcagca     840
   gctgcgtcct ccttcagctg cccctccccg gcgcgggggg cggcgtggat ttcagagtcg     900
   gggtttctgc tgcctccagc cctgtttgca tgtgccgggc cgcggcgagg agcctccgcc     960
   ccccacccgg ttgtttttcg gagcctccct ctgctcagcg ttggtggtgg cggtggcagc    1020
   atggcgagcc ctccggagag cgatggcttc tcggacgtgc gcaaggtggg ctacctgcgc    1080
   aaacccaaga gcatgcacaa acgcttcttc gtactgcgcg cggccagcga ggctgggggc    1140
   ccggcgcgcc tcgagtacta cgagaacgag aagaagtggc ggcacaagtc gagcgccccc    1200
   aaacgctcga tcccccttga gagctgcttc aacatcaaca agcgggctga ctccaagaac    1260
   aagcacctgg tggctctcta cacccgggac gagcactttg ccatcgcggc ggacagcgag    1320
   gccgagcaag acagctggta ccaggctctc ctacagctgc acaaccgtgc taagggccac    1380
   cacgacggag ctgcggccct cggggcggga ggtggtgggg gcagctgcag cggcagctcc    1440
   ggccttggtg aggctgggga ggacttgagc tacggtgacg tgcccccagg acccgcattc    1500
   aaagaggtct ggcaagtgat cctgaagccc aagggcctgg gtcagacaaa gaacctgatt    1560
   ggtatctacc gcctttgcct gaccagcaag accatcagct tcgtgaagct gaactcggag    1620
   gcagcggccg tggtgctgca gctgatgaac atcaggcgct gtggccactc ggaaaacttc    1680
   ttcttcatcg aggtgggccg ttctgccgtg acggggcccg gggagttctg gatgcaggtg    1740
   gatgactctg tggtggccca gaacatgcac gagaccatcc tggaggccat gcgggccatg    1800
   agtgatgagt tccgccctcg cagcaagagc cagtcctcgt ccaactgctc taaccccatc    1860
   agcgtccccc tgcgccggca ccatctcaac aatcccccgc ccagccaggt ggggctgacc    1920
   cgccgatcac gcactgagag catcaccgcc acctccccgg ccagcatggt gggcgggaag    1980
   ccaggctcct tccgtgtccg cgcctccagt gacggcgaag gcaccatgtc ccgcccagcc    2040
   tcggtggacg gcagccctgt gagtcccagc accaacagaa cccacgccca ccggcatcgg    2100
   ggcagcgccc ggctgcaccc cccgctcaac cacagccgct ccatccccat gccggcttcc    2160
   cgctgctcgc cttcggccac cagcccggtc agtctgtcgt ccagtagcac cagtggccat    2220
   ggctccacct cggattgtct cttcccacgg cgatctagtg cttcggtgtc tggttccccc    2280
   agcgatggcg gtttcatctc ctcggatgag tatggctcca gtccctgcga tttccggagt    2340
   tccttccgca gtgtcactcc ggattccctg ggccacaccc caccagcccg cggtgaggag 2400
   gagctaagca actatatctg catgggtggc aaggggccct ccaccctgac cgcccccaac 2460
   ggtcactaca ttttgtctcg gggtggcaat ggccaccgct gcaccccagg aacaggcttg 2520
   ggcacgagtc cagccttggc tggggatgaa gcagccagtg ctgcagatct ggataatcgg 2580
   ttccgaaaga gaactcactc ggcaggcaca tcccctacca ttacccacca gaagaccccg 2640
   tcccagtcct cagtggcttc cattgaggag tacacagaga tgatgcctgc ctacccacca 2700
   ggaggtggca gtggaggccg actgccggga cacaggcact ccgccttcgt gcccacccgc 2760
   tcctacccag aggagggtct ggaaatgcac cccttggagc gtcggggggg gcaccaccgc 2820
   ccagacagct ccaccctcca cacggatgat ggctacatgc ccatgtcccc aggggtggcc 2880
   ccagtgccca gtggccgaaa gggcagtgga gactatatgc ccatgagccc caagagcgta 2940
   tctgccccac agcagatcat caatcccatc agacgccatc cccagagagt ggaccccaat 3000
   ggctacatga tgatgtcccc cagcggtggc tgctctcctg acattggagg tggccccagc 3060
   agcagcagca gcagcagcaa cgccgtccct tccgggacca gctatggaaa gctgtggaca 3120
   aacggggtag ggggccacca ctctcatgtc ttgcctcacc ccaaaccccc agtggagagc 3180
   agcggtggta agctcttacc ttgcacaggt gactacatga acatgtcacc agtgggggac 3240
   tccaacacca gcagcccctc cgactgctac tacggccctg aggaccccca gcacaagcca 3300
   gtcctctcct actactcatt gccaagatcc tttaagcaca cccagcgccc cggggagccg 3360
   gaggagggtg cccggcatca gcacctccgc ctttccacta gctctggtcg ccttctctat 3420
   gctgcaacag cagatgattc ttcctcttcc accagcagcg acagcctggg tgggggatac 3480
   tgcggggcta ggctggagcc cagccttcca catccccacc atcaggttct gcagccccat 3540
   ctgcctcgaa aggtggacac agctgctcag accaatagcc gcctggcccg gcccacgagg 3600
   ctgtccctgg gggatcccaa ggccagcacc ttacctcggg cccgagagca gcagcagcag 3660
   cagcagccct tgctgcaccc tccagagccc aagagcccgg gggaatatgt caatattgaa 3720
   tttgggagtg atcagtctgg ctacttgtct ggcccggtgg ctttccacag ctcaccttct 3780
   gtcaggtgtc catcccagct ccagccagct cccagagagg aagagactgg cactgaggag 3840
   tacatgaaga tggacctggg gccgggccgg agggcagcct ggcaggagag cactggggtc 3900
   gagatgggca gactgggccc tgcacctccc ggggctgcta gcatttgcag gcctacccgg 3960
   gcagtgccca gcagccgggg tgactacatg accatgcaga tgagttgtcc ccgtcagagc 4020
   tacgtggaca cctcgccagc tgcccctgta agctatgctg acatgcgaac aggcattgct 4080
   gcagaggagg tgagcctgcc cagggccacc atggctgctg cctcctcatc ctcagcagcc 4140
   tctgcttccc cgactgggcc tcaaggggca gcagagctgg ctgcccactc gtccctgctg 4200
   gggggcccac aaggacctgg gggcatgagc gccttcaccc gggtgaacct cagtcctaac 4260
   cgcaaccaga gtgccaaagt gatccgtgca gacccacaag ggtgccggcg gaggcatagc 4320
   tccgagactt tctcctcaac acccagtgcc acccgggtgg gcaacacagt gccctttgga 4380
   gcgggggcag cagtaggggg cggtggcggt agcagcagca gcagcgagga tgtgaaacgc 4440
   cacagctctg cttcctttga gaatgtgtgg ctgaggcctg gggagcttgg gggagccccc 4500
   aaggagccag ccaaactgtg tggggctgct gggggtttgg agaatggtct taactacata 4560
   gacctggatt tggtcaagga cttcaaacag tgccctcagg agtgcacccc tgaaccgcag 4620
   cctcccccac ccccaccccc tcatcaaccc ctgggcagcg gtgagagcag ctccacccgc 4680
   cgctcaagtg aggatttaag cgcctatgcc agcatcagtt tccagaagca gccagaggac 4740
   cgtcagtagc tcaactggac atcacagcag aatgaagacc taaatgacct cagcaaatcc 4800
   tcttctaact catgggtacc cagactctaa atatttcatg attcacaact aggacctcat 4860
   atcttcctca tcagtagatg gtacgatgca tccatttcag tttgtttact ttatccaatc 4920
   ctcaggattt cattgactga actgcacgtt ctatattgtg ccaagcgaaa aaaaaaaatg 4980
   cactgtgaca ccagaataat gagtctgcat aaacttcatc ttcaacctta aggacttagc 5040
   tggccacagt gagctgatgt gcccaccacc gtgtcatgag agaatgggtt tactctcaat 5100
   gcattttcaa gatacatttc atctgctgct gaaactgtgt acgacaaagc atcattgtaa 5160
   attatttcat acaaaactgt tcacgttggg tggagagagt attaaatatt taacataggt 5220
   tttgatttat atgtgtaatt ttttaaatga aaatgtaact tttcttacag cacatctttt 5280
   ttttggatgt gggatggagg tatacaatgt tctgttgtaa agagtggagc aaatgcttaa 5340
   aacaaggctt aaaagagtag aatagggtat gatccttgtt ttaagattgt aattcagaaa 5400
   acataatata agaatcatag tgccatagat ggttctcaat tgtatagtta tatttgctga 5460
   tactatctct tgtcatataa acctgatgtt gagctgagtt ccttataaga attaatctta 5520
   attttgtatt ttttcctgta agacaatagg ccatgttaat taaactgaag aaggatatat 5580
   ttggctgggt gttttcaaat gtcagcttaa aattggtaat tgaatggaag caaaattata 5640
   agaagaggaa attaaagtct tccattgcat gtattgtaaa cagaaggaga tgggtgattc 5700
   cttcaattca aaagctctct ttggaatgaa caatgtgggc gtttgtaaat tctggaaatg 5760
   tctttctatt cataataaac tagatactgt tgatctttta aaaaaaaaaa aaaaaaaaaa 5820
   aaaaaaaa 5828
<210> 13
   <211> 1242
   <212> PRT
   <213> Homo sapiens
<400> 13
   Met Ala Ser Pro Pro Glu Ser Asp Gly Phe Ser Asp Val Arg Lys Val
   1               5                   10                  15
   Gly Tyr Leu Arg Lys Pro Lys Ser Met His Lys Arg Phe Phe Val Leu
               20                  25                  30
   Arg Ala Ala Ser Glu Ala Gly Gly Pro Ala Arg Leu Glu Tyr Tyr Glu
           35                  40                  45
   Asn Glu Lys Lys Trp Arg His Lys Ser Ser Ala Pro Lys Arg Ser Ile
   50 55 60
   Pro Leu Glu Ser Cys Phe Asn Ile Asn Lys Arg Ala Asp Ser Lys Asn
   65 70 75 80
   Lys His Leu Val Ala Leu Tyr Thr Arg Asp Glu His Phe Ala Ile Ala
   85 90 95
   Ala Asp Ser Glu Ala Glu Gln Asp Ser Trp Tyr Gln Ala Leu Leu Gln
   100 105 110
   Leu His Asn Arg Ala Lys Gly His His Asp Gly Ala Ala Ala Leu Gly
   115 120 125
   Ala Gly Gly Gly Gly Gly Ser Cys Ser Gly Ser Ser Gly Leu Gly Glu
   130 135 140
   Ala Gly Glu Asp Leu Ser Tyr Gly Asp Val Pro Pro Gly Pro Ala Phe
   145 150 155 160
   Lys Glu Val Trp Gln Val Ile Leu Lys Pro Lys Gly Leu Gly Gln Thr
   165 170 175
   Lys Asn Leu Ile Gly Ile Tyr Arg Leu Cys Leu Thr Ser Lys Thr Ile
   180 185 190
   Ser Phe Val Lys Leu Asn Ser Glu Ala Ala Ala Val Val Leu Gln Leu
   195 200 205
   Met Asn Ile Arg Arg Cys Gly His Ser Glu Asn Phe Phe Phe Ile Glu
   210 215 220
   Val Gly Arg Ser Ala Val Thr Gly Pro Gly Glu Phe Trp Met Gln Val
   225 230 235 240
   Asp Asp Ser Val Val Ala Gln Asn Met His Glu Thr Ile Leu Glu Ala
   245 250 255
   Met Arg Ala Met Ser Asp Glu Phe Arg Pro Arg Ser Lys Ser Gln Ser
   260 265 270
   Ser Ser Asn Cys Ser Asn Pro Ile Ser Val Pro Leu Arg Arg His His
   275 280 285
   Leu Asn Asn Pro Pro Pro Ser Gln Val Gly Leu Thr Arg Arg Ser Arg
   290 295 300
   Thr Glu Ser Ile Thr Ala Thr Ser Pro Ala Ser Met Val Gly Gly Lys
   305 310 315 320
   Pro Gly Ser Phe Arg Val Arg Ala Ser Ser Asp Gly Glu Gly Thr Met
   325 330 335
   Ser Arg Pro Ala Ser Val Asp Gly Ser Pro Val Ser Pro Ser Thr Asn
   340 345 350
   Arg Thr His Ala His Arg His Arg Gly Ser Ala Arg Leu His Pro Pro
   355 360 365
   Leu Asn His Ser Arg Ser Ile Pro Met Pro Ala Ser Arg Cys Ser Pro
   370 375 380
   Ser Ala Thr Ser Pro Val Ser Leu Ser Ser Ser Ser Thr Ser Gly His
   385 390 395 400
   Gly Ser Thr Ser Asp Cys Leu Phe Pro Arg Arg Ser Ser Ala Ser Val
   405 410 415
   Ser Gly Ser Pro Ser Asp Gly Gly Phe Ile Ser Ser Asp Glu Tyr Gly
   420 425 430
   Ser Ser Pro Cys Asp Phe Arg Ser Ser Phe Arg Ser Val Thr Pro Asp
   435 440 445
   Ser Leu Gly His Thr Pro Pro Ala Arg Gly Glu Glu Glu Leu Ser Asn
   450 455 460
   Tyr Ile Cys Met Gly Gly Lys Gly Pro Ser Thr Leu Thr Ala Pro Asn
   465 470 475 480
   Gly His Tyr Ile Leu Ser Arg Gly Gly Asn Gly His Arg Cys Thr Pro
   485 490 495
   Gly Thr Gly Leu Gly Thr Ser Pro Ala Leu Ala Gly Asp Glu Ala Ala
   500 505 510
   Ser Ala Ala Asp Leu Asp Asn Arg Phe Arg Lys Arg Thr His Ser Ala
   515 520 525
   Gly Thr Ser Pro Thr Ile Thr His Gln Lys Thr Pro Ser Gln Ser Ser
   530 535 540
   Val Ala Ser Ile Glu Glu Tyr Thr Glu Met Met Pro Ala Tyr Pro Pro
   545 550 555 560
   Gly Gly Gly Ser Gly Gly Arg Leu Pro Gly His Arg His Ser Ala Phe
   565 570 575
   Val Pro Thr Arg Ser Tyr Pro Glu Glu Gly Leu Glu Met His Pro Leu
   580 585 590
   Glu Arg Arg Gly Gly His His Arg Pro Asp Ser Ser Thr Leu His Thr
   595 600 605
   Asp Asp Gly Tyr Met Pro Met Ser Pro Gly Val Ala Pro Val Pro Ser
   610 615 620
   Gly Arg Lys Gly Ser Gly Asp Tyr Met Pro Met Ser Pro Lys Ser Val
   625 630 635 640
   Ser Ala Pro Gln Gln Ile Ile Asn Pro Ile Arg Arg His Pro Gln Arg
   645 650 655
   Val Asp Pro Asn Gly Tyr Met Met Met Ser Pro Ser Gly Gly Cys Ser
   660 665 670
   Pro Asp Ile Gly Gly Gly Pro Ser Ser Ser Ser Ser Ser Ser Asn Ala
   675 680 685
   Val Pro Ser Gly Thr Ser Tyr Gly Lys Leu Trp Thr Asn Gly Val Gly
   690 695 700
   Gly His His Ser His Val Leu Pro His Pro Lys Pro Pro Val Glu Ser
   705 710 715 720
   Ser Gly Gly Lys Leu Leu Pro Cys Thr Gly Asp Tyr Met Asn Met Ser
   725 730 735
   Pro Val Gly Asp Ser Asn Thr Ser Ser Pro Ser Asp Cys Tyr Tyr Gly
   740 745 750
   Pro Glu Asp Pro Gln His Lys Pro Val Leu Ser Tyr Tyr Ser Leu Pro
   755 760 765
   Arg Ser Phe Lys His Thr Gln Arg Pro Gly Glu Pro Glu Glu Gly Ala
   770 775 780
   Arg His Gln His Leu Arg Leu Ser Thr Ser Ser Gly Arg Leu Leu Tyr
   785 790 795 800
   Ala Ala Thr Ala Asp Asp Ser Ser Ser Ser Thr Ser Ser Asp Ser Leu
   805 810 815
   Gly Gly Gly Tyr Cys Gly Ala Arg Leu Glu Pro Ser Leu Pro His Pro
   820 825 830
   His His Gln Val Leu Gln Pro His Leu Pro Arg Lys Val Asp Thr Ala
   835 840 845
   Ala Gln Thr Asn Ser Arg Leu Ala Arg Pro Thr Arg Leu Ser Leu Gly
   850 855 860
   Asp Pro Lys Ala Ser Thr Leu Pro Arg Ala Arg Glu Gln Gln Gln Gln
   865 870 875 880
   Gln Gln Pro Leu Leu His Pro Pro Glu Pro Lys Ser Pro Gly Glu Tyr
   885 890 895
   Val Asn Ile Glu Phe Gly Ser Asp Gln Ser Gly Tyr Leu Ser Gly Pro
   900 905 910
   Val Ala Phe His Ser Ser Pro Ser Val Arg Cys Pro Ser Gln Leu Gln
   915 920 925
   Pro Ala Pro Arg Glu Glu Glu Thr Gly Thr Glu Glu Tyr Met Lys Met
   930 935 940
   Asp Leu Gly Pro Gly Arg Arg Ala Ala Trp Gln Glu Ser Thr Gly Val
   945 950 955 960
   Glu Met Gly Arg Leu Gly Pro Ala Pro Pro Gly Ala Ala Ser Ile Cys
   965 970 975
   Arg Pro Thr Arg Ala Val Pro Ser Ser Arg Gly Asp Tyr Met Thr Met
   980 985 990
   Gln Met Ser Cys Pro Arg Gln Ser Tyr Val Asp Thr Ser Pro Ala Ala
   995 1000 1005
   Pro Val Ser Tyr Ala Asp Met Arg Thr Gly Ile Ala Ala Glu Glu
   1010 1015 1020
   Val Ser Leu Pro Arg Ala Thr Met Ala Ala Ala Ser Ser Ser Ser
   1025 1030 1035
   Ala Ala Ser Ala Ser Pro Thr Gly Pro Gln Gly Ala Ala Glu Leu
   1040 1045 1050
   Ala Ala His Ser Ser Leu Leu Gly Gly Pro Gln Gly Pro Gly Gly
   1055 1060 1065
   Met Ser Ala Phe Thr Arg Val Asn Leu Ser Pro Asn Arg Asn Gln
   1070 1075 1080
   Ser Ala Lys Val Ile Arg Ala Asp Pro Gln Gly Cys Arg Arg Arg
   1085 1090 1095
   His Ser Ser Glu Thr Phe Ser Ser Thr Pro Ser Ala Thr Arg Val
   1100 1105 1110
   Gly Asn Thr Val Pro Phe Gly Ala Gly Ala Ala Val Gly Gly Gly
   1115 1120 1125
   Gly Gly Ser Ser Ser Ser Ser Glu Asp Val Lys Arg His Ser Ser
   1130 1135 1140
   Ala Ser Phe Glu Asn Val Trp Leu Arg Pro Gly Glu Leu Gly Gly
   1145 1150 1155
   Ala Pro Lys Glu Pro Ala Lys Leu Cys Gly Ala Ala Gly Gly Leu
   1160 1165 1170
   Glu Asn Gly Leu Asn Tyr Ile Asp Leu Asp Leu Val Lys Asp Phe
   1175 1180 1185
   Lys Gln Cys Pro Gln Glu Cys Thr Pro Glu Pro Gln Pro Pro Pro
   1190 1195 1200
   Pro Pro Pro Pro His Gln Pro Leu Gly Ser Gly Glu Ser Ser Ser
   1205 1210 1215
   Thr Arg Arg Ser Ser Glu Asp Leu Ser Ala Tyr Ala Ser Ile Ser
   1220 1225 1230
   Phe Gln Lys Gln Pro Glu Asp Arg Gln
   1235 1240
<210> 14
   <211> 1888
   <212> DNA
   <213> Homo sapiens
<400> 14
   gcggcgcggg gcgggcgcag cgggggtcgg ggcgctggga gcccgttggg ccgcgaacgc 60
   agccgccacg ccggggccgc cgagatcggg tgcccgggat gagcctcatc cggaaaaagg 120
   gcttctacaa gcaggacgtc aacaagaccg cctgggagct gcccaagacc tacgtgtccc 180
   cgacgcacgt cggcagcggg gcctatggct ccgtgtgctc ggccatcgac aagcggtcag 240
   gggagaaggt ggccatcaag aagctgagcc gaccctttca gtccgagatc ttcgccaagc 300
   gcgcctaccg ggagctgctg ctgctgaagc acatgcagca tgagaacgtc attgggctcc 360
   tggatgtctt caccccagcc tcctccctgc gcaacttcta tgacttctac ctggtgatgc 420
   ccttcatgca gacggatctg cagaagatca tggggatgga gttcagtgag gagaagatcc 480
   agtacctggt gtatcagatg ctcaaaggcc ttaagtacat ccactctgct ggggtcgtgc 540
   acagggacct gaagccaggc aacctggctg tgaatgagga ctgtgaactg aagattctgg 600
   attttgggct ggcgcgacat gcagacgccg agatgactgg ctacgtggtg acccgctggt 660
   accgagcccc cgaggtgatc ctcagctgga tgcactacaa ccagacagtg gacatctggt 720
   ctgtgggctg tatcatggca gagatgctga cagggaaaac tctgttcaag gggaaagatt 780
   acctggacca gctgacccag atcctgaaag tgaccggggt gcctggcacg gagtttgtgc 840
   agaagctgaa cgacaaagcg gccaaatcct acatccagtc cctgccacag acccccagga 900
   aggatttcac tcagctgttc ccacgggcca gcccccaggc tgcggacctg ctggagaaga 960
   tgctggagct agacgtggac aagcgcctga cggccgcgca ggccctcacc catcccttct 1020
   ttgaaccctt ccgggaccct gaggaagaga cggaggccca gcagccgttt gatgattcct 1080
   tagaacacga gaaactcaca gtggatgaat ggaagcagca catctacaag gagattgtga 1140
   acttcagccc cattgcccgg aaggactcac ggcgccggag tggcatgaag ctgtagggac 1200
   tcatcttgca tggcaccacc ggccagacac tgcccaagga ccagtatttg tcactaccaa 1260
   actcagccct tcttggaata cagcctttca agcagaggac agaagggtcc ttctccttat 1320
   gtgggaaatg ggcctagtag atgcagaatt caaagatgtc ggttgggaga aactagctct 1380
   gatcctaaca ggccacgtta aactgcccat ctggagaatc gcctgcaggt ggggcccttt 1440
   ccttcccgcc agagtggggc tgagtgggcg ctgagccagg ccgggggcct atggcagtga 1500
   tgctgtgttg gtttcctagg gatgctctaa cgaattacca caaacctggt ggattgaaac 1560
   agcagaactt gattccctta cagttctgga ggctggaaat ctgggatgga ggtgttggca 1620
   gggctgtggt ccctttgaag gctctgggga agaatccttc cttggctctt tttagcttgt 1680
   ggcggcagtg ggcagtccgt ggcattcccc agcttattgc tgcatcactc cagtctctgt 1740
   ctcttctgtt ctctcctctt ttaacaacag tcattggatt tagggcccac cctaatcctg 1800
   tgtgatctta tcttgatcct tattaattaa acctgcaaat actctagttc caaataaagt 1860
   cacattctca ggttccaggt ggacatga 1888
<210> 15
   <211> 365
   <212> PRT
   <213> Homo sapiens
<400> 15
   Met Ser Leu Ile Arg Lys Lys Gly Phe Tyr Lys Gln Asp Val Asn Lys
   1               5                   10                  15
   Thr Ala Trp Glu Leu Pro Lys Thr Tyr Val Ser Pro Thr His Val Gly
               20                  25                  30
   Ser Gly Ala Tyr Gly Ser Val Cys Ser Ala Ile Asp Lys Arg Ser Gly
           35                  40                  45
   Glu Lys Val Ala Ile Lys Lys Leu Ser Arg Pro Phe Gln Ser Glu Ile
       50                  55                  60
   Phe Ala Lys Arg Ala Tyr Arg Glu Leu Leu Leu Leu Lys His Met Gln
   65                  70                  75                  80
   His Glu Asn Val Ile Gly Leu Leu Asp Val Phe Thr Pro Ala Ser Ser
                   85                  90                  95
   Leu Arg Asn Phe Tyr Asp Phe Tyr Leu Val Met Pro Phe Met Gln Thr
   100 105 110
   Asp Leu Gln Lys Ile Met Gly Met Glu Phe Ser Glu Glu Lys Ile Gln
   115 120 125
   Tyr Leu Val Tyr Gln Met Leu Lys Gly Leu Lys Tyr Ile His Ser Ala
   130 135 140
   Gly Val Val His Arg Asp Leu Lys Pro Gly Asn Leu Ala Val Asn Glu
   145 150 155 160
   Asp Cys Glu Leu Lys Ile Leu Asp Phe Gly Leu Ala Arg His Ala Asp
   165 170 175
   Ala Glu Met Thr Gly Tyr Val Val Thr Arg Trp Tyr Arg Ala Pro Glu
   180 185 190
   Val Ile Leu Ser Trp Met His Tyr Asn Gln Thr Val Asp Ile Trp Ser
   195 200 205
   Val Gly Cys Ile Met Ala Glu Met Leu Thr Gly Lys Thr Leu Phe Lys
   210 215 220
   Gly Lys Asp Tyr Leu Asp Gln Leu Thr Gln Ile Leu Lys Val Thr Gly
   225 230 235 240
   Val Pro Gly Thr Glu Phe Val Gln Lys Leu Asn Asp Lys Ala Ala Lys
   245 250 255
   Ser Tyr Ile Gln Ser Leu Pro Gln Thr Pro Arg Lys Asp Phe Thr Gln
   260 265 270
   Leu Phe Pro Arg Ala Ser Pro Gln Ala Ala Asp Leu Leu Glu Lys Met
   275 280 285
   Leu Glu Leu Asp Val Asp Lys Arg Leu Thr Ala Ala Gln Ala Leu Thr
   290 295 300
   His Pro Phe Phe Glu Pro Phe Arg Asp Pro Glu Glu Glu Thr Glu Ala
   305 310 315 320
   Gln Gln Pro Phe Asp Asp Ser Leu Glu His Glu Lys Leu Thr Val Asp
   325 330 335
   Glu Trp Lys Gln His Ile Tyr Lys Glu Ile Val Asn Phe Ser Pro Ile
   340 345 350
   Ala Arg Lys Asp Ser Arg Arg Arg Ser Gly Met Lys Leu
   355 360 365
<210> 16
   <211> 7341
   <212> DNA
   <213> Homo sapiens
<400> 16
   gcgcgaggcc gtcgattcgc tcgcggctcc atcgcggcct ggccgggggg cggtgtctgc      60
   tgcgccaggt tcgctggccg cacgtcttca ggtcctcctg ttcctgggag gcgggcgcgg     120
   caggactggg aggtggcggc agcgggcgag gactcgccga ggacggggct ccggcccggg     180
   ataaccaact ctccttctct cttctttggt gcttccccag gcggcggcgg cggcgcccgg     240
   gagccggagc cttcgcggcg tccacgtccc tcccccgctg caccccgccc cggcgcgaga     300
   ggagagcgcg agagccccag ccgcgggcgg gcgggcggcg aagatggcag aggcaccggc     360
   ttccccggcc ccgctctctc cgctcgaagt ggagctggac ccggagttcg agccccagag     420
   ccgtccgcga tcctgtacgt ggcccctgca aaggccggag ctccaagcga gccctgccaa     480
   gccctcgggg gagacggccg ccgactccat gatccccgag gaggaggacg atgaagacga     540
   cgaggacggc gggggacggg ccggctcggc catggcgatc ggcggcggcg gcgggagcgg     600
   cacgctgggc tccgggctgc tccttgagga ctcggcccgg gtgctggcac ccggagggca     660
   agaccccggg tctgggccag ccaccgcggc gggcgggctg agcgggggta cacaggcgct     720
   gctgcagcct cagcaaccgc tgccaccgcc gcagccgggg gcggctgggg gctccgggca     780
   gccgaggaaa tgttcgtcgc ggcggaacgc ctggggaaac ctgtcctacg cggacctgat     840
   cacccgcgcc atcgagagct ccccggacaa acggctcact ctgtcccaga tctacgagtg     900
   gatggtgcgt tgcgtgccct acttcaagga taagggcgac agcaacagct ctgccggctg     960
   gaagaactcc atccggcaca acctgtcact gcatagtcga ttcatgcggg tccagaatga    1020
   gggaactggc aagagctctt ggtggatcat caaccctgat ggggggaaga gcggaaaagc    1080
   cccccggcgg cgggctgtct ccatggacaa tagcaacaag tataccaaga gccgtggccg    1140
   cgcagccaag aagaaggcag ccctgcagac agcccccgaa tcagctgacg acagtccctc    1200
   ccagctctcc aagtggcctg gcagccccac gtcacgcagc agtgatgagc tggatgcgtg    1260
   gacggacttc cgttcacgca ccaattctaa cgccagcaca gtcagtggcc gcctgtcgcc    1320
   catcatggca agcacagagt tggatgaagt ccaggacgat gatgcgcctc tctcgcccat    1380
   gctctacagc agctcagcca gcctgtcacc ttcagtaagc aagccgtgca cggtggaact    1440
   gccacggctg actgatatgg caggcaccat gaatctgaat gatgggctga ctgaaaacct    1500
   catggacgac ctgctggata acatcacgct cccgccatcc cagccatcgc ccactggggg    1560
   actcatgcag cggagctcta gcttcccgta taccaccaag ggctcgggcc tgggctcccc    1620
   aaccagctcc tttaacagca cggtgttcgg accttcatct ctgaactccc tacgccagtc    1680
   tcccatgcag accatccaag agaacaagcc agctaccttc tcttccatgt cacactatgg    1740
   taaccagaca ctccaggacc tgctcacttc ggactcactt agccacagcg atgtcatgat    1800
   gacacagtcg gaccccttga tgtctcaggc cagcaccgct gtgtctgccc agaattcccg    1860
   ccggaacgtg atgcttcgca atgatccgat gatgtccttt gctgcccagc ctaaccaggg 1920
   aagtttggtc aatcagaact tgctccacca ccagcaccaa acccagggcg ctcttggtgg 1980
   cagccgtgcc ttgtcgaatt ctgtcagcaa catgggcttg agtgagtcca gcagccttgg 2040
   gtcagccaaa caccagcagc agtctcctgt cagccagtct atgcaaaccc tctcggactc 2100
   tctctcaggc tcctccttgt actcaactag tgcaaacctg cccgtcatgg gccatgagaa 2160
   gttccccagc gacttggacc tggacatgtt caatgggagc ttggaatgtg acatggagtc 2220
   cattatccgt agtgaactca tggatgctga tgggttggat tttaactttg attccctcat 2280
   ctccacacag aatgttgttg gtttgaacgt ggggaacttc actggtgcta agcaggcctc 2340
   atctcagagc tgggtgccag gctgaaggat cactgaggaa ggggaagtgg gcaaagcaga 2400
   ccctcaaact gacacaagac ctacagagaa aaccctttgc caaatctgct ctcagcaagt 2460
   ggacagtgat accgtttaca gcttaacacc tttgtgaatc ccacgccatt ttcctaaccc 2520
   agcagagact gttaatggcc ccttaccctg ggtgaagcac ttacccttgg aacagaactc 2580
   taaaaagtat gcaaaatctt ccttgtacag ggtggtgagc cgcctgccag tggaggacag 2640
   cacccctcag caccacccac cctcattcag agcacaccgt gagcccccgt cggccattct 2700
   gtggtgtttt aatattgcga tggtttatgg gacgttttaa gtgttgttct tgtgtttgtt 2760
   ttcctttgac tttctgagtt tttcacatgc attaacttgc ggtatttttc tgttaaaatg 2820
   ttaaccgtcc ttcccctagc aaatttaaaa acagaaagaa aatgttgtac cagttaccat 2880
   tccgggttcg agcatcacaa gcttttgagc gcatggaact ccataaacta acaaattaca 2940
   taaactaaag ggggattttc tttcttcttt tgtttggtag aaaattatcc ttttctaaaa 3000
   actgaacaat ggcacaattg tttgctatgt gcacccgtcc aggacagaac cgtgcatagg 3060
   caaaaggagt ggagcacagc gtccggccca gtgtgtttcc ggttctgagt cagggtgatc 3120
   tgtggacggg accccagcac caagtctacg ggtgccagat cagtagggcc tgtgatttcc 3180
   tgtcagtgtc ctcagctaat gtgaacagtg ttggtctgct ggttagaaac tagaatattg 3240
   atattttcag gaaagaaatc agctcagctc tccactcatt gccaaatgtc actaaagggt 3300
   ttagttttaa ggagaaagaa aaggaaaaaa aaaaaaaaca aaaaagtcct gttttgcttt 3360
   gcagaacaaa tgaacttaca ggtgagcatt aagcttgcag tgagaaatgt gcgaagagta 3420
   aaaacccaag tcaatgctga ggcagttcta acttcactgt tttcctaaat acacatcctt 3480
   gattattttc agccttgcta tataatctga tctgctagaa gtgtatgagt gagaggcaat 3540
   agcatacaaa ctgatttttt aaatataagc ttaggttgta attgtacaag tgactcaatg 3600
   gaagtacaaa atagggcagt tttaactttt ttttctgctt ctatggattt cattttgttg 3660
   tgttttcaaa aagttatggt gctgtatagg tgctttctgt ttaacctgga aagtgtgatt 3720
   atattcgtta ccttctttgg tagacggaat agttgggacc acctttggta cataagaaat 3780
   tggtataacg atgctctgat tagcacagta tatgcatact tctccaaagt gatatatgaa 3840
   gactcttttc tttgcataaa aagcattagg catataaatg tataaatata ttttatcatg 3900
   tacagtacaa aaatggaacc ttatgcatgg gccttaggaa tacaggctag tatttcagca 3960
   cagacttccc tgcttgagtt cttgctgatg cttgcaccgt gacagtgggc accaacacag 4020
   acgtgccacc caaccccctg cacacaccac cggccaccag gggccccctt gtgcgccttg 4080
   gctttataac tcctctgggg gtgatattgg tggtgatcac agctcctagc ataatgagag 4140
   ttccatttgg tattgtcaca cgtctcctgc ctcgcttggg ttgccatgtt tgagcgatgg 4200
   ccctgttgat ttcaccctgc cttttactga atctgtaaat tgttgtgcaa ttgtggttat 4260
   agtagactgt agcacattgc cttttctaaa ctgctacatg tttataatct tcatttttaa 4320
   agtatgtgta atttttttaa gtatgtattc tattcatatg gtctgcttgt cagtgagcca 4380
   gacttgctta ctatattcct ttataataat gctagccact tcctggattc tttagtaatg 4440
   tgctgtatgc aagaactttc cagtagcagt gaaggagggt tgcctctcca agcttcctaa 4500
   gggatgctgc cctgtgtggg gatgcattgc agaggcacta gtagcatggg ggctagagtg 4560
   gggagcgaga tgtaaaaggg tggggggata ggagaattcc agagtgcttc cagcattagg 4620
   gtcctgagaa cttctgagtt cagagaaaca tgcaaagtga ctaacaaaat agctacttac 4680
   ctttgcagtt ttacagaccc tgggagctgc tttgggagtg agaaaggcaa ccctccaatg 4740
   tgtttcaact ttaaaatgtt gaattctttt cagacatggt atctcattta ttctcctttt 4800
   ctagcgtttg ttgaatttca ggcagaatgt cttacagaat gtcctagaac cagattatca 4860
   tttaatctga aacagctgag gaagggacag agaaggtaca agggcaaggc agcacaaaac 4920
   agatcaggag aatgaagagg gaatgctttg gttttttgtt ttgttttgtt ttttcttttt 4980
   caagtaacta aaacagcatc tacatgtaga gtgttgtgga gagctgagac cagggtaaag 5040
   tcaagtgcag catcagtact gcgagaccca ccagcccctg gagagggtca gccgagaatc 5100
   tggtagtgaa gcctgtctag ggtcccggca ccctcaccct cagccacctg cagagaggcc 5160
   agggccccag agactagcct ggttctgaag tgggcagggg tgctgccaga gccctctgcc 5220
   ccttatgttg agaccctgct ttcaggacag gccagccgtt ggccaccatg tcacattctg 5280
   agtgagtgtc acaggtccct aacaataatt ttctgatctg gagcatatca gcagaatgct 5340
   tagcctcaag gggcctggca gctgtaatgt ttgatttatg atgagaacta tccgaggcca 5400
   cccttggcct ctaaataagc tgctctaggg agccgcctac tttttgatga gaaattagaa 5460
   gagtacctaa tgttgaaaac atgacatgcg ctcttgggat ctgctgttct ctccagggct 5520
   ccagaacctg atacctgtta ccaaagctag gaaagagctt tatcacaagc cttcactgtc 5580
   ctggcatgag aactggctgc caggctcagt gtaccccatt aactgtgaat gaatctgagc 5640
   ttggtttcct ttattgcttc ctctgcaata tgattgctga aacacatttt aaaaattcag 5700
   aagcttgtca ctcctgttaa tgggaggatc agtcacacat gtgtagtaca aggcggactt 5760
   tgtgtttgtt tttggtgtta atttttagca ttgtgtgtgt tgcttcccca ccctgaggag 5820
   aggacaccat ggcttactac tcaggacaag tatgccccgc tcagggtgtg atttcaggtg 5880
   gcttccaaac ttgtacgcag tttaaagatg gtggggacag actttgcctc tacctagtga 5940
   accccactta aagaataagg agcatttgaa tctcttggaa aaggccatga agaataaagc 6000
   agtcaaaaag aagtcctcca tgttggtgcc aaggacttgc gaggggaaat aaaaatgtta 6060
   tccagcctga ccaacatgga gaaaccccgt ctccattaaa aatacaaaat tagcctggca 6120
   tggtggcgca tgcctgtaat cccagctact ctggaggctg aggcaggaga atcgcttgaa 6180
   cccaggaggc ggaggtcgca gtgagccgag atcatgccag tgcactccag cctgggtaac 6240
   aagagtgaaa ctccgtgtca aaaaaaaaaa aaaaatgtta ctcatcctct ctgaaagcaa 6300
   aaaggaaacc ctaacagctc tgaactctgg ttttattttt cttgctgtat ttgggtgaac 6360
   attgtatgat taggcataat gttaaaaaaa aaaatttttt tttggtagaa atgcaatcac 6420
   cagtaaagag gtacgaaaaa gctagcctct ctcagagacc ggggaggcag agtactacta 6480
   gaggaagtga agttctgatg gaatcatgcc tgtcaaatga ggtcttgaag cggatgccca 6540
   aataaaagag tatattttat ctaaatctta agtgggtaac attttatgca gtttaaatga 6600
   atggaatatt ttcctcttgt ttagttgtat ctgtttgtat ttttctttga tgaatgattg 6660
   gtcatgaggc ctcttgccac actccagaaa tacgtgtgcg gctgctttta agaactatgt 6720
   gtctggtcac ttatttctct aaaattatct cattgcctgg caatcagtct tctcttgtat 6780
   acttgtccta gcacattatg tacatgggaa atgtaaacaa atgtgaagga ggaccagaaa 6840
   aattagttaa tatttaaaaa aatgtattgt gcattttggc ttcacatgtt taactttttt 6900
   taagaaaaaa gttgcatgaa tggaaaaaaa aatctgtata cagtatctgt aaaaactatc 6960
   ttatctgttt caattccttg ctcatatccc atataatcta gaactaaata tggtgtgtgg 7020
   ccatatttaa acacctgaga gtcaagcagt tgagactttg atttgaagca cctcatcctt 7080
   ctttcaatgc gaacactatc atatggcatt cttactgagg attttgtcta accatatgtt 7140
   gccatgaatt aactctgccg cctttcttaa ggatcaaaac cagtttgatt tgggaatctt 7200
   cccctttcca aatgaaatag agatgcagta cttaactttc cttggtgttt gtagatattg 7260
   ccttgtgtat tccacttaaa accgtaatct agtttgtaaa agagatggtg acgcatgtaa 7320
   ataaagcatc agtgacactc t 7341
<210> 17
   <211> 673
   <212> PRT
   <213> Homo sapiens
<400> 17
   Met Ala Glu Ala Pro Ala Ser Pro Ala Pro Leu Ser Pro Leu Glu Val
   1               5                   10                  15
   Glu Leu Asp Pro Glu Phe Glu Pro Gln Ser Arg Pro Arg Ser Cys Thr
               20                  25                  30
   Trp Pro Leu Gln Arg Pro Glu Leu Gln Ala Ser Pro Ala Lys Pro Ser
           35                  40                  45
   Gly Glu Thr Ala Ala Asp Ser Met Ile Pro Glu Glu Glu Asp Asp Glu
   50 55 60
   Asp Asp Glu Asp Gly Gly Gly Arg Ala Gly Ser Ala Met Ala Ile Gly
   65 70 75 80
   Gly Gly Gly Gly Ser Gly Thr Leu Gly Ser Gly Leu Leu Leu Glu Asp
   85 90 95
   Ser Ala Arg Val Leu Ala Pro Gly Gly Gln Asp Pro Gly Ser Gly Pro
   100 105 110
   Ala Thr Ala Ala Gly Gly Leu Ser Gly Gly Thr Gln Ala Leu Leu Gln
   115 120 125
   Pro Gln Gln Pro Leu Pro Pro Pro Gln Pro Gly Ala Ala Gly Gly Ser
   130 135 140
   Gly Gln Pro Arg Lys Cys Ser Ser Arg Arg Asn Ala Trp Gly Asn Leu
   145 150 155 160
   Ser Tyr Ala Asp Leu Ile Thr Arg Ala Ile Glu Ser Ser Pro Asp Lys
   165 170 175
   Arg Leu Thr Leu Ser Gln Ile Tyr Glu Trp Met Val Arg Cys Val Pro
   180 185 190
   Tyr Phe Lys Asp Lys Gly Asp Ser Asn Ser Ser Ala Gly Trp Lys Asn
   195 200 205
   Ser Ile Arg His Asn Leu Ser Leu His Ser Arg Phe Met Arg Val Gln
   210 215 220
   Asn Glu Gly Thr Gly Lys Ser Ser Trp Trp Ile Ile Asn Pro Asp Gly
   225 230 235 240
   Gly Lys Ser Gly Lys Ala Pro Arg Arg Arg Ala Val Ser Met Asp Asn
   245 250 255
   Ser Asn Lys Tyr Thr Lys Ser Arg Gly Arg Ala Ala Lys Lys Lys Ala
   260 265 270
   Ala Leu Gln Thr Ala Pro Glu Ser Ala Asp Asp Ser Pro Ser Gln Leu
   275 280 285
   Ser Lys Trp Pro Gly Ser Pro Thr Ser Arg Ser Ser Asp Glu Leu Asp
   290 295 300
   Ala Trp Thr Asp Phe Arg Ser Arg Thr Asn Ser Asn Ala Ser Thr Val
   305 310 315 320
   Ser Gly Arg Leu Ser Pro Ile Met Ala Ser Thr Glu Leu Asp Glu Val
   325 330 335
   Gln Asp Asp Asp Ala Pro Leu Ser Pro Met Leu Tyr Ser Ser Ser Ala
   340 345 350
   Ser Leu Ser Pro Ser Val Ser Lys Pro Cys Thr Val Glu Leu Pro Arg
   355 360 365
   Leu Thr Asp Met Ala Gly Thr Met Asn Leu Asn Asp Gly Leu Thr Glu
   370 375 380
   Asn Leu Met Asp Asp Leu Leu Asp Asn Ile Thr Leu Pro Pro Ser Gln
   385 390 395 400
   Pro Ser Pro Thr Gly Gly Leu Met Gln Arg Ser Ser Ser Phe Pro Tyr
   405 410 415
   Thr Thr Lys Gly Ser Gly Leu Gly Ser Pro Thr Ser Ser Phe Asn Ser
   420 425 430
   Thr Val Phe Gly Pro Ser Ser Leu Asn Ser Leu Arg Gln Ser Pro Met
   435 440 445
   Gln Thr Ile Gln Glu Asn Lys Pro Ala Thr Phe Ser Ser Met Ser His
   450 455 460
   Tyr Gly Asn Gln Thr Leu Gln Asp Leu Leu Thr Ser Asp Ser Leu Ser
   465 470 475 480
   His Ser Asp Val Met Met Thr Gln Ser Asp Pro Leu Met Ser Gln Ala
   485 490 495
   Ser Thr Ala Val Ser Ala Gln Asn Ser Arg Arg Asn Val Met Leu Arg
   500 505 510
   Asn Asp Pro Met Met Ser Phe Ala Ala Gln Pro Asn Gln Gly Ser Leu
   515 520 525
   Val Asn Gln Asn Leu Leu His His Gln His Gln Thr Gln Gly Ala Leu
   530 535 540
   Gly Gly Ser Arg Ala Leu Ser Asn Ser Val Ser Asn Met Gly Leu Ser
   545 550 555 560
   Glu Ser Ser Ser Leu Gly Ser Ala Lys His Gln Gln Gln Ser Pro Val
   565 570 575
   Ser Gln Ser Met Gln Thr Leu Ser Asp Ser Leu Ser Gly Ser Ser Leu
   580 585 590
   Tyr Ser Thr Ser Ala Asn Leu Pro Val Met Gly His Glu Lys Phe Pro
   595 600 605
   Ser Asp Leu Asp Leu Asp Met Phe Asn Gly Ser Leu Glu Cys Asp Met
   610 615 620
   Glu Ser Ile Ile Arg Ser Glu Leu Met Asp Ala Asp Gly Leu Asp Phe
   625 630 635 640
   Asn Phe Asp Ser Leu Ile Ser Thr Gln Asn Val Val Gly Leu Asn Val
   645 650 655
   Gly Asn Phe Thr Gly Ala Lys Gln Ala Ser Ser Gln Ser Trp Val Pro
   660 665 670
   Gly
<210> 18
   <211> 1593
   <212> DNA
   <213> Homo sapiens
<400> 18
   gcggtgccct tgcggcgcag ctggggtcgc ggccctgctc cccgcgcttt cttaaggccc     60
   gcgggcggcg caggagcggc actcgtggct gtggtggctt cggcagcggc ttcagcagat    120
   cggcggcatc agcggtagca ccagcactag cagcatgttg agccgggcag tgtgcggcac    180
   cagcaggcag ctggctccgg ttttggggta tctgggctcc aggcagaagc acagcctccc    240
   cgacctgccc tacgactacg gcgccctgga acctcacatc aacgcgcaga tcatgcagct    300
   gcaccacagc aagcaccacg cggcctacgt gaacaacctg aacgtcaccg aggagaagta    360
   ccaggaggcg ttggccaagg gagatgttac agcccagata gctcttcagc ctgcactgaa    420
   gttcaatggt ggtggtcata tcaatcatag cattttctgg acaaacctca gccctaacgg    480
   tggtggagaa cccaaagggg agttgctgga agccatcaaa cgtgactttg gttcctttga    540
   caagtttaag gagaagctga cggctgcatc tgttggtgtc caaggctcag gttggggttg    600
   gcttggtttc aataaggaac ggggacactt acaaattgct gcttgtccaa atcaggatcc    660
   actgcaagga acaacaggcc ttattccact gctggggatt gatgtgtggg agcacgctta    720
   ctaccttcag tataaaaatg tcaggcctga ttatctaaaa gctatttgga atgtaatcaa    780
   ctgggagaat gtaactgaaa gatacatggc ttgcaaaaag taaaccacga tcgttatgct    840
   gagtatgtta agctctttat gactgttttt gtagtggtat agagtactgc agaatacagt    900
   aagctgctct attgtagcat ttcttgatgt tgcttagtca cttatttcat aaacaactta    960
   atgttctgaa taatttctta ctaaacattt tgttattggg caagtgattg aaaatagtaa   1020
   atgctttgtg tgattgaatc tgattggaca ttttcttcag agagctaaat tacaattgtc   1080
   atttataaaa ccatcaaaaa tattccatcc atatactttg gggacttgta gggatgcctt    1140
   tctagtccta ttctattgca gttatagaaa atctagtctt ttgccccagt tacttaaaaa   1200
   taaaatatta acactttccc aagggaaaca ctcggctttc tatagaaaat tgcacttttt   1260
   gtcgagtaat cctctgcagt gatacttctg gtagatgtca cccagtggtt tttgttaggt   1320
   caaatgttcc tgtatagttt ttgcaaatag agctgtatac tgtttaaatg tagcaggtga   1380
   actgaactgg ggtttgctca cctgcacagt aaaggcaaac ttcaacagca aaactgcaaa   1440
   aaggtggttt ttgcagtagg agaaaggagg atgtttattt gcagggcgcc aagcaaggag   1500
   aattgggcag ctcatgcttg agacccaatc tccatgatga cctacaagct agagtattta   1560
   aaggcagtgg taaatttcag gaaagcagaa gtt                                1593
<210> 19
   <211> 222
   <212> PRT
   <213> Homo sapiens
<400> 19
   Met Leu Ser Arg Ala Val Cys Gly Thr Ser Arg Gln Leu Ala Pro Val
   1               5                   10                  15
   Leu Gly Tyr Leu Gly Ser Arg Gln Lys His Ser Leu Pro Asp Leu Pro
               20                  25                  30
   Tyr Asp Tyr Gly Ala Leu Glu Pro His Ile Asn Ala Gln Ile Met Gln
           35                  40                  45
   Leu His His Ser Lys His His Ala Ala Tyr Val Asn Asn Leu Asn Val
       50                  55                  60
   Thr Glu Glu Lys Tyr Gln Glu Ala Leu Ala Lys Gly Asp Val Thr Ala
   65                  70                  75                  80
   Gln Ile Ala Leu Gln Pro Ala Leu Lys Phe Asn Gly Gly Gly His Ile
                   85                  90                  95
   Asn His Ser Ile Phe Trp Thr Asn Leu Ser Pro Asn Gly Gly Gly Glu
               100                 105                 110
   Pro Lys Gly Glu Leu Leu Glu Ala Ile Lys Arg Asp Phe Gly Ser Phe
           115                 120                 125
   Asp Lys Phe Lys Glu Lys Leu Thr Ala Ala Ser Val Gly Val Gln Gly
       130                 135                 140
   Ser Gly Trp Gly Trp Leu Gly Phe Asn Lys Glu Arg Gly His Leu Gln
   145                 150                 155                 160
   Ile Ala Ala Cys Pro Asn Gln Asp Pro Leu Gln Gly Thr Thr Gly Leu
                   165                 170                 175
   Ile Pro Leu Leu Gly Ile Asp Val Trp Glu His Ala Tyr Tyr Leu Gln
               180                 185                 190
   Tyr Lys Asn Val Arg Pro Asp Tyr Leu Lys Ala Ile Trp Asn Val Ile
           195                 200                 205
   Asn Trp Glu Asn Val Thr Glu Arg Tyr Met Ala Cys Lys Lys
       210                 215                 220
<210> 20
   <211> 235
   <212> PRT
   <213> Homo sapiens
<400> 20
   Met Gly Ile Gly Lys Ser Lys Ile Asn Ser Cys Pro Leu Ser Leu Ser
   1 5 10 15
   Trp Gly Lys Arg His Ser Val Asp Thr Ser Pro Gly Tyr His Glu Ser
   20 25 30
   Asp Ser Lys Lys Ser Glu Asp Leu Ser Leu Cys Asn Val Ala Glu His
   35 40 45
   Ser Asn Thr Thr Glu Gly Pro Thr Gly Lys Gln Glu Gly Ala Gln Ser
   50 55 60
   Val Glu Glu Met Phe Glu Glu Glu Ala Glu Glu Glu Val Phe Leu Lys
   65 70 75 80
   Phe Val Ile Leu His Ala Glu Asp Asp Thr Asp Glu Ala Leu Arg Val
   85 90 95
   Gln Asn Leu Leu Gln Asp Asp Phe Gly Ile Lys Pro Gly Ile Ile Phe
   100 105 110
   Ala Glu Met Pro Cys Gly Arg Gln His Leu Gln Asn Leu Asp Asp Ala
   115 120 125
   Val Asn Gly Ser Ala Trp Thr Ile Leu Leu Leu Thr Glu Asn Phe Leu
   130 135 140
   Arg Asp Thr Trp Cys Asn Phe Gln Phe Tyr Thr Ser Leu Met Asn Ser
   145 150 155 160
   Val Asn Arg Gln His Lys Tyr Asn Ser Val Ile Pro Met Arg Pro Leu
   165 170 175
   Asn Asn Pro Leu Pro Arg Glu Arg Thr Pro Phe Ala Leu Gln Thr Ile
   180 185 190
   Asn Ala Leu Glu Glu Glu Ser Arg Gly Phe Pro Thr Gln Val Glu Arg
   195 200 205
   Ile Phe Gln Glu Ser Val Tyr Lys Thr Gln Gln Thr Ile Trp Lys Glu
   210 215 220
   Thr Arg Asn Met Val Gln Arg Gln Phe Ile Ala
   225 230 235
<210> 21
   <211> 1670
   <212> DNA
   <213> Homo sapiens
<400> 21
   gtgtatcttt gcggggtggg cgccaacagc agtcaggcct gacaagcggc gacctccaag    60
   ggtgaggcct ctgcgggccc ccgactcacg cgcgtccggg ctctgcaagg gcggtgggga   120
   gcaggctgct gtggtcgcgg ggactgggtt gcggcgcgcc gcgtacggga cggccccaaa   180
   ctctcgacgc ccggggcaag acgcccaccc cctgggcgct ctcgctgggc cagaaaggaa   240
   gacagaaaag ccgcgggctg actgtggtgg cgctcgcctg cagattgaaa agaaatgctg   300
   agaaatacat aaagttttcc tcttctgcct tggatattta taatgggtat cgggaagtct   360
   aaaataaatt cctgccctct ttctctctct tggggtaaaa ggcacagtgt ggatacaagt   420
   ccaggatatc atgagtcaga ttccaagaag tctgaagatc tatccttgtg taatgttgct   480
   gagcacagca atacaacaga ggggccaaca ggaaagcagg agggagctca gagcgtggaa   540
   gagatgtttg aagaagaagc tgaagaagag gtgttcctca aatttgtgat attgcatgca   600
   gaagatgaca cagatgaagc cctcagagtc cagaatctgc tacaagatga ctttggtatc   660
   aaacccggaa taatctttgc tgagatgcca tgtggcagac agcatttaca gaatttagat   720
   gatgctgtaa atgggtctgc atggacaatc ttattactga ctgaaaactt tttaagagat   780
   acttggtgta atttccagtt ctatacgtcc ctaatgaact ccgttaacag gcagcataaa   840
   tacaactctg ttatacccat gcggcccctg aacaatcccc ttccccgaga aaggactccc   900
   tttgccctcc aaaccatcaa tgccttagag gaagaaagtc gtggatttcc tacacaagta   960
   gaaagaattt ttcaggagtc tgtgtataag acacaacaaa ctatatggaa agagacaaga  1020
   aatatggtac aaagacaatt tattgcctga gatgaaacat ataacatgtg gctggctctt  1080
   gttttgtaaa ccaaatgatt aatcttcact tgagaaagca gtttctagga aatgtttaaa  1140
   taaaagagag tcttcacctt aaagaaacct atggagcaca agaaagataa atttctgcag  1200
   gacagtctat aaaattgtgg tactttttga tgtttcagta aacttgacat tgtcagagtt  1260
   tcaaggactt ttctttcaca attttcctag ttcatggata tgaaaaagga attctcaatc  1320
   catattcctt gtattgaacc ttgaacaaaa acttgtatga cagacatttt taaaaatgtg  1380
   acaacacttt tattctctga attttgatct caaaggacac agaaaaaaaa tggccccagg  1440
   agatctgatc acacttcctc ctgaggcacc tctcatggat gttgcaataa gcattcgggt  1500
   actatcaccc agaaatatga attgccagaa tagaacattt agcatgttaa gcgttgatgc  1560
   atataaaatc agaaatagat gtgagaatgg tggaactttt taaaagaacc cagtcaaatg  1620
   tattttctgc tgaaatctgc atatttggag gcatttccca ccaccgattc             1670
<210> 22
   <211> 926
   <212> PRT
   <213> Homo sapiens
<400> 22
   Pro Ala Met Asp Val Leu Pro Thr Gly Gly Gly Arg Pro Gly Leu Arg
   1 5 10 15
   Thr Glu Leu Glu Phe Arg Gly Gly Gly Gly Glu Ala Arg Leu Glu Ser
   20 25 30
   Gln Glu Glu Glu Thr Ile Pro Ala Ala Pro Pro Ala Pro Arg Leu Arg
   35 40 45
   Gly Ala Ala Glu Arg Pro Arg Arg Ser Arg Asp Thr Trp Asp Gly Asp
   50 55 60
   Glu Asp Thr Glu Pro Gly Glu Ala Cys Gly Gly Arg Thr Ser Arg Thr
   65 70 75 80
   Ala Ser Leu Val Ser Gly Leu Leu Asn Glu Leu Tyr Ser Cys Thr Glu
   85 90 95
   Glu Glu Glu Ala Ala Gly Gly Gly Arg Gly Ala Glu Gly Arg Arg Arg
   100 105 110
   Arg Arg Asp Ser Leu Asp Ser Ser Thr Glu Ala Ser Gly Ser Asp Val
   115 120 125
   Val Leu Gly Gly Arg Ser Gly Ala Gly Asp Ser Arg Val Leu Gln Glu
   130 135 140
   Leu Gln Glu Arg Pro Ser Gln Arg His Gln Met Leu Tyr Leu Arg Gln
   145 150 155 160
   Lys Asp Ala Asn Glu Leu Lys Thr Ile Leu Arg Glu Leu Lys Tyr Arg
   165 170 175
   Ile Gly Ile Gln Ser Ala Lys Leu Leu Arg His Leu Lys Gln Lys Asp
   180 185 190
   Arg Leu Leu His Lys Val Gln Arg Asn Cys Asp Ile Val Thr Ala Cys
   195 200 205
   Leu Gln Ala Val Ser Gln Lys Arg Arg Val Asp Thr Lys Leu Lys Phe
   210 215 220
   Thr Leu Glu Pro Ser Leu Gly Gln Asn Gly Phe Gln Gln Trp Tyr Asp
   225 230 235 240
   Ala Leu Lys Ala Val Ala Arg Leu Ser Thr Gly Ile Pro Lys Glu Trp
   245 250 255
   Arg Arg Lys Val Trp Leu Thr Leu Ala Asp His Tyr Leu His Ser Ile
   260 265 270
   Ala Ile Asp Trp Asp Lys Thr Met Arg Phe Thr Phe Asn Glu Arg Ser
   275 280 285
   Asn Pro Asp Asp Asp Ser Met Gly Ile Gln Ile Val Lys Asp Leu His
   290 295 300
   Arg Thr Gly Cys Ser Ser Tyr Cys Gly Gln Glu Ala Glu Gln Asp Arg
   305 310 315 320
   Val Val Leu Lys Arg Val Leu Leu Ala Tyr Ala Arg Trp Asn Lys Thr
   325 330 335
   Val Gly Tyr Cys Gln Gly Phe Asn Ile Leu Ala Ala Leu Ile Leu Glu
   340 345 350
   Val Met Glu Gly Asn Glu Gly Asp Ala Leu Lys Ile Met Ile Tyr Leu
   355 360 365
   Ile Asp Lys Val Leu Pro Glu Ser Tyr Phe Val Asn Asn Leu Arg Ala
   370 375 380
   Leu Ser Val Asp Met Ala Val Phe Arg Asp Leu Leu Arg Met Lys Leu
   385 390 395 400
   Pro Glu Leu Ser Gln His Leu Asp Thr Leu Gln Arg Thr Ala Asn Lys
   405 410 415
   Glu Ser Gly Gly Gly Tyr Glu Pro Pro Leu Thr Asn Val Phe Thr Met
   420 425 430
   Gln Trp Phe Leu Thr Leu Phe Ala Thr Cys Leu Pro Asn Gln Thr Val
   435 440 445
   Leu Lys Ile Trp Asp Ser Val Phe Phe Glu Gly Ser Glu Ile Ile Leu
   450 455 460
   Arg Val Ser Leu Ala Ile Trp Ala Lys Leu Gly Glu Gln Ile Glu Cys
   465 470 475 480
   Cys Glu Thr Ala Asp Glu Phe Tyr Ser Thr Met Gly Arg Leu Thr Gln
   485 490 495
   Glu Met Leu Glu Asn Asp Leu Leu Gln Ser His Glu Leu Met Gln Thr
   500 505 510
   Val Tyr Ser Met Ala Pro Phe Pro Phe Pro Gln Leu Ala Glu Leu Arg
   515 520 525
   Glu Lys Tyr Thr Tyr Asn Ile Thr Pro Phe Pro Ala Thr Val Lys Pro
   530 535 540
   Thr Ser Val Ser Gly Arg His Ser Lys Ala Arg Asp Ser Asp Glu Glu
   545 550 555 560
   Asn Asp Pro Asp Asp Glu Asp Ala Val Val Asn Ala Val Gly Cys Leu
   565 570 575
   Gly Pro Phe Ser Gly Phe Leu Ala Pro Glu Leu Gln Lys Tyr Gln Lys
   580 585 590
   Gln Ile Lys Glu Pro Asn Glu Glu Gln Ser Leu Arg Ser Asn Asn Ile
   595 600 605
   Ala Glu Leu Ser Pro Gly Ala Ile Asn Ser Cys Arg Ser Glu Tyr His
   610 615 620
   Ala Ala Phe Asn Ser Met Met Met Glu Arg Met Thr Thr Asp Ile Asn
   625 630 635 640
   Ala Leu Lys Arg Gln Tyr Ser Arg Ile Lys Lys Lys Gln Gln Gln Gln
   645 650 655
   Val His Gln Val Tyr Ile Arg Ala Asp Lys Gly Pro Val Thr Ser Ile
   660 665 670
   Leu Pro Ser Gln Val Asn Ser Ser Pro Val Ile Asn His Leu Leu Leu
   675 680 685
   Gly Lys Lys Met Lys Met Thr Asn Arg Ala Ala Lys Asn Ala Val Ile
   690 695 700
   His Ile Pro Gly His Thr Gly Gly Lys Ile Ser Pro Val Pro Tyr Glu
   705 710 715 720
   Asp Leu Lys Thr Lys Leu Asn Ser Pro Trp Arg Thr His Ile Arg Val
   725 730 735
   His Lys Lys Asn Met Pro Arg Thr Lys Ser His Pro Gly Cys Gly Asp
   740 745 750
   Thr Ile Gly Leu Ile Asp Glu Gln Asn Glu Ala Ser Lys Thr Asn Gly
   755 760 765
   Leu Gly Ala Ala Glu Ala Phe Pro Ser Gly Cys Thr Ala Thr Ala Gly
   770 775 780
   Arg Glu Gly Ser Ser Pro Glu Gly Ser Thr Arg Arg Thr Ile Glu Gly
   785 790 795 800
   Gln Ser Pro Glu Pro Val Phe Gly Asp Ala Asp Val Asp Val Ser Ala
   805 810 815
   Val Gln Ala Lys Leu Gly Ala Leu Glu Leu Asn Gln Arg Asp Ala Ala
   820 825 830
   Ala Glu Thr Glu Leu Arg Val His Pro Pro Cys Gln Arg His Cys Pro
   835 840 845
   Glu Pro Pro Ser Ala Pro Glu Glu Asn Lys Ala Thr Ser Lys Ala Pro
   850 855 860
   Gln Gly Ser Asn Ser Lys Thr Pro Ile Phe Ser Pro Phe Pro Ser Val
   865 870 875 880
   Lys Pro Leu Arg Lys Ser Ala Thr Ala Arg Asn Leu Gly Leu Tyr Gly
   885 890 895
   Pro Thr Glu Arg Thr Pro Thr Val His Phe Pro Gln Met Ser Arg Ser
   900 905 910
   Phe Ser Lys Pro Gly Gly Gly Asn Ser Gly Thr Lys Lys Arg
   915 920 925
<210> 23
   <211> 93
   <212> PRT
   <213> Homo sapiens
<400> 23
   Met Ala Gly Asn Cys Gly Ala Arg Gly Ala Leu Ser Ala His Thr Leu
   1 5 10 15
   Leu Phe Asp Leu Pro Pro Ala Leu Leu Gly Glu Leu Cys Ala Val Leu
   20 25 30
   Asp Ser Cys Asp Gly Ala Leu Gly Trp Arg Gly Leu Val Ser Lys Asn
   35 40 45
   Lys Leu Cys Glu Asn Gly Val Leu Ile Leu Gln Gly Phe Trp His Val
   50 55 60
   Val Val Cys Lys Tyr Lys Asp Ser Lys Tyr Gly Cys Val Ala Leu Phe
   65 70 75 80
   Glu Lys Thr Pro Ser Glu Thr Tyr Leu His Met Glu Thr
   85 90
<210> 24
   <211> 5653
   <212> DNA
   <213> Homo sapiens
<400> 24
   gacaaagaga actaatgctt tgtgctgatt catatttgaa tcgaggcatt gggaaccctg 60
   tatgccttgt ttgtggaaag aaccagtgac accatcactg agcttcctaa aagttcgaag 120
   aagttagagg actatacact ttcttttgaa cttttataat aaatatttgc tctggttttt 180
   ggaacccagg gctgttagag gggtgagtga caagtcttac aagtggcctt attccaactc 240
   cagaaattgc ccaacggaac tttgagatta tatgcaatcg aaagtgacag gaaacatgcc 300
   aactcaatcc ctcttaatgt acatggatgg gccagaagtg attggcagct ctcttggcag 360
   tccgatggag atggaggatg ccttgtcaat gaaagggacc gctgttgttc cattccgagc 420
   tacacaagaa aaaaatgtca tccaaatcga ggggtatatg cccttggatt gcatgttctg 480
   cagccagacc ttcacacatt cagaagacct taataaacat gtcttaatgc aacaccggcc 540
   taccctctgt gaaccagcag ttcttcgggt tgaagcagag tatctcagtc cgcttgataa 600
   aagtcaagtg cgaacagaac ctcccaagga aaagaattgc aaggaaaatg aatttagctg 660
   tgaggtatgt gggcagacat ttagagtcgc ttttgatgtt gagatccaca tgagaacaca 720
   caaagattct ttcacttacg ggtgtaacat gtgcggaaga agattcaagg agccttggtt 780
   tcttaaaaat cacatgcgga cacataatgg caaatcgggg gccagaagca aactgcagca 840
   aggcttggag agtagtccag caacgatcaa cgaggtcgtc caggtgcacg cggccgagag 900
   catctcctct ccttacaaaa tctgcatggt ttgtggcttc ctatttccaa ataaagaaag 960
   tctaattgag caccgcaagg tgcacaccaa aaaaactgct ttcggtacca gcagcgcgca 1020
   gacagactct ccacaaggag gaatgccgtc ctcgagggag gacttcctgc agttgttcaa 1080
   cttgagacca aaatctcacc ctgaaacggg gaagaagcct gtcagatgca tccctcagct 1140
   cgatccgttc accaccttcc aggcttggca gctggctacc aaaggaaaag ttgccatttg 1200
   ccaagaagtg aaggaatcgg ggcaagaagg gagcaccgac aacgacgatt cgagttccga 1260
   gaaggagctt ggagaaacaa ataagggcag ttgtgcaggc ctctcgcaag agaaagagaa 1320
   gtgcaaacac tcccacggcg aagcgccctc cgtggacgcg gatcccaagt tacccagtag 1380
   caaggagaag cccactcact gctccgagtg cggcaaagct ttcagaacct accaccagct 1440
   ggtcttgcac tccagggtcc acaagaagga ccggagggcc ggcgcggagt cgcccaccat 1500
   gtctgtggac gggaggcagc cggggacgtg ttctcctgac ctcgccgccc ctctggatga 1560
   aaatggagcc gtggatcgag gggaaggtgg ttctgaagac ggatctgagg atgggcttcc 1620
   cgaaggaatc catctggata aaaatgatga tggaggaaaa ataaaacatc ttacatcttc 1680
   aagagagtgt agttattgtg gaaagttttt ccgttcaaat tattacctca atattcatct 1740
   cagaacgcat acaggtgaaa aaccatacaa atgtgaattt tgtgaatatg ctgcagccca 1800
   gaagacatct ctgaggtatc acttggagag acatcacaag gaaaaacaaa ccgatgttgc 1860
   tgctgaagtc aagaacgatg gtaaaaatca ggacactgaa gatgcactat taaccgctga 1920
   cagtgcgcaa accaaaaatt tgaaaagatt ttttgatggt gccaaagatg ttacaggcag 1980
   tccacctgca aagcagctta aggagatgcc ttctgttttt cagaatgttc tgggcagcgc 2040
   tgtcctctca ccagcacaca aagatactca ggatttccat aaaaatgcag ctgatgacag 2100
   tgctgataaa gtgaataaaa accctacccc tgcttacctg gacctgttaa aaaagagatc 2160
   agcagttgaa actcaggcaa ataacctcat ctgtagaacc aaggcggatg ttactcctcc 2220
   tccggatggc agtaccaccc ataaccttga agttagcccc aaagagaagc aaacggagac 2280
   cgcagctgac tgcagataca ggccaagtgt ggattgtcac gaaaaacctt taaatttatc 2340
   cgtgggggct cttcacaatt gcccggcaat ttctttgagt aaaagtttga ttccaagtat 2400
   cacctgtcca ttttgtacct tcaagacatt ttatccagaa gttttaatga tgcaccagag 2460
   actggagcat aaatacaatc ctgacgttca taaaaactgt cgaaacaagt ccttgcttag 2520
   aagtcgacgt accggatgcc cgccagcgtt gctgggaaaa gatgtgcctc ccctctctag 2580
   tttctgtaaa cccaagccca agtctgcttt cccggcgcag tccaaatccc tgccatctgc 2640
   gaaggggaag cagagccctc ctgggccagg caaggcccct ctgacttcag ggatagactc 2700
   tagcacttta gccccaagta acctgaagtc ccacagacca cagcagaatg tgggggtcca 2760
   aggggccgcc accaggcaac agcaatctga gatgtttcct aaaaccagtg tttcccctgc 2820
   accggataag acaaaaagac ccgagacaaa attgaaacct cttccagtag ctccttctca 2880
   gcccaccctc ggcagcagta acatcaatgg ttccatcgac taccccgcca agaacgacag 2940
   cccgtgggca cctccgggaa gagactattt ctgtaatcgg agtgccagca atactgcagc 3000
   agaatttggt gagccccttc caaaaagact gaagtccagc gtggttgccc ttgacgttga 3060
   ccagcccggg gccaattaca gaagaggcta tgaccttccc aagtaccata tggtcagagg 3120
   catcacatca ctgttaccgc aggactgtgt gtatccgtcg caggcgctgc ctcccaaacc 3180
   aaggttcctg agctccagcg aggtcgattc tccaaatgtg ctgactgttc agaagcccta 3240
   tggtggctcc gggccacttt acacttgtgt gcctgctggt agtccagcat ccagctcgac 3300
   gttagaagga aaaaggcctg tgtcatatca acacttatct aacagcatgg cacaaaagag 3360
   aaactatgag aattttattg ggaatgcaca ttatcgacca aatgacaaaa aaacttgatt 3420
   cactaattag gggaaaaaaa ggtcttggtg gatgtcagtg cttactcccc atgaaattaa 3480
   attttacttc atcctttgag aagcgaatgg tgaaagctac tgaaataagc tgtgattgta 3540
   ctgtacataa aacatatgag gaatctgcaa ggaacactac agttgtgtaa agttgttctg 3600
   ttaacttttg taccaaatag caatacaaac tagttggaac agttggaact tacatacatg 3660
   gggactggaa atctctattt tgtccctgaa taatattttt cttagaattg accaaataag 3720
   aagtggaatt tttgcatact tgagcgctgc tgaaaagaaa tcatttgggt tgggtggggc 3780
   gggatggggg aaaagtatat aatgcttgca cctcaggtaa aaatctgtaa atatctaagt 3840
   tgtaaacctg cttgttcaaa tactgtgtgt attccttttc tctaatgcag ctcatcactt 3900
   ggagcagttt ctgctattgt gctctttcat ttaaaatgta tgtttttttt tttttaaact 3960
   gtcaatgatt ttgtattatg ttgaatccac ccaaatctat tgttgtctta aaattgttaa 4020
   tggaagtatt gaccctctat gatatgtgct gcagatatcg aggtcagcca ttcggaagct 4080
   ggcagcattt tatcgcaact ttgagcatct cagatgggga aggcaccttc ttcctcgcct 4140
   ctccagattg tcctggaacc tccaggatcc ttgactgagg gcgttgggat ggcttgtagg 4200
   atttttaaga gagtgtgtct acagacaagc attttctctg tagagcagcc acacgttgta 4260
   taaacataaa ctgtatgtgc agttatttaa atttgtttct gtcaaattaa tcatttttgt 4320
   tggacgattc agtggcgggg gggttttgcc taaattagta tataaaaaca aaaatgctaa 4380
   attatatctg tgaattgcag gtattgggga acagttttaa gggaaatttt ggggggaaca 4440
   ttttaggttt gtatttggta gtcttaatgt atctggcatt tgggtgaact gtggacatac 4500
   tagagttgat tatagacaca ttgattttga ataaggaact gctggccgag cccgctggga 4560
   gtctagaaag agaaaatctg tttctagacc tcagttattt tcccattttt ggttgttttg 4620
   aagcagtaac atttttctca gtgcacatgc aatttgggtt ttagagaaga tggccaccag 4680
   ctggcttcct agatatttta aacttttgtt ctttaatatg ctgtccatgg ctgagtttat 4740
   tagtacatgg gcttagtgac cacaaaatat tttattaaga aactgtttca aaaataaatt 4800
   tgcactgttc atttttctgg cctcgctgtt ctccatagag caagggtaat cctagaaaaa 4860
   attttttttt tttaaattat gcaacgtaag atgtcctcct tgatagaagt cttagctcct 4920
   gtgttacaag ggagaactca tttgagatca gtctgttggc attgcaatga agtgctttgt 4980
   atcaggaaag tgtacactat tgaccttttt tcctgttcac aagctgagcc atatgtacat 5040
   aatctagatt ttgttttcat agttttgcac ttttatagcc tatttttgaa gattaacaca 5100
   tttgcaagat gattgactca atctttgcct aatccaatga gtgttacaga gagcttgctg 5160
   tgactagaac cataaatctt aaagggggta tgtgataata gagggctgga atttaaacct 5220
   gtatttaaaa aaaagaatca ccaaatctat ttgaaaacaa gtcgatttgt attatgctgg 5280
   aatttttggg gctttcagat ttctcttttt aaccacattt ctgaatgtat aaaaatacca 5340
   attattttcc tacagccctt tgtacttcaa aatatgtttt tgtgtccatc agtattaact 5400
   attggtatac tactggtttt atattttttt ttctttgaga caacagtaca tataatagag 5460
   gtacaattcg ttggattttt gtttatgtat ttatttcatt ccagtttgat ttattttaat 5520
   tgttgatact taagttgtcg aacagtagac attacttgtt ttatttatga tatatttcag 5580
   cttaaagtta tgttattata tgtggaagtg taaatataga tttggtgttt tgcaaaaaaa 5640
   aaaaaaaaaa aaa 5653
<210> 25
   <211> 1048
   <212> PRT
   <213> Homo sapiens
<400> 25
   Met Gln Ser Lys Val Thr Gly Asn Met Pro Thr Gln Ser Leu Leu Met
   1               5                   10                  15
   Tyr Met Asp Gly Pro Glu Val Ile Gly Ser Ser Leu Gly Ser Pro Met
               20                  25                  30
   Glu Met Glu Asp Ala Leu Ser Met Lys Gly Thr Ala Val Val Pro Phe
           35                  40                  45
   Arg Ala Thr Gln Glu Lys Asn Val Ile Gln Ile Glu Gly Tyr Met Pro
       50                  55                  60
   Leu Asp Cys Met Phe Cys Ser Gln Thr Phe Thr His Ser Glu Asp Leu
   65                  70                  75                  80
   Asn Lys His Val Leu Met Gln His Arg Pro Thr Leu Cys Glu Pro Ala
                   85                  90                  95
   Val Leu Arg Val Glu Ala Glu Tyr Leu Ser Pro Leu Asp Lys Ser Gln
               100                 105                 110
   Val Arg Thr Glu Pro Pro Lys Glu Lys Asn Cys Lys Glu Asn Glu Phe
           115                 120                 125
   Ser Cys Glu Val Cys Gly Gln Thr Phe Arg Val Ala Phe Asp Val Glu
       130                 135                 140
   Ile His Met Arg Thr His Lys Asp Ser Phe Thr Tyr Gly Cys Asn Met
   145                 150                 155                 160
   Cys Gly Arg Arg Phe Lys Glu Pro Trp Phe Leu Lys Asn His Met Arg
                   165                 170                 175
   Thr His Asn Gly Lys Ser Gly Ala Arg Ser Lys Leu Gln Gln Gly Leu
               180                 185                 190
   Glu Ser Ser Pro Ala Thr Ile Asn Glu Val Val Gln Val His Ala Ala
           195                 200                 205
   Glu Ser Ile Ser Ser Pro Tyr Lys Ile Cys Met Val Cys Gly Phe Leu
       210                 215                 220
   Phe Pro Asn Lys Glu Ser Leu Ile Glu His Arg Lys Val His Thr Lys
   225                 230                 235                 240
   Lys Thr Ala Phe Gly Thr Ser Ser Ala Gln Thr Asp Ser Pro Gln Gly
                   245                 250                 255
   Gly Met Pro Ser Ser Arg Glu Asp Phe Leu Gln Leu Phe Asn Leu Arg
   260 265 270
   Pro Lys Ser His Pro Glu Thr Gly Lys Lys Pro Val Arg Cys Ile Pro
   275 280 285
   Gln Leu Asp Pro Phe Thr Thr Phe Gln Ala Trp Gln Leu Ala Thr Lys
   290 295 300
   Gly Lys Val Ala Ile Cys Gln Glu Val Lys Glu Ser Gly Gln Glu Gly
   305 310 315 320
   Ser Thr Asp Asn Asp Asp Ser Ser Ser Glu Lys Glu Leu Gly Glu Thr
   325 330 335
   Asn Lys Gly Ser Cys Ala Gly Leu Ser Gln Glu Lys Glu Lys Cys Lys
   340 345 350
   His Ser His Gly Glu Ala Pro Ser Val Asp Ala Asp Pro Lys Leu Pro
   355 360 365
   Ser Ser Lys Glu Lys Pro Thr His Cys Ser Glu Cys Gly Lys Ala Phe
   370 375 380
   Arg Thr Tyr His Gln Leu Val Leu His Ser Arg Val His Lys Lys Asp
   385 390 395 400
   Arg Arg Ala Gly Ala Glu Ser Pro Thr Met Ser Val Asp Gly Arg Gln
   405 410 415
   Pro Gly Thr Cys Ser Pro Asp Leu Ala Ala Pro Leu Asp Glu Asn Gly
   420 425 430
   Ala Val Asp Arg Gly Glu Gly Gly Ser Glu Asp Gly Ser Glu Asp Gly
   435 440 445
   Leu Pro Glu Gly Ile His Leu Asp Lys Asn Asp Asp Gly Gly Lys Ile
   450 455 460
   Lys His Leu Thr Ser Ser Arg Glu Cys Ser Tyr Cys Gly Lys Phe Phe
   465 470 475 480
   Arg Ser Asn Tyr Tyr Leu Asn Ile His Leu Arg Thr His Thr Gly Glu
   485 490 495
   Lys Pro Tyr Lys Cys Glu Phe Cys Glu Tyr Ala Ala Ala Gln Lys Thr
   500 505 510
   Ser Leu Arg Tyr His Leu Glu Arg His His Lys Glu Lys Gln Thr Asp
   515 520 525
   Val Ala Ala Glu Val Lys Asn Asp Gly Lys Asn Gln Asp Thr Glu Asp
   530 535 540
   Ala Leu Leu Thr Ala Asp Ser Ala Gln Thr Lys Asn Leu Lys Arg Phe
   545 550 555 560
   Phe Asp Gly Ala Lys Asp Val Thr Gly Ser Pro Pro Ala Lys Gln Leu
   565 570 575
   Lys Glu Met Pro Ser Val Phe Gln Asn Val Leu Gly Ser Ala Val Leu
   580 585 590
   Ser Pro Ala His Lys Asp Thr Gln Asp Phe His Lys Asn Ala Ala Asp
   595 600 605
   Asp Ser Ala Asp Lys Val Asn Lys Asn Pro Thr Pro Ala Tyr Leu Asp
   610 615 620
   Leu Leu Lys Lys Arg Ser Ala Val Glu Thr Gln Ala Asn Asn Leu Ile
   625 630 635 640
   Cys Arg Thr Lys Ala Asp Val Thr Pro Pro Pro Asp Gly Ser Thr Thr
   645 650 655
   His Asn Leu Glu Val Ser Pro Lys Glu Lys Gln Thr Glu Thr Ala Ala
   660 665 670
   Asp Cys Arg Tyr Arg Pro Ser Val Asp Cys His Glu Lys Pro Leu Asn
   675 680 685
   Leu Ser Val Gly Ala Leu His Asn Cys Pro Ala Ile Ser Leu Ser Lys
   690 695 700
   Ser Leu Ile Pro Ser Ile Thr Cys Pro Phe Cys Thr Phe Lys Thr Phe
   705 710 715 720
   Tyr Pro Glu Val Leu Met Met His Gln Arg Leu Glu His Lys Tyr Asn
   725 730 735
   Pro Asp Val His Lys Asn Cys Arg Asn Lys Ser Leu Leu Arg Ser Arg
   740 745 750
   Arg Thr Gly Cys Pro Pro Ala Leu Leu Gly Lys Asp Val Pro Pro Leu
   755 760 765
   Ser Ser Phe Cys Lys Pro Lys Pro Lys Ser Ala Phe Pro Ala Gln Ser
   770 775 780
   Lys Ser Leu Pro Ser Ala Lys Gly Lys Gln Ser Pro Pro Gly Pro Gly
   785 790 795 800
   Lys Ala Pro Leu Thr Ser Gly Ile Asp Ser Ser Thr Leu Ala Pro Ser
   805 810 815
   Asn Leu Lys Ser His Arg Pro Gln Gln Asn Val Gly Val Gln Gly Ala
   820 825 830
   Ala Thr Arg Gln Gln Gln Ser Glu Met Phe Pro Lys Thr Ser Val Ser
   835 840 845
   Pro Ala Pro Asp Lys Thr Lys Arg Pro Glu Thr Lys Leu Lys Pro Leu
   850 855 860
   Pro Val Ala Pro Ser Gln Pro Thr Leu Gly Ser Ser Asn Ile Asn Gly
   865 870 875 880
   Ser Ile Asp Tyr Pro Ala Lys Asn Asp Ser Pro Trp Ala Pro Pro Gly
   885 890 895
   Arg Asp Tyr Phe Cys Asn Arg Ser Ala Ser Asn Thr Ala Ala Glu Phe
   900 905 910
   Gly Glu Pro Leu Pro Lys Arg Leu Lys Ser Ser Val Val Ala Leu Asp
   915 920 925
   Val Asp Gln Pro Gly Ala Asn Tyr Arg Arg Gly Tyr Asp Leu Pro Lys
   930 935 940
   Tyr His Met Val Arg Gly Ile Thr Ser Leu Leu Pro Gln Asp Cys Val
   945 950 955 960
   Tyr Pro Ser Gln Ala Leu Pro Pro Lys Pro Arg Phe Leu Ser Ser Ser
   965 970 975
   Glu Val Asp Ser Pro Asn Val Leu Thr Val Gln Lys Pro Tyr Gly Gly
   980 985 990
   Ser Gly Pro Leu Tyr Thr Cys Val Pro Ala Gly Ser Pro Ala Ser Ser
   995 1000 1005
   Ser Thr Leu Glu Gly Lys Arg Pro Val Ser Tyr Gln His Leu Ser
   1010 1015 1020
   Asn Ser Met Ala Gln Lys Arg Asn Tyr Glu Asn Phe Ile Gly Asn
   1025 1030 1035
   Ala His Tyr Arg Pro Asn Asp Lys Lys Thr
   1040 1045

## Description

### Background and Summary

### BACKGROUND OF THE INVENTION

### A. Field of the Invention

The present invention relates generally to a new cluster of correlating molecules in a tissue or at least one cell of a tissue for instance a cell of a blood tissue, preferably such myeloid cells and of identifying the condition of the genes expression said correlating molecules or of the expression levels of said molecules in a method or system for identifying the risk of obesity-related metabolic syndrome disorder phenotype characterized by dyslipidemia, hypertension, glucose intolerance, insulin resistance and diabetes, lipid homeostasis disorders and/or cardiovascular diseases This system of method provides information on how to modulate the correlating molecules to treat or prevent obesity and to treat or prevent the obesity-related metabolic syndrome disorders.

Several documents are cited throughout the text of this specification. However, there is no admission that any document cited is indeed prior art of the present invention.

### B. Description of the Related Art

Currently more than 1 billion adults are overweight-and at least 300 million of them are clinically obese. This suggests the role of relatively modem environmental or behavioral risk factors such as high caloric intake or sedentary lifestyle.

The epidemic of obesity is a global health issue across all age groups, especially in industrialized countries (American Obesity Association, 2006). According to WHO's estimate there are more than 300 million obese people (BMI>30) world-wide. Today, for example almost 65% of adult Americans (about 127 million) are categorized as being overweight or obese. There is also evidence that obesity is increasing problem among children, for example in the USA, the percentage of overweight children (aged 5-14 years) has doubled in the last 30 years, from 15% to 32%. The degree of health impairment of obesity is determined by three factors: 1) the amount of fat 2) the distribution of fat and 3) the presence of other risk factors. It is the second leading cause of preventable death in the Western society and an increasing cause on modernizing societies. Obesity affects all major bodily systems--heart, lung, muscle and bones-and is considered as a major risk factor for several chronic disease conditions, including coronary heart disease, type 2 diabetes mellitus, hypertension, stroke, and cancers of the breast, endometrium, prostate and colon (Burton & Foster 1985).

A large number of medical conditions have been associated with obesity. Health consequences are categorized as being the result of either increased fat mass (osteoarthritis, obstructive sleep apnea, social stigma) or increased number of fat cells (diabetes, cancer, cardiovascular disease, non-alcoholic fatty liver disease) (Bray GA (2004). J. Clin. Endocrinol. Metab. 89 (6): 2583-9). Mortality is increased in obesity, with a BMI of over 32 being associated with a doubled risk of death (Manson JE, et al (1995). N. Engl. J. Med. 333 (11): 677-85). There are alterations in the body's response to insulin (insulin resistance), a pro-inflammatory state and an increased tendency to thrombosis (pro-thrombotic state) (Bray GA (2004). J. Clin. Endocrinol. Metab. 89 (6): 2583-9). Disease associations may be dependent or independent of the distribution of adipose tissue. Central obesity (male-type or waist-predominant obesity, characterized by a high waist-hip ratio), is an important risk factor for the metabolic syndrome, the clustering of a number of diseases and risk factors that heavily predispose for cardiovascular disease. These are diabetes mellitus type 2, high blood pressure, high blood cholesterol, and triglyceride levels (combined hyperlipidemia) (Grundy SM (2004). J. Clin. Endocrinol. Metab. 89 (6): 2595-600. doi:10.1210/jc.2004-0372. PMID 15181029). Apart from the metabolic syndrome, obesity is also correlated with a variety of other complications. For some of these complaints, it has not been clearly established to what extent they are caused directly by obesity itself, or have some other cause (such as limited exercise) that causes obesity as well. Cardiovascular: congestive heart failure, enlarged heart and its associated arrhythmias and dizziness, varicose veins, and pulmonary embolism. Endocrine: polycystic ovarian syndrome (PCOS), menstrual disorders, and infertility (van der Steeg JW, Steures P, Eijkemans MJ, et al (2008). "Obesity affects spontaneous pregnancy chances in subfertile, ovulatory women". Hum. Reprod. 23 (2): 324-8.) Gastrointestinal: gastroesophageal reflux disease (GERD), fatty liver disease, cholelithiasis (gallstones), hernia, and colorectal cancer. Renal and genitourinary: erectile dysfunction (Esposito K, et al. (2004). JAMA 291 (24): 2978-84. doi:10.1001/jama.291.24.2978) urinary incontinence, chronic renal failure, (Ejerblad E, Fored CM, Lindblad P, Fryzek J, McLaughlin JK, Nyrén O (2006). J. Am. Soc. Nephrol. 17 (6): 1695-702) hypogonadism (male), breast cancer (female), uterine cancer (female), stillbirth. Integument (skin and appendages): stretch marks, acanthosis nigricans, lymphedema, cellulitis, carbuncles, intertrigo. Musculoskeletal: hyperuricemia (which predisposes to gout), immobility, osteoarthritis, low back pain. Neurologic: stroke, meralgia paresthetica, headache, carpal tunnel syndrome, dementia, (Whitmer RA, et al. (2005). BMJ 330 (7504): 1360) idiopathic intracranial hypertension. Respiratory: obstructive sleep apnea, obesity hypoventilation syndrome, asthma. Psychological: Depression, low self esteem, body dimorphic disorder, social stigmatization.

The economic cost attributable to obesity is substantial and is close to $100 billion/yr (Wolf & Colditz 1998). Obesity accounts for 2-6% of total health care costs in several developed countries; some estimates put the figure as high as 7%. The true costs are undoubtedly much greater as not all obesity-related conditions are included in the calculations.

There is thus a clear need in the art to have accurate predictive tools for the risk of obesity associated disorders and there is a need as well for accurate diagnostic tools that are indicative for a proper treatment of obesity associated disorders such as obesity associated metabolic syndrome, obesity related cardiovascular disorders and obesity related insulin resistance for person in need thereto.

Present invention provides such solution to these problems in the art. The present inventors have namely found that in particular IRAK3 expression or activity is very suitable for diagnosing, identifying, and/or monitoring a metabolic syndrome disorder phenotype in a subject. In addition, also combinations of expression or activity of IRAK3 and ZNF217; or IRAK3 and one or more of SOD2, TNFAIP3, TNFAIP3, TLR2, IRS2 and ZNF217 may serve the same purpose. None of said genes or expression products thereof have been previously linked to the metabolic syndrome disorder, in contrast to Myd88 (WO2006083798 - (0026)), of which the expression and function in hematopoietic cells was found to link obesity, to inflammation and insulin resistance. Rather, the IRAK (Interleukin 1 receptor associated kinase) family has been found to be involved in branching morphogenesis (WO2005051314), whereas overexpression of ZNF217 was found to be associated with cancer (WO03079748). It was therefore unexpected that IRAK3 and ZNF217 could have a prognostic value in diagnosing, identifying and/or monitoring the metabolic syndrome disorder.

### SUMMARY OF THE INVENTION

In accordance with the purpose of the invention, as embodied and broadly described herein, the invention is broadly drawn to a method of diagnosis or a diagnostic tool for assessing the condition of a subject or for testing the efficacy of his or her treatment.

The present invention solves the problems of the related art by providing an accurate diagnosis tool for the progression of a metabolic syndrome disorder and in particular for the progression of a lipid homeostasis disorder, such as an impaired glucose tolerance and/or insulin resistance condition seen with said lipid homeostasis disorder; and/or the progression of an adipocyte tissue disorder, such as an impaired adipose tissue accumulation or adipocyte function; hereinafter also referred to as an obesity-related metabolic syndrome, both with increased risk to related cardiovascular diseases.

In particular this invention demonstrates that IRAK3 is a causal biomarker for obesity-related metabolic syndrome disorders.

Accordingly, in a first embodiment the present invention provides a method of diagnosing a metabolic syndrome disorder phenotype in a subject, i.e. determining whether a sample is from tissue of a mammal having a metabolic syndrome disorder phenotype, said method comprising: (**a**) determine or analyze the level of IRAK3 expression or activity of an IRAK3 expression product in a biological sample isolated from said subject, and (**b**) compare said level of expression or activity of said expression product with the IRAK3 expression or activity in a control sample; whereby a decreased level of IRAK3 expression or activity is indicative for the progression to an impaired glucose tolerance and/or insulin resistance condition seen with said metabolic syndrome disorder both with increased risk to related cardiovascular diseases.

As provided in more details in the examples hereinafter, in addition to IRAK3, further genes of interest in diagnosing the progression of a metabolic syndrome disorder and in discriminating lean from obese subjects, include SOD2, TNFAIP6, TNFAIP3, TLR2, IRS2, and ZNF217.

Thus in a further aspect, the present invention provides a method of diagnosing a metabolic syndrome disorder phenotype in a subject, including discriminating lean from obese subjects, said method comprising: (**a**) obtaining an expression profile in a biological sample isolated from said subject, wherein said expression profile consists of the analysis of the level of IRAK3 expression or activity of an IRAK3 expression product in combination with the gene expression level or activity of a gene product of at least one gene selected from the group consisting of SOD2, TNFAIP6, TNFAIP3, TLR2, IRS2 and ZNF217; and (**b**) comparing said obtained expression profile to a reference expression profile to determine whether said sample is from subject having a metabolic syndrome disorder phenotype. A lower than reference expression profile of IRAK3 gene is indicative for said metabolic syndrome disorder phenotype. In a particular embodiment of the aforementioned method, the genes analyzed in the expression profile consist of any one of the following combinations; IRAK3 and SOD2; IRAK3 and TNFAIP6; IRAK3 and TNFAIP3; IRAK 3 and ZNF217; IRAK3 and TNFAIP6 and TNFAIP3; IRAK3 and SOD2 and TNFAIP3; IRAK3 and SOD2 and TNFAIP6; IRAK3 and SOD2 and ZNF217; IRAK3 and TNFAIP3 and ZNF217; IRAK3 and TNFAIP6 and ZNF217; IRAK3 and SOD2 and TNFAIP3 and TNFAIP6; IRAK3 and SOD2 and TNFAIP3 and ZNF217; IRAK3 and SOD2 and TNFAIP6 and ZNF217; IRAK3 and TNFAIP3, TNFAIP6, SOD2 and ZNF217. In said embodiment, a lower than reference expression profile of IRAK3 and a higher than reference expression profile of SOD2, TNFAIP3, TNFAIP6 and/or ZNF217 gene is indicative for said metabolic syndrome disorder phenotype.

In a particular embodiment of present invention, the metabolic syndrome disorder in the aforementioned methods is a lipid homeostasis disorder; more in particular a glucose intolerance and/or insulin resistance phenotype.

Yet another aspect of present invention is a method of diagnosing the risk for cardiovascular diseases in a subject, i.e. determining whether a sample is from tissue of a mammal having a high risk for cardiovascular diseases, said method comprising: (**a**) determine or analyze the level of IRAK3 expression or activity of an IRAK3 expression product in a biological sample isolated from said subject, and (**b**) compare said level of expression or activity of said expression product with the IRAK3 expression or activity in a control sample; whereby a decreased level of IRAK3 expression or activity is indicative for said risk for cardiovascular diseases. A higher than reference expression profile of SOD2, TNFAIP3, TNFAIP6 and/or ZNF217 gene is indicative for said cardiovascular diseases.

The present invention also concerns the above described methods for testing or evaluating whether a medicament, a diet or nutriceutical is efficient in curing or decreasing the disorders.

Further scope of applicability of the present invention will become apparent from the detailed description given hereinafter. However, it should be understood that the detailed description and specific examples, while indicating preferred embodiments of the invention, are given by way of illustration only, since various changes and modifications will become apparent to those skilled in the art from this detailed description. It is to be understood that both the foregoing general description and the following detailed description are exemplary and explanatory only and are not restrictive of the invention, as claimed.

### Illustrative embodiments of the invention

### DEFINITIONS

**Myeloid** refers to the nonlymphocytic groups of white blood cells, including the granulocytes, monocytes and platelets.

**Dyslipidemia** (From dys- + lipid (fat) + -emia (in the blood) = essentially, disordered lipids in the blood) is a disorder of lipoprotein metabolism. Dyslipidemias may be manifested by elevation of the triglyceride concentrations, and a decrease in the "good" high-density lipoprotein (HDL) cholesterol concentration in the blood. Dyslipidemia comes under consideration in many situations including diabetes, a common cause of lipidemia. For adults with diabetes, it has been recommended that the levels HDL-cholesterol, and triglyceride be measured every year. Optimal HDL-cholesterol levels are equal to or greater than 40 mg/dL (1.02 mmol/L), and desirable triglyceride levels are less than 150 mg/dL (1.7 mmol/L).

**Insulinemia** concerns an abnormally large concentration of insulin in the blood.

**Glycemia** concerns the presence of glucose in the blood. It is a medical term meaning that the blood glucose is elevated, typically above 100 mg/dl.

**Hypercholesterolemia** is manifested by elevation of the total cholesterol due to elevation of the "bad" low-density lipoprotein (LDL) cholesterol in the blood. Optimal LDL-cholesterol levels for adults with diabetes are less than 100 mg/dL (2.60 mmol/L),

**Triglycerides** are the major form of fat. A triglyceride consists of three molecules of fatty acid combined with a molecule of the alcohol glycerol. Triglycerides serve as the backbone of many types of lipids (fats). Triglycerides come from the food we eat as well as from being produced by the body. Triglyceride levels are influenced by recent fat and alcohol intake, and should be measured after fasting for at least 12 hours. A period of abstinence from alcohol is advised before testing for triglycerides. Markedly high triglyceride levels (greater than 500mg/dl) can cause inflammation of the pancreas (pancreatitis). Therefore, these high levels should be treated aggressively with low fat diets and medications, if needed. The word "triglyceride" reflects the fact that a triglyceride consists of three ("tri-") molecules of fatty acid combined with a molecule of the alcohol glycerol ("-glyceride") that serves as the backbone in many types of lipids (fats).

**HDL-cholesterol** concerns lipoproteins, which are combinations of lipids (fats) and proteins, are the form in which lipids are transported in the blood. The high-density lipoproteins transport cholesterol from the tissues of the body to the liver so it can be gotten rid of (in the bile). HDL-cholesterol is therefore considered the "good" cholesterol. The higher the HDL-cholesterol level, the lower the risk of coronary artery disease. Even small increases in HDL-cholesterol reduce the frequency of heart attacks. For each 1 mg/dl increase in HDL-cholesterol there is a 2 to 4% reduction in the risk of coronary heart disease. Although there are no formal guidelines, proposed treatment goals for patients with low HDL-cholesterol are to increase HDL-cholesterol to above 35 mg/dl in men and 45 mg/dl in women with a family history of coronary heart disease; and to increase HDL-cholesterol to approach 45 mg/dl in men and 55 mg/dl in women with known coronary heart disease. The first step in increasing HDL-cholesterol levels is life style modification. Regular aerobic exercise, loss of excess weight (fat), and cessation of cigarette smoking cigarettes will increase HDL-cholesterol levels. Moderate alcohol consumption (such as one drink a day) also raises HDL-cholesterol. When life style modifications are insufficient, medications are used. Medications that are effective in increasing HDL-cholesterol include nicotinic acid (niacin), gemfibrozil (Lopid), estrogen, and to a lesser extent, the statin drugs.

**Hypertension or High blood pressure** is defined as a repeatedly elevated blood pressure exceeding 140 over 90 mmHg -- a systolic pressure above 140 with a diastolic pressure above 90. Chronic hypertension is a "silent" condition. Stealthy as a cat, it can cause blood vessel changes in the back of the eye (retina), abnormal thickening of the heart muscle, kidney failure, and brain damage. For diagnosis, there is no substitute for measurement of blood pressure. Not having your blood pressure checked (or checking it yourself) is an invitation to hypertension. No specific cause for hypertension is found in 95% of cases. Hypertension is treated with regular aerobic exercise, weight reduction (if overweight), salt restriction, and medications. **Diabetes, type 2** is one of the two major types of diabetes, the type in which the beta cells of the pancreas produce insulin but the body is unable to use it effectively because the cells of the body are resistant to the action of insulin. Although this type of diabetes may not carry the same risk of death from ketoacidosis, it otherwise involves many of the same risks of complications as type 1 diabetes (in which there is a lack of insulin). The aim of treatment is to normalize the blood glucose in an attempt to prevent or minimize complications. People with type 2 diabetes may experience marked hyperglycemia, but most do not require insulin injections. In fact, 80% of all people with type 2 diabetes can be treated with diet, exercise, and, if needed be, oral hypoglycemic agents (drugs taken by mouth to lower the blood sugar). Type 2 diabetes requires good dietary control including the restriction of calories, lowered consumption of simple carbohydrates and fat with increased consumption of complex carbohydrates and fiber. Regular aerobic exercise is also an important method for treating both type 2 diabetes since it decreases insulin resistance and helps burn excessive glucose. Regular exercise also may help lower blood lipids and reduce some effects of stress, both important factors in treating diabetes and preventing complications. Type 2 diabetes is also known as insulin-resistant diabetes, non-insulin dependent diabetes, and adult-onset diabetes.

**Systolic**: The blood pressure when the heart is contracting. It is specifically the maximum arterial pressure during contraction of the left ventricle of the heart. The time at which ventricular contraction occurs is called systole. In a blood pressure reading, the systolic pressure is typically the first number recorded. For example, with a blood pressure of 120/80 ("120 over 80"), the systolic pressure is 120. By "120" is meant 120 mm Hg (millimeters of mercury). A systolic murmur is a heart murmur heard during systole, the time the heart contracts, between the normal first and second heart sounds. "Systolic" comes from the Greek systole meaning "a drawing together or a contraction." The term has been in use since the 16th century to denote the contraction of the heart muscle.

**Osteoarthritis** is a type of arthritis caused by inflammation, breakdown, and eventual loss of cartilage in the joints. It is also known as degenerative arthritis.

**An ischemic stroke** is death of an area of brain tissue (cerebral infarction) resulting from an inadequate supply of blood and oxygen to the brain due to blockage of an artery. Ischemic stroke usually results when an artery to the brain is blocked, often by a blood clot or a fatty deposit due to atherosclerosis. Symptoms occur suddenly and may include muscle weakness, paralysis, lost or abnormal sensation on one side of the body, difficulty speaking, confusion, problems with vision, dizziness, and loss of balance and coordination. Diagnosis is usually based on symptoms and results of a physical examination, imaging tests, and blood tests. Treatment may include drugs to break up blood clots or to make blood less likely to clot and surgery, followed by rehabilitation. About one third of people recover all or most of normal function after an ischemic stroke. Ischemic stroke occurs when local blood flow is suddenly limited by vessel occlusion. The rate of neuronal death varies with blood flow. If blood flow falls to less than 15 mL/100 g/min, energy failure and subsequent cell death occur within minutes. Even suboptimal flow for longer periods may cause the cells to die by an apoptotic mechanism over days to weeks. Rapid restoration of blood flow is essential to save brain tissue. The mechanism of stroke involving the PCA territory is variable. It is commonly due to embolization from the heart, the aortic arch, the vertebral artery, or the basilar artery. Other mechanisms include intrinsic atherosclerotic disease and vasospasm. Migrainous strokes tend to involve PCAs preferentially. Less commonly, the anterior circulation is to blame (e.g., internal carotid stenosis), when a fetal PCA is present. Rare causes of stroke may be considered when usual culprits such as coagulation abnormalities, vasculitis, sympathomimetic drugs, and metabolic disorders are not present.

**Insulin resistance** is the diminished ability of cells to respond to the action of insulin in transporting glucose (sugar) from the bloodstream into muscle and other tissues. Insulin resistance typically develops with obesity and heralds the onset of type 2 diabetes. It is as if insulin is "knocking" on the door of muscle. The muscle hears the knock, opens up, and lets glucose in. But with insulin resistance, the muscle cannot hear the knocking of the insulin (the muscle is "resistant"). The pancreas makes more insulin, which increases insulin levels in the blood and causes a louder "knock." Eventually, the pancreas produces far more insulin than normal and the muscles continue to be resistant to the knock. As long as one can produce enough insulin to overcome this resistance, blood glucose levels remain normal. Once the pancreas is no longer able to keep up, blood glucose starts to rise, initially after meals, eventually even in the fasting state. Insulin resistance is an early feature and finding in the pathogenesis of type 2 diabetes associated with obesity is the development is insulin resistance, defined as impaired insulin-mediated glucose clearance in insulin-sensitive tissues (skeletal muscle, liver and adipose tissue) (Martin BC et al. Lancet. 1992 Oct 17;340(8825):925-9 and Warram J H et al, Ann Intern Med. 1990 Dec 15;113(12):909-15). Insulin resistance is the condition in which normal amounts of insulin are inadequate to produce a normal insulin response from fat, muscle and liver cells. Insulin resistance in fat cells reduces the effects of insulin and results in elevated hydrolysis of stored triglycerides in the absence of measures which either increase insulin sensitivity or which provide additional insulin. Increased mobilization of stored lipids in these cells elevates free fatty acids in the blood plasma. Insulin resistance in muscle cells reduces glucose uptake (and so local storage of glucose as (glycogen), whereas insulin resistance in liver cells reduces storage of glycogen, making it unavailable for release into the blood when blood insulin levels fall (normally only when blood glucose levels are at low storage: Both lead to elevated blood glucose levels. High plasma levels of insulin and glucose due to insulin resistance often lead to metabolic syndrome and type 2 diabetes, including its complications. In 2000, there were approximately 171 million people, worldwide, with diabetes. The numbers of diabetes patients will expectedly more than double over the next 25 years, to reach a total of 366 million by 2030 (WHO/IDF, 2004). The two main contributors to the worldwide increase in prevalence of diabetes are population ageing and urbanization, especially in developing countries, with the consequent increase in the prevalence of obesity (WHO/IDF, 2004).

**Cardiovascular diseases** refer to the class of diseases that involve the heart or blood vessels (arteries and veins). While the term technically refers to any disease that affects the cardiovascular system, it is usually used to refer to those related to atherosclerosis (arterial disease). The circulatory system (or cardiovascular system) is an organ system that moves nutrients, gases, and wastes to and from cells, helps fight diseases and helps stabilize body temperature and pH to maintain homeostasis. While humans, as well as other vertebrates, have a closed circulatory system (meaning that the blood never leaves the network of arteries, veins and capillaries), some invertebrate groups have open circulatory system. The present diagnostic invention is particularly suitable for a cardiovascular disease of the group consisting of hypertension, coronary heart disease, heart failure, congestive heart failure, atherosclerosis, arteriosclerosis, stroke, cerebrovascular disease, myocardial infarction and peripheral vascular disease.

The following terms are similar, yet distinct, in both spelling and meaning, and can be easily confused: arteriosclerosis, arteriolosclerosis and atherosclerosis.

**Arteriosclerosis** also called hardening of the arteries chronic disease is characterized by abnormal thickening and hardening of the walls of arteries, with a resulting loss of elasticity. The major form of arteriosclerosis is atherosclerosis, in which plaques of consisting of macrophages, fatty deposits in foam cells, or atheromas, form on the inner walls of the arteries. These fatty acids are largely due to the uptake of oxidized LDL by macrophages. Arteriosclerosis is a general term describing any hardening (and loss of elasticity) of medium or large arteries (in Greek, "Arterio" meaning artery and "sclerosis" meaning hardening); arteriolosclerosis is arteriosclerosis mainly affecting the arterioles (small arteries); atherosclerosis is a hardening of an artery specifically due to an atheromatous plaque. Therefore, atherosclerosis is a form of arteriosclerosis. Arteriosclerosis ("hardening of the artery") results from a deposition of tough, rigid collagen inside the vessel wall and around the atheroma. This increases the stiffness, decreases the elasticity of the artery wall. **Arteriolosclerosis** (hardening of small arteries, the arterioles) is the result of collagen deposition, but also muscle wall thickening and deposition of protein ("hyaline").Calcification, sometimes even ossification (formation of complete bone tissue) occurs within the deepest and oldest layers of the sclerosed vessel wall.

**Atherosclerosis** causes two main problems. First, the atheromatous plaques, though long compensated for by artery enlargement, eventually lead to plaque ruptures and stenosis (narrowing) of the artery and, therefore, an insufficient blood supply to the organ it feeds. If the compensating artery enlargement is excessive, a net aneurysm occurs. Atherosclerosis chronic disease is caused by the deposition of fats, cholesterol, calcium, and other substances in the innermost layer (endothelium) of the large and medium-sized arteries. Atherosclerosis is a disease affecting the arterial blood vessel. It is commonly referred to as a "hardening" or "furring" of the arteries. It is caused by the formation of multiple plaques within the arteries.
These complications are chronic, slowly progressing and cumulative. Most commonly, soft plaque suddenly ruptures (see vulnerable plaque), causing the formation of a thrombus that will rapidly slow or stop blood flow, e.g. 5 minutes, leading to death of the tissues fed by the artery. This catastrophic event is called an infarction. One of the most common recognized scenarios is called coronary thrombosis of a coronary artery causing myocardial infarction (a heart attack). Another common scenario in very advanced disease is claudication from insufficient blood supply to the legs, typically due to a combination of both stenosis and aneurismal segments narrowed with clots. Since atherosclerosis is a body wide process, similar events also occur in the arteries to the brain, intestines, kidneys, legs, etc.
Pathologically, the atheromatous plaque is divided into three distinct components: the nodular accumulation of a soft, flaky, yellowish material at the centre of large plaques composed of macrophages nearest the lumen of the artery; sometimes with underlying areas of cholesterol crystals; and possibly also calcification at the outer base of older/more advanced lesions.

**Thrombogenicity** refers to the tendency of a material in contact with the blood to produce a thrombus, or clot. It not only refers to fixed thrombi but also to emboli, thrombi which have become detached and travel through the bloodstream. Thrombogenicity can also encompass events such as the activation of immune pathways and the complement system. All materials are considered to be thrombogenic with the exception of the endothelial cells which line the vasculature. Certain medical implants appear non-thrombogenic due to high flow rates of blood past the implant, but in reality, all are thrombogenic to a degree. A thrombogenic implant will eventually be covered by a fibrous capsule, the thickness of this capsule can be considered one measure of thrombogenicity, and if extreme can lead to the failure of the implant.

**Low-density lipoprotein** (LDL) belongs to the lipoprotein particle family. Its size is approx. 22 nm and its mass is about 3 million Daltons; but, since LDL particles contain a changing number of fatty acids, they actually have a mass and size distribution. Each native LDL particle contains a single apolipoprotein B-100 molecule (Apo B-100, a protein with 4536 amino acid residues) that circles the fatty acids, keeping them soluble in the aqueous environment. In addition, LDL has a highly-hydrophobic core consisting of polyunsaturated fatty acid known as linoleate and about 1500 esterified cholesterol molecules. This core is surrounded by a shell of phospholipids and unesterified cholesterol as well as a single copy of B-100 large protein (514 kD)[ Segrest, J. P. et al (September 2001ture of apolipoprotein B-100 in low density lipoproteins). Journal of Lipid Research 42: 1346-1367]. Cholesterol is an animal sterol that is normally synthesized by the liver. The main types, low-density lipoprotein (LDL) and high-density lipoprotein (HDL) carry cholesterol from and to the liver, respectively. LDL-cholesterol concerns thus the cholesterol in low-density lipoproteins. Cholesterol is required in the membrane of mammalian cells for normal cellular function, and is either synthesized in the endoplasmic reticulum, or derived from the diet, in which case it is delivered by the bloodstream in low-density lipoproteins. These are taken into the cell by LDL receptor-mediated endocytosis in clathrin-coated pits, and then hydrolyzed in lysosomes. Oxidized LDL-cholesterol concerns a LDL-cholesterol that has been bombarded by free radicals; it is thought to cause atherosclerosis; the 'bad' cholesterol; a high level in the blood is thought to be related to various pathogenic conditions

**Metabolic syndrome** is a combination of medical disorders that increase the risk of developing cardiovascular disease and type 2 diabetes. It affects a large number of people, and prevalence increases with age. Some studies estimate the prevalence in the USA to be up to 25% of the population. Metabolic syndrome is also known as metabolic syndrome X, syndrome X, insulin resistance syndrome, Reaven's syndrome or CHAOS. Metabolic syndrome components were defined as detailed in the Third Report of the National Cholesterol Education Program Expert Panel on Detection, Evaluation, and Treatment of High Blood Cholesterol in adults (ATPIII) report: 1) waist circumference ≥102 cm in men and ≥ 88 cm in women; 2) fasting triglycerides ≥ 150 mg/dl (1.70 mmol/l); 3) HDL-cholesterol <40 mg/dl (1.03 mmol/l) in men and < 50 mg/dl (1.29 mmol/l) in women; 4) blood pressure ≥ 130/85 mmHg or on anti-hypertensive medication; 5) fasting-glucose ≥ 100 mg/dl (5.55 mmol/l) or on anti-diabetic medication.

**"Sample"** or **"biological sample"** as used herein can be any organ, tissue, cell, or cell extract isolated from a subject, such as a sample isolated from a mammal having a metabolic syndrome disorder or at risk for a metabolic syndrome disorder (e.g., based on family history or personal history). For example, a sample can include, without limitation, cells or tissue (e.g., from a biopsy or autopsy), peripheral blood, whole blood, red cell concentrates, platelet concentrates, leukocyte concentrates, blood cell proteins, blood plasma, platelet-rich plasma, a plasma concentrate, a precipitate from any fractionation of the plasma, a supernatant from any fractionation of the plasma, blood plasma protein fractions, purified or partially purified blood proteins or other components, serum, tissue or fine needle biopsy samples, or any other specimen, or any extract thereof, obtained from a patient (human or animal), test subject, healthy volunteer, or experimental animal. A subject can be a human, rat, mouse, non-human primate, etc. A sample may also include sections of tissues such as frozen sections taken for histological purposes. A "sample" may also be a cell or cell line created under experimental conditions, that is not directly isolated from a subject.

In a particular embodiment the sample is selected from the group consisting of (a) a liquid containing cells; (b) a tissue-sample; (c) a cell-sample and (d) a cell biopsy; more in particular the sample comprises hematopoietic cells or blood cells; even more in particular the sample comprises at least one myeloid cell or debris thereof. In an even further embodiment the sample comprises at least one of monocytes or peripheral blood mononuclear cells or debris thereof.

A **"control"** or **"reference"** includes a sample obtained for use in determining base-line expression or activity. Accordingly, a control sample may be obtained by a number of means including from subjects not having a metabolic syndrome disorder; from subjects not suspected of being at risk for developing a metabolic syndrome disorder; or from cells or cell lines derived from such subjects. A control also includes a previously established standard, such as a previously characterized pool of RNA or protein extracts from monocytes of at least 20 subjects without any of the metabolic syndrome components as defined above. Accordingly, any test or assay conducted according to the invention may be compared with the established standard and it may not be necessary to obtain a control sample for comparison each time.

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), Microarrays in Clinical Diagnostics (© 2005 Humana Press Inc.) provide one skilled in the art with a general guide to many of the terms used in the present application.

For purposes of the present invention, the following terms are defined below.
The term **"array"** or **"microarray"** in general refers to an ordered arrangement of hybridizable array elements such as polynucleotide probes on a substrate. An "array" is typically a spatially or logically organized collection, e.g., of oligonucleotide sequences or nucleotide sequence products such as RNA or proteins encoded by an oligonucleotide sequence. In some embodiments, an array includes antibodies or other binding reagents specific for products of a candidate library. The array element may be an oligonucleotide, DNA fragment, polynucleotide, or the like, as defined below. The array element may include any element immobilized on a solid support that is capable of binding with specificity to a target sequence such that gene expression may be determined, either qualitatively or quantitatively.

When referring to a pattern of expression, a **"qualitative"** difference in gene expression refers to a difference that is not assigned a relative value. That is, such a difference is designated by an "all or nothing" valuation. Such an all or nothing variation can be, for example, expression above or below a threshold of detection (an on/off pattern of expression). Alternatively, a qualitative difference can refer to expression of different types of expression products, e.g., different alleles (e.g., a mutant or polymorphic allele), variants (including sequence variants as well as post-translationally modified variants), etc. In contrast, a "quantitative" difference, when referring to a pattern of gene expression, refers to a difference in expression that can be assigned a value on a graduated scale, (e.g., a 0-5 or 1-10 scale, a + +++ scale, a grade 1 grade 5 scale, or the like; it will be understood that the numbers selected for illustration are entirely arbitrary and in no-way are meant to be interpreted to limit the invention). Microarrays are useful in carrying out the methods disclosed herein because of the reproducibility between different experiments. DNA microarrays provide one method for the simultaneous measurement of the expression levels of large numbers of genes. Each array consists of a reproducible pattern of capture probes attached to a solid support. Labeled RNA or DNA is hybridized to complementary probes on the array and then detected for instance by laser scanning. Hybridization intensities for each probe on the array are determined and converted to a quantitative value representing relative gene expression levels. See the patent publications Nos. US6040138, US5800992 and US6020135, US6033860, US 6344316, US7439346, US7371516, US7353116, US7348181, US7347921, US7335762 , US7335470, US7323308, US7321829, US7302348, US7276592, US7264929, US7244559, US7221785, US7211390, US7189509, US7138506, US7052842, US7047141 and US7031845. High-density oligonucleotide arrays are particularly useful for determining the gene expression profile for a large number of RNA's in a sample.

A **"DNA fragment"** includes polynucleotides and/or oligonucleotides and refers to a plurality of joined nucleotide units formed from naturally-occurring bases and cyclofuranosyl groups joined by native phosphodiester bonds. This term effectively refers to naturally-occurring species or synthetic species formed from naturally-occurring subunits. "DNA fragment" also refers to purine and pyrimidine groups and moieties which function similarly but which have no naturally-occurring portions. Thus, DNA fragments may have altered sugar moieties or inter-sugar linkages. Exemplary among these are the phosphorothioate and other sulfur containing species. They may also contain altered base units or other modifications, provided that biological activity is retained. DNA fragments may also include species that include at least some modified base forms. Thus, purines and pyrimidines other than those normally found in nature may be so employed. Similarly, modifications on the cyclofuranose portions of the nucleotide subunits may also occur as long as biological function is not eliminated by such modifications.

The term "**polynucleotide**," when used in singular or plural generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of cells, including simple and complex cells.

The term "**oligonucleotide**" refers to a relatively short polynucleotide, including, without limitation, single-stranded deoxyribonucleotides, single- or double-stranded ribonucleotides, RNA: DNA hybrids and double-stranded DNAs. Oligonucleotides, such as single-stranded DNA oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells.

The terms **"differentially expressed gene,"** "differential gene expression" and their synonyms, which are used interchangeably, refer to a gene whose expression is activated to a higher or lower level in a subject, relative to its expression in a normal or control subject. A differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes, or a comparison of the ratios of the expression between two or more genes, or even a comparison of two differently processed products of the same gene, which differ between normal subjects and subjects suffering from a disease, or between various stages of the same disease. Differential expression includes both quantitative, as well as qualitative, differences in the temporal or cellular expression pattern in a gene or its expression products. As used herein, "differential gene expression" can be present when there is, for example, at least an about a one to about two-fold, or about two to about four-fold, or about four to about six-fold, or about six to about eight-fold, or about eight to about ten-fold, or greater than about 11-fold difference between the expression of a given gene in a patient of interest compared to a suitable control. However, folds change less than one is not intended to be excluded and to the extent such change can be accurately measured, a fold change less than one may be reasonably relied upon in carrying out the methods disclosed herein.
In some embodiments, the fold change may be greater than about five or about 10 or about 20 or about 30 or about 40.

The phrase **"gene expression profile"** as used herein, is intended to encompass the general usage of the term as used in the art, and generally means the collective data representing gene expression with respect to a selected group of two or more genes, wherein the gene expression may be upregulated, downregulated, or unchanged as compared to a reference standard A gene expression profile is obtained via measurement of the expression level of many individual genes. The expression profiles can be prepared using different methods. Suitable methods for preparing a gene expression profile include, but are not limited to reverse transcription loop-mediated amplification (RT-LAMP), for instance one-step RT-LAMP, quantitative RT-PCR, Northern Blot, in situ hybridization, slot-blotting, nuclease protection assay, nucleic acid arrays, and immunoassays. The gene expression profile may also be determined indirectly via measurement of one or more gene products (whether a full or partial gene product) for a given gene sequence, where that gene product is known or determined to correlate with gene expression.

The phrase **"gene product"** is intended to have the meaning as generally understood in the art and is intended to generally encompass the product(s) of RNA translation resulting in a protein and/or a protein fragment. The gene products of the genes identified herein may also be used for the purposes of diagnosis or treatment in accordance with the methods described herein.

A **"'reference gene expression profile"** as used herein, is intended to indicate the gene expression profile, as defined above, for a pre selected group which is useful for comparison to the gene expression profile of a subject of interest. For example, the reference gene expression profile may be the gene expression profile of a single individual known to not have an metabolic syndrome disorder phenotype or a propensity thereto (i.e.. a "normal" subject) or the gene expression profile represented by a collection of RNA samples from "'normal" individuals that has been processed as a single sample. The "reference gene expression profile" may vary and such variance will be readily appreciated by one of ordinary skill in the art.

The phrase **"reference standard"** as used herein may refer to the phrase **"reference gene expression profile"** or may more broadly encompass any suitable reference standard which may be used as a basis of comparison with respect to the measured variable. For example, a reference standard may be an internal control, the gene expression or a gene product of a "healthy" or "'normal" subject, a housekeeping gene, or any unregulated gene or gene product. The phrase is intended to be generally non-limiting in that the choice of a reference standard is well within the level of skill in the art and is understood to vary based on the assay conditions and reagents available to one using the methods disclosed herein.
"Gene expression profiling" as used herein, refers to any method that can analyze the expression of selected genes in selected samples.
The phrase "gene expression system" as used herein, refers to any system, device or means to detect gene expression and includes diagnostic agents, candidate libraries, oligonucleotide sets or probe sets.

The terms **"diagnostic oligonucleotide"** or **"diagnostic oligonucleotide set"** generally refers to an oligonucleotide or to a set of two or more oligonucleotides that, when evaluated for differential expression their corresponding diagnostic genes, collectively yields predictive data.

Such predictive data typically relates to diagnosis, prognosis, selection of therapeutic agents, monitoring of therapeutic outcomes, and the like. In general, the components of a diagnostic oligonucleotide or a diagnostic oligonucleotide set are distinguished from oligonucleotide sequences that are evaluated by analysis of the DNA to directly determine the genotype of an individual as it correlates with a specified trait or phenotype, such as a disease, in that it is the pattern of expression of the components of the diagnostic oligonucleotide set, rather than mutation or polymorphism of the DNA sequence that provides predictive value. It will be understood that a particular component (or member) of a diagnostic oligonucleotide set can, in some cases, also present one or more mutations, or polymorphisms that are amenable to direct genotyping by any of a variety of well known analysis methods, e.g., Southern blotting, RFLP, AFLP, SSCP, SNP, and the like.
The phrase "gene amplification" refers to a process by which multiple copies of a gene or gene fragment are formed in a particular cell or cell line. The duplicated region (a stretch of amplified DNA) is often referred to as "amplicon." Usually, the amount of the messenger RNA (mRNA) produced, i.e., the level of gene expression, also increases in the proportion of the number of copies made of the particular gene expressed.

A **"gene expression system"** refers to any system, device or means to detect gene expression and includes diagnostic agents, candidate libraries oligonucleotide, diagnostic gene sets, oligonucleotide sets, array sets, or probe sets.

As used herein, a **"gene probe"** refers to the gene sequence arrayed on a substrate.

As used herein, a **"nucleotide probe"** refers to the oligonucleotide, DNA fragment, polynucleotide sequence arrayed on a substrate.
The terms "splicing" and "RNA splicing" are used interchangeably and refer to RNA processing that removes introns and joins exons to produce mature mRNA with continuous coding sequence that moves into the cytoplasm of a eukaryotic cell.

**"Stringency"** of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995) and in Current Protocols in Molecular Biology Copyright © 2007 by John Wiley and Sons, Inc., 2008.

As used herein, a **"gene target"** refers to the sequence derived from a biological sample that is labeled and suitable for hybridization to a gene probe affixed on a substrate and a "nucleotide target" refers to the sequence derived from a biological sample that is labeled and suitable for hybridization to a nucleotide probe affixed on a substrate.

The term **"treatment"** refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented. The practice of the present invention will employ, unless otherwise indicated, conventional techniques of molecular biology (including recombinant techniques), microbiology, cell biology and biochemistry, which are within the skill of the art.

### DETAILED DESCRIPTION OF EMBODIMENTS OF THE INVENTION

The following detailed description of the invention refers to the accompanying drawings. Also, the following detailed description does not limit the invention. Instead, the scope of the invention is defined by the appended claims and equivalents thereof.

The risk for developing heart disease is directly related to the concomitant burden of obesity-related cardiovascular risk factors clustered in the metabolic syndrome (MetSyn)¹: dyslipidemia (i.e. high triglycerides and low HDL-cholesterol), hypertension, and type 2 diabetes.

Persons with the metabolic syndrome (MetSyn) are at increased risk of developing coronary heart diseases (CHD) as well as increased mortality from CHD and any other cause ^{2,3}.

The Third Report of the National Cholesterol Education Program Expert Panel on Detection, Evaluation, and Treatment of High Blood Cholesterol in adults (ATPIII), draws attention to the importance of MetSyn and provides a working definition of this syndrome ⁴.

Findings from the Third National Health and Nutrition Examination Survey showed that MetSyn is highly prevalent in the United States. Its prevalence has increased from 6.7% among participants aged 20 to 29 years, to 43.5% and 42.0% for participants aged 60 to 69 years and aged at least 70 years, respectively ⁵.

Over 75% of hypertension cases are reported to be directly attributable to obesity, and the risk of developing hypertension is five to six times greater in obese adult Americans age 20 to 45 compared to non-obese individuals of the same age. Obesity and insulin resistance, and the interaction between these two components, are associated with a high cardiovascular risk.^{6, 7} As many as 90% of individuals with type 2 diabetes are overweight or obese. ⁸ Obesity-related type 2 diabetes is a leading cause of morbidity and mortality in western societies, and is quickly approaching pandemic proportions ⁹. In addition to heart disease, obesity is reported to increase the risk of ischemic stroke independent of other risk factors, including age and systolic blood pressure. The incidence of osteoarthritis increases with BMI and is associated with arthritis of the hand, hip, back and, in particular, the knee. Increased weight adds stress to bones and joints due to increased load. Lastly, there is evidence that some cancers (endometrial, breast and colon) are associated with obesity.
Although obesity and insulin resistance, and the interaction between these two components, are associated with a high cardiovascular risk ^{6, 7,} the severity of insulinemia and glycaemia during the diabetic phase can only to a minor extent explain this increased cardiovascular risk.

Therefore, the pathogenic mechanisms which link obesity with type-2 diabetes and with cardiovascular risk remain to be elucidated. One possible link is inflammation. Indeed, obesity is associated with increased infiltration in the adipose tissue of monocytes/macrophages that also produce inflammatory chemokines.¹⁰
Increased oxidative stress causes increased monocyte infiltration and is an early instigator of MetSyn. Several findings support this hypothesis. We demonstrated that MetSyn is associated with elevated levels of circulating oxidized LDL (oxLDL), a marker of oxidative stress. High triglycerides, low HDL-cholesterol, and high glucose and insulin predicted elevated levels of oxLDL independent of LDL-cholesterol levels. The association between MetSyn and elevated levels of oxLDL has been confirmed in European and Japanese cohorts ¹¹⁻¹³. Persons with high oxLDL levels showed a greater disposition to myocardial infarction, adjusting for all established cardiovascular risk factors.¹⁴ Two other studies confirmed that elevated levels of circulating oxLDL predict future cardiovascular events even after adjustment for traditional cardiovascular risk factors and C-reactive protein ^{15, 16}. Recently, we have shown that persons with high oxLDL showed a 4.5-fold greater disposition to future MetSyn after 5 years follow-up, adjusted for age, gender, race, study centre, cigarette smoking, BMI, physical activity, and LDL-cholesterol, little changed by further adjustment for C-reactive protein, and adiponectin. In particular, oxLDL predicted the development of obesity, dyslipidemia and prediabetes.
We hypothesized that the identification of a cluster of genes and associated proteins which are associated with inflammation and oxidative stress and of which the expression pattern is improved by weight loss that significantly reduces cardiovascular risk could lead to a better estimate of the risk for cardiovascular disease for obese persons. We started from the observation in obese miniature pigs on an atherogenic diet that toll-like receptor 2 (TLR2) was over expressed in plaque macrophages isolated by laser capture micro dissection and correlated with atherosclerotic plaque complexity ¹⁷. Then, we performed micro array analysis of RNA extracted from monocytes of obese women. Because we found that TLR2 was over expressed, we searched for genes that correlated with TLR2. Structural modeling predicted a cluster of genes that besides TLR2 contains the following genes and associated proteins: IL1 receptor-associated kinase 3 (IRAK3), Tumor Necrosis Factor (TNF)-Associated Factor 6 (TRAF6), the myeloid differentiation marker MYD88, TNF-alpha-induced protein 3 and 6 (TNFAIP3; TNFAIP6), the Insulin Receptor Substrate 2 (IRS2), mitogen-activated protein kinase 13 (MAPK13), the Forkhead Box O3A (FOXO3A), and superoxide dismutase 2 (SOD2). These genes and associated proteins form the backbone of a pathway that links the toll-like receptor-mediated inflammation with the protection against oxidative stress by means of SOD2. Recently, we identified a protein correlating with that cluster that may be of importance for regulating the expression of that cluster: zinc finger 217 (ZNF217).

Present application first summarizes knowledge about the function of relevant genes and associated proteins. Thereafter examples which support our findings that some of these predicted molecules indeed are novel (bio)markers of cardiovascular risk in association with obesity, lipid homeostais disorder related cardiovascular disease and/or an impaired glucose tolerance condition and that some are even causal biomarkers.

### The genes in the new cluster of correlating molecules

### Toll-like receptors

The Toll like /Interleukin 1 receptor family consists of a large number of transmembrane proteins which are involved in host defense and have conserved intracellular domains. This superfamily is divided into 2 subgroups based on the components of the extracellular domains: the Toll like receptors (TLRs) with leucine-rich repeats, and the Interleukin-1 receptors (IL1Rs) with immunoglobulin-like motifs. Signal transduction pathways in these receptor families ultimately lead to activation of members of the Rel and APlfamily of transcription factors. An important mediator in this pathway is IL1 receptor-associated kinase (IRAK1). Using a murine EST sequence encoding a polypeptide with significant homology to IRAK1 to screen a human phytohemagglutinin-activated peripheral blood leukocyte (PBL) cDNA library, Wesche et al. ¹⁸ isolated a full-length cDNA clone encoding a 596-amino acid protein. Sequence analysis revealed an overall sequence similarity of 30 to 40% with IRAK1 and IRAK2 as well as structural similarity in an N-terminal death domain and a central kinase domain. Northern blot analysis revealed expression of 3 mRNA transcripts (an approximately 8-kb doublet and a 2.5-kb species that matched the isolated cDNA) predominantly in PBL and the monocytic cell lines U937 and THP-1, in contrast to the other IRAKs that are expressed in most cell types. Because of the restriction of expression of this IRAK to monocytic cells, the authors termed the protein IRAKM, now called IRAK3. The IRAK3 (or IRAKM or **I**nterleukin-1 **R**eceptor-**A**ssociated **K**inase **3** or **I**nterleukin-1 **R**eceptor-**A**ssociated **K**inase **M**) gene consists of 12 exons spanning a region of approximately 60 kb in chromosome 12q14.3 ¹⁹. Like IRAK2, the expression of IRAK3 in THP-1 cells is upregulated in the presence of phorbol ester and ionomycin, which also induce differentiation of these cells into more mature macrophages ¹⁸. IRAK-3 (IRAK-M) is a member of the interleukine-1 receptor-associated kinase (IRAK) family. The IRAK family is implicated in the Toll-like receptor (TLR) and Il-1R signaling pathway. IRAK3 interacts with the myeloid differentiation (MYD) marker MYD88 and TRAF6 signaling proteins in a manner similar to the other IRAKs. However, Kobayashi et al. ²⁰ showed that IRAK3, in contrast to other IRAKs, is induced upon TLR stimulation but negatively regulates TLR signaling. IRAK3 -/- cells exhibited increased cytokine production upon TLR/IL1 stimulation and bacterial challenge, and Irakm -/- mice showed increased inflammatory responses to bacterial infection. Endotoxin tolerance, a protection mechanism against endotoxin shock, was significantly reduced in IRAKM -/- cells. Thus, the authors concluded that IRAK3 regulates TLR signaling and innate immune homeostasis. IRAK-2 and IRAK-M. Data with IRAK-M knockout mice have revealed that IRAK-M serves as a negative regulator of IL-1R/TLR signaling. In contrast to other member of the interleukine-1 receptor-associated kinase (IRAK) family, IRAK-1 and IRAK-4 IRAK-M do not possess any detectable kinase activity. Moreover IRAK-M expression is mainly restricted to cells of a myeloid origin.

The Homo sapiens interleukin-1 receptor-associated kinase 3 (IRAK3) mRNA has been deposited in the NCBI database as under the accession number ACCESSION NM_007199, VERSION NM_007199.1 (LOCUS:NM_007199 2288 bp mRNA linear PRI 11-FEB-2008) with the nucleotide sequence as in sequence ID 1.

The Homo sapiens interleukin-1 receptor-associated kinase 3 (IRAK3) protein has been deposited in the NCBI database as under the accession number ACCESSION NP_009130 VERSION NP_009130.1 GI:6005792 (LOCUS NP_009130 596 aa linear PRI 11-FEB-2008) with the amino acid sequence as in sequence ID 2. The isoform hPA5312.2 [924 aa] Entry created: 2007-04-02 and as published by the Last update of 2007-12-12 has been depicted in Seq. ID 22 and the IRAK3 10 kDa protein - Length: 93 AS in sequence ID 23.

### Tumor Necrosis Factor-Associated Factor (TRAF)

The transcription factor NF-kappa-B (NFκB) is also activated by many cytokines which signal through different cell surface receptors. Members of the Tumor Necrosis Factor-Associated Factor (TRAF) protein family have been implicated in the activation of this transcription factor by the tumor necrosis factor (TNF) superfamily. TRAF2 is required for activation of this transcription factor by 2 TNF receptors, TNFR1 and TNFR2, as well as CD40, and TRAF5 may be responsible for NFκB activation signaled by the lymphotoxin B receptor. Cao et al. ²¹ identified a new member of the TRAF family, designated TRAF6. They showed that when over expressed in cultured human cells, TRAF6 activates NFκB. A dominant-negative mutant of TRAF6 inhibited this activation signaled by interleukin-1 (IL1A). IL1A treatment of the same cells induced the association of TRAF6 with IRAK. The findings were interpreted as indicating that TRAF proteins function as signal transducers for distinct receptor families in that TRAF6 participates in AL1A signaling. TRAF6 is a signal transducer in the NFκB pathway that activates I-kappa-B kinase (IKK) in response to pro-inflammatory cytokines. Cell-permeable peptides with the TRAF6-binding motif inhibited TRAF6 signaling, which indicated their potential as therapeutic modulators. It was concluded that their studies identified a universal mechanism by which TRAF6 regulates several signaling cascades in adaptive immunity, innate immunity, and bone homeostasis. TRAF6 is required for IL1 signaling. IL1B stimulation failed to induce NFκB or JNK/SAPK activation in cells from Traf6 -/- mice. Inducible nitrous oxide synthase (INOS) production in response to TNF plus IFNG, but not to IL1, was intact in Traf6-deficient mice. King et al. ²² generated healthy mice lacking Traf6 specifically in T lymphocytes. At 10 to 12 weeks of age, these mice developed splenomegaly and lymphadenopathy, with increased B-cell and Cd4-positive T-cell numbers, but fewer Cd8-positive T cells. Histopathologic analysis showed systemic inflammation in multiple organs of mutant mice. Cd4-positive/Cd25-positive regulatory T cells were present and appeared functional in mutant mice, but proliferation of Traf6 -/- T cells could not be suppressed by wild-type or mutant regulatory T cells, suggesting the presence of a responder T-cell mechanism necessary to render T cells susceptible to regulation. The resistance to suppression was accompanied by hyperactivation of PI3K-dependent pathways. It was concluded that TRAF6 is important in maintaining the balance between immune activation and suppression.

Variant (1) contains an additional segment in the 5' UTR compared to variant 2. Both variants encode the same protein. Source sequence(s) BC031052,BI463192 There are two transcript Variant: This variant (2) lacks a segment in the 5' UTR compared to variant 1. Both variants encode the same protein. Source sequence(s) BC031052,BI463192,U78798 Consensus CDS CCDS7901.1 UniProtKB/TrEMBL A8KAB3 UniProtKB/Swiss-Prot Q9Y4K3 The Homo sapiens TNF receptor-associated factor 6 (TRAF6) protein has been deposited in the NCBI database as under the accession number ACCESSION NP_004611, VERSION NP_004611.1 GI:4759254, DBSOURCE REFSEQ: accession NM_004620.2 with the amino acid sequence as in sequence ID 3.

mRNA of Homo sapiens TNF receptor-associated factor 6 (TRAF6), transcript variant 2 has been deposited in the NCBI database as under the accession number ACCESSION NM_004620 REGION: 248..1816 VERSION NM_004620.2 GI:22027628 (LOCUS NM_004620 1569 bp mRNA linear PRI 10-FEB-2008) with the nucleotide sequence as in sequence ID 4.

mRNA of Homo sapiens TNF receptor-associated factor 6 (TRAF6), transcript variant 1 has been deposited in the NCBI database as under the accession number ACCESSION BC031052 VERSION BC031052.1 GI:21410268 (LOCUS BC031052 2594 bp mRNA linear PRI 17-JUL-2006) with the nucleotide sequence as in sequence ID 5.

### Myeloid differentiation marker MYD88

The myeloid differentiation (MYD) marker MYD88 was first characterized during a study of the early genetic responses of murine myeloid cells to various differentiation and growth inhibitory stimuli ²³. Myeloid differentiation primary response genes are activated in M1 myeloleukemic cells in response to IL6, which induces both growth arrest and terminal differentiation. Northern blot analysis revealed widespread expression of the gene in many adult mouse tissues, and RT-PCR detected MYD88 mRNA in T- and B-cell lines and differentiating embryonic stem cells. The broad expression pattern demonstrated that mouse MYD88 expression is not restricted to cells of myeloid lineage as was originally believed. Medzhitov et al.²⁴ showed that signaling by the human TLR4 employs an adaptor protein, MYD88, and induces activation of NFκB via IRAK1 and TRAF6 protein. These findings implicate MYD88 as a general adaptor/regulator molecule for the Toll/IL1R family of receptors for innate immunity. Adachi et al. ²⁵ observed that mice with a targeted disruption of the MYD88 gene were unable to respond to IL1, as determined by defective T-cell proliferation and the production of cytokines. Likewise, MYD88-deficient mice were unable to produce IFNG and mediate natural killer cell activity in response to IL18. NFκB activation in response to IL1 or IL18 was also impaired. These results indicated that MYD88 is a critical component in the IL1R and IL18R signaling cascades. Kawai et al. ²⁶ extended these studies to show that responses to lipopolysaccharide, mediated by TLR4 and CD14, were lost or delayed in MYD88-deficient mice, establishing that MYD88 is part of the TLR signaling cascade as well, acting just upstream of IRAK. Bjorkbacka et al. ²⁷ examined atherosclerotic lesion development in uninfected Apoe single-null mice and Apoe -/- MYD88 - /- double-null mice, and found that the MYD88-deficient mice showed a marked reduction in early atherosclerosis. Inactivation of the MYD88 pathway led to a reduction in atherosclerosis through a decrease in macrophage recruitment to the artery wall that was associated with reduced chemokine levels. The findings linked elevated serum lipid levels to a pro-inflammatory signaling cascade that is also engaged by microbial pathogens.

mRNA of Homo sapiens *Myeloid differentiation primary response gene (88) MYD88* has been deposited in the NCBI database as under the accession number ACCESSION BC013589 VERSION BC013589.1 GI:15488922 (LOCUS BC013589, 2678 bp mRNA linear PRI 19-JUN-2006) with the mRNA nucleotide sequence as in sequence ID 6.

The MYD88 translation product of the MYD88 gene CDS 40.930 of clone "MGC: 9601 IMAGE: 3900951" is depicted in sequence ID 7.

### TNF-alpha-induced protein 3 (TNFAIP3)

TNF-alpha-induced protein 3 (TNFAIP3; or A20) is a cytoplasmic zinc finger protein that inhibits NFκB activity and TNF-mediated programmed cell death. TNF dramatically increases TNFAIP3 mRNA expression in all tissues^{28, 29}. Cytokines such as TNF profoundly affect endothelial cell function, promoting, for example, interaction with leukocytes and inducing a procoagulant phenotype. Changes of this nature are likely to be central to the pro-inflammatory effects of TNF. Dixit et al. ²⁸ analyzed TNF-induced primary response genes in human umbilical vein endothelial cells. Of the 6 induced cDNAs identified, 2 encoded paracrine factors, neutrophil chemotactic factor and monocyte chemotactic factor; 1 encoded a membrane receptor for neutrophils, endothelial leukocyte adhesion molecule 1 (ELAM1); and 3 encoded hitherto undescribed TNF primary response genes. On exposure of endothelial cells to TNF, there was a rapid and substantial increase in the levels of mRNA encoding the 6 genes, which were further superinduced by cycloheximide. Thus these represent primary response genes, as their induction does not depend on protein synthesis. One of the 3 new proteins, designated A20 (new name TNFAIP3), was found on sequence analysis to code for a novel zinc finger protein ³⁰. ³¹ expressed TNFAIP3 and TLR4 in a human embryonic kidney cell line and observed inhibition of NFκB activation after LPS stimulation. Mutation analysis showed that the C-terminal zinc finger domain of A20 was sufficient for NFκB inhibition, whereas the full-length protein was required for inhibition of AP1 activation and for induction of IL8. They concluded that TNFAIP3 (A20) modulates TLR4 signaling at or downstream of MEKK1 (MAP3K1). Wertz et al. ³² demonstrated that A20 down regulates NFκB signaling through the cooperative activity of its 2 ubiquitin-editing domains. The N-terminal domain of TNFAIP3, which is a deubiquitinating enzyme of the OTU (ovarian tumor) family, removes lysine-63-linked ubiquitin chains from receptor-interacting protein (RIP), an essential mediator of the proximal TNF receptor-1 (TNFR1) signaling complex. The C-terminal domain of TNFAIP3 composed of 7 C2/C2 zinc fingers, then functions as an ubiquitin ligase by polyubiquitinating RIP with lysine-48-linked ubiquitin chains, thereby targeting RIP for proteasomal degradation. They defined a novel ubiquitin ligase domain and identified 2 sequential mechanisms by which A20 downregulates NFκB signaling. They also provided an example of a protein containing separate ubiquitin ligase and deubiquitinating domains, both of which participate in mediating a distinct regulatory effect. Lee et al. ²⁹ generated A20-deficient mice by targeted disruption. A20 +/- mice appeared normal without evidence of pathology. A20 -/- mice, born from interbred A20 +/- mice in Mendelian ratios, developed runting as early as 1 week of age. Mice deficient for A20 developed severe inflammation and cachexia, were hypersensitive to both lipopolysaccharide and TNF, and died prematurely. A20-deficient cells failed to terminate TNF-induced NFκB responses. These cells were also more susceptible than control cells to undergo TNF-mediated program cell death. Thus, A20 is critical for limiting inflammation by terminating TNF-induced NFκB responses in vivo. Using mice doubly deficient in either A20 and Tnf or A20 and Tnfr1, Boone et al. ³³ showed that, in addition to terminating TNF-induced signals, A20 is required for terminating TLR (e.g., TLR4)-induced activity of NFκB. Mutation and immunoblot analyses indicated that A20 acts, via its conserved OTU-like domain, as a deubiquitinating enzyme on ubiquitinated TRAF6. In mice subjected to aortic banding, Cook et al. ³⁴ detected greater than 4-fold A20 upregulation (p less than 0.05) at 3 hours, coinciding with peak NFκB activation. Cardiomyocytes infected with an adenoviral vector (Ad) encoding A20 inhibited TNF-stimulated NFκB signaling with an efficacy comparable to dominant-negative inhibitor of kappa-B kinase-beta (IKBKB). Ad-IKBKB-infected cardiomyocytes exhibited increased apoptosis when serum-starved or subjected to hypoxia-reoxygenation, whereas Ad-A20-infected cardiomyocytes did not. Expression of Ad-A20 inhibited the hypertrophic response in cardiomyocytes stimulated with phenylephrine or endothelin-1. They concluded that A20 is dynamically regulated during acute biomechanical stress in the heart and functions to attenuate cardiac hypertrophy through the inhibition of NFκB signaling without sensitizing cardiomyocytes to apoptosis.

mRNA of Homo sapiens TNF-alpha-induced protein 3 (TNFAIP3; or A20) has been deposited in the NCBI database as under the accession number ACCESSION M59465 J05610 VERSION M59465.1 GI: 177865 (LOCUS HUMA20 4426 bp mRNA linear PRI 30-OCT-1994) with the mRNA nucleotide sequence as in sequence ID 8.

The human tumor necrosis factor alpha inducible protein A20 translation product of the CDS 67.2439 of the TNFAIP1 gene is depicted in sequence ID 9.

### TNF-alpha-induced protein 6 (TNFAIP6)

Lee et al. ³⁵ described a gene, which they designated TSG6 (current name TNFAIP6), that is transcribed in normal fibroblasts and activated by binding of TNF-alpha and IL1 at AP-1 and NF-IL6 sites in its promoter. The cDNA was isolated from a library made from TNF-treated human fibroblasts. TNFAIP6 is a member of the hyaluronan-binding protein family, which includes cartilage link protein, proteoglycan core protein, and the adhesion receptor CD44. The predicted polypeptide is 277 amino acids long and includes a typical cleavage signal peptide. TNFAIP6 is highly homologous to CD44, particularly in the hyaluronic acid-binding domain. Western blots with antibodies made to a TNFAIP6 fusion protein detected a 39-kD glycoprotein in TNF-treated cells, and hyaluronate binding was shown by co-precipitation. TNFAIP6 expression is rapidly activated by TNF-alpha, IL1, and lipopolysaccharide in normal fibroblasts, peripheral blood mononuclear cells, synovial cells, and chondrocytes.

mRNA of Homo sapiens Homo sapiens tumor necrosis factor, alpha-induced protein 6 (TNFAIP6) has been deposited in the NCBI database as under the accession number ACCESSION NM_007115 VERSION NM_007115.2 GI: 26051242 (LOCUS NM_007115 1440 bp mRNA linear PRI 11-FEB-2008) with the mRNA nucleotide sequence as in sequence ID 10.

The translated product from the CDS 77.910 of the TNFAIP6 gene is depicted in Sequence ID 11.

### Insulin Receptor Substrates

The Insulin Receptor Substrate 1 (IRS1) acts as an interface between signaling proteins with Src homology-2 domains (SH2 proteins) and the receptors for insulin, IGF2, growth hormone, several interleukins, and other cytokines. It regulates gene expression and stimulates mitogenesis and appears to mediate insulin/IGF1-stimulated glucose transport. Thus, the finding that the homozygous Irs1 KO mouse survives with only mild resistance to hypertension was surprising. This dilemma was provisionally resolved by the discovery by Sun et al. ³⁶ of a second IRS signaling protein in mouse. They purified and cloned a likely candidate from mouse myeloid progenitor cells and, because of its resemblance to IRS1, they designated it IRS2. Withers et al. ³⁷ demonstrated that homozygous absence of the Irs2 gene results in type II diabetes in mice. Heterozygous and wild type animals were unaffected. The authors demonstrated profound insulin resistance in both skeletal muscle and liver in the homozygous Irs2 -/mice. Male mice lacking the Irs2 locus showed polydypsia and polyuria without ketosis and died from dehydration and hyperosmolar coma. A similar disease progression was observed in female mice, with the exception that the females rarely died. The authors concluded that dysfunction of IRS2 may contribute to the pathogenesis of human type II diabetes. Tobe et al. ³⁸ observed that Irs2-deficient mice showed increased adiposity with increased serum leptin level, suggesting leptin resistance before the mice developed diabetes. Using oligonucleotide micro array and Northern blot analyses to analyze gene expression they detected increased expression of SREBP1, a downstream target of insulin, in Irs2-deficient mouse liver. Using high dose leptin administration, they provided evidence that leptin resistance in Irs2-deficient mice is causally related to SREBP1 gene induction. The authors concluded that Irs2 gene disruption results in leptin resistance, causing SREBP1 gene induction, obesity, fatty liver, and diabetes. Taguchi et al. ³⁹ showed that, in mice, less Irs2 signaling throughout the body or only in brain extended life span up to 18%. At 22 months of age, brain-specific Irs2 knockout mice were overweight, hyperinsulinemic, and glucose intolerant; however, compared with control mice, they were more active and displayed greater glucose oxidation, and during meals they displayed stable SOD2 concentrations in the hypothalamus. Thus, they concluded that less Irs2 signaling in aging brains can promote healthy metabolism, attenuate meal-induced oxidative stress, and extend the life span of overweight and insulin-resistant mice.

mRNA of Homo sapiens insulin receptor substrate 1 (IRS1) has been deposited in the NCBI database as under the accession number ACCESSION NM_005544 VERSION NM_005544.1 GI: 5031804 (LOCUS NM_005544 5828 bp mRNA linear PRI 16-MAR-2008) with the mRNA nucleotide sequence as in sequence ID 12.

The translation product of the CDS 1021.4749 IRS1gene is depicted in sequence ID 13.

### Mitogen-activated protein kinase 13

Mitogen-activated protein kinase (MAPK) cascades represent one of the major signal systems used by eukaryotic cells to transduce extracellular signals into cellular responses. Goedert et al. ⁴⁰ isolated cDNAs encoding a protein that they designated SAPK4 (current name MAPK13). The sequence of the predicted 365-amino acid protein is approximately 60% identical to those of SAPK3 (or p38-gamma), SAPK2a (or p38), and SAPK2b. Like those SAPKs, SAPK4 has a TGY dual phosphorylation motif and is activated in response to cellular stresses and pro-inflammatory cytokines. Wang et al. ⁴¹ also isolated SAPK4 cDNAs. Using Northern blot analysis, they compared the expression patterns of SAPK4 (designated p38-delta by them), SAPK2a, SAPK2b, and SAPK3 in 50 human tissues. They detected 2 SAPK4 transcripts: a predominant 1.8-kb mRNA and a lower abundance 6-kb mRNA. The highest levels of expression were observed in exocrine/endocrine tissues including salivary gland, pituitary gland, adrenal gland and placenta. Recently, it has been shown that MAP kinase activation is contributing to atherosclerosis ⁴², 43

mRNA of Homo sapiens mitogen-activated protein kinase 13 (MAPK13), has been deposited in the NCBI database as under the accession number ACCESSION NM_002754 VERSION NM_002754.3 GI: 20986527 (LOCUS NM_002754 1888 bp mRNA linear PRI 21-MAR-2008) with the mRNA nucleotide sequence as in sequence ID 14.

The translation product of the CDS 99.1196 of the MAPK13 gene is depicted in the sequence ID 15.

### FOXO3A

Survival factors can suppress apoptosis in a transcription-independent manner by activating the serine/threonine kinase AKT1, which then phosphorylates and inactivates components of the apoptotic machinery, including BAD and caspase-9. Brunet et al. ⁴⁴ demonstrated that AKT1 also regulates the activity of FKHRL1 (current name FOXO3A). In the presence of survival factors, AKT1 phosphorylates FKHRL1, leading to the association of FKHRL1 with 14-3-3 proteins and its retention in the cytoplasm. Survival factor withdrawal leads to FKHRL1 dephosphorylation, nuclear translocation, and target gene activation. Within the nucleus, FKHRL1 most likely triggers apoptosis by inducing the expression of genes that are critical for cell death, such the TNF ligand superfamily 6 (TNFSF6). Nemoto and Finkel ⁴⁵ observed that exposure to intracellular ROS induced an increase in phosphorylated Fkhrl1 and a shift from a nuclear to a cytosolic localization. They found that serum starvation, a stimulus that increases oxidative stress, resulted in lower levels of hydrogen peroxide in Shc1 -/- cells or in cells expressing a ser36-to-ala (S36A) Shc1 mutant compared with wild type cells. Serum starvation also increased Fkhrl1-dependent transcriptional activity, which was further augmented in the Shc1-deficient cells. Increased ROS exposure failed to induce increased Fkhrl1 phosphorylation in the mutant cells. Essers et al. ⁴⁶ reported an evolutionarily conserved interaction of beta-catenin with FOXO transcription factors, which are regulated by insulin and oxidative stress signaling. In mammalian cells, beta-catenin binds directly to FOXO and enhances FOXO transcriptional activity. In C. elegans, loss of the beta-catenin BAR1 reduces the activity of the FOXO ortholog DAF16 in dauer formation and life span. Association of beta-catenin with FOXO was enhanced in cells exposed to oxidative stress. Furthermore, BAR1 was required for the oxidative stress-induced expression of the DAF16 target gene sod3 and for resistance to oxidative damage. They concluded that their results demonstrated a role for beta-catenin in regulating FOXO function that is particularly important under conditions of oxidative stress.

mRNA of Homo sapiens forkhead box 03 (FOXO3), transcript variant 1, has been deposited in the NCBI database as under the accession number ACCESSION NM_001455 VERSION NM_001455.3 GI: 146260266 with the mRNA nucleotide sequence as in sequence ID 16.

The translation product of the CDS 344.2365 of the human forkhead box 03 (FOXO3), transcript variant 1 is depicted in sequence ID 17.

### Superoxide dismutase 2

The superoxide dismutase 2 (SOD2; or manganese or mitochondrial superoxide dismutase) gene encodes an intramitochondrial free radical scavenging enzyme that is the first line of defense against superoxide produced as a by-product of oxidative phosphorylation. Li et al. ⁴⁷ inactivated the Sod2 gene in transgenic mice by homologous recombination. Homozygous mutant mice died within the first 10 days of life with a dilated cardiomyopathy, accumulation of lipid in liver and skeletal muscle, and metabolic acidosis. The findings suggested SOD2 that is required for normal biologic function of tissues by maintaining the integrity of mitochondrial enzymes susceptible to direct inactivation by superoxide. Oxidative stress has been implicated in many diseases. The chief source of reactive oxygen species (ROS) within the cell is the mitochondrion. ROS have been implicated in a wide range of degenerative processes including amyotrophic lateral sclerosis, ischemic heart disease, Alzheimer disease, Parkinson disease, and aging. ROS are generated by mitochondria as the toxic by-products of oxidative phosphorylation, their energy generating pathway. Melov et al. ⁴⁸ characterized a variety of biochemical and metabolic effects of inactivation of the mouse sod2 gen. The Sod2 mutant mice exhibited a tissue-specific inhibition of the respiratory chain enzymes NADH-dehydrogenase (complex I) and succinate dehydrogenase (complex II), inactivation of the tricarboxylic acid cycle enzyme aconitase, development of a urinary organic aciduria in conjunction with a partial defect in 3-hydroxy-3-methylglutaryl-CoA lyase, and accumulation of oxidative DNA damage. Treatment with an SOD mimetic, MnTBAP, rescued Sod2 -/- mutant mice from this systemic pathology and dramatically prolonged their survival. Surviving animals developed a pronounced movement disorder progressing to total debilitation by 3 weeks of age. Neuropathologic evaluation showed a striking spongiform degeneration of the cortex and specific brainstem nuclei, associated with gliosis and intramyelinic vacuolization similar to that observed in cytotoxic edema and disorders associated with mitochondrial abnormalities such as Leigh disease and Canavan disease. Their data suggested that because of the failure of MnTBAP to cross the blood-brain barrier progressive neuropathology is caused by excessive mitochondrial production of ROS ⁴⁸.

mRNA of Homo sapiens superoxide dismutase 2, mitochondrial (SOD2), nuclear gene encoding mitochondrial protein, transcript variant 1, has been deposited in the NCBI database as under the accession number ACCESSION NM_000636 VERSION NM_000636.2 GI:67782304 (LOCUS NM_000636 1593 bp mRNA linear PRI 30-MAR-2008) with the mRNA nucleotide sequence as in sequence ID 18.

The translation product of the CDS 155.823 Homo sapiens superoxide dismutase 2, mitochondrial (SOD2), nuclear gene encoding mitochondrial protein, transcript variant 1 gene is depicted in sequence ID 19.

The biomarkers, i.e. the genes identified hereinbefore, according to the invention include substantially identical homologues and variants of the nucleic acid molecules and expression products thereof described herein, for example, a molecule that includes nucleotide sequences encoding polypeptides functionally equivalent to the biomarkers of the invention, e.g., sequences having one or more nucleotide substitutions, additions, or deletions, such as allelic variants or splice variants or species variants or molecules differing from the nucleic acid molecules and polypeptides referred to in the Tables herein due to the degeneracy of the genetic code. Species variants are nucleic acid sequences that vary from one species to another, although the resulting polypeptides generally will have significant amino acid identity and functional similarity relative to each other. A polymorphic variant (e.g., a single nucleotide polymorphism or SNP) is a variation in the nucleic acid sequence of a particular gene between individuals of a given species.

A "substantially identical" sequence is an amino acid or nucleotide sequence that differs from a reference sequence only by one or more conservative substitutions, as discussed herein, or by one or more non-conservative substitutions, deletions, or insertions located at positions of the sequence that do not destroy the biological function of the amino acid or nucleic acid molecule. Such a sequence can be any integer from 10% to 99%, or more generally at least 10%, 20%, 30%, 40%, 50, 55% or 60%, or at least 65%, 75%, 80%, 85%, 90%, or 95%, or as much as 96%, 97%, 98%, or 99% identical when optimally aligned at the amino acid or nucleotide level to the sequence used for comparison using, for example, the Align Program (Myers and Miller, CABIOS, 1989, 4:11-17) or FASTA. For polypeptides, the length of comparison sequences may be at least 2, 5, 10, or 15 amino acids, or at least 20, 25, or 30 amino acids. In alternate embodiments, the length of comparison sequences may be at least 35, 40, or 50 amino acids, or over 60, 80, or 100 amino acids. For nucleic acid molecules, the length of comparison sequences may be at least 5, 10, 15, 20, or 25 nucleotides, or at least 30, 40, or 50 nucleotides. In alternate embodiments, the length of comparison sequences may be at least 60, 70, 80, or 90 nucleotides, or over 100, 200, or 500 nucleotides. Sequence identity can be readily measured using publicly available sequence analysis software (e.g., Sequence Analysis Software Package of the Genetics Computer Group, University of Wisconsin Biotechnology Center, 1710 University Avenue, Madison, Wis. 53705, or BLAST software available from the National Library of Medicine, or as described herein). Examples of useful software include the programs Pile-up and Pretty Box. Such software matches similar sequences by assigning degrees of homology to various substitutions, deletions, substitutions, and other modifications. Alternatively, or additionally, two nucleic acid sequences may be "substantially identical" if they hybridize under high stringency conditions. In some embodiments, high stringency conditions are, for example, conditions that allow hybridization comparable with the hybridization that occurs using a DNA probe of at least 500 nucleotides in length, in a buffer containing 0.5 M NaHPO4, pH 7.2, 7% SDS, 1 mM EDTA, and 1% BSA (fraction V), at a temperature of 65[deg.]C, or a buffer containing 48% formamide, 4.8x SSC, 0.2 M Tris-Cl, pH 7.6, Ix Denhardt's solution, 10% dextran sulfate, and 0.1% SDS, at a temperature of 42[deg.]C. (These are typical conditions for high stringency northern or Southern hybridizations.) Hybridizations may be carried out over a period of about 20 to 30 minutes, or about 2 to 6 hours, or about 10 to 15 hours, or over 24 hours or more. High stringency hybridization is also relied upon for the success of numerous techniques routinely performed by molecular biologists, such as high stringency PCR, DNA sequencing, single strand conformational polymorphism analysis, and in situ hybridization. In contrast to northern and Southern hybridizations, these techniques are usually performed with relatively short probes (e.g., usually about 16 nucleotides or longer for PCR or sequencing and about 40 nucleotides or longer for in situ hybridization). The high stringency conditions used in these techniques are well known to those skilled in the art of molecular biology, and examples of them can be found, for example, in Ausubel et al., Current Protocols in Molecular Biology, John Wiley & Sons, New York, N. Y., 1998.

### Zinc finger 217 (ZNF217)

Studies in which comparative genomic hybridization was used revealed approximately 20 regions of recurrent increased DNA sequence copy number in breast tumors ^{49, 50}. These regions are predicted to encode dominantly acting genes that may play a role in tumor progression or response to therapy. Three of these regions had been associated with established oncogenes: ERBB2 at 17q12, MYC at 8q24, and CCND1 and EMS1 at 11q13. In breast cancer, ERBB2 and CCND1/EMS1 amplification and overexpression are associated with decreased life expectancy, whereas MYC amplification has been associated with lymph node involvement, advanced stage, and an increased rate of relapse. Amplification at 20q13 occurs in a variety of tumor types and is associated with aggressive tumor behavior. Kallioniemi et al. ⁵⁰ found increased copy number involving 20q13 in 40% of breast cancer cell lines and 18% of primary breast tumors. Other comparative genomic hybridization studies revealed copy number gains at 20q13 in greater than 25% of cancers of the ovary, colon, head and neck, brain, and pancreas. Collins et al. ⁵¹ reported the molecular cloning of an approximately 1-Mb region at 20q13.2 involved in recurrent amplification in breast cancer and other tumors, and the delineation of a 260-kb common region of amplification. Analysis of the 1-Mb region produced evidence of 5 genes, of which ZNF217 and NABC1 (novel amplified in breast cancer-1) emerged as strong candidate oncogenes and were characterized in detail. ZNF217 was found to be centrally located in the 260-kb common region of amplification, transcribed in multiple normal tissues, and overexpressed in all cell lines and tumors in which it was amplified and in 2 in which it was not. ZNF217 was predicted to encode alternatively spliced, Kruppel-like transcription factors of 1,062 and 1,108 amino acids, each having a DNA-binding domain (8 C2H2 zinc fingers) and a proline-rich transcription activation domain. One of the previously known genes identified in the region of amplification was the CYP24 gene.

Over-expression of the zinc-finger protein 217 (ZNF217), a candidate oncogene on 20q13.2, in cultured human mammary and ovarian epithelial cells can lead to their immortalization, indicating that selection for ZNF217 expression may drive 20q13 amplification during critical early stages of cancer progression. ZNF217 can also attenuate apoptotic signals resulting from exposure to doxorubicin, suggesting that ZNF217 expression may also be involved in resistance to chemotherapy. Recent findings indicate that ZNF217 binds specific DNA sequences, recruits the co-repressor C-terminal binding protein (CtBP), and represses the transcription of a variety of genes. Inappropriate expression of ZNF217 may lead to aberrant down-regulation of genes involved in limiting the proliferation, survival, and/or invasiveness of cancer cells. Better understanding of ZNF217 and its associated pathways may provide new targets for therapeutic intervention in human cancers ⁵².

Huang et al ⁵³ presented evidence that ZNF217 can attenuate apoptotic signals resulting from telomere dysfunction as well as from doxorubicin-induced DNA damage and that silencing ZNF217 with siRNA restores sensitivity to doxorubicin. Moreover, elevated ZNF217 leads to increased phosphorylation of Akt, whereas inhibition of the phosphatidylinositol 3 kinase pathway and Akt phosphorylation decreases ZNF217 protein levels and increases sensitivity to doxorubicin. These results suggest that ZNF217 may promote neoplastic transformation by increasing cell survival during telomeric crisis and may promote later stages of malignancy by increasing cell survival during chemotherapy.
Transforming growth factor-beta (TGF-beta) is a tumor suppressor, the function of which is compromised in many types of human cancer, including breast cancer. The tumor suppressive effects of TGF-beta are caused by potent inhibition of cell proliferation due to cell cycle arrest in the G1 phase. Such antiproliferative responses are mediated by a signaling system that includes two types of cell surface receptors and intracellular signal transducers, the SMAD proteins. Different molecular mechanisms can lead to loss of antiproliferative TGF-beta responses in tumor cells, including mutations in components of the signaling system and inhibition of the SMAD signaling pathway by aberrant activities of various regulatory molecules ⁵⁴. Thillainadesan et al ⁵⁵ demonstrated that stimulation of HaCaT cells with transforming growth factor beta (TGF-beta) resulted in a release of ZNF217 and a concomitant binding of SMAD2 to the proximal promoter, which preceded increases in ink4b protein expression. Furthermore, the changes in chromatin marks at the p15 (ink4b) promoter following TGF-beta stimulation were similar to those observed following ZNF217 down-regulation. Collectively, these results establish the ZNF217 complex as a novel negative regulator of the p15 (ink4b) gene and may constitute an important link between amplification of ZNF217 and the loss of TGF-beta responsiveness in breast cancer. The regulation of TGF-beta in human monocytes is also important in regulating the balance between macrophage deactivating and macrophage activating effects ⁵⁶.

mRNA and protein of Homo sapiens Zinc finger 217 (ZNF217), has been deposited in the NCBI database as under the accession number ACCESSION NM_006526.2 (deposited 21-DEC-2008) and NP_006517.1 (deposited 21-DEC-2008) respectively with the mRNA nucleotide sequence as in sequence ID 24 (Zinc finger 217 (ZNF217) 1..5653, organism="Homo sapiens" mRNA") and the protein as in sequence ID 25 (zinc finger protein 217 organism="Homo sapiens" protein" 1..1048)

### Methods to determine the gene expression / activity

### Preparation of Reagents Using Biomarkers

The biomarkers described herein may be used to prepare oligonucleotide probes and antibodies that hybridize to or specifically bind the biomarkers mentioned herein, and homologues and variants thereof.

### Antibodies or other binding agents

The in vitro method of analyzing the level of IRAK3 expression or activity of expression product in a biological sample isolated from said subject, an of analyzing the level of IRAK3 expression or activity of an IRAK3 expression product in combination with the gene expression level or activity of a gene product of at least one gene selected from the group consisting of SOD2, TNFAIP6, TNFAIP3, TLR2, and IRS2 may for present invention also be carried of a binding assay. Methods, techniques and equipment for performing such binding assays will also be clear to the skilled person. For the binding assay, the binding agent may also be immobilized on a suitable support, as will be again clear to the skilled person.

An "antibody" includes molecules having antigen binding regions, such as whole antibodies of any isotype (IgG, IgA, IgM, IgE, etc.) and fragments thereof. Antibody fragments include Fab', Fab, F(ab')2, single domain antibodies, Fv, scFv, etc. Antibodies may be prepared using standard techniques of preparation as, for example, described in Harlow and Lane (Harlow and Lane Antibodies; A Laboratory Manual, Cold Spring Harbor Laboratory, Cold Spring Harbor, N.Y., 1988), or known to those skilled in the art. For example, a coding sequence for a polypeptide biomarker of the invention may be purified to the degree necessary for immunization of rabbits. To attempt to minimize the potential problems of low affinity or specificity of antisera, two or three polypeptide constructs may be generated for each protein, and each construct may be injected into at least two rabbits. Antisera may be raised by injections in a series, preferably including at least three booster injections. Primary immunizations may be carried out with Freund's complete adjuvant and subsequent immunizations with Freund's incomplete adjuvant. Antibody titers may be monitored by Western blot and immunoprecipitation analyses using the purified protein. Immune sera may be affinity purified using CNBr-Sepharose-coupled protein. Antiserum specificity may be determined using a panel of unrelated proteins. Antibody fragments may be prepared recombinantly or by proteolytic cleavage. Peptides corresponding to relatively unique immunogenic regions of a polypeptide biomarker of the invention may be generated and coupled to keyhole limpet hemocyanin (KLH) through an introduced C-terminal lysine. Antiserum to each of these peptides may be affinity purified on peptides conjugated to BSA, and specificity tested in ELISA and Western blots using peptide conjugates and by Western blot and immunoprecipitation.

Monoclonal antibodies which specifically bind any one of the polypeptide biomarkers of the invention are prepared according to standard hybridoma technology (see, e.g., Kohler et al., Nature 256:495, 1975; Kohler et al., Eur. J. Immunol. 6:511, 1976; Kohler et al., Eur. J. Immunol. 6:292, 1976; Hammerling et al., In Monoclonal Antibodies and T Cell Hybridomas, Elsevier, N. Y., 1981). Alternatively monoclonal antibodies may be prepared using the polypeptides of the invention and a phage display library (Vaughan et al., Nature Biotech 14:309-314, 1996). Once produced, monoclonal antibodies may also be tested for specific recognition by Western blot or immunoprecipitation.

In some embodiments, antibodies may be produced using polypeptide fragments that appear likely to be immunogenic, by criteria such as high frequency of charged residues. Antibodies can be tailored to minimize adverse host immune response by using chimerical antibodies contain an antigen binding domain from one species and the Fc portion from another species, or by using antibodies made from hybridomas of the appropriate species.

An antibody "specifically binds" an antigen when it recognizes and binds the antigen, for example, a biomarker as described herein, but does not substantially recognize and bind other molecules in a sample. Such an antibody has, for example, an affinity for the antigen which is at least 2, 5, 10, 100, 1000 or 10000 times greater than the affinity of the antibody for another reference molecule in a sample. Specific binding to an antibody under such conditions may require an antibody that is selected for its specificity for a particular biomarker. For example, a polyclonal antibody directed against a biomarker from a specific species such as rat, mouse, or human may be selected for only those polyclonal antibodies that are specifically immunoreactive with the biomarker and not with other proteins, except for polymorphic variants and alleles of the biomarker. In some embodiments, a polyclonal antibody raised to a biomarker from a specific species such as rat, mouse, or human may be selected for only those polyclonal antibodies that are specifically immunoreactive with the biomarker from that species and not with other proteins, including polymorphic variants and alleles of the biomarker.

In principle any suitable binding agent that can specifically binds the IRAK3 expression product and for the combined assay any suitable binding agent that can specifically binds SOD2, TNFAIP6, TNFAIP3, TLR2, and/or IRS2 can be used in the binding assay of present invention. For example, the binding agent can be a protein or polypeptide that is capable of specifically binding, such as an antibody that is capable of specifically binding; a part of fragment of an antibody, in which said part or fragment is capable of specifically binding; or a protein or polypeptide that contains and/or comprises one or more parts of fragments of an antibody, in which at least one of said parts or fragments is capable of specifically binding. In particular, said part or fragment may be a variable domain, such as a heavy chain variable domain and/or a light chain variable domain, or a ScFv comprising both a heavy chain variable domain and/or a light chain variable domain. Such antibodies and fragments, and methods for obtaining the same, will be clear to the skilled person; reference is for example made to Roitt et al., "Immunology" (6th. Ed.), Mosby/Elsevier, Edinburgh (2001); and Janeway et al., "Immunobiology" (6th Ed.), Garland Science Publishing/Churchill Livingstone, New York (2005).
According to one preferred, but non-limiting embodiment, the binding agent can be a so-called "heavy chain antibody" that is capable of specifically binding the expression products IRAK3, SOD2, TNFAIP6, TNFAIP3, TLR2, and/or IRS2; a part of fragment of a heavy chain antibody, in which said part or fragment is capable of specifically binding to these expression products; or a protein or polypeptide that contains and/or comprises one or more parts of fragments of a heavy chain antibody, in which at least one of said parts or fragments is capable of specifically binding these expression products(for example, a multivalent protein containing two or more such fragments. Heavy chain antibodies and methods-for obtaining the same have been described in the art, see for example the following references, that are cited as general background art: WO 94/04678 (=EP 656 946), WO 96/34103 (=EP 0 822 985) and WO 97/49805 by Vrije Universiteit Brussel; WO 97/49805 by Vlaams Interuniversitair Instituut voor Biotechnologie; WO 94/25591 (=EP 0 698 097) and WO 00/43507 by Unilever N.V.; WO 01/90190 by the National Research Council of Canada; WO 03/025020 (=EP 1 433 793) by the Institute of Antibodies; WO 04/062551, WO 04/041863, WO 04/041865, WO 04/041862 by applicant; as well as for example Hamers-Casterman et al., Nature, Vol. 363, p. 446 (1993) and Riechmann and Muyldermans, Journal of Immunological Methods, 231 (1999), p. 25-38. For example, heavy chain antibodies against a desired antigen can be obtained from a species of Camelid or of Chondrostei immunized with said antigen, as described in the general prior art mentioned above. As also mentioned in the above references, naturally occurring heavy chain antibodies do not contain the light chains present in naturally occurring conventional 4-chain antibodies (that natively contain both heavy chains and light chains). Because of this, such naturally occurring heavy chain antibodies have also been referred to in the art as "single chain antibodies" (see for example WO 02/085945; and not to be confused with so-called "single chain Fv's" or "scFv's", which are synthetic polypeptides comprising a V_{H} domain covalently linked to a V_{L} domain) and as "immunoglobulins devoid of light chains" (see for example EP 0 656 946 and some of the further general background art mentioned above), which terms for the purposes of the present description should be considered equivalent to the term "heavy chain antibody" as used herein. As also mentioned in these references, the heavy chains of naturally occurring heavy chain antibodies contain CH3 domains, CH2 domains and a variable domain, but-in addition to the light chains-lack the CH1 domains present in the heavy chains of naturally occurring conventional 4-chain antibodies. Herein, the variable domains from naturally occurring heavy chain antibodies will also be referred to as "V_{HH} domains", in order to distinguish said variable domains from the variable domains from conventional 4-chain antibodies, which are commonly referred to as "V_{H} domains". Generally, V_{HH} domains have a structure that retains the immunoglobulin fold of conventional V_{H} domains. However, compared to V_{H} domains, V_{HH} domains contain one or more substitutions in their amino acid sequence (and in particular in their framework regions) that make the region(s)/residues of the V_{HH} domain that in a V_{H} domain would form the V_{H}/V_{L} interphase more hydrophobic (see the general background art cited above). Also, as mentioned in the general background art cited above, heavy chain antibodies and V_{HH} domains have the major advantage that they are capable of binding an antigen without the presence of any light chains or light chain variable domains, respectively. This makes heavy chain antibodies and V_{HH} domains easier to obtain, to develop, to prepare (in particular) on a large scale, to use and/or to bind to a support than conventional 4-chain antibodies or light chain or heavy chain variable domains thereof. For example, the immobilization of V_{HH} domains on a solid support has been described in the International application WO 01/40310 by Hindustan Lever Limited.
According to a particularly preferred embodiment, the binding agent is a variable domain sequences of heavy chain antibodies, for which term reference is made to the prior art mentioned above, to US20080096223, US20070077249, US20060062784, US20050271663, WO2007063311, WO2007063308, WO2006122786 and WO2005033130 Generally, such ligands can be described as proteins that have some of the functional properties and structural features that are characteristic of naturally occurring V_{HH} domains. They may for example be a naturally occurring V_{HH} domain, a "humanized" or human V_{HH} domains or a "camelized" V_{H} domains, as well as a partially or fully synthetic protein, as long as the foregoing have (at least some of) the functional properties and structural features that are characteristic of naturally occurring V_{HH} domains.

Alternatively in the binding assays and diagnostic method of present invention aptamer sequences which specifically bind to RNA or DNA sequences or expression products of SOD2, TNFAIP6, TNFAIP3, TLR2, and/or IRS2 are used. As used herein, the terms "aptamer(s)" or "aptamer sequence(s)" are meant to refer to single stranded nucleic acids (RNA or DNA) whose distinct nucleotide sequence determines the folding of the molecule into a unique three dimensional structure. Aptamers comprising 15 to 120 nucleotides can be selected in vitro from a randomized pool of oligonucleotides (1014-1015 molecules). Any aptamers of the invention as described herein further contemplates the use of both native and modified DNA and RNA bases, such as beta-D-Glucosyl-Hydroxymethyluracil. Aptamer sequences can be isolated through methods such as those disclosed in co-pending U.S. patent application Ser. No. 10/934,856, entitled, "Aptamers and Methods for their In vitro Selection and Uses Thereof," . It is contemplated that the sequences described herein may be varied to result in substantially homologous sequences which retain the same function as the original. As used herein, a polynucleotide or fragment thereof is "substantially homologous" (or "substantially similar") to another if, when optimally aligned (with appropriate nucleotide insertions or deletions) with the other polynucleotide (or its complementary strand), using an alignment program such as BLASTN (Altschul, S. F., Gish, W., Miller, W., Myers, E. W. & Lipman, D. J. (1990) "Basic local alignment search tool." J. Mol. Biol. 215:403-410), and there is nucleotide sequence identity in at least about 80%, preferably at least about 90%, and more preferably at least about 95-98% of the nucleotide bases. Nucleic acids encoding sequences of SOD2, TNFAIP6, TNFAIP3, TLR2 and/or IRS2 or their expression products can also be isolated from expression libraries using antibodies as probes. Such polyclonal or monoclonal antibodies can be raised using methods known in the art (see, e.g., Harlow and Lane, Antibodies: A Laboratory Manual (1988).

### Probes and Primers

A "probe" or "primer" is a single-stranded DNA or RNA molecule of defined sequence that can base pair to a second DNA or RNA molecule that contains a complementary sequence (the target). The stability of the resulting hybrid molecule depends upon the extent of the base pairing that occurs, and is affected by parameters such as the degree of complementarity between the probe and target molecule, and the degree of stringency of the hybridization conditions. The degree of hybridization stringency is affected by parameters such as the temperature, salt concentration, and concentration of organic molecules, such as formamide, and is determined by methods that are known to those skilled in the art. Probes or primers specific for the nucleic acid biomarkers described herein, or portions thereof, may vary in length by any integer from at least 8 nucleotides to over 500 nucleotides, including any value in between, depending on the purpose for which, and conditions under which, the probe or primer is used. For example, a probe or primer may be 8, 10, 15, 20, or 25 nucleotides in length, or may be at least 30, 40, 50, or 60 nucleotides in length, or may be over 100, 200, 500, or 1000 nucleotides in length. Probes or primers specific for the nucleic acid biomarkers described herein may have greater than 20-30% sequence identity, or at least 55-75% sequence identity, or at least 75-85% sequence identity, or at least 85-99% sequence identity, or 100% sequence identity to the nucleic acid biomarkers described herein. Probes or primers may be derived from genomic DNA or cDNA, for example, by amplification, or from cloned DNA segments, and may contain either genomic DNA or cDNA sequences representing all or a portion of a single gene from a single individual. A probe may have a unique sequence (e.g., 100% identity to a nucleic acid biomarker) and/or have a known sequence. Probes or primers may be chemically synthesized. A probe or primer may hybridize to a nucleic acid biomarker under high stringency conditions as described herein.

### Diagnostic Use

In a preferred embodiment, the invention involves methods to assess quantitative and qualitative aspects of the biomarker gene expression(s). In one example the decreased expression of an IRAK3 gene or gene product as provided by the present invention is indicative for the progression of a metabolic syndrome disorder in a subject or the increased risk to develop related cardiovascular diseases in said subject. Techniques well known in the art, e.g., quantitative or semiquantitative RT PCR for instance real time RT PCR, for instance mRNA analysis by the fluorescence-based real-time reverse transcription polymerase chain reaction (qRT-PCR or RT-qPCR) or reverse transcription loop-mediated amplification (RT-LAMP), for instance one-step RT-LAMP, or real-time NASBA for detection, quantification and differentiation of the RNA and DNA targets (LOENS K. et al. Journal of microbiological methods 2006, vol. 67, no3, pp. 408-415, or Northern blot, can be used to measure expression levels of IRAK3.

The measurement of IRAK3 gene expression levels may include measuring naturally occurring IRAK3 transcripts and variants thereof as well as non-naturally occurring variants thereof. The diagnosis and/or prognosis of metabolic syndrome disorder in a subject, however is preferably directed to detecting a naturally occurring IRAK3 gene product or variant thereof. Thus, the invention relates to methods of diagnosing and/or predicting metabolic syndrome disorder in a subject by measuring the expression of an IRAK3 gene in a subject. For example a decreased level of mRNA encoded by an IRAK3 nucleic acid sequence (e.g., SEQ ID NO: 1).

Diagnostic methods for the detection of IRAK3 nucleic acid molecules, in patient samples or other appropriate cell sources, may involve the pre-amplification of specific gene sequences, e.g., by PCR (SeeMullis, K. B., 1987, U. S. Patent No.4, 683,202), followed by the analysis of the amplified molecules using techniques well known to those of skill in the art, such as, for example, those listed above. Utilizing analysis techniques such as these, amplified sequences can be compared to the levels in control samples.

In a particular embodiment, the analyzing techniques include the application of detectably-labeled probes or primers. The probes or primers can be detectably-labeled, either radioactively or non-radioactively, by methods that are known to those skilled in the art, and their use in the methods according to the invention, involves nucleic acid hybridization, such as nucleic acid sequencing, nucleic acid amplification by the polymerase chain reaction (e.g., RT-PCR), single stranded conformational polymorphism (SSCP) analysis, restriction fragment polymorphism (RFLP) analysis, Southern hybridization, northern hybridization, in situ hybridization, electrophoretic mobility shift assay (EMSA), fluorescent in situ hybridization (FISH), and other methods that are known to those skilled in the art.

By "detectably labeled" is meant any means for marking and identifying the presence of a molecule, e.g., an oligonucleotide probe or primer, a gene or fragment thereof, or a cDNA molecule. Methods for detectably-labeling a molecule are well known in the art and include, without limitation, radioactive labeling (e.g., with an isotope such as 32P or 35S) and nonradioactive labeling such as, enzymatic labeling (for example, using horseradish peroxidase or alkaline phosphatase), chemiluminescent labeling, fluorescent labeling (for example, using fluorescein), bioluminescent labeling, or antibody detection of a ligand attached to the probe. Also included in this definition is a molecule that is detectably labeled by an indirect means, for example, a molecule that is bound with a first moiety (such as biotin) that is, in turn, bound to a second moiety that may be observed or assayed (such as fluorescein-labeled streptavidin). Labels also include digoxigenin, luciferases, and aequorin.

### Immunoassays

Antibodies that specifically bind any of the biomarkers described herein may be employed in an immunoassay by contacting a sample with the antibody and detecting the presence of a complex of the antibody bound to the biomarker in the sample. The antibodies used in an immunoassay may be produced as described herein or known in the art, or may be commercially available from suppliers, such as Dako Canada, Inc., Mississauga, ON. The antibody may be fixed to a solid substrate (e.g., nylon, glass, ceramic, plastic, etc.) before being contacted with the sample, to facilitate subsequent assay procedures. The antibody-biomarker complex may be visualized or detected using a variety of standard procedures, such as detection of radioactivity, fluorescence, luminescence, chemiluminescence, absorbance, or by microscopy, imaging, etc. Immunoassays include immunohistochemistry, enzyme- linked immunosorbent assay (ELISA), western blotting, and other methods known to those of skill in the art. Immunoassays can be used to determine presence or absence of a biomarker in a sample as well as the amount of a biomarker in a sample. The amount of an antibody-biomarker complex can be determined by comparison to a reference or standard, such as a polypeptide known to be present in the sample. The amount of an antibody-biomarker complex can also be determined by comparison to a reference or standard, such as the amount of the biomarker in a reference or control sample. Accordingly, the amount of a biomarker in a sample need not be quantified in absolute terms, but may be measured in relative terms with respect to a reference or control.

Detection of the biomarkers described herein may enable a medical practitioner to determine the appropriate course of action for a subject (e.g., further testing, drug or dietary therapy, surgery, no action, etc.) based on the diagnosis. Detection of the biomarkers described herein may also help determine the presence or absence of a metabolic syndrome disorder, early diagnosis of a metabolic syndrome disorder, prognosis of a metabolic syndrome disorder, efficacy of a therapy for a metabolic syndrome disorder, or monitoring a metabolic syndrome disorder therapy in a subject. In alternative aspects, the biomarkers and reagents prepared using the biomarkers may be used to identify metabolic syndrome disorder therapeutics. The methods according to the invention allow a medical practitioner to monitor a metabolic syndrome disorder therapy in a subject, enabling the medical practitioner to modify the treatment based upon the results of the test.

A particular embodiment of present invention is also an in vitro method of diagnosing a metabolic syndrome disorder phenotype in a subject, said method comprising: (a) analyzing the level of ZNF217 expression or activity of expression product in a biological sample isolated from said subject, and (b) compare said level of expression or activity with the ZNF217 expression or activity in a control sample; whereby a increased level of ZNF217 expression or activity relative to a control sample is an indication of such metabolic syndrome disorder phenotype or a propensity thereto.

### Measuring ZNF217 expression

Another particular embodiment of present invention is an in vitro method of diagnosing a metabolic syndrome disorder, or a propensity thereto in a subject, said method (a) obtaining an expression profile in a biological sample isolated from said subject, wherein said expression profile consists of the analysis of the level of ZNF217 expression or activity of an ZNF217 expression product in combination with the gene expression level or activity of a gene product of at least one gene selected from the group consisting of SOD2, TNFAIP6, TNFAIP3, TLR2, IRAK3 and IRS2; and (b) comparing said obtained expression profile to a reference expression profile to determine whether said sample is from subject having a metabolic syndrome disorder phenotype or a propensity thereto.

The present invention can comprise the above described in vitro methods wherein the metabolic syndrome disorder is an impaired adipose tissue accumulation or adipocyte function, an impaired glucose tolerance condition, an insulin resistance or type II diabetes disorder, a lipid homeostasis disorder or a cardiovascular disease related thereto. Such cardiovascular disease can hereby be of the group consisting of hypertension, coronary heart disease, heart failure, congestive heart failure, atherosclerosis, arteriosclerosis, stroke, cerebrovascular disease, myocardial infarction and peripheral vascular disease.

Such in vitro methods as described hereabove can comprise analyzing the level of ZNF217 expression or activity of expression product in a sample isolated from said subject, whereby a decreased level of ZNF217 expression or activity relative to a control sample is an indication of such adipose tissue disorder, lipid homeostasis disorder and/or an impaired glucose tolerance or insulin resistance condition and/or related cardiovascular disease or a propensity thereto. The expression product can be a nucleic acid molecule selected from the group consisting of mRNA and cDNA mRNA or derived polypeptides. Moereover the in vitro method can be carried out on a sample isolated form said subject is selected from a group consisting of a) a liquid containing cells; (b) a tissue-sample; (c) a cell-sample and (a) a cell biopsy. Such sample can comprise haematopoietic cells or blood cells. For instance the sample can comprise at least one myeloid cell or debris thereof or the sample can comprise at least one of monocytes or peripheral blood mononuclear cells or debris thereof. Moreover the in-vitro method as described above can be by carrying out the detection of the level of the nucleic acids or polypeptides is carried using at least one binding agent specifically binding to the nucleic acids or polypeptides to be detected. The binding agent can be detectably labelled and the label can be selected from the group consisting of a radioisotope, a bioluminescent compound, a chemiluminescent compound, a fluorescent compound, a metal chelate, biotin, digoxygenin and an enzyme. The in vitro methods described above can be wherein at least one binding agent is an aptamer or an antibody selected from a group comprising
- (a) a monoclonal antibody;
- (b) a polyclonal antibody;
- (c) a Fab-Fragment; and
- (d) a single chain antibody.
- (e) an antibody variable domain sequence.

The detection can comprise an immuno-cytochemical detection procedure or a procdure wherein at least one binding agent being a nucleic acid hybridising to a nucleic acid for the detection of the marker molecules, in particular for the detection of ZNF217 alone or in combination with a gene of the group consisting of IRAK3, SOD2, TNFAIP6, TNFAIP3, TLR2 and ISR2. Furthermore the detection reaction can comprise a nucleic acid amplification reaction. Finally the abobve described in vitro method can be for in-situ detection.

The present invention also comprises an vitro method for identifying or monitoring a metabolic syndrome disorder therapy in a subject said method comprising analysing the level of ZNF217 expression or activity of expression product in a sample isolated from said subject before and after treatment with said therapy, whereby an increased level of ZNF217 compared to a sample of said subject before the therapy is indicative for the efficacy of said therapy.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

### Drawing Description

### BRIEF DESCRIPTION OF THE DRAWINGS

The present invention will become more fully understood from the detailed description given herein below and the accompanying drawings which are given by way of illustration only, and thus are not limitative of the present invention, and wherein:

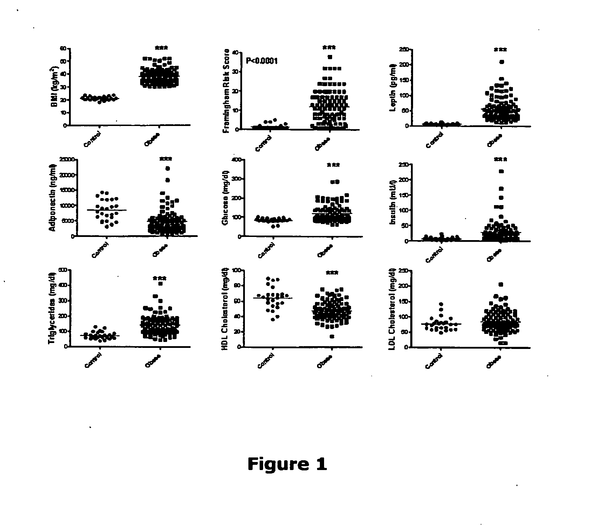

**Figure 1** demonstrates the predicted cardiovascular risk (by Framingham risk scoring or FRS) and metabolic syndrome-related characteristics of controls (n=25), and obese women (n=95). *** P<0.001 compared to controls.

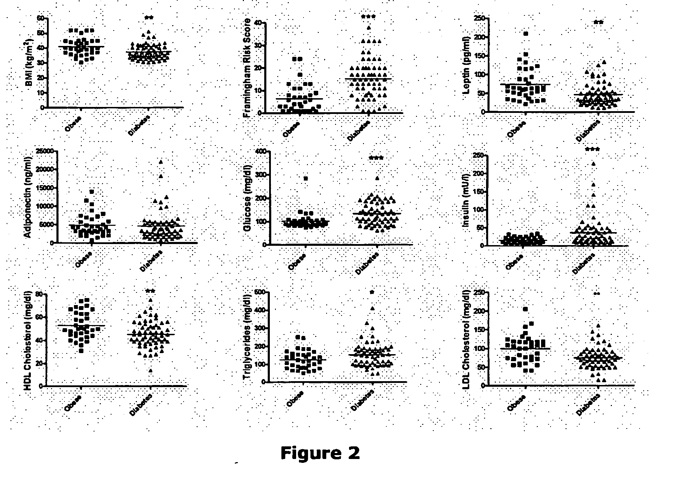

**Figure 2****:** displays the predicted cardiovascular risk (by Framingham risk scoring or FRS) and metabolic syndrome-related components of obese women without (n=35) and with (n=60) diabetes. *, **, *** P<0.05, P<0.01, and P<0.001 compared to obese women without diabetes.

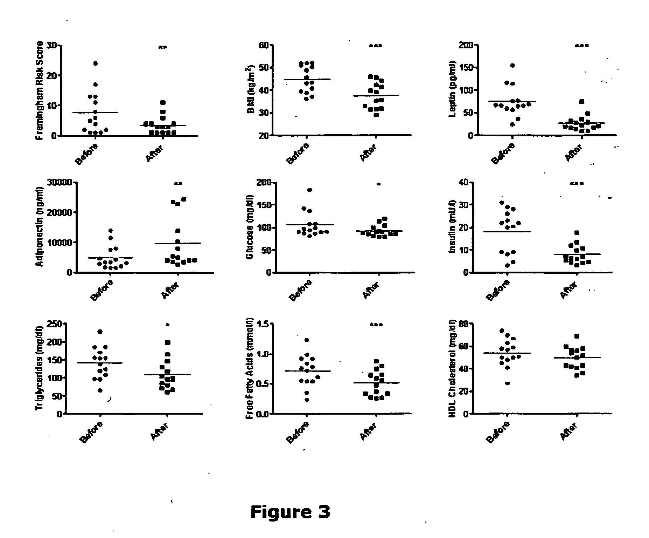

**Figure 3****:** demonstrates the predicted cardiovascular risk (by Framingham risk scoring or FRS) and metabolic syndrome-related characteristics of obese women (N=14) without diabetes before and 3 months after bariatric surgery. *, **, *** P<0.05, P<0.01, and P<0.001 compared to values before weight loss.

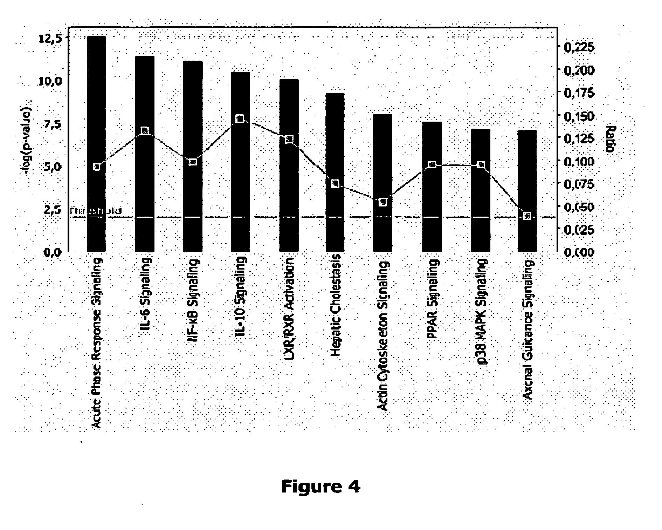

**Figure 4****:** provides the top ten canonical signaling pathways which were identified by means of Ingenuity Pathways Analysis (Ingenuity Systems® Ingenuity *IPA* 5.5 - 802, www.ingenuity.com). The significance of the association between the dataset and the canonical pathway was measured in 2 ways. First, a ratio (yellow squares) of the number of genes which were differentially expressed in monocytes of obese women and were assigned to a particular signaling pathway to the total number of genes that belong to this signaling canonical pathway and were present on the microchip. Second, the Fischer's exact test was used to calculate a p-value determining the probability that the association between the differentially expressed genes in the dataset and the assigned genes in the canonical pathway is not explained by chance alone. The threshold was P=0.01.

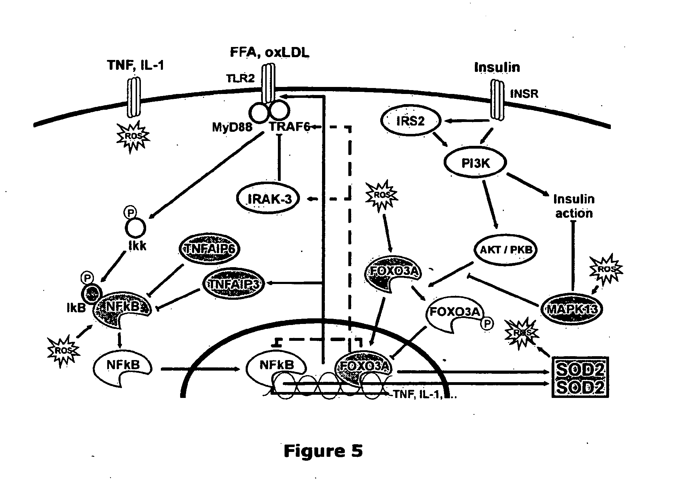

**Figure 5****:** displays the proposed structural model that predicts the interactions between the TLR2 and the PI3K/AKT pathway. Flow of the pathways at the protein interaction level is indicated by black arrows. Blunted arrows indicate inhibition. Phosphorylation is indicated by a circled P. Note that both Foxo3a and NFκB are regulated at the level of nuclear transport. NFκB is translocated to the nucleus after dissociation from the phosphorylated IκB. Foxo3a is a direct target of AKT/PKB and the phosphorylated transcription is unable to enter the nucleus and become active. Red arrows indicate downstream transcriptional events. Dashed arrows indicate the hypothetical involvement of Foxo3a in the transcription of TRAF6 and IRAK3. Dark grey: unregulated genes; light grey; down regulated genes; white: genes of which the expression is not different in monocytes of lean controls and obese women.

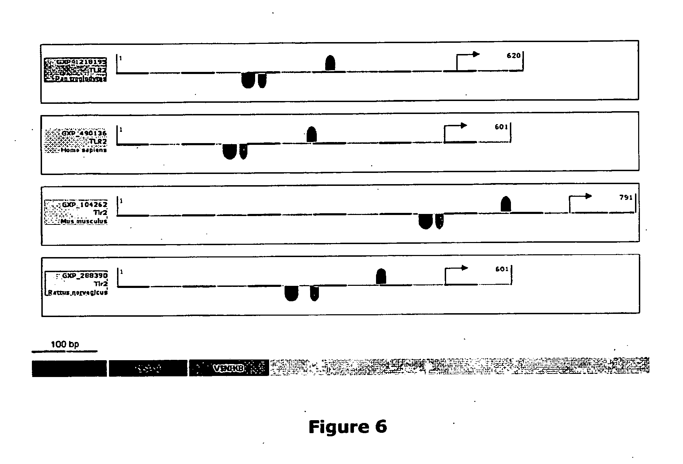

**Figure 6** is a schematic view of the conserved NFKβ-binding sites in the TLR2 promoter.

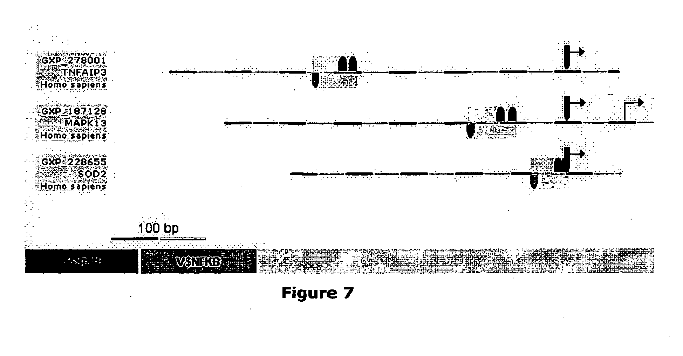

**Figure 7****:** displays common transcription factor binding sites in the promoters of TNFAIP3, MAPK13, and SOD2.

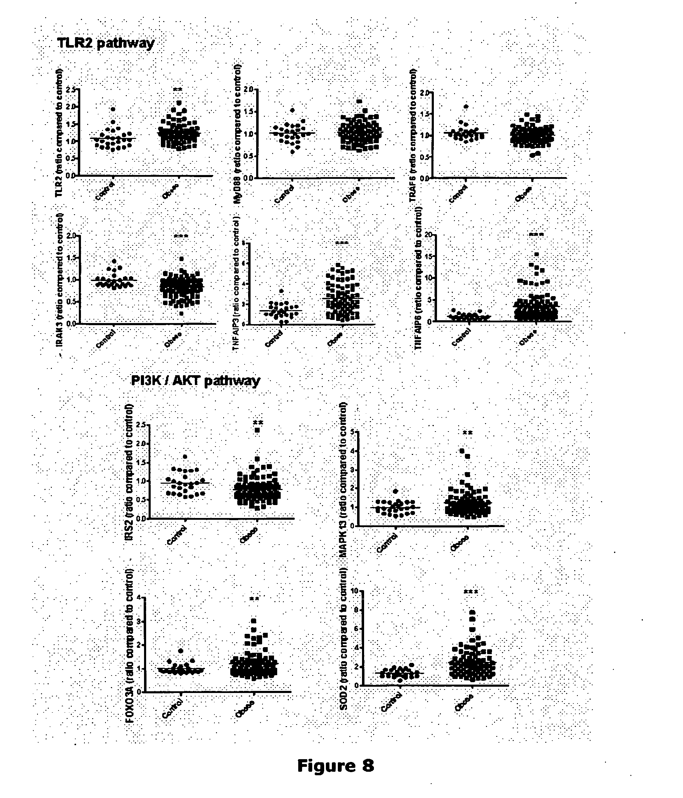

**Figure 8****:** displays the results of RT-PCR analysis of indicated genes in monocytes from control and obese women; obese women without and with diabetes are combined. **, *** P<0.01, and P<0.001 compared to controls

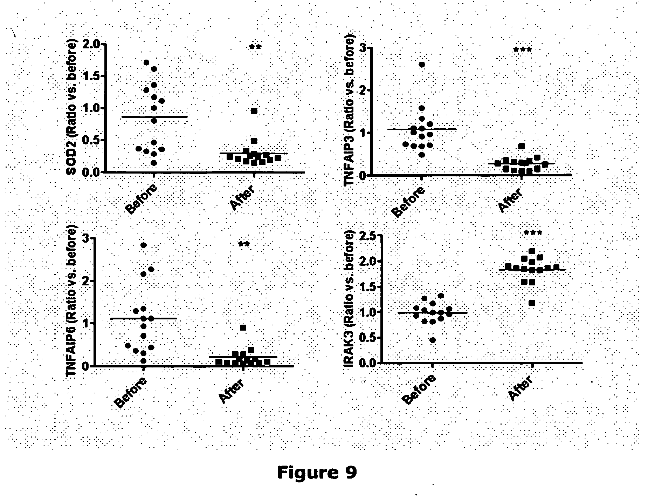

**Figure 9****:** provides a comparison of RNA expression before and after weight loss. Individual data before weight loss were expressed as a ratio compared to a pool of 29 samples of non-obese women. Individual data after weight loss were expressed as a ratio compared to values before weight loss. **, *** P<0.01, and P<0.001 compared to before.

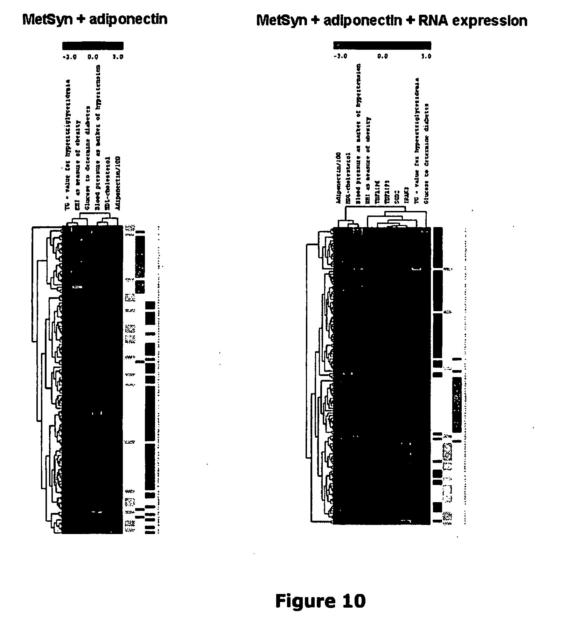

**Figure 10****:** An example of hierarchical clustering analysis based on Euclidean distance after normalization. Group attribution: lean controls (green), obese persons without cardiovascular risk equivalents (yellow), and obese persons with cardiovascular risk equivalents (red). Cardiovascular risk equivalents are: an at least 10% risk of developing a cardiovascular disease within the next 10 years based on Framingham scoring and/or diabetes and or metabolic syndrome (at least 3 metabolic syndrome or MetSyn components as defined above).

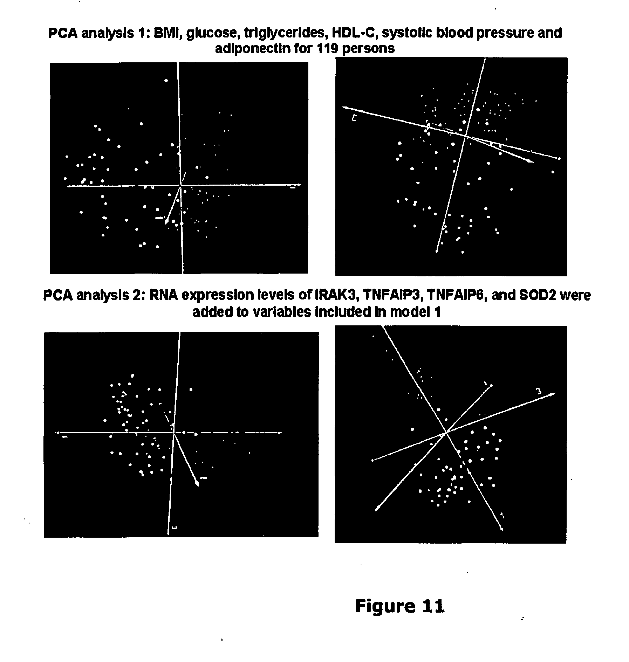

**Figure 11****:** An example of Principal component analysis of 6-10 variables (indicated on figure 10) for 119 participants. Color attributions are as in figure 10. We are showing two views for the same PCA along two different factorial axes.

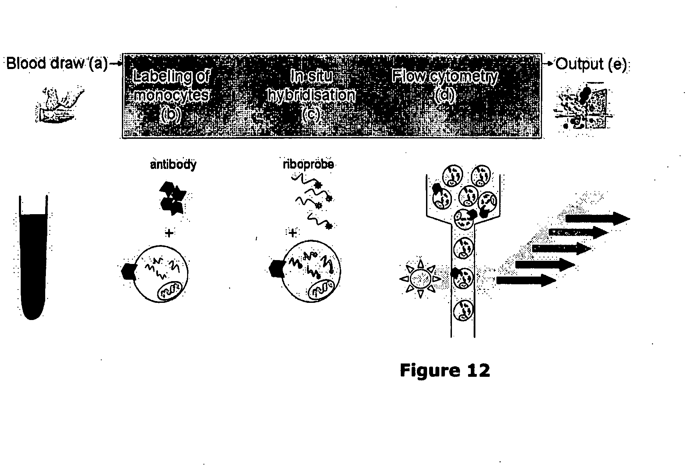

**Figure 12****:** Determination of RNA expression of specific cells in suspension combines flow cytometry with in situ hybridization. After blood draw (a), monocytes in whole blood are labeled with a specific monocyte specific antibody carrying a marker (b). Next, riboprobes that carry a marker for detection are added for in situ hybridization (c). Multi-color flow cytometry detects markers simultaneously (d). Data is analyzed to generate a risk profile (e).

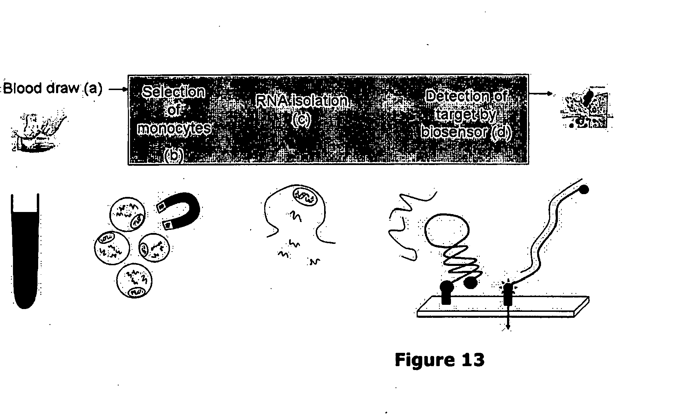

**Figure 13**: Example 1 of biosensor approach. After blood draw (a), monocytes in whole blood are labeled with a monocyte specific antibody carrying a magnet to remove them from whole blood (b). Next, RNA is isolated from the monocytes (c). Target RNAs hybridize with their complementary molecular beacons immobilized onto the recognition layer of the biosensor, generating a fluorescent signal that is transduced by the transductor layer of the biosensor (d).

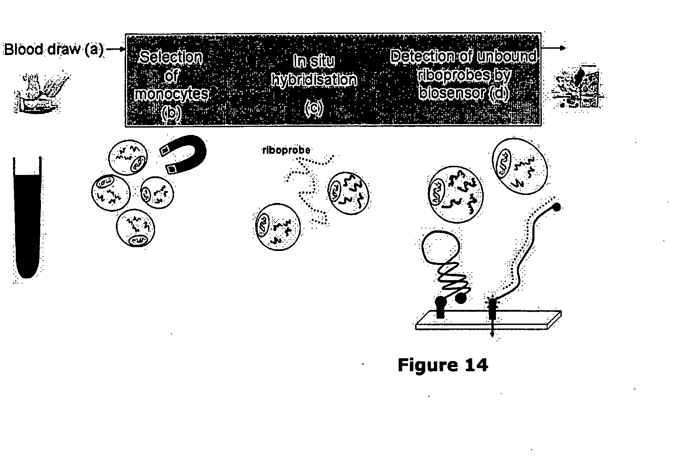

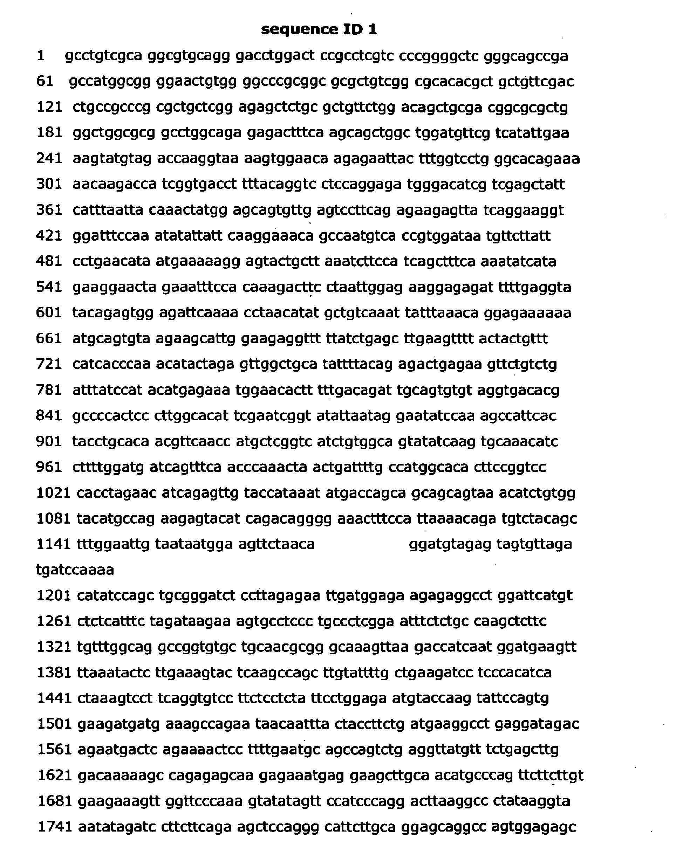

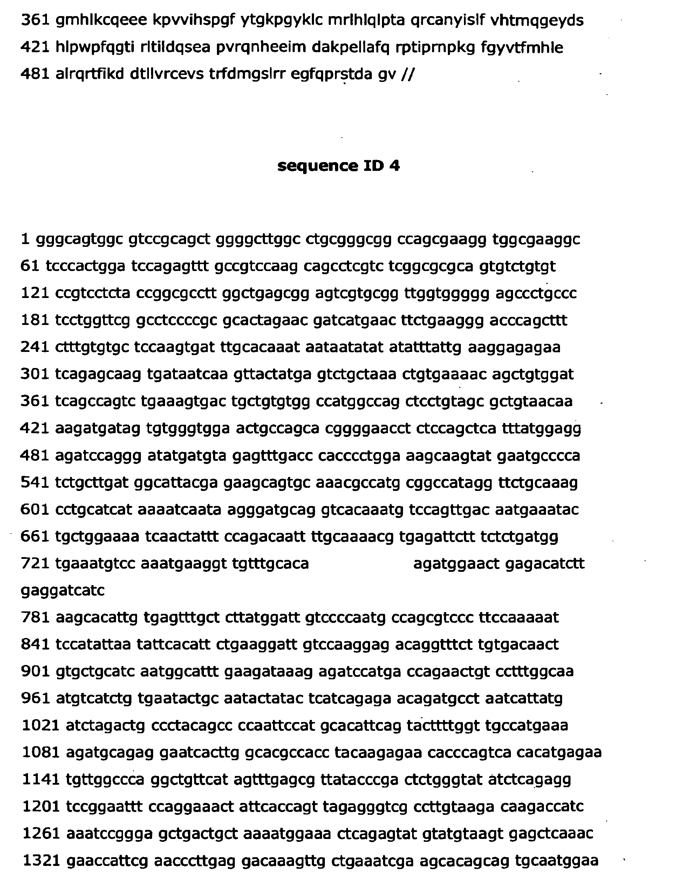

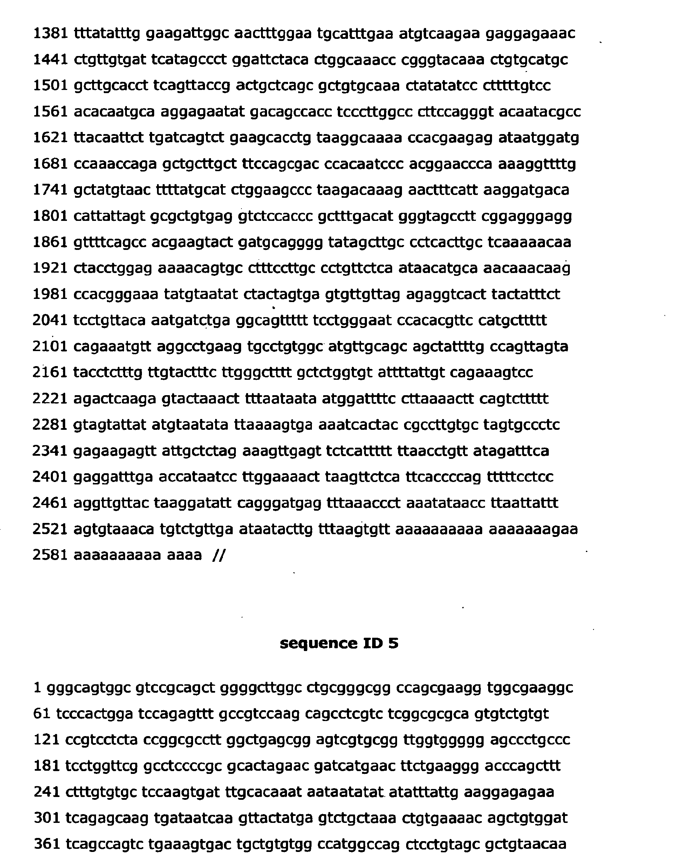

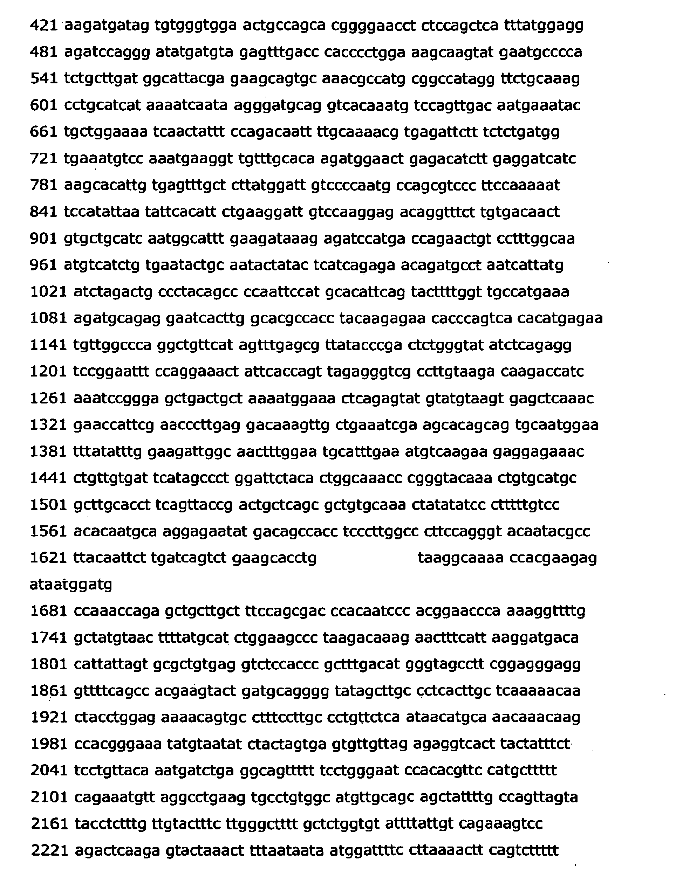

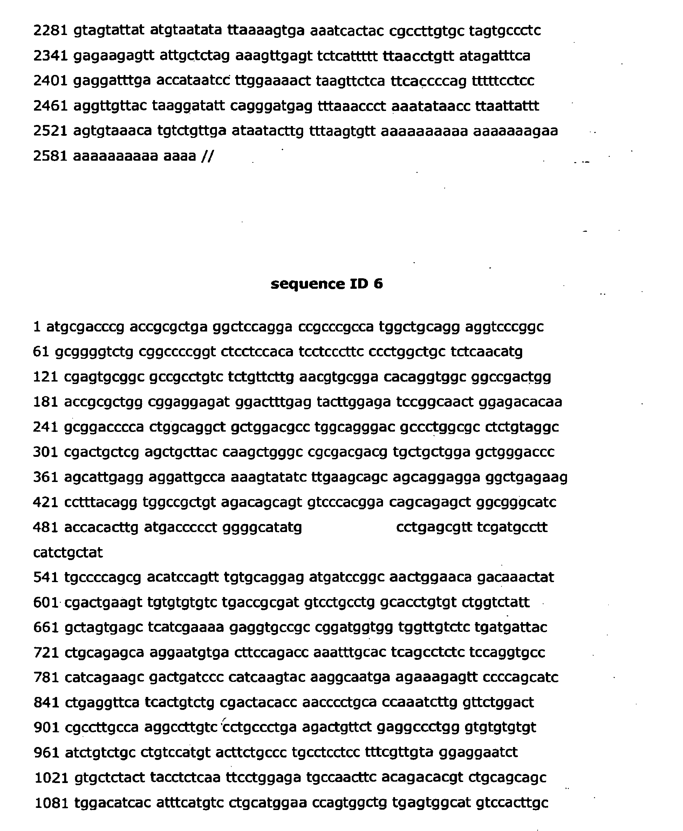

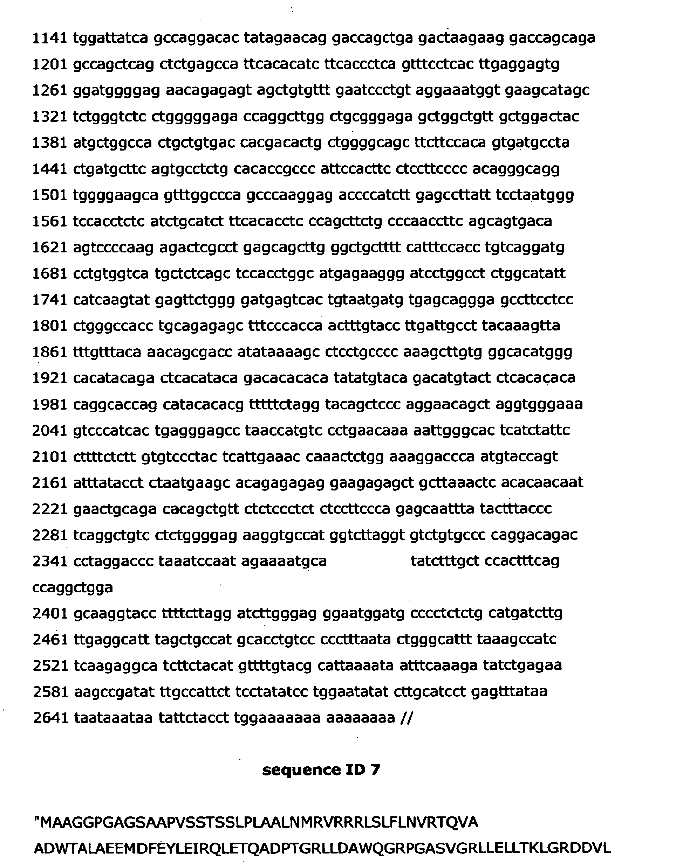

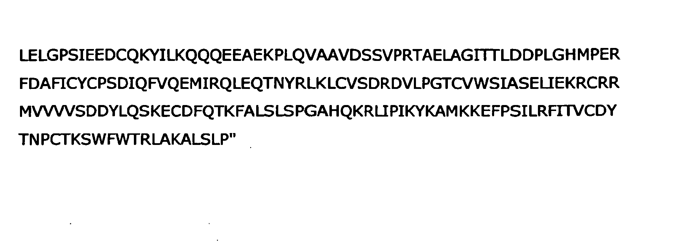

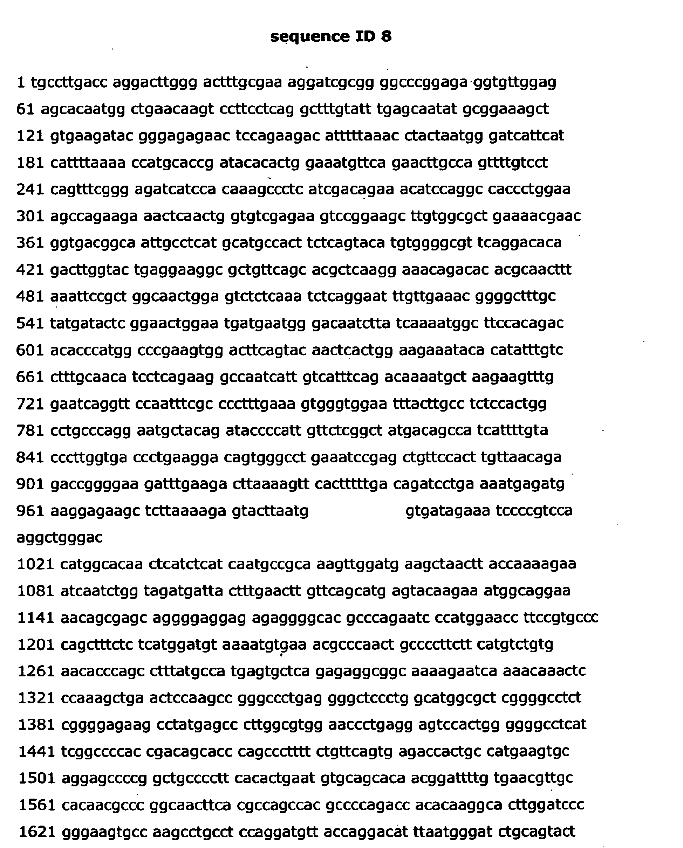

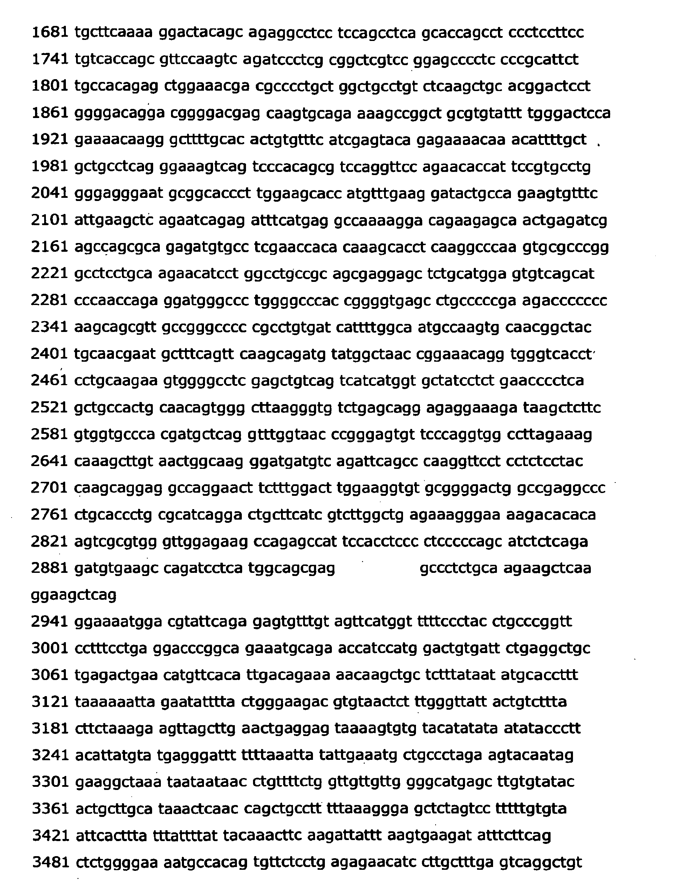

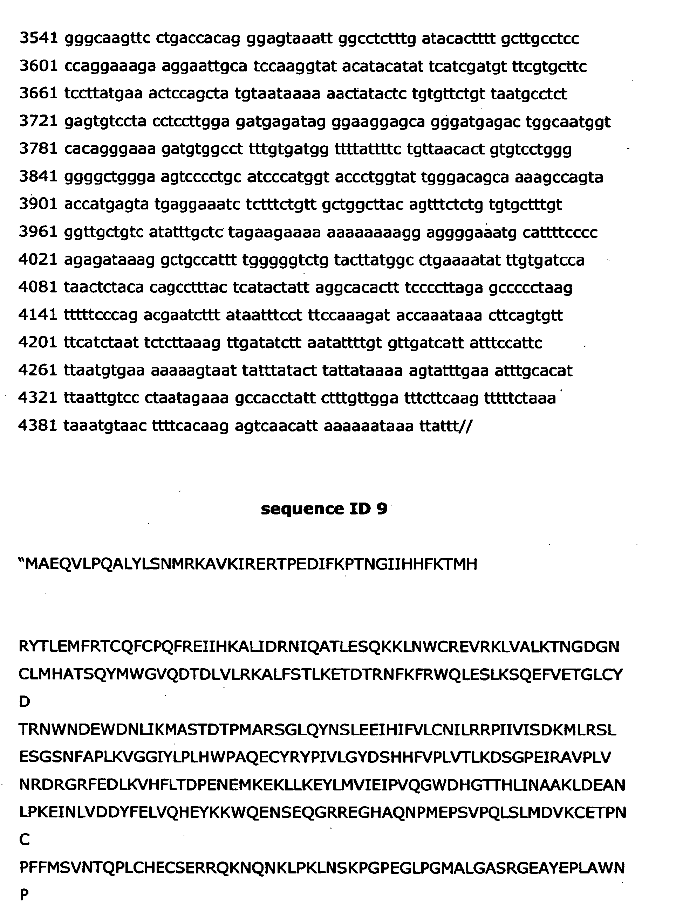

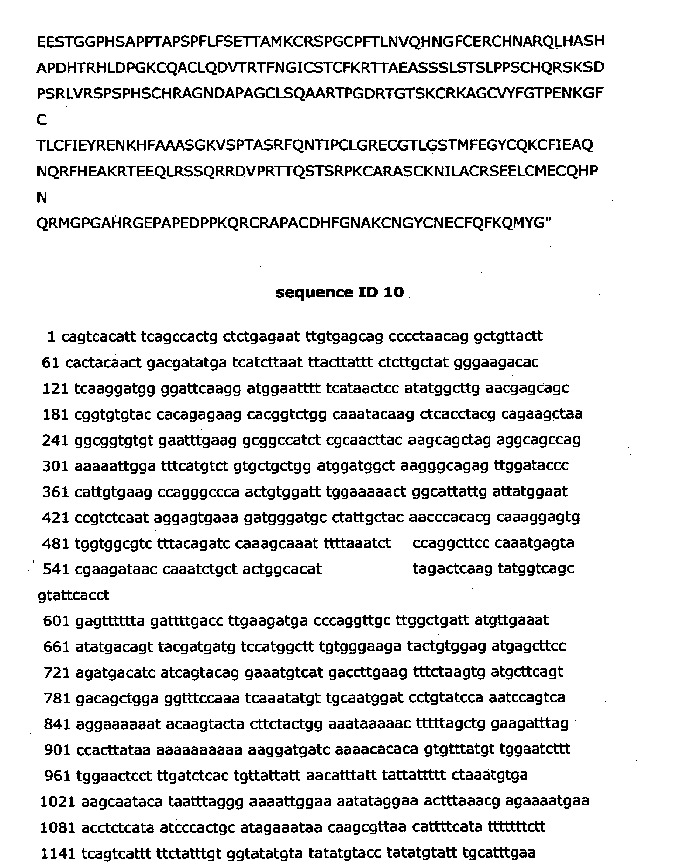

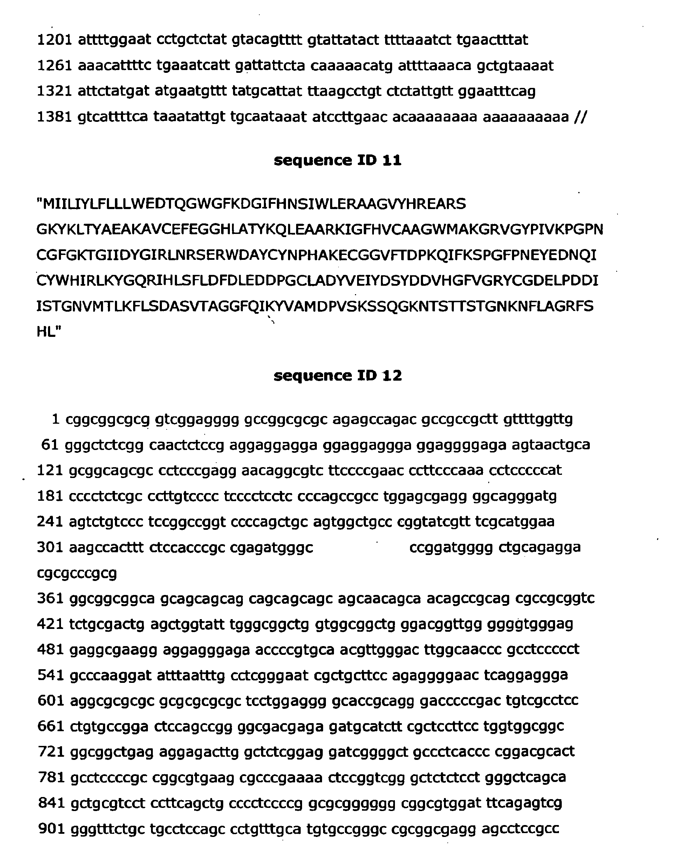

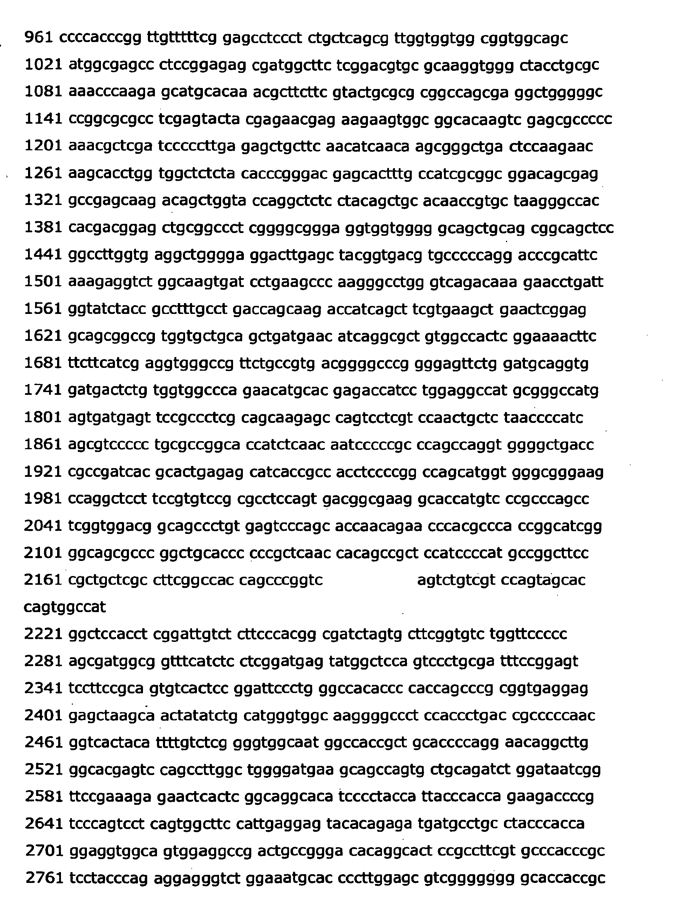

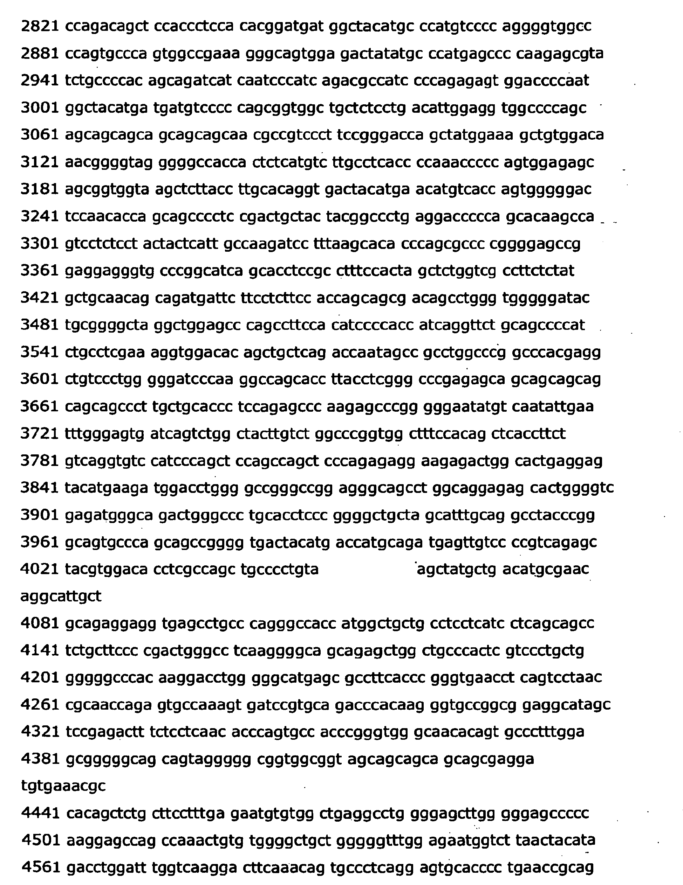

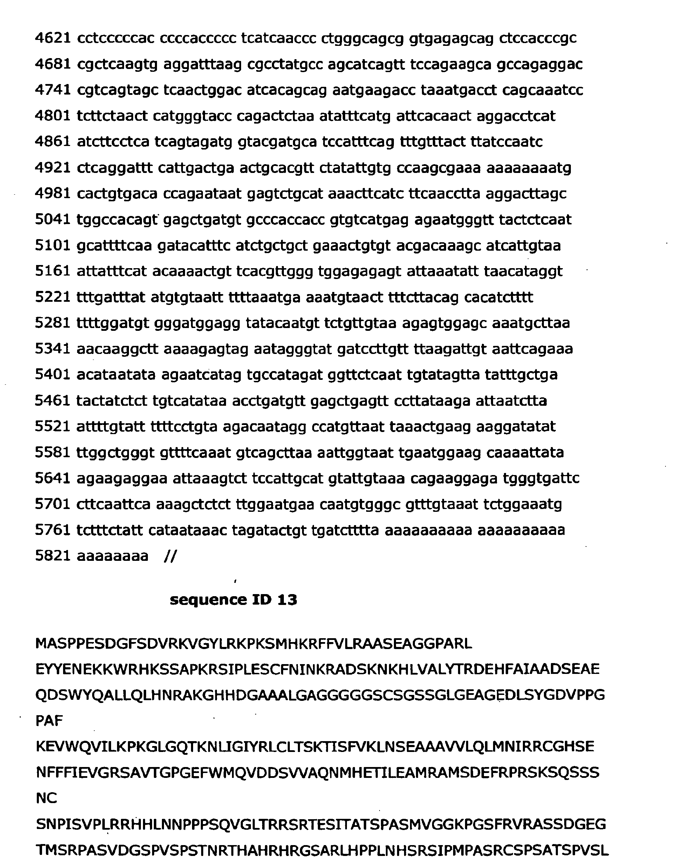

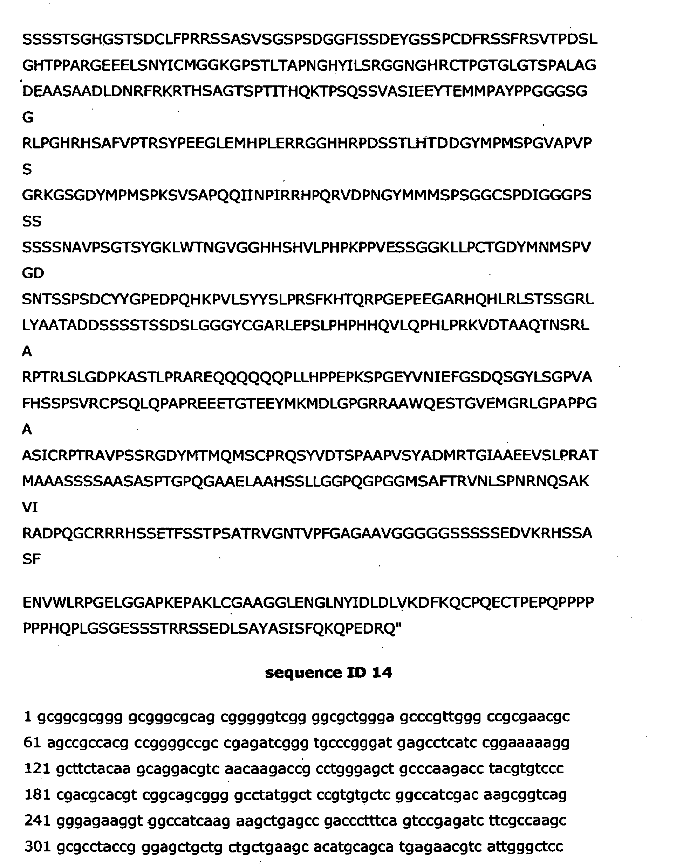

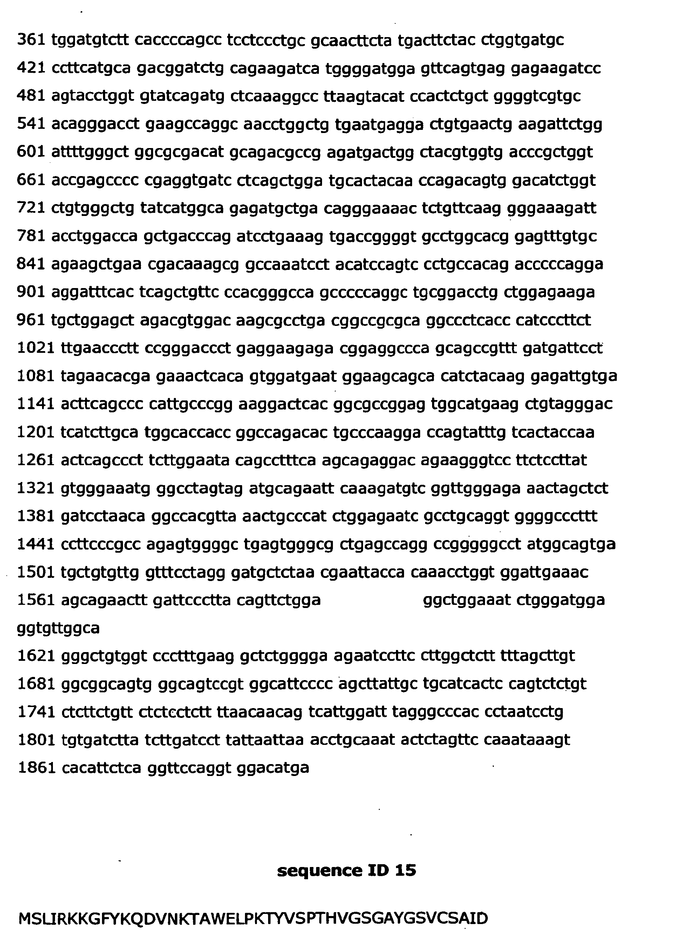

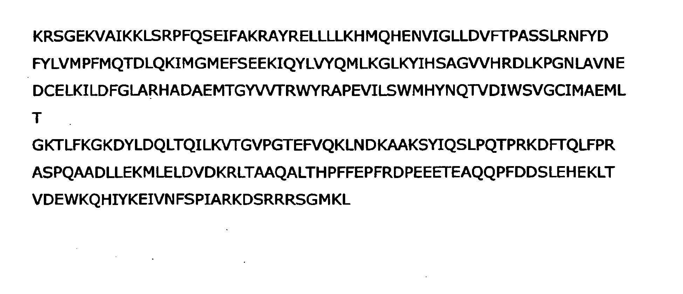

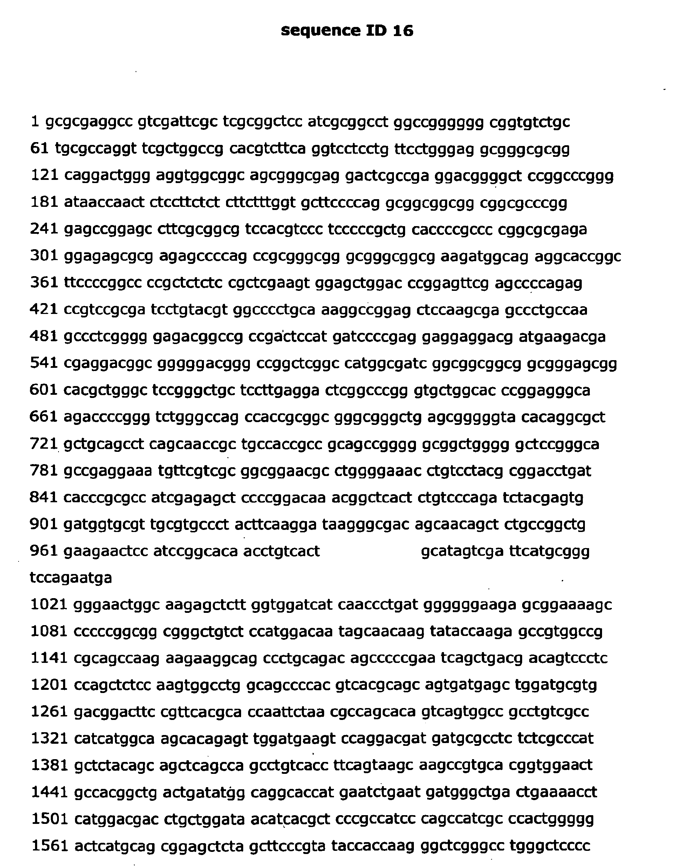

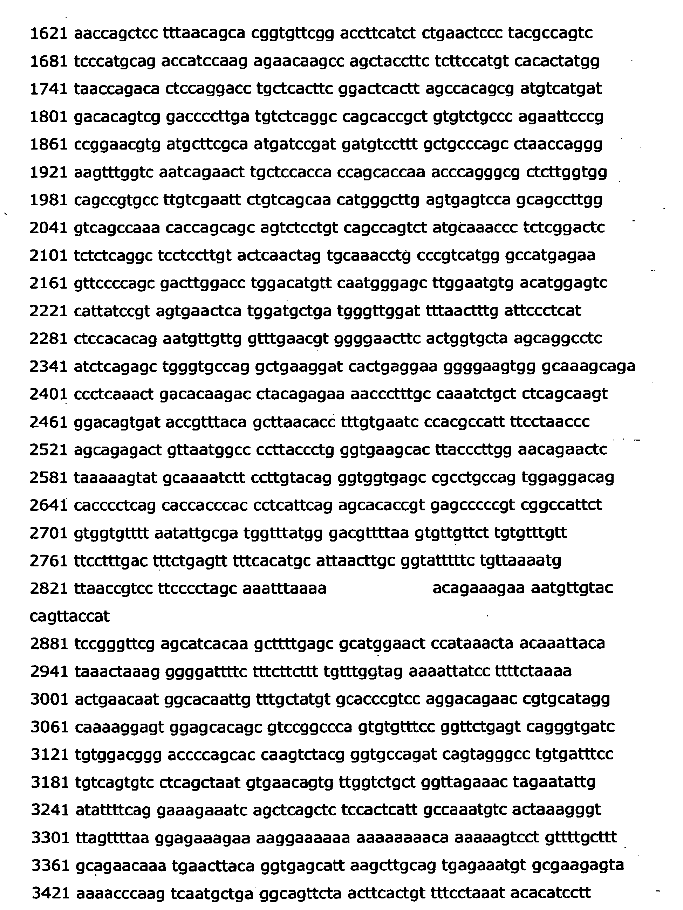

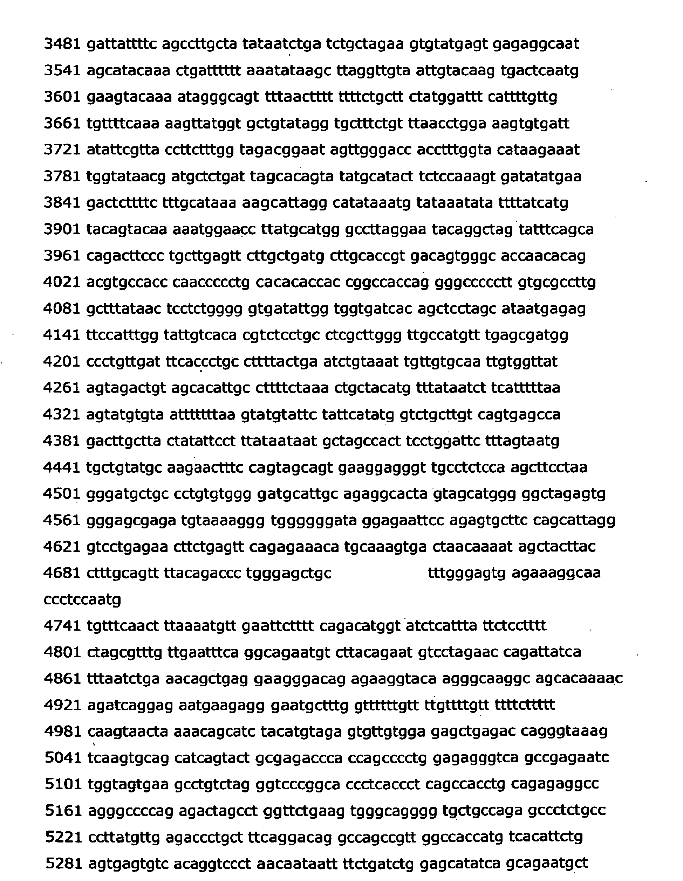

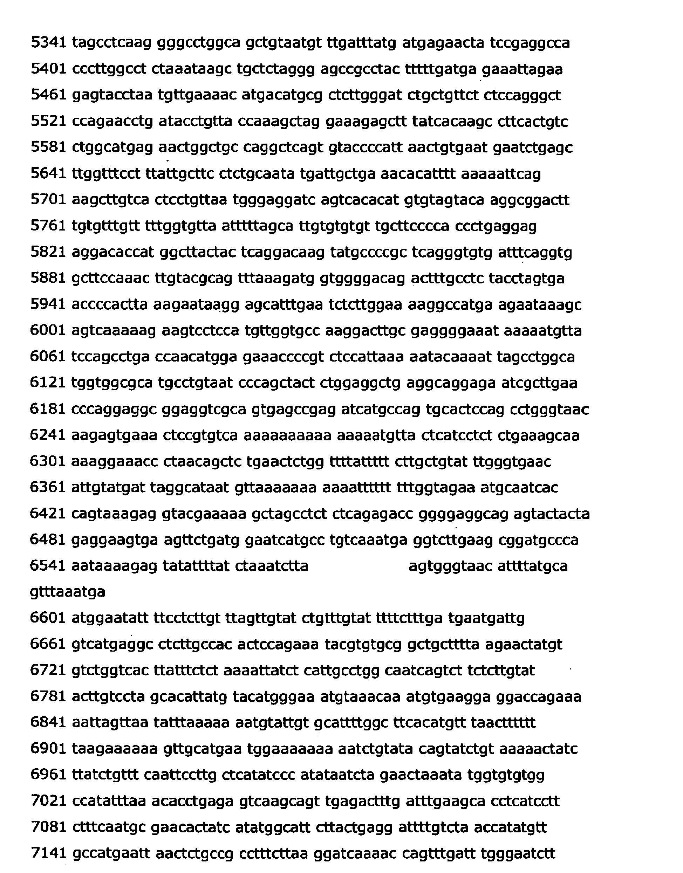

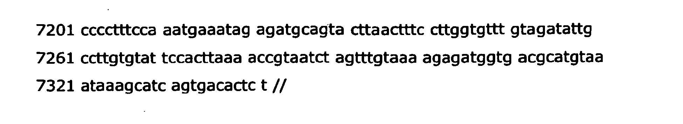

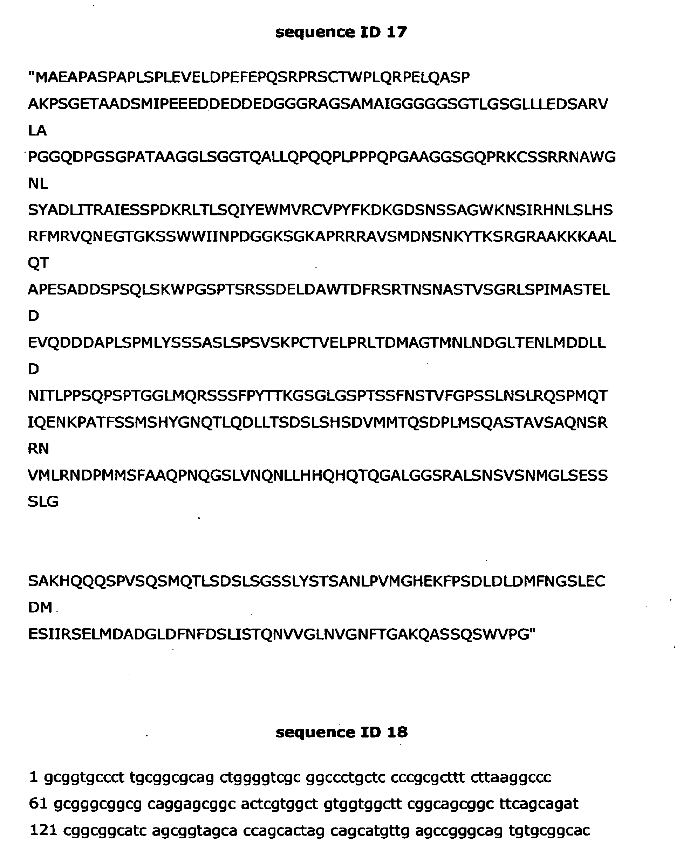

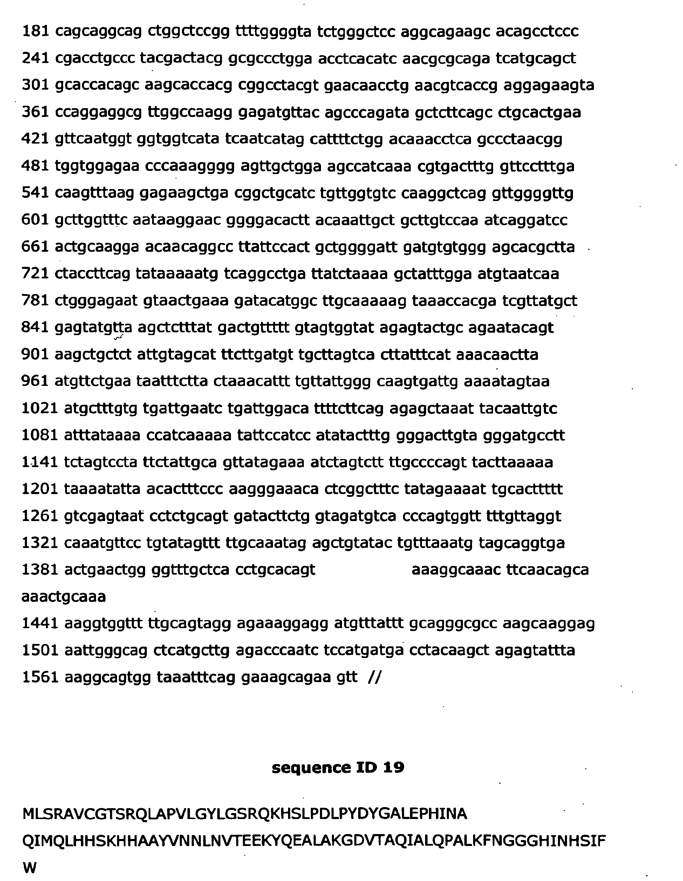

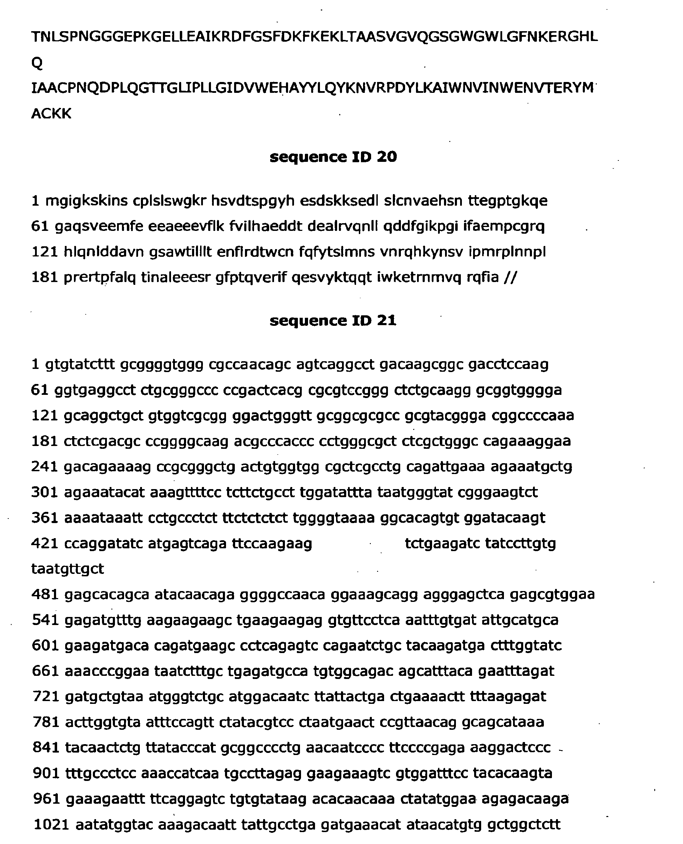

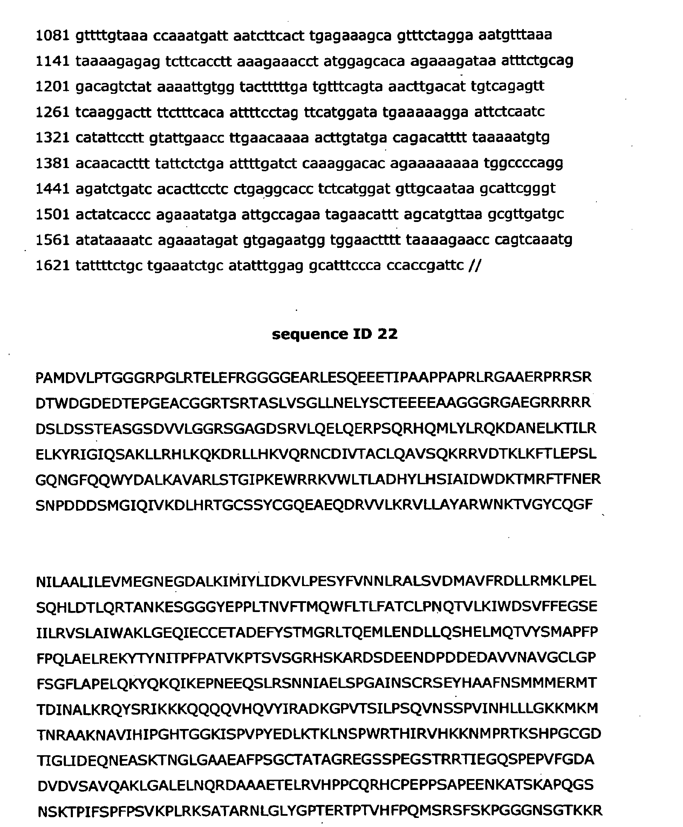

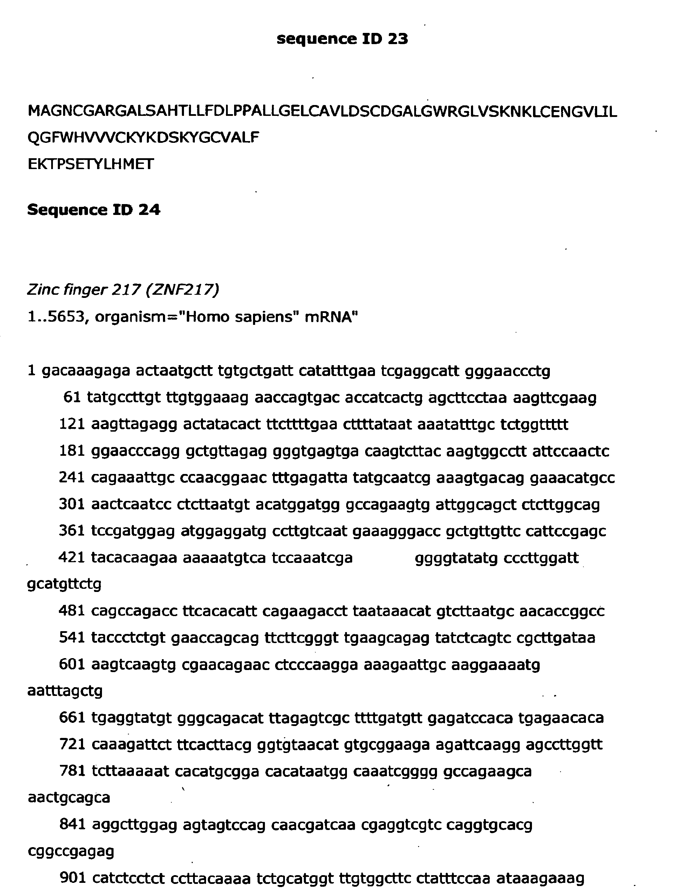

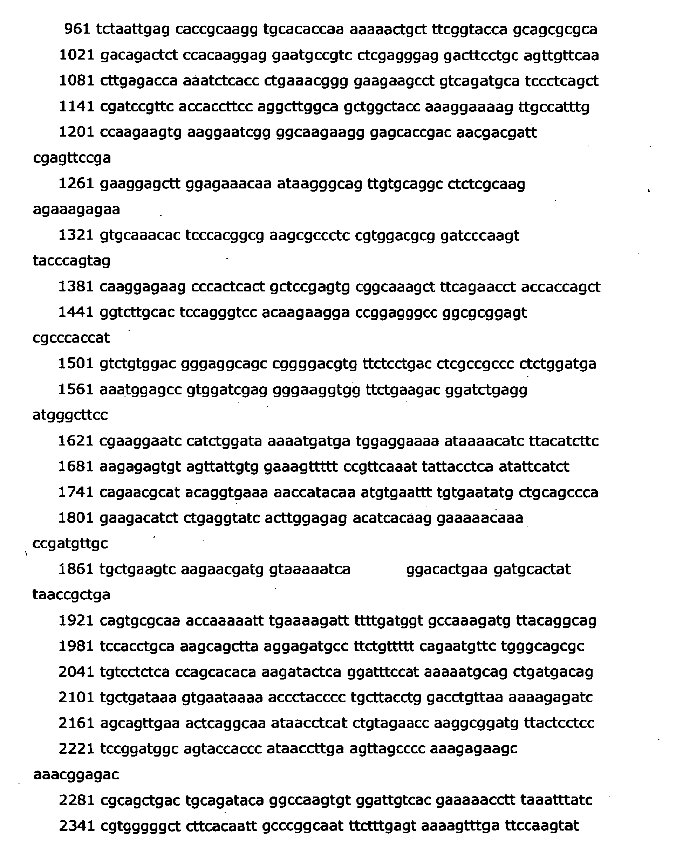

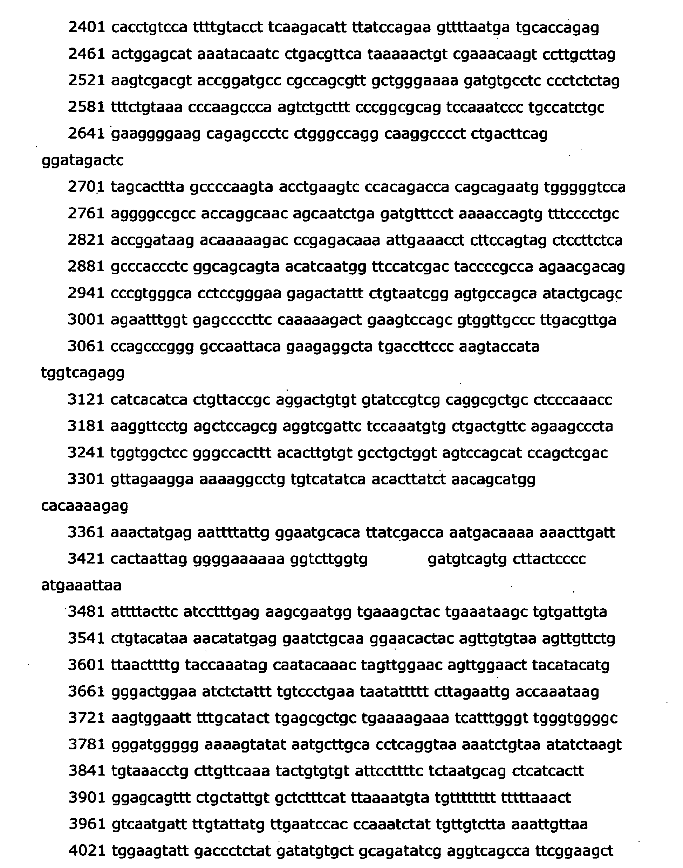

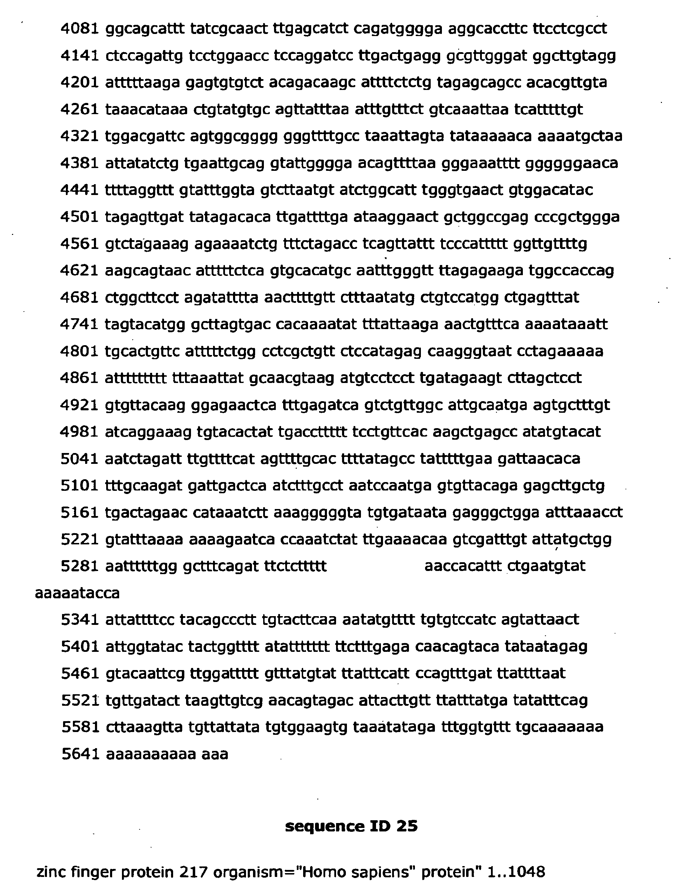

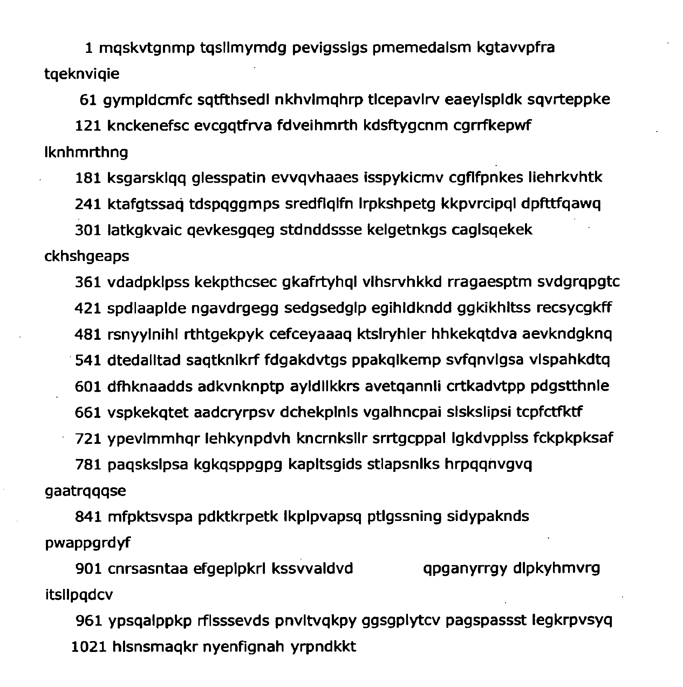

**Figure 14****:** Example 2 of biosensor approach. After blood draw (a), monocytes in whole blood are labeled with a monocyte specific antibody carrying a magnet to remove them from whole blood (b). Next, unlabelled rib probes are added to the cell suspension for in situ hybridization with target mRNAs (c). Unbound rib probes can hybridize with their complementary molecular beacons immobilized onto the recognition layer of the biosensor, generating a fluorescent signal that is inversely related to the expression of the target mRNAs (d).

### Gene Expression Profiling

The present invention relates to an in vitro method of diagnosing a metabolic syndrome disorder phenotype in a subject, said method comprising: (a) analyzing the level of IRAK3 expression or activity of expression product in a biological sample isolated from said subject, and (b) compare said level of expression or activity with the IRAK3 expression or activity in a control sample; whereby a decreased level of IRAK3 expression or activity relative to a control sample is an indication of such metabolic syndrome disorder phenotype or a propensity thereto or to an in vitro method of diagnosing a metabolic syndrome disorder, or a propensity thereto in a subject, said method (a) obtaining an expression profile in a biological sample isolated from said subject, wherein said expression profile consists of the analysis of the level of IRAK3 expression in combination with the gene expression level of at least one gene selected from the group consisting of SOD2, TNFAIP6, TNFAIP3, TLR2, IRS2 and ZNF217; and (b) comparing said obtained expression profile to a reference expression profile to determine whether said sample is from subject having a metabolic syndrome disorder phenotype or a propensity thereto. Furthermore the present invention relates to an in vitro method of diagnosing adipose tissue disorder, lipid homeostasis disorder and/or an impaired glucose tolerance or insulin resistance condition and/or related cardiovascular disease or a propensity thereto in a subject, said method comprising:
(a) analyzing the level of IRAK3 expression in a biological sample isolated from said subject, and (b) compare said level of expression with the IRAK3 expression in a control sample; whereby a decreased level of IRAK3 expression relative to a control sample is an indication of such adipose tissue disorder, lipid homeostasis disorder and/or an impaired glucose tolerance or insulin resistance condition and/or related cardiovascular disease or a propensity thereto or to an in vitro method of diagnosing an adipose tissue disorder, lipid homeostasis disorder and/or an impaired glucose tolerance or insulin resistance condition and/or related cardiovascular disease or a propensity thereto, or a propensity thereto in a subject, said method (a) obtaining an expression profile in a biological sample isolated from said subject, wherein said expression profile consists of the analysis of the level of IRAK3 expression in combination with the gene expression level or activity of a gene product of at least one gene selected from the group consisting of SOD2, TNFAIP6, TNFAIP3, TLR2, IRS2 and ZNF217; and
(b) comparing said obtained expression profile to a reference expression profile to determine whether said sample is from subject having an adipose tissue disorder, lipid homeostasis disorder and/or an impaired glucose tolerance or insulin resistance condition and/or related cardiovascular disease or a propensity thereto
Furthermore the present invention concerns a method for classifying individuals having or suspected of having a metabolic syndrome disorder phenotype as a responder or a non-responder to first line treatment, comprising the steps of measuring the gene expression of IRAK3 in a biological sample obtained from the individual to obtain a gene expression profile, and comparing the gene expression profile to that of a suitable control.
Another embodiment of present invention is method for classifying individuals having or suspected of having a metabolic syndrome disorder phenotype as a responder or a non-responder to first line treatment, comprising the steps of measuring the gene expression of IRAK3 expression or activity of an IRAK3 expression product in combination with the gene expression level or activity of a gene product of at least one gene selected from the group consisting of SOD2, TNFAIP6, TNFAIP3, TLR2, IRS2 and ZNF217; in a biological sample obtained from the individual to obtain a gene expression profile, and comparing the gene expression profile to that of a suitable control.
This method is used for predicting the optimal course of therapy for patients having an adipose tissue disorder, lipid homeostasis disorder and/or an impaired glucose tolerance or insulin resistance condition and/or related cardiovascular disease or a propensity thereto using a diagnostic oligonucleotide set or gene expression profile as described herein, via classification of a individual having or suspected of having a an adipose tissue disorder, lipid homeostasis disorder and/or an impaired glucose tolerance or insulin resistance condition and/or related cardiovascular disease or a propensity thereto as being either a "responder"' or 'non-responder" to first-line therapy. In one embodiment, the methods described herein may be used to predict the optimal course of therapy, or identify the efficacy of a given treatment in an individual having, or suspected of having an adipose tissue disorder, lipid homeostasis disorder and/or an impaired glucose tolerance or insulin resistance condition and/or related cardiovascular disease or a propensity thereto. In other embodiments, the methods described herein may be used to predict the optimal course of therapy post-diagnosis, for example, after treatment of an individual having an adipose tissue disorder, lipid homeostasis disorder and/or an impaired glucose tolerance or insulin resistance condition and/or related cardiovascular disease or a propensity thereto has begun, such that the therapy may be changed or adjusted, in accordance with the outcome of the diagnostic methods.
The present invention also relates to diagnostic oligonucleotides and diagnostic oligonucleotide sets and methods of using the diagnostic oligonucleotides and oligonucleotide sets to diagnose or monitor disease, assess severity of disease, predict future occurrence of disease, predict future complications of disease, determine disease prognosis, evaluate the patient's risk, "stratify" or classify a group of patients, assess response to current drug therapy, assess response to current non-pharmacological therapy, identify novel therapeutic compounds, determine the most appropriate medication or treatment for the patient, predict whether a patient is likely to respond to a particular drug, and determine most appropriate additional diagnostic testing for the patient, as well as other clinically and epidemiologically relevant applications. As set forth above, the term "diagnostic oligonucleotide set" generally refers to a set of two or more oligonucleotides that, when evaluated for differential expression of their products, collectively yields predictive data. Such predictive data typically relates to diagnosis, prognosis, monitoring of therapeutic outcomes, and the like. In general, the components of a diagnostic oligonucleotide set are distinguished from nucleotide sequences that are evaluated by analysis of the DNA to directly determine the genotype of an individual as it correlates with a specified trait or phenotype, such as a disease, in that it is the pattern of expression of the components of the diagnostic nucleotide set, rather than mutation or polymorphism of the DNA sequence that provides predictive value. It will be understood that a particular component (or member) of a diagnostic nucleotide set can, in some cases, also present one or more mutations, or polymorphisms that are amenable to direct genotyping by any of a variety of well known analysis methods, e.g., Southern blotting, RFLP, AFLP, SSCP, SNP, and the like.
In another embodiment of the present invention, a gene expression system useful for carrying out the described methods is also provided. This gene expression system can be conveniently used for determining a diagnosis, prognosis, or selecting a treatment for patients having or suspected of having an adipose tissue disorder, lipid homeostasis disorder and/or an impaired glucose tolerance or insulin resistance condition and/or related cardiovascular disease or a propensity thereto In one embodiment, the methods disclosed herein allow one to classify an individual of interest as either a "responder" or a "non-responder" to first-line treatment using a gene expression profile. For purposes of the methods disclosed herein, the term "responder" refers to a patient that responds to first line therapy and does not require a second induction of remission during the year following the induction of remission. In contrast, the term "non-responder" refers to a patient having an adipose tissue disorder, lipid homeostasis disorder and/or an impaired glucose tolerance or insulin resistance condition and/or related cardiovascular disease or a propensity thereto that will require a second induction of remission using any therapy. For example, treatment non-responders may require more than one course of a medicament of the group consisting of poglitazone, rosiglitazone, netoglitazone, rivoglitazone (CS-011), FK-614, tesaglitazar (AZ-242), ragaglitazar (N,N-622), muraglitazar (BMS-298585), edaglitazone (BM-13-1258), metaglidasen (MBX-102), naveglitazar (LY-519818), MX-6054, LY-510929, AMG-131(T-131), THR-0921), voglibose, acarbose, miglitol, emiglitate, phenformin, metformin, buformin, Vidagliptin (LAF237), P32/98, Sitagliptin (MK-431), P93/01, PT-100, saxagliptin (BMS-477118), T-6666, TS-021), AJ-9677, GLP-1, GLP-1MR agent, N,N-2211, AC-2993 (exendin-4), BIM-51077, Aib(8,35)hGLP-1 (7,37)NH2, CJC-[131], pramlintide, sodium vanadate, BVT-3498, AS-2868 Ro-28-1675, and GIP (Glucose-dependent insulinotropic peptide) or a medicament of the group consisting of pravastatin, simvastatin, lovastatin, atorvastatin, fluvastatin, rosuvastatin, pitavastatin N--[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl] piperidine-4-acetic acid, ciprofibrate, fenofibrate, bezafibrate, clofibrate, simfibrate, clinofibrate, Avasimibe, Eflucimibe, colestyramine, probucol, nicomol, niceritrol, ethyl icosapentate, soysterol and γ-oryzanol or a medicament of the group consisting of dexfenfluramine, fenfluramine, phentermine, sibutramine, amfepramone, dexamphetamine, mazindol, phenylpropanolamine, clobenzorex, SB-568849; SNAP-7941, CP-422935, SR-141716, SR-147778, BVT-3498, orlistat, cetilistat (ATL-962)), AJ-9677,leptin, CNTF (Ciliary Neurotropic Factor), lintitript, FPL-15849 or a nutricuetical.
Thus, in accordance with the methods, a classification of an individual as a "responder" indicates that first line treatment is likely to be successful in treating the adipose tissue disorder, lipid homeostasis disorder and/or an impaired glucose tolerance or insulin resistance condition and/or related cardiovascular disease or a propensity thereto, and as such, may be the treatement of choice, while an individual identified as being a non-responder would generally not be an ideal candidate for traditional first-line therapies.
Rather, an individual identified as a non-responder would likely benefit from more aggressive, or second-line therapies typically reserved for individuals that have not responded to first-line treatment.
Classifying patients as either a "responder" or a "non-responder" is advantageous, in that it allows one to predict the optimal course of therapy for the patient. This classification may be useful at the outset of therapy (at the time of diagnosis) or later, when first-line therapy has already been initiated, such that treatment may be altered to the benefit of the patient.
In general, the method of using a gene expression profile or gene expression system for diagnosing an individual as a responder or a non-responder comprises measuring the gene expression of IRAK3 and a gene identified in any of tables 3 - 12 or the sequence listing. Gene expression, as used herein, may be determined using any method known in the art reasonably calculated to determine whether the expression of a gene is upregulated, down-regulated, or unchanged, and may include measurement of RNA or the gene product itself.
In one embodiment, an individual is characterized as a responder or nonresponder to first line therapy via measurement of the expression of IRAK3 one or more genes of tables 3 - 12 in the individual as compared to the expression of one or more genes of tables 3 - 12 in a suitable control (such as an individual previously determined to be a responder or non responder). In another embodiment the one or more genes are selected from Table 3. In another embodiment the one or more genes are selected from Table 4. In another embodiment the one or more genes are selected from Table 5. In another embodiment the one or more genes are selected from Table 6. In another embodiment the one or more genes are selected from Table 7. In another embodiment the one or more genes are selected from Table 8. In another embodiment the one or more genes are selected from Table 9. In another embodiment the one or more genes are selected from Table 10. In another embodiment the one or more genes are selected from Table 11. In another embodiment the one or more genes are selected from Table 12. The genes selected for measurement of expression may be selected on the basis of fold difference.
In yet another embodiment, the method of identifying an individual having or suspected of having an adipose tissue disorder, lipid homeostasis disorder and/or an impaired glucose tolerance or insulin resistance condition and/or related cardiovascular disease or a propensity thereto such as comprises the steps of: 1) providing an array set immobilized on a substrate, wherein the array set comprises one or more oligonucleotides derived from IRAK3 or derived from SOD2, TNFAIP6, TNFAIP3, TLR2, IRS2 or ZNF217, or the Sequence Listing in this application, 2) providing a labeled target obtained from mRNA isolated from a biological sample from a patient having an adipose tissue disorder, lipid homeostasis disorder and/or an impaired glucose tolerance or insulin resistance condition and/or related cardiovascular disease or a propensity thereto, 3) hybridizing the labeled target to the array set under suitable hybridization conditions such that the labeled target hybridizes to the array elements, 4) determining the relative amounts of gene expression in the patient's biological sample as compared to a reference sample by detecting labeled target that is hybridized to the array set; 5) using the gene expression profile to classify the patient as a responder or a non-responder; and 6) predicting the optimal course of therapy based on said classification.
The one or more sequences that comprise the array elements may be selected from any of the sequences listed in Tables 3-12 or the Sequence Listing in this application. In one embodiment, the gene expression system comprises one or more array elements wherein the one or more array elements correspond to sequences selected from those sequences listed in Tables 3-12, or the Sequence Listing. In one embodiment, the array set comprises the sequences listed on IRAK3. In another embodiment, the array set comprises the sequences listed on of SOD2, TNFAIP6, TNFAIP3, TLR2, IRS2 and ZNF217.
The present invention also relates to an apparatus for predicting the optimal course of therapy in a patient having an adipose tissue disorder, lipid homeostasis disorder and/or an impaired glucose tolerance or insulin resistance condition and/or related cardiovascular disease or a propensity thereto. The apparatus comprises a solid support having an array set immobilized thereon, wherein labeled target derived from mRNA from a patient of interest is hybridized to the one or more sequences of the array set on the solid support, such that a change in gene expression for each sequence compared to a reference sample or other suitable control may be determined, permitting a determination of the optimal course of therapy for the patient. The array set comprises one or more sequences selected from those sequences IRAK3, SOD2, TNFAIP6, TNFAIP3, TLR2, IRS2 or ZNF217 listed in the Sequence Listing described herein.

In yet another embodiment, the method of classifying an individual having or suspected of having an adipose tissue disorder, lipid homeostasis disorder and/or an impaired glucose tolerance or insulin resistance condition and/or related cardiovascular disease or a propensity thereto as a responder or non-responder comprises the steps of: 1) obtaining mRNA isolated from a biological sample from a patient having or suspected of having an adipose tissue disorder, lipid homeostasis disorder and/or an impaired glucose tolerance or insulin resistance condition and/or related cardiovascular disease or a propensity thereto, 2) reverse transcribing mRNA to obtain the corresponding DNA; 3) selecting suitable oligonucleotide primers corresponding to IRAK3 and one or more genes selected from SOD2, TNFAIP6, TNFAIP3, TLR2, IRS2 and ZNF217 which sequence listed herein, 4) combining the DNA and oligonucleotide primers in a suitable hybridization solution; 5) incubating the solution under conditions that permit amplification of the sequences corresponding to the primers; and 6) determining the relative amounts of gene expression in the patient' s biological sample as compared to a reference sample or other suitable control; wherein the resulting gene expression profile can be used to classify the patient as a responder or a non-responder. In other embodiments, real time PCR methods or any other method useful in measuring mRNA levels as known in the art may also be used. Alternatively, measurement of one or more gene products using any standard method of measuring protein (such as radioimmunoassay methods or Western blot analysis) may be used to determine a gene expression profile.
The methods of gene expression profiling that may be used with the methods and apparatus described herein are well-known in the art. In general, methods of gene expression profiling can be divided into methods based on hybridization analysis of polynucleotides, and methods based on sequencing of polynucleotides. Commonly used methods known in the art for the quantification of mRNA expression in a sample include northern blotting and in situ hybridization (Parker & Barnes, Methods in Molecular Biology 106:247 283 (1999)), RNAse protection assays (Hod, Biotechniques 13:852 854 (1992)), and reverse transcription polymerase chain reaction (RT-PCR) (Weis et al., Trends in Genetics 8:263 264 (1992)), or modified RT-PCR methods, such as that described in US Patent 6,618,679 or in Methods in Molecular Biology # 193: RT-PCR Protocols by Joe O'connell Publisher: Humana Press or Real-Time PCR: Current Technology and Applications Publisher: Caister Academic Press January 2009. Alternatively, antibodies, antibody fragments, domain antibodies or nanobodies may be employed that can recognize specific duplexes, including DNA duplexes, RNA duplexes, and DNA-RNA hybrid duplexes or DNA-protein duplexes. Representative methods for sequencing-based gene expression analysis include Serial Analysis of Gene Expression (SAGE) and gene expression analysis by massively parallel signature sequencing (MPSS). In one embodiment described herein, gene array technology such as microarray technology is used to profile gene expression.

### Arrays and Microarray Technologies

Array and microarray techniques known in the art to determine gene expression may be employed with the invention described herein. Where used herein, array refers to either an array or microarray. An array is commonly a solid-state grid containing sequences of polynucleotides or oligonucleotides (array elements) of known sequences are immobilized at a particular position (also referred to as an "address") on the grid. Microarrays are a type of array termed as such due to the small size of the grid and the small amounts of nucleotide (such as nanogram, nanomolar or nanoliter quantities) that are usually present at each address. The immobilized array elements (collectively, the "array set") serve as hybridization probes for cDNA or cRNA derived from messenger RNA (mRNA) isolated from a biological sample. An array set is defined herein as one or more DNA fragments or oligonucleotides, as defined above, that are immobilized on a solid support to form an array.
In one embodiment, for example, the array is a "chip" composed, e.g., of one of the above specified materials. Polynucleotide probes, e.g., RNA or DNA, such as cDNA, synthetic oligonucleotides, and the like, or binding proteins such as antibodies, that specifically interact with expression products of individual components of the candidate library are affixed to the chip in a logically ordered manner, i.e., in an array. In addition, any molecule with a specific affinity for either the sense or anti-sense sequence of the marker nucleotide sequence (depending on the design of the sample labeling), can be fixed to the array surface without loss of specific affinity for the marker and can be obtained and produced for array production, for example, proteins that specifically recognize the specific nucleic acid sequence of the marker, ribozymes, peptide nucleic acids (PNA), or other chemicals or molecules with specific affinity.
The techniques described herein, including array and microarray techniques, may be used to compare the gene expression profile of a biological sample from a patient of interest to the gene expression profile of a reference sample or other suitable control. The gene expression profile is determined by first extracting RNA from a biological sample of interest, such as from a patient diagnosed with an adipose tissue disorder, lipid homeostasis disorder and/or an impaired glucose tolerance or insulin resistance condition and/or related cardiovascular disease or a propensity thereto. The RNA is then reverse transcribed into cDNA and labeled. In another embodiment, the cDNA may be transcribed into cRNA and labeled. The labeled cDNA or cRNA forms the target that may be hybridized to the array set comprising probes selected according the methods described herein. The reference sample obtained from a control patient is prepared in the same way. In one embodiment, both a test sample and reference sample may be used, the targets from each sample being differentially labeled (for example, with fluorophores having different excitation properties), and then combined and hybridized to the array under controlled conditions. In general, the labeled target and immobilized array sets are permitted, under appropriate conditions known to one of ordinary skill in the art, to hybridize such that the targets hybridize to complementary sequences on the arrays. After the array is washed with solutions of appropriately determined stringency to remove or reduce non-specific binding of labeled target, gene expression may be determined. The ratio of gene expression between the test sample and reference sample for a given gene determines the color and/or intensity of each spot, which can then be measured using standard techniques as known in the art. Analysis of the differential gene expression of a given array set provides an "expression profile" or "gene signature" for that array set.
The expression profile is the pattern of gene expression produced by the experimental sample, wherein transcription of some genes are increased or decreased compared to the reference sample. Amplification methods using in vitro transcription may also be used to yield increased quantities of material to array where sample quantities are limited. In one embodiment, the Nugen Ovation amplification system may be incorporated into the protocol, as described below. Commercially-produced, high-density arrays such as those manufactured by Affymetrix GeneChip (available from Affymetrix, Santa Clara, CA) containing synthesized oligonucleotides may be used with the methods disclosed herein. In one embodiment, the HGU133 Plus Version 2 Affymetrix GeneChip may be used to determine gene expression of an array sets comprising sequences listed in Tables 4-8 or the Sequence Listing. In another embodiment, customized cDNA or oligonucleotide arrays may be manufactured by first selecting one or more array elements to be deposited on the array, selected from one or more sequences of IRAK3, SOD2, TNFAIP6, TNFAIP3, TLR2, IRS2 or ZNF217 listed in this application or of natural sequence variations on these human genes or homologs and variants of the disclosed nucleic acid molecules. Purified PCR products or other suitably derived oligonucleotides having the selected sequence may then be spotted or otherwise deposited onto a suitable matrix. The support may be selected from any suitable support known in the art, for example, microscope slides, glass, plastic or silicon chips, membranes such as nitrocellulose or paper, fibrous mesh arrangement, nylon filter arrays, glass-based arrays or the like. The array may be a chip array, a plate array, a bead array, a pin array, a membrane array, a solid surface array, a liquid array, an oligonucleotide array, a polynucleotide array, a cDNA array, a microfilter plate, a membrane or a chip. Where transparent surfaces such as microscope slides are used, the support provides the additional advantage of two-color fluorescent labeling with low inherent background fluorescence. The gene expression systems described above, such as arrays or microarrays, may be manufactured using any techniques known in the art, including, for example, printing with fine-pointed pins onto glass slides, photolithograpahy using dynamic micromirror devices, ink-jet printing, or electrochemistry on microelectrode arrays.
Oligonucleotide adherence to the slide may be enhanced, for example, by treatment with polylysine or other cross-linking chemical coating or by any other method known in the art. The DNA or oligonucleotide may then be cross-linked by ultraviolet irradiation and denatured by exposure to either heat or alkali. The microarray may then be hybridized with labeled target derived from mRNA from one or more samples to be analyzed. For example, in one embodiment, cDNA or cRNA obtained from mRNA from colon samples derived from both a patient diagnosed with metabolic syndrome and a healthy control sample is used. The samples may be labeled with different detectable labels such as, for example, fluorphores that exhibit different excitation properties. The samples may then be mixed and hybridized to a single microarray that is then scanned, allowing the visualization of up-regulated or down- regulated genes. The DualChip TM platform available from Eppendorf is an example of this type of array.
The probes affixed to the solid support in the gene expression system comprising the array elements may be a candidate library, a diagnostic agent, a diagnostic oligonucleotide set or a diagnostic probe set. In one embodiment of the present invention, the one or more array elements comprising the array set are selected from those sequences of IRAK3, SOD2, TNFAIP6, TNFAIP3, TLR2, IRS2 or ZNF217 listed in this application or of natural sequence variations on these human genes or homologs and variants of the disclosed nucleic acid molecules.

### Determination of Array Sets

A global pattern of gene expression in biological samples such as blood cells, blood monocyte preferable a myeloid cell from patients with adipose tissue disorder, lipid homeostasis disorder and/or an impaired glucose tolerance or insulin resistance condition and/or related cardiovascular disease at diagnosis (CDD), treated CD patients refractory to first line therapy (chronic refractory, CDT), and healthy controls can be determined. cRNA can be prepared from biopsies obtained from endoscopically affected segments, predominantly the ascending colon, with control biopsies obtained from matched segments in healthy patients. cRNA can be labeled and then hybridized. RNA obtained from a pool of RNA from one normal specimen can be labeled and hybridized with each batch of new samples to serve as an internal control for batch to batch variability in signal intensity. Results can be interpreted utilizing a proper software e.g. GeneSpring™ 7.3 Software (Silicon Genetics). Differentially expressed genes are then identified by filtering levels of gene-specific signal intensity for statistically significant differences when grouped by clinical forms (e.g. healthy control versus diagnosis for adipose tissue disorder, lipid homeostasis disorder and/or an impaired glucose tolerance or insulin resistance condition and/or related cardiovascular disease and healthy control versus chronic refractory to first line therapy).

### Determination of a Gene Expression Profile

The present invention is related to methods of detecting gene expression using a gene expression system having one or more array elements wherein the array elements comprise one or more sequence that corresponds to sequence selected from IRAK3, SOD2, TNFAIP6, TNFAIP3, TLR2, IRS2 or ZNF217 listed in this application or of natural sequence variations on these human genes or homologs and variants of the disclosed nucleic acid molecules, forming an array set. From IRAK3, SOD2, TNFAIP6, TNFAIP3, TLR2, IRS2 or ZNF217 listed in this application or of natural sequence variations on these human genes or homologs and variants of the disclosed nucleic acid molecules, it should be understood by one of ordinary skill in the art that standard methods of data analysis or using the disclosed methods (such as cluster analysis, K-nearest neighbors class prediction algorithms, or class prediction analysis using appropriately selected parameters) can be used to identify a smaller number of array elements, while still retaining the predictive characteristics of the array sets disclosed herein. Non-limiting examples of data analysis that may be used are listed below. In one embodiment, an array may be used to determine gene expression as described above. For example, PCR amplified inserts of cDNA clones may be applied to a substrate in a dense array. These cDNA may be selected from one or more of those sequences of IRAK3, SOD2, TNFAIP6, TNFAIP3, TLR2, IRS2 or ZNF217 listed in this application or of natural sequence variations on these human genes or homologs and variants of the disclosed nucleic acid molecules.
The probes, immobilized on the selected substrate, are suitable for hybridization under conditions with appropriately determined stringency, such that targets binding non-specifically to the substrate or array elements are substantially removed. Appropriately labeled targets generated from mRNA are generated using any standard method as known in the art. For example, the targets may be cDNA targets generated through incorporation of fluorescent nucleotides by reverse transcription of RNA extracted from tissues of interest. Alternatively, biotin labeled targets may be used, such as using the method described herein. It should be clear that any suitable oligonucleotide-based target may be used. In another embodiment, suitably labeled cRNA targets may be used. Regardless of the type of target, the targets are such that the labeled targets applied to the chip hybridize to complementary probes on the array. After washing to minimize non-specific binding, the chip may be scanned by confocal laser microscopy or by any other suitable detection method known in the art, for example, a CCD camera. Quantification of hybridization at each spot in the array allows a determination of corresponding mRNA expression. With dual color fluorescence, separately labeled cDNA targets generated from two sources of RNA are hybridized pairwise to the array. The relative abundance of the transcripts from the two sources corresponding to each specified gene can then be determined simultaneously. (See Schena et al., Proc. Natl. Acad. Sci. USA 93(2): 106 149 (1996)). Microarray analysis can be performed by commercially available equipment, following manufacturer's protocols, such as by using the Affymetrix GenChip technology, or Incyte's microarray technology, or using any other methods as known in the art.
Homologs and variants of these nucleic acid molecules such of IRAK3, SOD2, TNFAIP6, TNFAIP3, TLR2, IRS2 or ZNF217 listed in this application typically possess a relatively high degree of sequence identity when aligned using standard methods. Sequences suitable for use in the methods described herein have at least about 40-50, about 50-60, about 70-80, about 80-85, about 85-90, about 90-95 or about 95-100% sequence identity to the sequences disclosed herein. It is understood that for determination of a gene expression profile, variations in the disclosed sequences will still permit detection of gene expression. The degree of sequence identity required to detect gene expression varies depending on the length of the oligomer. For example, in a 60-mer, (an oligonucleotide with about 60 nucleotides), about 6 to about 8 random mutations or about 6 to about 8 random deletions in a 60-mer do not affect gene expression detection. Hughes, T R, et al. "Expression profiling using microarrays fabricated by an ink-jet oligonucleotide synthesizer. Nature Biotechnology, 19:343-347(2001). As the length of the DNA sequence is increased, the number of mutations or deletions permitted while still allowing gene expression detection is increased. As will be appreciated by those skilled in the art, the sequences of the present invention may contain sequencing errors. That is, there may be incorrect nucleotides, frame shifts, unknown nucleotides, or other types of sequencing errors in any of the sequences; however, the correct sequences will fall within the homology and stringency definitions herein.

### Additional Methods of Determining Gene Expression

The array sets disclosed herein may also be used to determine a gene expression profile such that a patient may be classified as a responder or a nonresponder any other techniques that measure gene expression. For example, the expression of genes disclosed in the array sets herein may be detected using RT-PCR methods or modified RT-PCR methods. In this embodiment, RT-PCR is used to detect gene expression of genes selected from one or more genes selected from of IRAK3, SOD2, TNFAIP6, TNFAIP3, TLR2, IRS2 or ZNF217 listed in this application or of natural sequence variations on these human genes or homologs and variants of the disclosed nucleic acid molecules Various methods using RT-PCR may be employed. For example, standard RT-PCR methods may be used. Using this method, well- known in the art, isolated RNA may be reverse transcribed using into cDNA using standard methods as known in the art. This cDNA is then exponentially amplified in a PCR reaction using standard PCR techniques. The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, Calif., USA), following the manufacturer's instructions. The derived cDNA can then be used as a template in the subsequent PCR reaction. Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide is designed to detect nucleotide sequence located between the two PCR primers. The third oligonucleotide is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the third oligonucleotide in a template-dependent manner. The resultant fragments dissociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data. TaqMan® RT-PCR can be performed using commercially available equipment, such as, for example, ABI PRISM 7700™ Sequence Detection System™ (Perkin-Elmer-Applied Biosystems, Foster City, Calif., USA), or Lightcycler (Roche Molecular Biochemicals, Mannheim, Germany). In a preferred embodiment, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data. To minimize errors and the effect of sample-to- sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3- phosphate-dehydrogenase (GAPDH) and β-actin, although any other housekeeping gene or other gene established to be expressed at constant levels between comparison groups can be used. Real time quantitative PCR techniques, which measure PCR product accumulation through a dual-labeled fluorigenic target (i.e., TaqMan® probe), may also be used with the methods disclosed herein to determine a gene expression profile. The Stratagene Brilliant SYBR Green QPCR reagent, available from 11011 N. Torrey Pines Road, La Jolla, CA 92037, may also be used. The SYBR® Green dye binds specifically to double-stranded PCR products, without the need for sequence-specific targets. Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986 994 (1996). Alternatively, a modified RT-PCR method such as express Profiling™ (XP) technology for high-throughput gene expression analysis, available from Althea Technologies, Inc. 11040 Roselle Street, San Diego, California 92121 U.S.A. may be used to determine a gene expression profiles of a patient diagnosed with metabolic syndrome. The gene expression analysis may be limited to one or more array sets as disclosed herein. This technology is described in U.S. Patent 6,618,679. This technology uses a modified RT-PCR process that permits simultaneous, quantitative detection of expression levels of about 20 genes. This method may be complementary to or used in place of array technology or PCR and RT-PCR methods to determine or confirm a gene expression profile, for example, when classifying the status of a patient as a responder or non-responder. Multiplex mRNA assays may also be used, for example, that described in Tian, et al., "Multiplex mRNA assay using Electrophoretic tags for high-throughput gene expression analysis," Nucleic Acids Research 2004, Vol. 32, No. 16, published online September 8, 2004 and Elnifro, et al. "Multiplex PCR; Optimization and Application in Diagnostic Virology," Clinical Microbiology Reviews, Oct. 2000, p. 559-570. In multiplex CR, more than one target sequence can be amplified by including more than one pair of primers In the reaction. Alternatively, Real-Time NASBA can be used. This is an isothermal nucleic acid amplification method which is particularly suited to detection and quantification of genomic, ribosomal or messenger RNA and which has been has proved to be the basis of sensitive and specific assays for detection, quantification and differentiation of RNA and DNA targets. The product of NASBA is single-stranded RNA of opposite sense to the original target. The fist developed NASBA methods relied on liquid or gel-based probe-hybridization for post-amplification detection of products. But more recently, real-time procedures incorporating amplification and detection in a single step have been reported and applied to a wide range of RNA and some DNA targets *(*Loens K. et al. Journal of microbiological methods. 2006, vol. 67, no3, pp. 408-415*,* Schneider et al. Real-time nucleic acid sequence-based amplification is more convenient than real-time PCR for quantification of Plasmodium falciparum. J Clin Microbiol. 2005;43(1):402-5 *and* Keightley et al. Real-time NASBA detection of SARS-associated coronavirus and comparison with real-time reverse transcription-PCR. J Med Virol. 2005;77(4):602-8*.)* Another alternative is that can be used is the one-step, reverse transcription loop-mediated amplification (RT-LAMP) assay. RT-LAMP is sensitive, rapid, specific, and cost effective, with the potential for field deployment and use in first line diagnosis. (Horibe, D., et al. Rapid detection of metastasis of gastric cancer using reverse transcription loop-mediated isothermal amplification. Int. J. Cancer (2007)

The methods disclosed herein employ a biological sample derived from patients diagnosed with metabolic syndrome. The samples may include, for example, tissue samples obtained by biopsy of endoscopically affected colonic segments including the cecum/ascending, transverse/descending or sigmoid/rectum; small intestine; ileum; intestine; cell lysates; serum; or blood samples. Colon epithelia cells and lamina propria cells may be used for mRNA isolation. Control biopsies are obtained from the same source. Sample collection will depend on the target tissue or sample to be assayed.
Immediately after collection of a biological sample, for instance the blood cell blood cells, blood monocyte preferable a myeloid cell from patients with adipose tissue disorder, lipid homeostasis disorder and/or an impaired glucose tolerance or insulin resistance condition and/or related cardiovascular disease or an healthy patient the sample may be placed in a medium appropriate for storage of the sample such that degradation of mRNA is minimized and stored on ice. For example, a suitable medium for storage of sample until processing is RNALater®, available from Applied Biosystems, 850 Lincoln Centre Drive, Foster City CA 94404, U.S.A. Total RNA may then be prepared from a target sample using standard methods for RNA extraction known in the art and disclosed in standard textbooks of molecular biology, including Ausubel et al., Current Protocols of Molecular Biology, John Wiley and Sons (1997). For example, RNA isolation can be performed using purification kit, buffer set and protease from commercial manufacturers, such as Qiagen, according to the manufacturer's instructions. In one embodiment, total RNA is prepared utilizing the Qiagen RNeasy mini-column, available from QIAGEN Inc., 27220 Turnberry Lane Suite 200, Valencia, CA 91355. Other commercially available RNA isolation kits include MasterPure™ Complete DNA and RNA Purification Kit (EPICENTRE@, Madison, Wis.), or Paraffin Block RNA Isolation Kit (Ambion, Inc.). Total RNA from tissue samples may also be isolated using RNA Stat-60 (Tel- Test). RNA may also be prepared, for example, by cesium chloride density gradient centrifugation. RNA quality may then be assessed. RNA quality may be determined using, for example, the Agilent 2100 Bioanalyzer. Acceptable RNA samples have distinctive 18S and 28S Ribosomal RNA Bands and a 28S/18S ribosomal RNA ratio of about 1.5 to about 2.0. In one embodiment, about 400 to about 500 nanograms of total RNA per sample is used to prepare labeled mRNA as targets. The RNA may be labeled using any methods known in the art, including for example, the TargetAmp 1-Round Aminoallyl-aRNA Amplification Kit available from Epicentre to prepare cRNA, following the manufacturer's instructions. The TargetAmp 1-Round Aminoallyl-aRNA Amplification Kit (Epicentre) is used to make double-stranded cDNA from total RNA. An in vitro transcription reaction creates cRNA target. Biotin-X-X-NHS (Epicentre) is used to label the aminoallyl-aRNA with biotin following the manufacturer's instructions. In one embodiment, the biotin-labeled cRNA target is then chemically fragmented and a hybridization cocktail is prepared and hybridized to a suitable array set immobilized on a suitable substrate. In another embodiment, the total RNA may be used to prepare cDNA targets. The targets may be labeled using any suitable labels known in the art. The labeled cDNA targets may then be hybridized under suitable conditions to any array set or subset of an array set described herein, such that a gene expression profile may be obtained.

### Normalization

Normalization is an adjustment made to microarray gene expression values to correct for potential bias or error introduced into an experiment. With respect to array-type analyses, such errors may be the result of unequal amounts of cDNA probe, differences in dye properties, differences in dye incorporation etc. Where appropriate, the present methods include the step of normalizing data to minimize the effects of bias or error. The type of normalization used will depend on the experimental design and the type of array being used. The type of normalization used will be understood by one of ordinary skill in the art.

### Levels of Normalization

There may be two types of normalization levels used with the methods disclosed herein: "within slide" (this compensates, for example, for variation introduced by using different printing pins, unevenness in hybridization or, in the case of two channel arrays, differences in dye incorporation between the two samples) or "between slides," which is sometimes referred to as "scaling" and permits comparison of results of different slides in an experiment, replicates, or different experiments.

### Normalization methods

Within slide normalization can be accomplished using local or global methods as known in the art. Local normalization methods include the use of "housekeeping genes" and "spikes" or "internal controls". "Housekeeping" genes are genes which are known, or expected, not to change in expression level despite changes in disease state or phenotype or between groups of interest (such as between known non- responders and responders). For example, common housekeeping genes used to normalize data are those that encode for ubiquitin, actin and elongation factors. Where housekeeping genes are used, expression intensities on a slide are adjusted such that the housekeeping genes have the same intensity in all sample assays. Normalization may also be achieved using spikes or internal controls that rely on RNA corresponding to particular probes on the microarray slide being added to each sample. These probes may be from a different species than the sample RNAs and optimally should not cross-hybridize to sample RNAs. For two channel arrays, the same amount of spike RNA is added to each sample prior to labeling and normalization is determined via measurement of the spiked features. Spikes can also be used to normalize spatially across a slide if the controls have been printed by each pin - the same controls on different parts of the slide should hybridize equally. Spikes may also be used to normalize between slides..
Reference samples may be any suitable reference sample or control as will be readily understood by one of skill in the art. For example, the reference sample may be selected from normal patients, "responder" patients, "non-responder" patients, or "chronic-refractory patients." Normal patients are those not diagnosed with an adipose tissue disorder, lipid homeostasis disorder and/or an impaired glucose tolerance or insulin resistance condition and/or related cardiovascular disease. "Responder" patients and "non-responder" patients are described above. "Chronic refractory" patients are patients with moderate to severe disease that require a second induction of remission using any drug. In one embodiment of the present invention, the control sample comprises cDNA from one or more patients that do not have adipose tissue disorder, lipid homeostasis disorder and/or an impaired glucose tolerance or insulin resistance condition and/or related cardiovascular disease. In this embodiment, the cDNA of multiple normal samples are combined prior to labeling, and used as a control when determining gene expression of experimental samples. The data obtained from the gene expression analysis may then be normalized to the control cDNA.
A variety of global normalization methods may be used including, for example, linear regression. This method is suitable for two channel arrays and involves plotting the intensity values of one sample against the intensity values of the other sample. A regression line is then fitted to the data and the slope and intercept calculated. Intensity values in one channel are then adjusted so that the slope = 1 and the intercept is 0. Linear regression can also be carried out using MA plots. These are plots of the log ratio between the Cy5 and Cy3 channel values against the average intensity of the two channels. Again regression lines are plotted and the normalized log ratios are calculated by subtracting the fitted value from the raw log ratio. In the alternative, Lowess regression (locally weighted polynomial regression) may be used. This regression method again uses MA plots but is a non-linear regression method. This normalization method is suitable if the MA plots show that the intensity of gene expression is influencing the log ratio between the channels. Lowess essentially applies a large number of linear regressions using a sliding window of the data. Yet another alternative method of normalization is "print tip normalization." This is a form of spatial normalization that relies on the assumption that the majority of genes printed with individual print tips do not show differential expression. Either linear or non-linear regression can be used to normalize the data. Data from features printed by different print tips are normalized independently. This type of normalization is especially important when using single channel arrays. Yet another method of normalization is "2D Lowess normalization." This form of spatial normalization uses a 2d polynomial Lowess regression that is fitted to the data using a false color plot of log ratio or intensity as a function of the position of the feature on the array. Values are adjusted according to this polynomial. "Between slide normalization" enables you to compare results from different slides, whether they are two channel or single channel arrays.
Centering and scaling may also be used. This adjusts the distributions of the data (either of log ratios or signal intensity) on different slides such that the data is more similar. These adjustments ensure that the mean of the data distribution on each slide is zero and the standard deviation is 1. For each value on a slide, the mean of that slide is subtracted and the resulting value divided by the standard deviation of the slide. This ensures that the "spread" of the data is the same in each slide you are comparing. Quantile normalization is yet another method that is particularly useful for comparing single channel arrays. Using this method, the data points in each slide are ranked from highest to lowest and the average computed for the highest values, second highest values and so on. The average value for that position is then assigned to each slide, i.e. the top ranked data point in each slide becomes the average of the original highest values and so on. This adjustment ensures that the data distributions on the different slides are identical. Various tools for normalizing data are known in the art, and include GenePix, Excel, GEPAS, TMeV/MIDAS and R.

### Hybridization Techniques

[Where array techniques are used to determine a gene expression profile, the targets must be hybridized to the array sets under suitable hybridization conditions using hybridization and wash solutions having appropriate stringency, such that labeled targets may hybridize to complementary probe sequences on the array. Washes of appropriate stringency are then used to remove non-specific binding of target to the array elements or substrate. Determination of appropriate stringency is within the ordinary skill of one skilled in the art. In one embodiment of the present invention, the array set is that of the Affymetrix Genechip Array (HGU133 Plus Version 2 Affymetrix GeneChip, available from Affymetrix, 3420 Central Expressway, Santa Clara, CA 95051). In this embodiment, suitably labeled cRNA and hybridization cocktail are first prepared. In this embodiment, the hybridization cocktail contains about 0.034 ug/uL fragmented cRNA, about 50 pM Control Oligonucleotide B2 (available from Affymetrix), 2OX Eukaryotic Hybridization Controls (1.5 pM bioB, 5 pM bioC, 25 pM bioD, 100 pM ere) (available from Affymetrix), about 0.1 mg/mL Herring Sperm DNA (Promega), about 0.5 mg/mL Acetylated BSA (Invitrogen). The hybridization cocktail is heated to 99°C for 5 minutes, to 45°C for 5 minutes, and spun at maximum speed in a microcentrifuge for 5 minutes. The probe array is then filled with 200 uL of IX Hybridization Buffer (available from Affymetrix) and incubated at 45°C for 10 minutes while rotating at 60 rpm. The IX Hybridization Buffer is removed and the probe array filled with 200 uL of the hybridization cocktail. The probe array is then incubated at 45°C for about 16 hours in a hybridization oven rotating at 60 rpm. The array is then washed and stained using any method as known in the art. In one embodiment, the Fluidics Station 450 (Affymetrix) and the fluidics protocol EukGE-WS2v4_450 is used. This protocol comprises the steps of a first post-hybridization wash (10 cycles of 2 mixes/cycle with Affymetrix Wash Buffer A at 25°C), a second post- hybridization wash (4 cycles of 15 mixes/cycle with Affymetrix Wash Buffer B at 50°C), a first stain (staining the probe array for 10 minutes with Affymetrix Stain Cocktail 1 at 25°C), a post-stain wash (10 cycles of 4 mixes/cycle with Affymetrix Wash Buffer A at 25°C), a second stain (stain the probe array for 10 minutes with Stain Cocktail 2 at 25°C), a third stain (stain the probe array for 10 minutes with Stain Cocktail 3 at 25°C) and a final wash (15 cycles of 4 mixes/cycle with Wash Buffer A at 30°C. The holding temperature is 25°C). All Wash Buffers and Stain Cocktails are those provided in the GeneChip® Hybridization, Wash and Stain Kit, Manufactured for Affymetrix, Inc., by Ambion, Inc., available from Affymetrix. In one embodiment, the stain used is R-Phycoerythrin Streptavidin, available from Molecular Probes. The antibody used is anti-streptavidin antibody (goat) biotinylated, available from Vector Laboratories.

### Data Collection and Processing

When using an array to determine a gene expression profile, the data from the array must be obtained and processed. The data may then be used for any of the purposes set forth herein, such as to predict the outcome of a therapeutic treatment or to classify a patient as a responder or nonresponder. Following appropriate hybridization and wash steps, the substrate containing the array set and hybridized target is scanned. Data is then collected and may be saved as both an image and a text file. Precise databases and tracking of files should be maintained regarding the location of the array elements on the substrates. Information on the location and names of genes should also be maintained. The files may then be imported to software programs that perform image analysis and statistical analysis functions. The gene expression profile of a patient of interest is then determined from the collected data. This may be done using any standard method that permits qualitative or quantitative measurements as described herein. Appropriate statistical methods may then be used to predict the significance of the variation in the gene expression profile, and the probability that the patient's gene expression profile is within the category of non-responder or responder. For example, in one embodiment, the data may be collected, then analyzed such that a class determination may be made (i.e., categorizing a patient as a responder or nonresponder) using a class prediction algorithm and GeneSpring™ software as described below. Expression patterns can be evaluated by qualitative and/or quantitative measures. Qualitative methods detect differences in expression that classify expression into distinct modes without providing significant information regarding quantitative aspects of expression. For example, a technique can be described as a qualitative technique if it detects the presence or absence of expression of a candidate nucleotide sequence, i.e., an on/off pattern of expression. Alternatively, a qualitative technique measures the presence (and/or absence) of different alleles, or variants, of a gene product. In contrast, some methods provide data that characterize expression in a quantitative manner. That is, the methods relate expression on a numerical scale, e.g., a scale of 0-5, a scale of 1-10, a scale of +- +++, from grade 1 to grade 5, a grade from a to z, or the like. It will be understood that the numerical, and symbolic examples provided are arbitrary, and that any graduated scale (or any symbolic representation of a graduated scale) can be employed in the context of the present invention to describe quantitative differences in nucleotide sequence expression. Typically, such methods yield information corresponding to a relative increase or decrease in expression. Any method that yields either quantitative or qualitative expression data is suitable for evaluating expression. In some cases, e.g., when multiple methods are employed to determine expression patterns for a plurality of candidate nucleotide sequences, the recovered data, e.g., the expression profile, for the nucleotide sequences is a combination of quantitative and qualitative data. In some applications, expression of the plurality of candidate nucleotide sequences is evaluated sequentially. This is typically the case for methods that can be characterized as low- to moderate- throughput. In contrast, as the throughput of the elected assay increases, expression for the plurality of candidate nucleotide sequences in a sample or multiple samples is assayed simultaneously.
Again, the methods (and throughput) are largely determined by the individual practitioner, although, typically, it is preferable to employ methods that permit rapid, e.g. automated or partially automated, preparation and detection, on a scale that is time-efficient and cost-effective.
It is understood that the preceding discussion is directed at both the assessment of expression of the members of candidate libraries and to the assessment of the expression of members of diagnostic nucleotide sets. Many techniques have been applied to the problem of making sense of large amounts of gene expression data. Cluster analysis techniques (e.g., K-Means), self-organizing maps (SOM), principal components analysis (PCA), and other analysis techniques are all widely available in packaged software used in correlating this type of gene expression data.

### Class Prediction

In one embodiment, the data obtained may be analyzed using a class prediction algorithm to predict whether a subject is a non-responder or a responder, as defined above. Class prediction is a supervised learning method in which the algorithm learns from samples with known class membership (the training set) and establishes a prediction rule to classify new samples (the test set). Class prediction consists of several steps. The first is a feature selection i.e. a process by which genes within a defined gene set are scored for their ability to distinguish between classes (responders and non-responders) in the training set. Genes may be selected for uses as predictors, by individual examination and ranking based on the power of the gene to discriminate responders from non-responders. Genes may then be scored on the basis of the best prediction point for responders or non-responders. The score function is the negative natural logarithm of the p-value for a hyper geometric test of predicted versus actual group membership for responder versus non-responder. A combined list for responders and non-responders for the most discriminating genes may then be produced, up to the number of predictor genes specified by the user. The Golub method may then be used to test each gene considered for the predictor gene set for its ability to discriminate responders from non-responders using a signal-to-noise ratio. Genes with the highest scores may then be kept for subsequent calculations. A subset of genes with high predictive strength may then used in class prediction, with cross validation performed using the known groups from the training set. The K-nearest neighbors approach may be used to classify training set samples during cross validation, and to classify test set samples once the predictive rule had been established. In this system, each sample is classified by finding the K-nearest neighboring training set samples (where K is the number of neighbors defined by the user) plotted based in Euclidean space over normalized expression intensity for each of the genes in the predictor set. For example, a predictive gene set of twenty members may be selected using four nearest neighbors. Depending on the number of samples available, the k value may vary. The class membership of the selected number of nearest neighbors to each sample is enumerated and p-values computed to determine the likelihood of seeing at least the observed number of neighbors from each class relative to the whole training set by chance in a K-sized neighborhood. With this method, the confidence in class prediction is best determined by the ratio of the smallest p-value and the second smallest p-value, termed the decision cut-off p-value. If it is lower, the test sample is classified as the class corresponding to the smallest p-value. If it is higher, a prediction is not made. In one embodiment, a decision cut-off p-value ratio of about 0.5 may be used. Cross validation in GeneSpring may then be then done by a drop-one-out algorithm, in which the accuracy of the prediction rule is tested. This approach removes one sample from the training set and uses it as a test sample. By predicting the class of a given sample only after it is removed from the training set, the rule makes unbiased prediction of the sample class. Once performance of the predictive rule has been optimized in this fashion, it may be tested using additional samples.

### Cluster Analysis

Cluster analysis is a loose term covering many different algorithms for grouping data. Clustering can be divided into two main types: top-down and bottom-up. Top-down clustering starts with a given number of clusters or classes and proceeds to partition the data into these classes. Bottom-up clustering starts by grouping data at the lowest level and builds larger groups by bringing the smaller groups together at the next highest level's-Means is an example of top-down clustering. K-means groups data into K number of best-fit clusters. Before using the algorithm, the user defines the number of clusters that are to be used to classify the data (K clusters). The algorithm randomly assigns centers to each cluster and then partitions the nearest data into clusters with those centers. The algorithm then iteratively finds new centers by averaging over the data in the cluster and reassigning data to new clusters as the centers change. The analysis iteratively continues until the centers no longer move (Sherlock, G., Current Opinion in Immunology, 12:201, 2000).Tree clustering is an example of bottom-up clustering. Tree clustering joins data together by assigning nearest pairs as leaves on the tree. When all pairs have been assigned (often according to either information-theoretical criteria or regression methods), the algorithm progresses up to the next level joining the two nearest groups from the prior level as one group. Thus, the number and size of the clusters depends on the level. Often, the fewer clusters, the larger each cluster will be. The stoppage criteria for such algorithms varies, but often is determined by an analysis of the similarity of the members inside the cluster compared to the difference across the clusters. Self-organizing maps (SOMs) are competitive neural networks that group input data into nearest neighbors (Torkkola, K., et al., Information Sciences, 139:79, 2001; Toronen, P., et al., FEBS Letters, 451:142 146, 1999). As data is presented to the neural network, neurons whose weights currently are capable of capturing that data (the winner neuron) are updated toward the input. Updating the weights, or training the neural net, shifts the recognition space of each neuron toward a center of similar data. SOMs are similar to K-means with the added constraint that all centers are on a 1 or 2 dimensional manifold (i.e., the feature space is mapped into a 1 or 2 dimensional array, where new neighborhoods are formed). In SOM, the number of neurons is chosen to be much larger than the possible number of the clusters. It is hoped that the clusters of trained neurons will provide a good estimation of the number of the neurons. In many cases, however, a number of small clusters are formed around the larger clusters, and there is no practical way of distinguishing such smaller clusters from, or of merging them into, the larger clusters. In addition, there is no guarantee that the resulting clusters of genes actually exhibit statistically independent expression profiles. Thus, the members of two different clusters may exhibit similar patterns of gene expression. Principal component analysis (PCA), although not a clustering technique in its nature (Jolliffe, I. T., Principal Component Analysis, New York: Springer-Verlag, 1986) can also be used for clustering (Yeung, K. Y., et al., Bioinformatics, 17:763, 2001). PCA is a stepwise analysis that attempts to create a new component axis at each step that contains most of the variation seen for the data. Thus, the first component explains the first most important basis for the variation in the data, the second component explains the second most important basis for the variation in the data, the third component the third most important basis, and so on. PCA projects the data into a new space spanned by the principal components. Each successive principal component is selected to be orthogonal to the previous ones, and to capture the maximum information that is not already present in the previous components. The principal components are therefore linear combinations (or eigenarrays) of the original data. These principal components are the classes of data in the new coordinate generated by PCA. If the data is highly non-correlated, then the number of significant principal components can be as high as the number of original data values. If, as in the case of DNA microarray experiments, the data is expected to correlate among groups, than the data should be described by a set of components which is fewer than the full complement of data points. A variety of systems known in the art may be used for image analysis and compiling the data. For example, where the mRNA is labeled with a fluorescent tag, and fluorescence imaging system (such as the microarray processor commercially available from AFFYMETRIX®, Santa Clara, Calif.) may be used to capture, and quantify the extent of hybridization at each address. Or, in the case where the mRNA is radioactive, the array may be exposed to X-ray film and a photographic image made. Once the data is collected, it may be compiled to quantify the extent of hybridization at each address as for example, using software to convert the measured signal to a numerical value. Any publicly available imaging software may be used. Examples include BioDiscovery (ImaGene), Axon Instruments (GenePix Pro 6.0), EisenLab -Stanford University (ScanAlyze), Spotfinder (TIGR), Imaxia (ArrayFox), F-Scan (Analytical Biostatistics Section - NIH), MicroDiscovery (GeneSpotter), CLONDIAG(IconoClust), Koada Technology (Koadarray), Vigene Tech (MicroVigene), Nonlinear Dynamics (Phoretix), CSIRO Mathematical and Information Sciences (SPOT) Niles Scientific (SpotReader).Any commercially available data analysis software may also be used. Examples include, BRB Array Tools (Biometric Research Branch - NCI), caGEDA (University of Pittsburgh), Cleaver 1.0 (Stanford Biomedical Informatics), ChipSC2C ( Peterson Lab - Baylor College of Medicine), Cluster (Eisen Lab - Stanford/UC Berkeley), DNA-Chip Analyzer (dChip) (Wong Laboratory - Harvard University), Expression Profiler (European Bioinformatics Institute), FuzzyK (Eisen Lab - Stanford/UC Berkeley), GeneCluster 2.0 (Broad Institute), GenePattern (Broad Institute), GeneXPress (Stanford University), Genesis (Alexander Sturn - Graz University of Technology), GEPAS (Spanish National Cancer Center), GLR (University of Utah), GQL (Max Planck Institute for Molecular Genetics), INCLUSive (Katholieke Universiteit Leuven), Maple Tree (Eisen Lab - Stanford/UC Berkeley) MeV (TIGR) MIDAS (TIGR), Onto-Tools (Sorin Draghici - Wayne State University), Short Time- series Expression Miner (Carnegie Mellon University), Significance Analysis of Microarrays (Rob Tibshirani - Stanford University), SNOMAD (Johns Hopkins Schools of Medicine and Public Health), SparseLOGREG (Shevade & Keerthi - National University of Singapore), SuperPC Microarrays (Rob Tibshirani - Stanford University), TableView (University of Minnesota), TreeView (Eisen Lab - Stanford/UC Berkeley), Venn Mapper (Universitais Medisch Centrum Rotterdam), Applied Maths (GeneMaths XT), Array Genetics (AffyMate), Axon Instruments (Acuity 4.0) BioDiscovery (GeneSight), BioSieve (ExpressionSieve), CytoGenomics (SilicoCyte), Microarray Data Analysis (GeneSifter), MediaCybernetics (ArrayPro Analyzer), Microarray Fuzzy Clustering (BioRainbow), Molmine (J-Express Pro), Optimal Design (ArrayMiner), Partek (Partek Pro) Predictive Patterns Software (GeneLinker), Promoter Extractor (BioRainbow) SAS Microarray Silicon Genetics (GeneSpring), Spotfire (Spotfire), Strand Genomics (Avadis) Vialogy Corp. It should also be understood that confounding factors may exist in individual subjects that may affect the ability of a given gene set to predict responders versus non-responders. These cofounding variables include variation in medications, such as cases in which concurrent 6- MP with infliximab overcomes the adverse effects of an unfavorable FasL polymorphism on response, the CARD15 genotype status, or the location of the biopsy, due to variation of gene expression along the colon. To account for this variation, outliers may be identified, and subsequently determined whether the outliers may be accounted for by variations in medication use, CARD15 genotype, or the location of the colon biopsy.

### Kits

In an additional aspect, the present invention provides kits embodying the methods, compositions, and systems for analysis of gene expression as described herein. Kits of the present invention may comprise one or more of the following: a) at least one pair of universal primers; b) at least one pair of target-specific primers, wherein the primers are specific to one or more sequences listed in Tables 4-8 or the sequence listing; c) at least one pair of reference gene-specific primers; and d) one or more amplification reaction enzymes, reagents, or buffers. The universal primers provided in the kit may include labeled primers. The target-specific primers may vary from kit to kit, depending upon the specified target gene(s) to be investigated, and may also be labeled. Exemplary reference gene-specific primers (e.g., target-specific primers for directing transcription of one or more reference genes) include, but are not limited to, primers for β-actin, cyclophilin, GAPDH, and various rRNA molecules. The kits of the invention optionally include one or more preselected primer sets that are specific for the genes to be amplified. The preselected primer sets optionally comprise one or more labeled nucleic acid primers, contained in suitable receptacles or containers.
Exemplary labels include, but are not limited to, a fluorophore, a dye, a radiolabel, an enzyme tag, etc., that is linked to a nucleic acid primer itself. In addition, one or more materials and/or reagents required for preparing a biological sample for gene expression analysis are optionally included in the kit. Furthermore, optionally included in the kits are one or more enzymes suitable for amplifying nucleic acids, including various polymerases (RT, Taq, etc.), one or more deoxynucleotides, and buffers to provide the necessary reaction mixture for amplification. In one embodiment of the invention, the kits are employed for analyzing gene expression patterns using mRNA as the starting template. The mRNA template may be presented as either total cellular RNA or isolated mRNA. In other embodiments, the methods and kits described in the present invention allow quantification of other products of gene expression, including tRNA, rRNA, or other transcription products. In still further embodiments, other types of nucleic acids may serve as template in the assay, including genomic or extragenomic DNA, viral RNA or DNA, or nucleic acid polymers generated by non-replicative or artificial mechanism, including PNA or RNA/DNA copolymers. Optionally, the kits of the present invention further include software to expedite the generation, analysis and/or storage of data, and to facilitate access to databases. The software includes logical instructions, instructions sets, or suitable computer programs that can be used in the collection, storage and/or analysis of the data. Comparative and relational analysis of the data is possible using the software provided.

### Examples

### STUDIES IN OBESE WOMEN

### Study design, materials and methods

We studied 25 healthy controls, and 35 successive obese (BMI > 35 kg/m² + co-morbidities) women without diabetes, and 60 obese women with diabetes. Fourteen obese women without diabetes were referred to our hospital for bariatric surgery. Before they were included, all women were evaluated by an endocrinologist, an abdominal surgeon, a psychologist and a dietician. Only after multidisciplinary deliberation the selected patients received a gastric bypass. This is a procedure in which the stomach is divided into a small, proximal pouch and a separate, large, distal remnant. The upper pouch is joined to the proximal jejunum through a narrow gastrojejunal anastomosis (Roux-en-Y configuration). The storage capacity of the stomach is reduced to approximately 5 % of its normal value, and ingested food bypasses approximately 95 % of the stomach, the entire duodenum, and a small portion (15-20 cm) of the proximal jejunum. Patients typically lose 35-40 % of total body weight and most of this is maintained for at least 10-15 years ⁵⁷⁻⁵⁹.
All participants did not have symptoms of clinical cardiovascular disease. However, their predicted cardiovascular risk based on their Framingham Risk Score (FRS) was elevated. This score was calculated according to Wilson⁶⁰ using the MedCalc (http://www.medcalc.com/heartrisk.html) program. Blood samples were collected, and peripheral blood mononuclear cells (PBMC) were prepared from the anti-coagulated blood using gradient separation on Histopaque-1077 performed directly in the blood collection tubes. Cells were washed three times in Ca²⁺ and Mg²⁺-free Hanks's balanced salt solution. PBMC were incubated for 20 min at 4°C with CD14 microbeads at 20 µl/1 × 10⁷ cells. The cells were washed once, resuspended in 500 µl Ca²⁺ and Mg²⁺-free PBS containing 5% FBS/1 × 10⁸ cells. The suspension was then applied to MidiMACS Separator (Miltenyi, Bergisch Gladbach, Germany) ^{61,62}. Blood samples were also centrifuged to prepare plasma samples for analysis. Total and HDL-cholesterol and triglyceride levels were determined with enzymatic methods (Boehringer Mannheim, Vilvoorde, Belgium). LDL-cholesterol levels were calculated with the Friedewald formula. Free fatty acids (FFA) were measured with a FFA-Half Microtest kit (Roche Applied Science). Plasma glucose was measured with the glucose oxidase method (on Vitros 750XRC, Johnson & Johnson), and insulin with an immunoradiometric assay (Biosource Technologies, Inc., Fleurus, Belgium). Oxidized LDL (oxLDL) and interleukin-6 (IL6) were measured with ELISA. Blood pressure was taken three times with the participant in a seated position after 5 minutes quiet rest. The average of the last two measurements was used for systolic and diastolic blood pressure.
Total RNA was extracted from 5.3 x 10⁶± 1.7 x 10⁶ (mean±s.d.) monocytes per person with the Trizol reagent (InVitrogen, Merelbeke, Belgium) and purified on an RNeasy Mini Kit column (Qiagen, Antwerpen, Belgium). Extraction yielded 6.5±2.8 µg of RNA. After phenol-chloroform extraction and RNeasy Mini Kit cleanup of the RNA, its quality was assessed with the RNA 6000 Nano assay kit using the Agilent 2100 Bioanalyzer. The adjusted S28/S18 ratios of extracted RNA were 1.60±0.07. First-strand cDNA was generated from total RNA by RT using random primers from Takara and Superscript III reverse transcriptase (InVitrogen). RNA expression levels were expressed as percentage of controls as previously described ⁶³.
RNA isolated from monocytes of obese women and non obese controls was analyzed on Illumina's Sentrix Human-6 v2 Expression BeadChip Kit containing around 46,713 probes/array targeting genes and known alternative splice variants from the RefSeq database release 17 and UniGene build 188. RNA was labeled, hybridized and scan according to Illumina GLP standards by Aros AB laboratory (Aarhus, Denmark). The raw data were normalized with the rank invariant method (Illumina BeadStudio V2). This method uses a linear scaling of the populations being compared. The scaling factor is determined by rank-invariant genes. 'Rank-invariant' genes are those genes whose expression values show a consistent order relative to other genes in the population. Of the 46,713 transcripts, 1,725 transcripts which were mapped in the Ingenuity Pathway Analysis (IPA) program 5.5-802 were differentially expressed in monocytes of obese women compared to controls at the P-value <= 0.05 level. Out of these genes, we selected a subpopulation of the 592 genes with a ratio threshold of + 1.2. The Ingenuity V5 Pathway analysis program (IPA 5.0) was used to model the canonical and metabolic function pathways that may be activated in the monocytes of obese women compared to controls. Networks were built by means of the "Connect" and the "Path explorer" tool in IPA 5.0. Canonical pathways were derived using the "Analysis" tool in IPA 5.0.

Quantitative real-time PCR was performed on a 7500 fast Real-Time PCR system using the power SYBR Green Master mix (Applied Biosystems, Lennik, Belgium). Table 1 summarizes forward and reverse primers used in RT-PCR analysis. The RNA expression level was calculated with the threshold cycle (C*ₜ*) value, i.e., the number of PCR cycles at which the fluorescent signal reaches a fixed threshold. For each sample, both the C*ₜ* for the gene of interest and for the housekeeping gene β-actin were determined to calculate Δ*C*_{*t*,sample} (*C*_{*t*,target gene} - *C*_{*t*,housekeeping gene}), thus normalizing the data and correcting for differences in amount and/or quality among the different RNA samples. The expression levels were related to an external calibrator. Subsequently, ΔΔ*Cₜ* (Δ*C*_{*t*,sample} - Δ*C*_{*t*,calibrator}) was determined, and the relative expression levels were calculated from 2^{-ΔΔ*Ct*}, according to the manufacturer's instructions (Applied Biosystems). RNA expression levels were expressed as percentage of controls as previously described ⁶⁴.
MM6 and THP-1 cells were cultured in RMPI-1640 medium containing 10% FBS (low LPS), 0,2 mM Streptomycin, 100 U/ml Penicillin, 2 mM L-glutamine, 1x MEM NEAA, 1 mM MEM sodium Pyruvate Solution. At day 1, cells were seeded in a 24 well plate at a cell density of 1 x 10⁶ cells/ml. At day 2, we replaced growth medium with complete growth medium (- FBS) + HSA (1mg/ml). At day 3 we added ox-LDL (50 µg/ml) or FFA (710.9 µM final concentration) or placebo. At day 4, cells were collected, washed and RNA was extracted as described above. IRAK3 RNA expression was assessed as described above. Groups were compared with unpaired t-test with Welch's correction (two groups) or with paired t-test (GraphPad 5.0). Pearson correlation coefficients were calculated to determine the relations between gene expressions and of gene expressions with risk factors. Receiver operating characteristic (ROC) curve analysis was performed using the MedCalc program. Fisher exact test analysis was performed to determine relative risks, sensitivity and specificity, and positive and negative predictive values using cut-offs which were determined by ROC curve analysis. A *P*-value of less than 0.05 was considered statistically significant. Principal component analysis (PCA) was performed using the TMEV 4-2 program ⁶⁵. Data are normalized in TMEV4 and the 119 patients are clustered according to the values of the 6 to 10 variables selected with the Hierarchical Clustering (HCL) with the average linkage method on Euclidean distances ^{66, 67} Tree leaves ordering were optimized using the "Gene Leaf Order" option. The hierarchical trees obtained are shown in figure 10. A principal Component Analysis (PCA) is then used to attribute the overall variability in the data to a reduced set of variables termed principal components⁶⁸. The centering mode is the median and the number for neighbors for KNN imputation is 10. (Available *at* *http:*//*smiweb.stanford.edu*/*pubs*/*SMI_Abstracts*/*SMI-1999-0804.html*).

### Characteristics of study cohort and effect of weight loss

We studied 25 healthy lean control women (age: 39±7 years), and 95 obese women (age: 51±11, P<0.001). Figure 1 shows that obese women had a higher BMI associated with higher FRS. Leptin concentrations were higher; adiponectin was lower. Glucose, insulin, and triglyceride concentrations were higher; HDL-cholesterol was lower. LDL-cholesterol levels were not different. Plasma concentrations of oxLDL (50±20 vs. 32±10 U/I; P<0.001) and interleukin-6 (5.4±2.9 vs. 2.7±0.94 pg/ml) were also higher.
Among obese women 35 were without diabetes (age: 45±12, P<0.05; BMI: 41.0.3±5.9, P<0.0001 vs. controls); 60 women had diabetes (age: 55±9, P<0.001 vs. controls and vs. obese women without diabetes; BMI: 37.2±4.8; P<0.0001 vs. controls, and P<0.05 vs. obese without diabetes). Figure 2 shows that diabetics had lower BMI but higher FRS. Their leptin concentrations were higher; adiponectin was not different. Diabetics had higher glucose, insulin, and triglyceride concentrations; their HDL-cholesterol was lower. Their LDL-cholesterol was lower due to more frequent use of statins (73% vs. 24% of women without diabetes).
Controls had lower systolic and diastolic blood pressure (121±11, 71±9.3 mm_{Hg}) than obese women without (135±18, 88±12 mm_{Hg}; P<0.01 and <0.001 vs. controls) and obese women with diabetes (139±16, 82±10 mm_{Hg}; P<0.001 vs. controls). Diabetics were more often treated for arterial hypertension (57%) than obese women without diabetes (42%). OxLDL concentrations in diabetics were lower (46±17 vs. 57±23 U/I; P<0.05) likely due to more frequent statin use and associated lower LDL-cholesterol. Interleukin-6 concentrations were not different.
We collected samples from 14 out of 35 obese women without diabetes after bariatric surgery. Their BMI was 17% lower 3 months after bariatric surgery (Figure 3). Their FRS predicted a decrease of the cardiovascular risk to 4±3% (compared to 8±7% before weight loss; P<0.01).
Weight loss was associated with a significant decrease of leptin, glucose, insulin, and triglyceride concentrations, and of systolic and diastolic blood pressure (118±4, 61±3 mm_{Hg}; P<0.001 vs. before). Adiponectin levels were higher. Weight loss did not affect LDL- and HDL-cholesterol, oxLDL and interleukin-6 concentrations.

### Microanalysis of RNA extracts of blood monocytes of obese women

We performed microarray analysis of RNA extracts from monocytes of 14 obese women. Table 2 summarizes the most significant gene functions of the 592 genes which were overexpressed compared to age-matched controls (N=10). Especially genes which mediate cell-to-cell signaling and immune response were overexpressed. They are known to be involved in hematological, immunological, neurological diseases, and in cancer. Figure 4 summarizes the top ten canonical signaling pathways which were identified by means of Ingenuity Pathways Analysis (IPA 5.0) program. The acute phase response, the interleukin, the NFκB, the LXRL/RXR, and the PPAR signaling pathways were among them.

### Proposal of a structural network

In order to identify the most representative structural network the following software tools were used together with the IPA program:

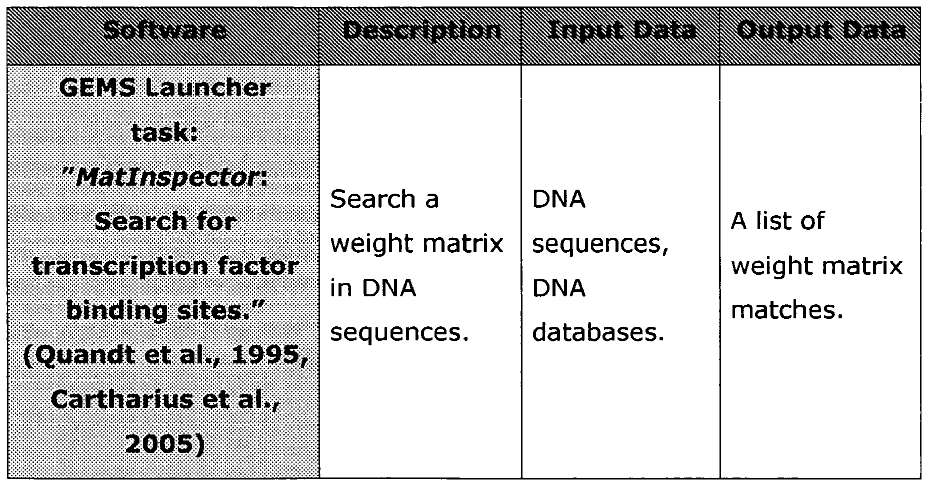

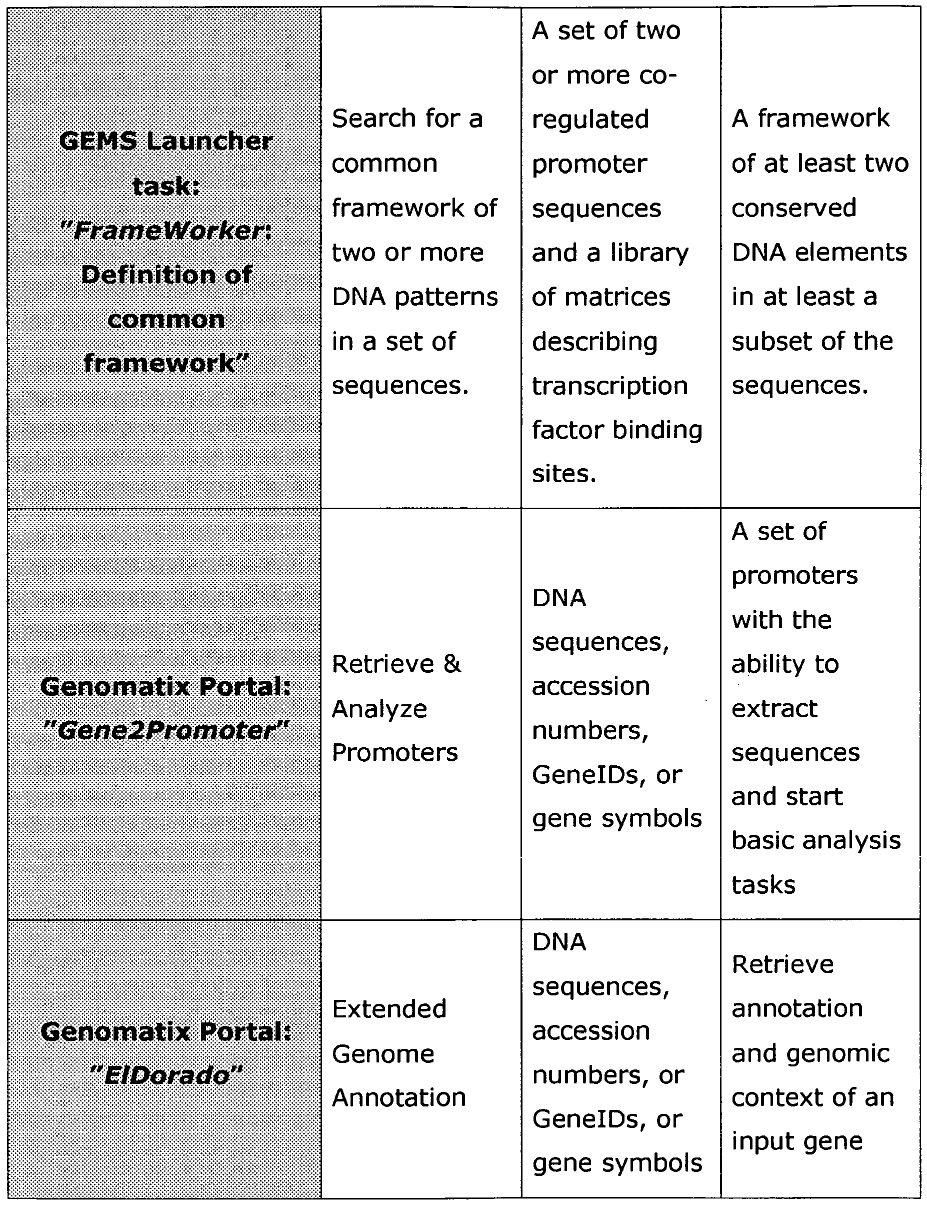

Figure 5 shows a predicted structural model in which two pathways interact:
- Activation of the TLR2 receptor pathway by free fatty acids (FFA) and oxLDL, which are elevated in blood of obese persons, occurs through activation of MYD88 and TRAF6 resulting in the activation of NFκB and associated expression of the inflammatory TNF and IL-1 which contribute to oxidative stress and release of ROS ⁶⁹. TRAF6 induces the expression and the activation of NFκB that then further induces the expression of TLR2. In support of this model, several NFκB-binding sites have been identified of the promoter of TLR2 (figure 6). This feedback may be used to couple the TLR2-pathway of innate immune response to other NFκB activating response mechanisms thereby generally amplifying the immune response. ROS activate directly NFκB. This pathway can be inhibited at two sites. IRAK3 is inhibiting TRAF6 ⁷⁰; TNFAIP3 ⁷¹ and TNFAIP6 ⁷² are inhibiting NFκB.
- Insulin through interaction with its receptor (INSR) and IRS2 activates the PI3K / AKT pathway. This activation is required for proper insulin action. ROS induces the expression of MAPK13 that blocks proper insulin action ⁷³. ROS induces the expression of the forkhead transcription factor FOXO3A that induces the expression of SOD2. FOXO3A, like NFκB, is regulated at the level of nuclear transport. In the nucleus FOXO3A blocks NFκB ⁷⁴. Promoter analysis suggested that FOXO3A is also involved in the regulation of TRAF6 and IRAK3. Indeed, a forkhead binding site was identified within a strongly conserved TFBS model shared by two promoters for these genes. Activation of the PI3K / AKT pathway results in the phosphorylation of FOXO3A, thereby blocking its transport into the nucleus. This can result in activation of NFκB, and the repression of the expression of SOD2 (directly), and of IRAK3, TRAF6, and TNFAIP3 (indirectly). This inactivation of FOXO3A can be reverted by phosphorylation of FOXO3A at another site by MAPK13 ⁷⁵ thereby restoring SOD2 expression. As discussed above, SOD2 is essential for neutralizing ROS. We postulated that MAPK13 is an important link between the two pathways, other than that it affects FOXO3A activity, because it contains common transcription factor binding sites (TFBS) with a gene upstream of NFκB, i.e. TNFAIP3, and two genes downstream of NFκB, i.e. MAPK13 and SOD2 (figure 7).

Although the indicated references suggested separate interactions proposed in the model, the complete model has not been presented in its present form and its relationship with obesity and related disorders has never been validated.

We also used the IPA program for searching other correlating proteins which may be important regulators of the expression of the identified cluster. We identified ZNF217 as a possible candidate.

### Validation of the structural model:

We measured the RNA expression of TLR2, MYD88, TRAF6, IRAK3, TNFAIP3, TNFAIP6, and IRS2, MAPK13, FOXO3A, SOD2, and ZNF217 in extracts of monocytes from healthy controls, and obese women without and with diabetes. Figure 8 shows that the expressions of TLR2, TNFAIP3, TNFAIP6, MAPK13, FOXO3A, and SOD2 were higher in monocytes of obese women; the expressions of IRAK3, IRS2 and ZNF217 were lower. Those of MYD88 and TRAF6 were not different. Lack of overexpression of MYD88 and TRAF6 does not exclude their activation. In support of the latter, we observed an 18±4.2% (P<0.01) increase in NFκB in monocytes from obese women compared to controls. This increase was observed in spite of the increase in TNFAIP3, TNFAIP6, and of FOXO3A, which are inhibitors of NFκB. The increase in NFκB was observed despite the unexpected increase of the NFκB inhibitor (39.4±6.0% higher in monocytes from obese women; P<0.001). Although we did not find an upregulation of MYD88, we observed an indirect evidence of the action of MYD88. Recent insights ⁷⁶ have revealed additional functions for MYD88 apart from NFκB activation, including activation of the transcription factors IRF1, IRF5 and IRF7, and also a role outside the TLRs in interferon-γ signaling. The expression of IRF1 was 94±6.3% higher in monocytes from obese women.

According to the proposed model, the increase in MAPK13 can explain why the increase in FOXO3A, despite the increase in NFκB, was associated with an increase in SOD2.

According to the proposed model **IRAK3** was the only inhibitor of the TLR2 pathway of which the expression was significantly lowered suggesting a functional role of IRAK3 in the regulation of this pathway. The expressions of the other predicted inhibitors TNFAIP3 and TNFAIP6 were elevated. In spite of their increases we observed an activation of the TLR2 pathway. These data suggest that TNFAIP3 and TNFAIP6 are markers of stress which cannot prevent stress. Despite the increase of the antioxidant SOD2 we observed an increase in oxLDL suggesting that SOD2 is also a marker of stress that by itself can not prevent oxidative stress.
The expression of ZNF217 was lower in obese women than in lean controls: 0.73±0.092 vs. 0.93±0.03 (P<0.0001).

### Determination of diagnostic values

To determine the value of the above mentioned genes to discriminate between lean control and obese women, we performed ROC curve analysis. Table 3 indicates that SOD2, TNFAIP6, TNFAIP3, IRAK3, TLR2, ZNF217, IRS2, and MAPK13 (ranked according to their areas under the curve) significantly discriminated between control and obese women; MYD88, FOXO3A, and TRAF6 did not.
In aggregate, we identified a cluster of genes containing IRAK3, SOD2, TNFAIP6, TNFAIP3, TLR2, ZNF217, IRS2, and MAPK13 with diagnostic value. Importantly, the expressions of these genes were not different between obese women with and with no diabetes, indicating that they are differentially regulated early in the onset of the above mentioned disorders.
As an alternative, we determined the relative risks, sensitivities and specificities, and positive and negative predictive values by Fisher exact test analysis. Low IRAK3 and ZNF217, and high SOD2, TNFAIP6, TNFAIP3 discriminated between lean controls and obese women (Table 4). The odds ratios of TLR2, MAPK13 and IRS2 were lower. We then investigated whether IRAK3, SOD2, TNFAIP6, TNFAIP3, and ZNF217 had an additive diagnostic value. Table 4 shows that the sensitivity of the combination of low IRAK3 and high SOD2 was 76%; specificity was 86%. Similar values were obtained when combining IRAK3 with TNFAIP3, TNFAIP6 or ZNF217. This was expected because of the high correlation between SOD2, TNFAIP3, and TNFAIP6 (correlation coefficients 0.88 and 0.83, respectively; P<0.001). Although ZNF217 correlated with TNFAIP3, TNFAIP6 and SOD2, the correlation coefficients were lower (Table 5). In contrast IRAK3 did not correlate with any of the 3 genes. Adding TNFAIP6 and TNFAIP3 to the IRAK3 - SOD2 pair increased sensitivity to 95% or above and increased specificity 76%. Similar values were obtained when replacing SOD2 with ZNF217. Further additions of genes did not significantly increase sensitivities and specificities. Table 6 shows the correlation between gene expressions and risk factors.
We also investigated if statin treatment had an effect on the discrimination between controls and obese persons. Table 7 shows the outcome of the ROC curve analysis for obese persons who were not treated with a cholesterol lowering statin compared to lean controls; table 8 shows the outcome for obese persons who were treated with a statin. SOD2, TNFAIP3, TNFAIP6, IRAK3, TLR2, and IRS2 discriminated between lean controls and obese persons whether they were treated or not with a statin; MAPK13 did not when treated with statin.
Table 9 shows the outcome of the Fisher exact test analysis comparing lean controls with obese persons who were not on statin therapy. Table 10 shows the analysis for obese persons on statin therapy. Overall, there were no significant differences.
To further assess the relation between gene expression and obesity, we measured the expressions of those genes in monocytes from obese women before and after weight loss. We demonstrated above that weight loss was associated with a significant decrease of their predicted risk as indicated by their lower FRS. Figure 9 shows that the expressions of SOD2, TNFAIP3, and TNFAIP6 decreased after weight loss; the expressions of IRAK3 increased. The expression of ZNF217 increased from 0.68±0.04 before to 0.98±0.09 after weight loss.
Table 11 shows the association between gene signatures in circulating monocytes and the occurrence of the metabolic syndrome. It shows that prediction of the metabolic syndrome improves by combining genes from the cluster particularly IRAK3, SOD2, TNFAIP3, TNFAIP6, and ZNF217. The relative risks of having the metabolic syndrome increase from 1.2 (when the RNA expression level of 1 gene is different from control; cut-point determined by ROC analysis) to 6.8 (when the RNA expression level of at least three genes are different from control). The clinical sensitivities (the percentages of positive tests when the clinical disorder is present) increase from 52% (when the RNA expression level of 1 gene is positive) to 97% (when the RNA expression level of at least three genes are positive). In the current cohort, ROC analysis did not show a significant relationship between ZNF217 expression and cardiovascular risk. Therefore, ZNF217 was as yet not included in further analyses.
Table 12 shows the association between gene signatures in circulating monocytes and the present cardiovascular risk equivalents. It shows that the risk prediction improves by combining genes from the cluster particularly IRAK3, SOD2, TNFAIP3, and TNFAIP6. The relative risks increase from 1.4 (when the RNA expression level of 1 gene is different from control; cut-point determined by ROC analysis) to 7.2 (when the RNA expression level of at least three genes are different from control); the sensitivities increase from 52% to 95%. The positive predictive values can be as high as 80%. The negative predictive values can be as high as 92%.
In addition to ROC and Fisher exact analysis, we performed PCA analysis to reduce the number of dimensions to 3 per 119 (number of patients) to map the distance between lean controls, obese persons without cardiovascular risk equivalents, and obese persons with cardiovascular risk equivalents. These are: an at least 10% risk of developing a cardiovascular disease within the next 10 years based on Framingham scoring and/or diabetes and or metabolic syndrome (at least 3 metabolic syndrome or MetSyn components as defined above).This type of analysis allows determining the additive value of emerging biomarkers on top of established risk factors: high BMI (obesity), high glucose (diabetes), high blood pressure (hypertension), and low HDL cholesterol and/or high triglycerides (dyslipidemia), and low adiponectin. Addition of RNA expression data for genes from the cluster particularly IRAK3, SOD2, TNFAIP3, and TNFAIP6 to the metabolic syndrome components together with adiponectin improved clearly the hierarchical clustering and the PCA representation of the 3 groups. Not only the lean controls (green) were separated better from the obese persons at low risk (yellow), but also the obese persons at low risk were separated better from the obese persons at high risk (red).

### Assessment of FFA and oxLDL on the expression of IRAK3 in a human monocyte cell line

As an example of a relevant human monocytic cell line we have chosen the MM6 cell. If we put the expression of IRAK3 at baseline at 100 %, then the expression in the presence of FFA at a concentration equal to the mean FFA concentration in the plasma of obese persons before weight loss was 80%. The expression in the presence of in vitro oxidized LDL (50 µg/ml) was 49%. As another example of a human monocyic cell line we have used THP-1 cells. Incubation of THP-1 cells with oxidized LDL reduced IRAK3 expression with 58%.

### Examples of tests for diagnosis and prognosis of metabolic syndrome disorders and cardiovascular diseases and for assessing the effect of interventions

A first approach for determining mRNA expression of specific cells in suspension combines flow cytometry with in situ hybridization ⁷⁷. This method maintains cellular integrity since it does not require the extraction and amplification of mRNA. Furthermore, it allows for the detection of the cellular source of the message in a heterogeneous population of cells thus avoiding monocyte isolation from whole blood. This way, the method provides the advantage to avoid 2 laborious and time-consuming steps. Indeed, the combination of flow cytometry with in situ hybridization makes a rapid analysis of large numbers of cells at a rate of approx 2000 cells/s feasible. Further, multicolor flow cytometry allows the resolution of very complex analytical mixtures. A schematic overview of the method is shown in figure 12. A small volume of blood is drawn from the patient. In a first step, monocytes in whole blood are stained with an antibody specific for monocyte surface markers e.g. CD14. This antibody is labeled with a marker for detection. In a second step, in situ hybridization is performed. Riboprobes that carry a marker for detection are added and hybridize with the corresponding mRNA of interest in a concentration-dependent way, which allows for quantitative detection of gene expression. The use of different color markers for each riboprobe set that hybridizes to one gene, allows for simultaneous detection of different messages. Finally, cells are guided through a flow cytometer and lasers excite the markers associated with the riboprobes and the monocyte specific antibody. The marker specific fluorescent signals are measured and allow simultaneous message detection in the same cell.
Two other approaches make use of biosensors. The first approach requires the extraction of mRNA and depending on the sensitivity of the bioreceptor, amplification of messages. A possible work flow for this approach is depicted in figure 13. To detect mRNA messages specifically in monocytes, we first need to select the monocytes out of whole blood. This can easily be done by adding magnetic bead coupled antibody specific for monocytes, e.g. CD14. Through magnetic forces, the monocytes can be moved out whole blood suspension. Next, the monocytes have to be lysed and RNA is extracted. Depending on the sensitivity of the bioreceptor, RNA can be amplified isothermally using NASBA ⁷⁸.
The bioreceptor for the detection of nucleic acids is a complementary nucleic acid ⁷⁹. Hybridization of the target is then detected through the generation of signal such as a current, fluorescence,... There are several possibilities. In this figure/In our proposal, the bioreceptor is a molecular beacon immobilized to the recognition layer of the biosensor 80
A molecular beacon is a single-stranded oligonucleotide hybridization probe that forms a stem-and-loop structure. A fluorophore is covalently linked to one end and a quencher is covalently linked to the other end. Molecular beacons do not fluoresce when they are unbound because the stem places the fluorophore close to the quencher that inhibits the fluorophore to fluoresce. When the probe encounters a target molecule, it forms a probe-target hybrid. Consequently, the molecular beacon undergoes a spontaneous conformational reorganization that forces the stem hybrid to dissociate and the fluorophore and the quencher to move away from each other, enabling them to fluoresce brightly.
Taking advantage of the properties of molecular beacons, we propose an alternative competitive assay that circumvents the need for nucleic acid extraction in figure 14. After monocyte selection as described above, unlabeled riboprobes are added to the monocyte suspension and can bind to the bioreceptor or to the intracellular target mRNA. The higher the expression of our target mRNA, the lower the signal detected by the biosensor.

**Table 1: Primers for RT-PCR analysis**

| **Gene Symbol** | **FORWARD** | **REVERSE** |
|---|---|---|
| **BCL2L11** | GGGCCACCGTGTCCATTA | CCTGTGCCAATTCCCATGA |
| **FOXO3A** | CAACAAAATGAAATCCATAGAAGCA | AGTGTATGAGTGAGAGGCAATAGCA |
| **IRAK3** | TGCAACGCGGGCAAA | TTTAGTGATGTGGGAGGATCTTCA |
| **IRS2** | GCTTCCCCAGTGCCTATCTTC | AAACCAACAACTTACATCTCCAATGA |
| **MAPK13** | AAAGCGGCCAAATCCTACATC | GGGAACAGCTGAGTGAAATCCT |
| **MST1** | TCTCAGCAAACACGGGACTGT | AATCCCCTCTACCCCTGTGTGTA |
| **MYD88** | TGCATATCTTTGCTCCACTTTCA | ATTCCCTCCCAAGATCCTAAGAA |
| **SOD2** | TGGAAGCCATCAAACGTGACT | TTTGTAAGTGTCCCCGTTCCTT |
| **TLR2** | TGCAAGTACGAGCTGGACTTCTC | GTGTTCATTATCTTCCGCAGCTT |
| **TNFAIP3** | TCCCTGCTCCTTCCCTATCTC | ATGTTTCGTGCTTCTCCTTATGAA |
| **TNFAIP6** | GGCGATCTCGCAACTTACAAG | GCAGCACAGACATGAAATCCA |
| **TRAF6** | CATGAAAAGATGCAGAGGAATCAC | GAACAGCCTGGGCCAACAT |
| **ZNF217** | CGCCTGCGACGGATACAC | TGGTCAGAGGCATCACATCAC |
| **BACTIN** | GGACCTGACCGACTACCTCATG | CGACGTAGCAGAGCTTCTCCTT |

**Table 2: Top ten of gene functions which are overexpressed in monocytes of obese women**

| **Functions of 592 overexpressed genes** | **Rank** | **Number of overexpressed genes** | **Total number of genes with this function present on microchip** | **Percentage of overexpressed genes** |
|---|---|---|---|---|
| Gene, cellular development, immune and lymphatic system development | **1** | 29 | 35 | 83% |
| Cell-to-cell signalling and interaction, haematological system development and function, Immune | **2** | 29 | 35 | 83% |
| Cancer, immunological disease, tumour | **3** | 28 | 35 | 80% |
| Cell signalling, lipid, small molecule biochemistry | **4** | 27 | 35 | 77% |
| Cancer, dermatological diseases and conditions, cellular development | **5** | 23 | 35 | 66% |
| Cancer, cell-to-cell signalling and interaction, neurological disease | **6** | 23 | 35 | 66% |
| Cancer, cell cycle, immunological disease | **7** | 21 | 35 | 60% |
| Cell death, immunological disease, cellular growth | **8** | 21 | 35 | 60% |
| Cellular assembly and cellular compromise, cellular function | **9** | 20 | 35 | 57% |

**Table 3: Receiver Operating Characteristic Curve analysis of relation with obesity**

| **Gene** | **AUC** | **Sensitivity %** | **Specificity %** | **PPV %** | **NPV %** |
|---|---|---|---|---|---|
| SOD2 | 0.81 (0.73 - 0.88)*** | 72 | 84 | 94 | 44 |
| TNFAIP6 | 0.81 (0.73 - 0.88)*** | 70 | 88 | 96 | 44 |
| TNFAIP3 | 0.76 (0.67 - 0.83)*** | 51 | 96 | 98 | 34 |
| IRAK3 | 0.72 (0.63 - 0.80)*** | 39 | 92 | 96 | 33 |
| TLR2 | 0.72 (0.63 - 0.80)*** | 76 | 64 | 88 | 41 |
| ZNF217 | 0.74 (0.65 - 0.82)*** | 54 | 96 | 98 | 36 |
| IRS2 | 0.70 (0.61 - 0.78)** | 78 | 56 | 87 | 40 |
| MAPK13 | 0.64 (0.55 - 0.73)** | 37 | 96 | 97 | 29 |
| TRAF6 | 0.60 (0.51 - 0.69) | 44 | 92 | 92 | 29 |
| FOXO3A | 0.60(0.50-0.70) | 55 | 76 | 87 | 40 |
| MYD88 | 0.52(0.42-0.62 | 24 | 88 | 86 | 27 |

| | | | | | |
|---|---|---|---|---|---|
| Data present area under the curve and 95% confidence intervals; *P<0.05, **P<0.01, ***P<0.001. AUC: area under the curve; PPV: positive predictive value; NPV: negative predictive value | | | | | |

**Table 4: Fisher exact test analysis of relation with obesity**

| **Gene** | **Cut point** | **OR** | **Sensitivity %** | **Specificity %** | **NPV%** | **PPV%** |
|---|---|---|---|---|---|---|
| IRAK3 | ≤ 0.85 | 11 | 92 (74-99) | 49 (39-60) | 32 (22-45) | 96 (86-100) |
| SOD2 | ≥ 1.67 | 10 | 80 (59-93) | 72 (61-80) | 43 (28-58) | 93 (85-98) |
| TNFAIP6 | ≥ 1.72 | 9.6 | 80 (59-93) | 70 (60-79) | 42 (28-57) | 93 (85-98) |
| TNFAIP3 | ≥ 2.14 | 7.8 | 88 (69-98) | 51 (41-62) | 32 (22-45) | 94 (84-99) |
| ZNF217 | ≤0.70 | 28 | 54(44-65) | 96(80-99) | 36(24-48) | 98(90-99) |
| IRAK3+SOD2 | Same as above | 20 | 76 (55-91) | 86 (78-93) | 59 (41-76) | 93 (86-97) |
| IRAK3+TNFAIP3 | Same as above | 17 | 80 (59-93) | 82 (72-88) | 53 (36-69) | 94 (86-98) |
| IRAK3+TNFAIP6 | Same as above | 22 | 76 (55-91) | 87 (79-93) | 61 (42-78) | 93 (86-97) |
| IRAK3+ZNF217 | Same as above | 23 | 76(66-84) | 88(69-97) | 49(34-64) | 96(89-99) |
| SOD2+ZNF217 | Same as above | 19 | 79(69-86) | 84(64-95) | 51(35-67) | 95(87-99) |
| TNFAIP3+ZNF217 | Same as above | 22 | 81(71-88) | 84(64-95) | 54(37-70) | 95(88-99) |
| TNFAIP6+ZNF217 | Same as above | 22 | 87(79-93) | 76(55-91) | 61(42-78) | 93(86-97) |
| IRAK3+SOD2+TN FAIP3 | Same as above | 56 | 95(88-98) | 76(55-91) | 79(58-93) | 94(87-98) |
| IRAK3+SOD2+TN FAIP6 | Same as above | 96 | 97(91-99) | 76(55-91) | 86(65-97) | 94(87-98) |
| IRAK3+TNFAIP3+TN FAIP6 | Same as above | 24 | 88(80-96) | 76(55-91) | 63(44-80) | 93(86-97) |
| IRAK3+SOD2+ ZNF217 | Same as above | 18 | 85(77-92) | 76(55-91) | 58(39-75) | 93(86-97) |
| IRAK3+ TNFAIP3+ZNF217 | Same as above | 21 | 84(75-91) | 80(59-93) | 57(39-74) | 94(87-98) |
| IRAK3+ TNFAIP6+ZNF217 | Same as above | 96 | 97(91-99) | 76(55-91) | 86(65-97) | 94(87-98) |
| SOD2+TNFAIP3+ ZNF217 | Same as above | 50 | 93(85-97) | 80(59-93) | 74(54-89) | 95(88-98) |
| SOD2+TNFAIP6+ ZNF217 | Same as above | 56 | 95(88-98) | 76(55-91) | 79(58-93) | 94(87-98) |
| TNFAIP3+TNFAIP6+ ZNF217 | Same as above | 30 | 90(83-96) | 76(55-91) | 68(86-98) | 93(86-98) |
| IRAK3+SOD2+TNFA IP3+TNFAIP6 | Same as above | 47 | 76 (55-91) | 94 (87-98) | 76 (55-91) | 94 (87-98) |
| IRAK3+SOD2+TNFA IP3+ZNF217 | Same as above | 294 | 99(94-99) | 76(55-91) | 95(75-99) | 94(87-98) |
| IRAK3+SOD2+TNFA IP6+ZNF217 | Same as above | 466 | 100(96-100) | 72(51-88) | 100(81-100) | 93(86-97) |
| IRAK3+TNFAIP3+TN FAIP6+ZNF217 | Same as above | 118 | 98(93-99) | 72(51-88) | 90(68-99) | 93(86-97) |
| IRAK3+SOD2+TNFA IP3+TNFAIP6 +ZNF217 | Same as above | 466 | 100(96-100) | 72(51-88) | 100(81-100) | 93(86-97) |

| | | | | | | |
|---|---|---|---|---|---|---|
| Cut-off values were determined by ROC curve analysis (see table 3). Sensitivities, specificities, and positive and negative predictive values were presented as means and 95% confidence intervals. OR: chance of being obese when having high or low (for IRAK3 and NF217) expressions compared with those having low or high expressions. | | | | | | |

**Table 5: Correlation between expressions of genes within the selected cluster**

| Genes | IRAK3 | TNFAIP3 | TNFAIP6 | SOD2 |
|---|---|---|---|---|
| IRAK3 | - | NS | NS | NS |
| TNFAIP3 | NS | - | 0.76**** | 0.83**** |
| | | | | |
| TNFAIP6 | NS | 0.76**** | - | 0.88**** |
| | | | | |
| SOD2 | NS | 0.83**** | 0.88**** | - |
| | | | | |
| ZNF217 | NS | -0.39 *** | -0.47*** | -0.48*** |

| | | | | |
|---|---|---|---|---|
| ***P<0.001; **** P<0.0001 | | | | |

**Table 6: Correlations between gene expressions of genes within the selected cluster and risk factors**

| Genes | BMI | Leptin | Adiponectin | Glucose | Insulin | HDL-C | TG | OxLDL | IL6 |
|---|---|---|---|---|---|---|---|---|---|
| IRAK3 | -0.40*** | -0.34*** | 0.19* | NS | -0.21* | NS | -0.23* | -0.33*** | NS |
| SOD2 | 0.23* | 0.25** | NS | NS | NS | 0.22* | 0.19 | 0.26** | 0.19* |
| TNFAIP6 | 0.30*** | 0.29** | NS | NS | NS | NS | NS | 0.27 | NS |
| TNFAIP3 | NS | NS | NS | NS | NS | NS | NS | NS | NS |
| ZNF217 | NS | -0.20* | NS | NS | -0.21* | NS | NS | NS | -0.22* |

| | | | | | | | | | |
|---|---|---|---|---|---|---|---|---|---|
| * P<0.05; ** P<0.01; *** P<0.001; NS: not significant | | | | | | | | | |

**Table 7: Receiver Operating Characteristic Curve analysis of obese persons not treated with a statin**

| **Gene** | **AUC** | **Sensitivity %** | **Specificity %** | **PPV %** | **NPV %** |
|---|---|---|---|---|---|
| SOD2 | 0.82 (0.71 - 0.90)*** | 63 | 96 | 97 | 58 |
| TNFAIP6 | 0.85 (0.75 - 0.92)*** | 76 | 88 | 92 | 66 |
| TNFAIP3 | 0.77 (0.65 - 0.86)*** | 50 | 96 | 96 | 51 |
| IRAK3 | 0.73 (0.62 - 0.83)*** | 46 | 100 | 100 | 55 |
| TLR2 | 0.78 (0.66 - 0.87)*** | 76 | 76 | 85 | 63 |
| IRS2 | 0.67 (0.54 - 0.77)* | 39 | 92 | 90 | 45 |
| MAPK13 | 0.65 (0.57 - 0.80)** | 46 | 96 | 96 | 49 |
| ZNF217 | 0.75 (0.65 - 0.83)*** | 55 | 96 | 98 | 36 |

| | | | | | |
|---|---|---|---|---|---|
| Data present area under the curve and 95% confidence intervals; *P<0.05, **P<0.01, ***P<0.001. AUC: area under the curve; PPV: positive predictive value; NPV: negative predictive value | | | | | |

**Table 8: Receiver Operating Characteristic Curve analysis of obese persons treated with a statin**

| **Gene** | **AUC** | **Sensitivity %** | **Specificity %** | **PPV %** | **NPV %** |
|---|---|---|---|---|---|
| SOD2 | 0.80 (0.69 - 0.88)*** | 69 | 84 | 89 | 58 |
| TNFAIP6 | 0.78 (0.67 - 0.87)*** | 63 | 88 | 91 | 55 |
| TNFAIP3 | 0.75 (0.63 - 0.84)*** | 51 | 96 | 96 | 58 |
| IRAK3 | 0.70 (0.59 - 0.81)** | 47 | 92 | 92 | 47 |
| TLR2 | 0.68 (0.56 - 0.78)** | 80 | 56 | 78 | 59 |
| IRS2 | 0.73 (0.61 - 0.82)*** | 84 | 56 | 79 | 64 |
| MAPK13 | 0.60 (0.48 - 0.71) | 33 | 92 | 89 | 41 |
| ZNF217 | 0.72 (0.63 - 0.81)*** | 53 | 94 | 96 | 36 |

| | | | | | |
|---|---|---|---|---|---|
| Data present area under the curve and 95% confidence intervals; *P<0.05, **P<0.01, ***P<0.001. AUC: area under the curve; PPV: positive predictive value; NPV: negative predictive value | | | | | |

**Table 9: Fisher exact test analysis of obese persons not treated with a statin**

| **Gene** | **Cut point** | **OR** | **Sensitivity %** | **Specificity %** | **PPV%** | **NPV%** |
|---|---|---|---|---|---|---|
| IRAK3 | ≤ 0.77 | 39.5 | 100 (86-100) | 43 (29-59) | 49 (35-63) | 100 (83-100) |
| SOD2 | ≥ 1.82 | 19.6 | 92 (74-99) | 63 (48-77) | 58 (41-73) | 94 (79-99) |
| TNFAIP6 | ≥ 1.72 | 12.7 | 80 (59-93) | 76 (61-87) | 65 (45-81) | 88 (73-96) |
| TNFAIP3 | ≥ 2.14 | 6.7 | 88 (69-97) | 48 (33-63) | 48 (33-63) | 88 (69-98) |
| IRAK3+ SOD2 | Same as above | 41.4 | 92 (74-99) | 78 (64-89) | 70 (51-84) | 95 (82-99) |
| IRAK3+TNFAIP3 | Same as above | 41.4 | 92 (74-99) | 78 (64-89) | 70 (51-84) | 95 (82-99) |
| IRAK3+TNFAIP6 | Same as above | 26.7 | 80 (59-93) | 87 (74-95) | 77 (56-91) | 89 (76-95) |
| IRAK3+SOD2+ TNFAIP6+ TNFAIP3 | Same as above | 26.7 | 80 (59-93) | 87 (74-95) | 77 (56-91) | 89 (76-96) |

| | | | | | | |
|---|---|---|---|---|---|---|
| Cut-off values were determined by ROC curve analysis (see table 3). Sensitivities, specificities, and positive and negative predictive values were presented as means and 95% confidence Intervals. OR: chance of being obese when having high or low (for IRAK3) expressions. | | | | | | |

**Table 10: Fisher exact test analysis of obese persons treated with a statin**

| **Gene** | **Cut point** | **OR** | **Sensitivity %** | **Specificity %** | **PPV%** | **NPV%** |
|---|---|---|---|---|---|---|
| IRAK3 | ≤ 0.85 | 10.0 | 92 (74-99) | 47 (33-62) | 47 (33-62) | 92 (74-99) |
| SOD2 | ≥ 1.67 | 9.1 | 80 (59-93) | 69 (55-82) | 57 (39-74) | 87 (73-96) |
| TNFAIP6 | ≥ 1.72 | 7.5 | 80 (59-93) | 65 (50-78) | 54 (37-71) | 86 (71-95) |
| TNFAIP3 | ≥ 2.14 | 7.6 | 88 (69-97) | 51 (36-66) | 48 (33-63) | 89 (72-98) |
| IRAK3+ SOD2 | Same as above | 16 | 76 (55-91) | 84 (70-93) | 70 (50-86) | 87 (74-95) |
| IRAK3+TNFAIP3 | Same as above | 15.6 | 80 (59-93) | 80 (66-90) | 67 (47-83) | 89 (75-96) |
| IRAK3+TNFAIP6 | Same as above | 16 | 76 (55-91) | 84 (70-93) | 70 (58-86) | 87 (74-95) |
| IRAK3+SOD2+ TNFAIP6+ TNFAIP3 | Same as above | 18 | 72 (51-88) | 88 (75-95) | 75 (53-90) | 86 (73-94) |

| | | | | | | |
|---|---|---|---|---|---|---|
| Cut-off values were determined by ROC curve analysis (see table 3). Sensitivities, specificities, and positive and negative predictive values were presented as means and 95% confidence intervals. OR: chance of being obese when having high or low (for IRAK3) expressions. | | | | | | |

**Table 11: Association between gene expression in monocytes and the occurrence of metabolic syndrome determined by Fisher exact test**

| **Gene** | **Cut point** | **RR** | **Sensitivity %** | **Specificity %** | **PPV%** | **NPV%** |
|---|---|---|---|---|---|---|
| SOD2 | ≥ 1.67 | 1.2 (0.9-1.8) | 60 (46-72) | 53 (39-65) | 58 (45-70) | 54 (40-68) |
| IRAK3 | ≤ 0.77 | 1.6 (1.1-2.2) | 52 (39-65) | 72 (58-83) | 67 (52-80) | 58 (45-69) |
| TNFAIP6 | ≥ 1.72 | 1.7 (1.1-2.5) | 71 (58-82) | 53 (39-66) | 62 (50-73) | 63 (47-76) |
| TNFAIP3 | ≥ 2.14 | 1.7 (1.2-2.4) | 53 (40-66) | 74 (60-84) | 69 (54-81) | 59 (47-71) |
| ZNF217 | ≤ 0.77 | 1.4 (098-1.9) | 52 (39-65) | 65 (51-77) | 62 (47-75) | 55 (43-67) |
| IRAK3+SOD2 | Same as above | 1.7 (1.0-2.8) | 81 (69-90) | 40 (28-54) | 60 (48-70) | 66 (48-80) |
| IRAK3+TNFAIP3 | Same as above | 2.1 (1.3-3.3) | 79 (67-88) | 50 (37-64) | 64 (52-74) | 69 (53-82) |
| IRAK3+TNFAIP6 | Same as above | 2.0 (1.1-3.6) | 85 (74-93) | 38 (25-51) | 60 (49-71) | 70 (51-85) |
| IRAK3+ZNF217 | Same as above | 1.7 (1.1-2.7) | 74 (62-84) | 51 (37-64) | 62 (50-73) | 64 (49-78) |
| SOD2+TNFAIP6 | Same as above | 2.1 (1.1-3.8) | 87 (76-94) | 35 (23-49) | 59 (48-70) | 71 (51-87) |
| SOD2+TNFAIP3 | Same as above | 2.7 (1.4-5.1) | 87 (76-94) | 46 (32-59) | 64 (52-74) | 76 (59-89) |
| SOD2+ZNF217 | Same as above | 1.7 (1.1-2.6) | 76 (63-86) | 46 (32-59) | 60 (49-71) | 63 (47-78) |
| TNFAIP3+TNFAIP6 | Same as above | 1.8 (1.2-2.8) | 74 (62-84) | 53 (39-66) | 63 (51-74) | 65 (50-79) |
| TNFAI3+ZNF217 | Same as above | 2.4 (1.4-4.1) | 82 (70-91) | 53 (39-66) | 65 (54-76) | 73 (57-86) |
| TNFAIP6+ZNF217 | Same as above | 1.8 (1.0-3.1) | 85 (74.93) | 33 (21-47) | 60 (49-70) | 67 (46-83) |
| lRAK3+SOD2+TNFAIP3 | Same as above | 3.9 (1.5-9.6) | 94 (84-98) | 37 (24-51) | 62 (51-72) | 84 (64-95) |
| IRAK3+SOD2+TNFAIP6 | Same as above | 4.5 (1.5-13) | 95 (87-99) | 33 (21-47) | 61 (51-71) | 86 (65-97) |
| IRAK3+SOD2+ZNF217 | Same as above | 1.9 (1.1-3.3) | 88 (72-92) | 37 (26-52) | 60 (49-70) | 69 (50-84) |
| IRAK3+TNFAIP3+TNFAIP6 | Same as above | 2.3 (1.2-4.2) | 87 (76-94) | 39 (26-52) | 61 (50-71) | 73 (54-88) |
| IRAK3+TNFAIP3+ZNF217 | Same as above | 3.8 (1.7-8.7) | 92 (82-97) | 44 (31-58) | 64 (53-74) | 83 (65-94) |
| | | | | | | |
| IRAK3+TNFAIP6+ZNF217 | Same as above | 4.5 (1.5-13) | 95 (86-99) | 33 (21-47) | 61 (50-71) | 86 (65-97) |
| SOD2+TNFAIP3+TNPAIP6 | Same as above | 1.6 (0.99-2.7) | 82 (70-91) | 35 (23-49) | 58 (47-68) | 65 (45-81) |
| SOD2+TNFAIP3+ZNF217 | Same as above | 4.2 (1.7-11) | 94 (84-98) | 41 (28-54) | 63 (52-73) | 85 (66-96) |
| SOD2+TNFAIP6+ZNF217 | Same as above | 2.9 (1.3-6.4) | 92 (82-97) | 33 (21-47) | 60 (49-70) | 79 (58-93) |
| TNFAIP3+TNFAIP6+ZNF217 | Same as above | 2.4 (1.2-4.7) | 89 (78-95) | 37 (24-51) | 60 (50-71) | 75 (55-89) |
| IRAK3+SOD2+TNFAIP3+T NFAIP6 | Same as above | 4.5 (1.5-13) | 95 (87-99) | 33 (21-47) | 61 (50-71) | 86 (65-97) |
| IRAK3+SOD2+TNFAIP3+Z NF217 | Same as above | 13 (1.9-89) | 98 (91-99) | 35 (23-49) | 62 (52-72) | 95 (76-100) |
| IRAK3+SOD2+TNFAIP6+Z NF217 | Same as above | 12 (1.7-79) | 98 (91-99) | 32 (20-45) | 61 (51-71) | 95 (74-100) |
| IRAK3+TNFAIP3+TNFAIP6+ZNF217 | Same as above | 4.5 (1.5-13) | 95 (86-99) | 33 (21.47) | 61 (50-71) | 86 (65-97) |
| IRAK3+SOD2+TNFAIP3+T NFAIP6+ZNF217 | Same as above | 12 (1.9-89) | 98 (91-99) | 32 (20-45) | 61 (51-71) | 95 (74-100) |

| | | | | | | |
|---|---|---|---|---|---|---|
| Cut points were determined by ROC curve analysis. Sensitivities, specificities, and positive and negative predictive values were presented as mean and 95% confidence intervals. RR: relative risk. NPV: negative predictive value; PPV: positive predictive value. Persons treated and not treated with a statin were included in analysis. | | | | | | |

**Table 12 Association between gene expression in monocytes and cardiovascular risk equivalents**

| **Gene** | **Cut point** | **RR** | **Sensitivity %** | **Specificity %** | **PPV%** | **NPV%** |
|---|---|---|---|---|---|---|
| SOD2 | ≥ 1.85 | 1.4 (1.1-1.9) | 61 (49-72) | 61 (49-72) | 73 (60-83) | 48 (35-62) |
| TNFAIP3 | ≥ 2.14 | 1.7 (1.3-2.3) | 54 (42-66) | 82 (67-92) | 83 (70-93) | 51 (39-64) |
| IRAK3 | ≤ 0.77 | 1.7 (1.3-2.2) | 51 (39-62) | 84 (70-93) | 84 (70-94) | 50 (38-62) |
| TNFAIP6 | ≥ 1.77 | 1.9 (1.4-2.7) | 72 (60-82) | 68 (52-81) | 79 (68-88) | 59 (44-72) |
| TNFAIP3+TNFAIP6 | Same as above | 1.8 (1.2-2.6) | 75 (63-84) | 59 (43-74) | 76 (64-85) | 58 (42-72) |
| IRAK3+TNFAIP3 | Same as above | 2.2 (1.5-3.4) | 80 (69-88) | 62 (47-67) | 78 (67-87) | 65 (49-79) |
| IRAK3+SOD2 | Same as above | 2.2 (1.4-3.4) | 83 (72-90) | 55 (39-70) | 76 (65-84) | 65 (47-80) |
| TNFAIP6+SOD2 | Same as above | 2.8 (1.5-5.2) | 89 (80-95) | 50 (35-65) | 75 (65-84) | 73 (54-88) |
| TNFAIP3 +SOD2 | Same as above | 2.9 (1.7-5.2) | 88 (78-94) | 57 (41-72) | 78 (67-86) | 74 (56-87) |
| IRAK3+TNFAIP6 | Same as above | 3.2 (1.8-5.7) | 88 (78-94) | 61 (45-76) | 80 (69-88) | 75 (58-88) |
| IRAK3+TNFAIP3+TNFAIP6 | Same as above | 3.2 (1.7-5.9) | 89 (80-95) | 56 (40-70) | 77 (67-85) | 76 (58-89) |
| IRAK3+TNFAIP3+SOD2 | Same as above | 5.0 (2.0-1.2) | 95 (87-99) | 50 (35-65) | 76 (66-85) | 85 (65-96) |
| IRAK3+TNFAIP6+SOD2 | Same as above | 6.4 (2.2-18) | 96 (89-99) | 50 (35-65) | 77 (67-85) | 88 (69-97) |
| TNFAIP3+TNFAIP6+SOD2 | Same as above | 9.2 (2.4-3.5) | 97 (91-99) | 50 (35-65) | 77 (67-85) | 92 (73-99) |
| IRAK3+TNFAIP3+ TNFAIP6+SOD2 | Same as above | 8.7 (2.3-33) | 97 (91-99) | 48 (32-65) | 76 (66-84) | 91 (72-99) |

| | | | | | | |
|---|---|---|---|---|---|---|
| Cut points were determined by ROC curve analysis. Sensitivities, specificities, and positive and negative predictive values were presented as mean and 95% confidence intervals. RR: relative risk. NPV: negative predictive value; PPV: positive predictive value. | | | | | | |

### Reference List

(1) Grundy SM, Brewer HB, Jr., Cleeman JI, Smith SC, Jr., Lenfant C. Definition of metabolic syndrome: Report of the National Heart, Lung, and Blood Institute/American Heart Association conference on scientific issues related to definition. Circulation 2004 January 27;109(3):433-438.
(2) Isomaa B, Almgren P, Tuomi T, Forsen B, Lahti K, Nissen M, Taskinen MR, Groop L. Cardiovascular morbidity and mortality associated with the metabolic syndrome. Diabetes Care 2001 April;24(4):683-689.
(3) Trevisan M, Liu J, Bahsas FB, Menotti A. Syndrome X and mortality: a population-based study. Risk Factor and Life Expectancy Research Group. Am JEpidemiol 1998 November 15;148(10):958-966.
(4) National Institutes of Health. Third Report of the National Cholesterol Education Program Expert Panel on Detection, Evaluation, and Treatment of High Blood Cholesterol in Adults (Adult Treatment Panel III). Bethesda, Md: National Institutes of Health; 2002. Report No.: NIH Publication 01-3670.
(5) Ford ES, Giles WH, Dietz WH. Prevalence of the metabolic syndrome among US adults: findings from the third National Health and Nutrition Examination Survey. JAMA 2002 January 16;287(3):356-359.
(6) Fagerberg B, Bondjers L, Nilsson P. Low birth weight in combination with catch-up growth predicts the occurrence of the metabolic syndrome in men at late middle age: the Atherosclerosis and Insulin Resistance study. J Intern Med 2004 September;256(3):254-259.
(7) Sinaiko AR, Steinberger J, Moran A, Prineas RJ, Vessby B, Basu S, Tracy R, Jacobs DR, Jr. Relation of body mass index and insulin resistance to cardiovascular risk factors, inflammatory factors, and oxidative stress during adolescence. Circulation 2005 April 19;111(15):1985-1991.
(8) Sjoholm A, Nystrom T. Inflammation and the etiology of type 2 diabetes. Diabetes Metab Res Rev 2005 July 1.
(9) Sjoholm A, Nystrom T. Inflammation and the etiology of type 2 diabetes. Diabetes Metab Res Rev 2005 July 1.
(10) Reilly MP, Lehrke M, Wolfe ML, Rohatgi A, Lazar MA, Rader DJ. Resistin is an inflammatory marker of atherosclerosis in humans. Circulation 2005 February 22;111(7):932-939.
(11) Lapointe A, Couillard C, Piche ME, Weisnagel SJ, Bergeron J, Nadeau A, Lemieux S. Circulating oxidized LDL is associated with parameters of the metabolic syndrome in postmenopausal women. Atherosclerosis 2006 May 3.
(12) Weinbrenner T, Schroder H, Escurriol V, Fito M, Elosua R, Vila J, Marrugat J, Covas MI. Circulating oxidized LDL is associated with increased waist circumference independent of body mass index in men and women. Am J Clin Nutr 2006 January;83(1):30-35.
(13) Yamagishi SI, Matsuoka H, Kitano S, Hibi N, Jinnouchi Y, Umei H, Iida S, Takenaka K, Matsui T, Nakamura K, Imaizumi T. Elevated circulating oxidized LDL levels in Japanese subjects with the metabolic syndrome. Int J Cardiol 2006 October 5.
(14) Holvoet P, Kritchevsky SB, Tracy RP, Mertens A, Rubin SM, Butler J, Goodpaster B, Harris TB. The metabolic syndrome, circulating oxidized LDL, and risk of myocardial infarction in well-functioning elderly people in the health, aging, and body composition cohort. Diabetes 2004 April;53(4):1068-1073.
(15) Johnston N, Jernberg T, Lagerqvist B, Siegbahn A, Wallentin L. Improved identification of patients with coronary artery disease by the use of new lipid and lipoprotein biomarkers. Am J Cardiol 2006 March 1;97(5):640-645.
(16) Meisinger C, Baumert J, Khuseyinova N, Loewel H, Koenig W. Plasma oxidized low-density lipoprotein, a strong predictor for acute coronary heart disease events in apparently healthy, middle-aged men from the general population. Circulation 2005 August 2;112(5):651-657.
(17) Holvoet P, Davey PC, De KD, Doukoure M, Deridder E, Bochaton-Piallat ML, Gabbiani G, Beaufort E, Bishay K, Andrieux N, Benhabiles N, Marguerie G. Oxidized low-density lipoprotein correlates positively with toll-like receptor 2 and interferon regulatory factor-1 and inversely with superoxide dismutase-1 expression: studies in hypercholesterolemic swine and THP-1 cells. Arterioscler Thromb Vasc Biol 2006 July;26(7):1558-1565.
(18) Wesche H, Gao X, Li X, Kirschning CJ, Stark GR, Cao Z. IRAK-M is a novel member of the Pelle/interleukin-1 receptor-associated kinase (IRAK) family. J Biol Chem 1999 July 2;274(27):19403-19410.
(19) Balaci L, Spada MC, Olla N, Sole G, Loddo L, Anedda F, Naitza S, Zuncheddu MA, Maschio A, Altea D, Uda M, Pilia S, Sanna S, Masala M, Crisponi L, Fattori M, Devoto M, Doratiotto S, Rassu S, Mereu S, Giua E, Cadeddu NG, Atzeni R, Pelosi U, Corrias A, Perra R, Torrazza PL, Pirina P, Ginesu F, Marcias S, Schintu MG, Del Giacco GS, Manconi PE, Malerba G, Bisognin A, Trabetti E, Boner A, Pescollderungg L, Pignatti PF, Schlessinger D, Cao A, Pilia G. IRAK-M is involved in the pathogenesis of early-onset persistent asthma. Am J Hum Genet 2007 June;80(6):1103-1114.
(20) Kobayashi K, Hernandez LD, Galan JE, Janeway CA, Jr., Medzhitov R, Flavell RA. IRAK-M is a negative regulator of Toll-like receptor signaling. Cell 2002 July 26;110(2):191-202.
(21) Cao Z, Xiong J, Takeuchi M, Kurama T, Goeddel DV. TRAF6 is a signal transducer for interleukin-1. Nature 1996 October 3;383(6599):443-446.
(22) King CG, Kobayashi T, Cejas PJ, Kim T, Yoon K, Kim GK, Chiffoleau E, Hickman SP, Walsh PT, Turka LA, Choi Y. TRAF6 is a T cell-intrinsic negative regulator required for the maintenance of immune homeostasis. Nat Med 2006 September;12(9):1088-1092.
(23) Lord KA, Hoffman-Liebermann B, Liebermann DA. Sequence of MyD116 cDNA: a novel myeloid differentiation primary response gene induced by IL6. Nucleic Acids Res 1990 May 11;18(9):2823.
(24) Medzhitov R, Preston-Hurlburt P, Kopp E, Stadlen A, Chen C, Ghosh S, Janeway CA, Jr. MyD88 is an adaptor protein in the hToll/IL-1 receptor family signaling pathways. Mol Cell 1998 August;2(2):253-258.
(25) Adachi O, Kawai T, Takeda K, Matsumoto M, Tsutsui H, Sakagami M, Nakanishi K, Akira S. Targeted disruption of the MyD88 gene results in loss of IL-1- and IL-18-mediated function. Immunity 1998 July;9(1):143-150.
(26) Kawai T, Adachi O, Ogawa T, Takeda K, Akira S. Unresponsiveness of MyD88-deficient mice to endotoxin. Immunity 1999 July;11(1):115-122.
(27) Bjorkbacka H, Kunjathoor VV, Moore KJ, Koehn S, Ordija CM, Lee MA, Means T, Halmen K, Luster AD, Golenbock DT, Freeman MW. Reduced atherosclerosis in MyD88-null mice links elevated serum cholesterol levels to activation of innate immunity signaling pathways. Nat Med 2004 April;10(4):416-421.
(28) Dixit VM, Green S, Sarma V, Holzman LB, Wolf FW, O'Rourke K, Ward PA, Prochownik EV, Marks RM. Tumor necrosis factor-alpha induction of novel gene products in human endothelial cells including a macrophage-specific chemotaxin. J Biol Chem 1990 February 15;265(5):2973-2978.
(29) Lee EG, Boone DL, Chai S, Libby SL, Chien M, Lodolce JP, Ma A. Failure to regulate TNF-induced NF-kappaB and cell death responses in A20-deficient mice. Science 2000 September 29;289(5488):2350-2354.
(30) Opipari AW, Jr., Boguski MS, Dixit VM. The A20 cDNA induced by tumor necrosis factor alpha encodes a novel type of zinc finger protein. J Biol Chem 1990 September 5;265(25):14705-14708.
(31) O'Reilly SM, Moynagh PN. Regulation of Toll-like receptor 4 signalling by A20 zinc finger protein. Biochem Biophys Res Commun 2003 April 4;303(2):586-593.
(32) Wertz IE, O'Rourke KM, Zhou H, Eby M, Aravind L, Seshagiri S, Wu P, Wiesmann C, Baker R, Boone DL, Ma A, Koonin EV, Dixit VM. Deubiquitination and ubiquitin ligase domains of A20 downregulate NF-kappaB signalling. Nature 2004 August 5;430(7000):694-699.
(33) Boone DL, Turer EE, Lee EG, Ahmad RC, Wheeler MT, Tsui C, Hurley P, Chien M, Chai S, Hitotsumatsu O, McNally E, Pickart C, Ma A. The ubiquitin-modifying enzyme A20 is required for termination of Toll-like receptor responses. Nat Immunol 2004 October;5(10): 1052-1060.
(34) Cook SA, Novikov MS, Ahn Y, Matsui T, Rosenzweig A. A20 is dynamically regulated in the heart and inhibits the hypertrophic response. Circulation 2003 August 12;108(6):664-667.
(35) Lee TH, Klampfer L, Shows TB, Vilcek J. Transcriptional regulation of TSG6, a tumor necrosis factor- and interleukin-1-inducible primary response gene coding for a secreted hyaluronan-binding protein. J Biol Chem 1993 March 25;268(9):6154-6160.
(36) Sun XJ, Wang LM, Zhang Y, Yenush L, Myers MG, Jr., Glasheen E, Lane WS, Pierce JH, White MF. Role of IRS-2 in insulin and cytokine signalling. Nature 1995 September 14;377(6545):173-177.
(37) Withers DJ, Burks DJ, Towery HH, Altamuro SL, Flint CL, White MF. Irs-2 coordinates Igf-1 receptor-mediated beta-cell development and peripheral insulin signalling. Nat Genet 1999 September;23(1):32-40.
(38) Tobe K, Suzuki R, Aoyama M, Yamauchi T, Kamon J, Kubota N, Terauchi Y, Matsui J, Akanuma Y, Kimura S, Tanaka J, Abe M, Ohsumi J, Nagai R, Kadowaki T. Increased expression of the sterol regulatory element-binding protein-1 gene in insulin receptor substrate-2(-/-) mouse liver. J Biol Chem 2001 October 19;276(42):38337-38340.
(39) Taguchi A, Wartschow LM, White MF. Brain IRS2 signaling coordinates life span and nutrient homeostasis. Science 2007 July 20;317(5836):369-372.
(40) Goedert M, Cuenda A, Craxton M, Jakes R, Cohen P. Activation of the novel stress-activated protein kinase SAPK4 by cytokines and cellular stresses is mediated by SKK3 (MKK6); comparison of its substrate specificity with that of other SAP kinases. EMBO J 1997 June 16;16(12):3563-3571.
(41) Wang XS, Diener K, Manthey CL, Wang S, Rosenzweig B, Bray J, Delaney J, Cole CN, Chan-Hui PY, Mantlo N, Lichenstein HS, Zukowski M, Yao Z. Molecular cloning and characterization of a novel p38 mitogen-activated protein kinase. J Biol Chem 1997 September 19;272(38):23668-23674.
(42) Jagavelu K, Tietge UJ, Gaestel M, Drexler H, Schieffer B, Bavendiek U. Systemic deficiency of the MAP kinase-activated protein kinase 2 reduces atherosclerosis in hypercholesterolemic mice. Circ Res 2007 November 26;101(11):1104-1112.
(43) Morris JB, Olzinski AR, Bernard RE, Aravindhan K, Mirabile RC, Boyce R, Willette RN, Jucker BM. p38 MAPK inhibition reduces aortic ultrasmall superparamagnetic iron oxide uptake in a mouse model of atherosclerosis: MRI assessment. Arterioscler Thromb Vasc Biol 2008 February;28(2):265-271.
(44) Brunet A, Bonni A, Zigmond MJ, Lin MZ, Juo P, Hu LS, Anderson MJ, Arden KC, Blenis J, Greenberg ME. Akt promotes cell survival by phosphorylating and inhibiting a Forkhead transcription factor. Cell 1999 March 19;96(6):857-868.
(45) Nemoto S, Finkel T. Redox regulation of forkhead proteins through a p66shc-dependent signaling pathway. Science 2002 March 29;295(5564):2450-2452.
(46) Essers MA, de Vries-Smits LM, Barker N, Polderman PE, Burgering BM, Korswagen HC. Functional interaction between beta-catenin and FOXO in oxidative stress signaling. Science 2005 May 20;308(5725):1181-1184.
(47) Li Y, Huang TT, Carlson EJ, Melov S, Ursell PC, Olson JL, Noble LJ, Yoshimura MP, Berger C, Chan PH, Wallace DC, Epstein CJ. Dilated cardiomyopathy and neonatal lethality in mutant mice lacking manganese superoxide dismutase. Nat Genet 1995 December;11(4):376-381.
(48) Melov S, Coskun P, Patel M, Tuinstra R, Cottrell B, Jun AS, Zastawny TH, Dizdaroglu M, Goodman SI, Huang TT, Miziorko H, Epstein CJ, Wallace DC. Mitochondrial disease in superoxide dismutase 2 mutant mice. Proc Natl Acad Sci U S A 1999 February 2;96(3):846-851.
(49) Isola JJ, Kallioniemi OP, Chu LW, Fuqua SA, Hilsenbeck SG, Osborne CK, Waldman FM. Genetic aberrations detected by comparative genomic hybridization predict outcome in node-negative breast cancer. Am J Pathol 1995 October;147(4):905-911.
(50) Kallioniemi A, Kallioniemi OP, Piper J, Tanner M, Stokke T, Chen L, Smith HS, Pinkel D, Gray JW, Waldman FM. Detection and mapping of amplified DNA sequences in breast cancer by comparative genomic hybridization. Proc Natl Acad Sci US A 1994 March 15;91(6):2156-2160.
(51) Collins C, Rommens JM, Kowbel D, Godfrey T, Tanner M, Hwang SI, Polikoff D, Nonet G, Cochran J, Myambo K, Jay KE, Froula J, Cloutier T, Kuo WL, Yaswen P, Dairkee S, Giovanola J, Hutchinson GB, Isola J, Kallioniemi OP, Palazzolo M, Martin C, Ericsson C, Pinkel D, Albertson D, Li WB, Gray JW. Positional cloning of ZNF217 and NABCl: genes amplified at 20q13.2 and overexpressed in breast carcinoma. Proc Natl Acad Sci U S A 1998 July 21;95(15):8703-8708.
(52) Quinlan KG, Verger A, Yaswen P, Crossley M. Amplification of zinc finger gene 217 (ZNF217) and cancer: when good fingers go bad. Biochim Biophys Acta 2007 June;1775(2):333-340.
(53) Huang G, Krig S, Kowbel D, Xu H, Hyun B, Volik S, Feuerstein B, Mills GB, Stokoe D, Yaswen P, Collins C. ZNF217 suppresses cell death associated with chemotherapy and telomere dysfunction. Hum Mol Genet 2005 November 1;14(21):3219-3225.
(54) Kretzschmar M. Transforming growth factor-beta and breast cancer: Transforming growth factor-beta/SMAD signaling defects and cancer. Breast Cancer Res 2000;2(2):107-115.
(55) Thillainadesan G, Isovic M, Loney E, Andrews J, Tini M, Torchia J. Genome analysis identifies the p15ink4b tumor suppressor as a direct target of the ZNF217/CoREST complex. Mol Cell Biol 2008 October;28(19):6066-6077.
(56) Bogdan C, Nathan C. Modulation of macrophage function by transforming growth factor beta, interleukin-4, and interleukin-10. Ann N Y Acad Sci 1993 June 23;685:713-739.
(57) Cummings DE, Overduin J, Foster-Schubert KE. Gastric bypass for obesity: mechanisms of weight loss and diabetes resolution. J Clin Endocrinol Metab 2004 June;89(6):2608-2615.
(58) Klein S, Burke LE, Bray GA, Blair S, Allison DB, Pi-Sunyer X, Hong Y, Eckel RH. Clinical implications of obesity with specific focus on cardiovascular disease: a statement for professionals from the American Heart Association Council on Nutrition, Physical Activity, and Metabolism: endorsed by the American College of Cardiology Foundation. Circulation 2004 November 2;110(18):2952-2967.
(59) Sjostrom L, Lindroos AK, Peltonen M, Torgerson J, Bouchard C, Carlsson B, Dahlgren S, Larsson B, Narbro K, Sjostrom CD, Sullivan M, Wedel H. Lifestyle, diabetes, and cardiovascular risk factors 10 years after bariatric surgery. N Engl J Med 2004 December 23;351(26):2683-2693.
(60) Wilson PW, D'Agostino RB, Levy D, Belanger AM, Silbershatz H, Kannel WB. Prediction of coronary heart disease using risk factor categories. Circulation 1998 May 12;97(18):1837-1847.
(61) Pickl WF, Majdic O, Kohl P, Stockl J, Riedl E, Scheinecker C, Bello-Fernandez C, Knapp W. Molecular and functional characteristics of dendritic cells generated from highly purified CD14+ peripheral blood monocytes. J Immunol 1996 November 1;157(9):3850-3859.
(62) Salio M, Cerundolo V, Lanzavecchia A. Dendritic cell maturation is induced by mycoplasma infection but not by necrotic cells. Eur J Immunol 2000 February;30(2):705-708.
(63) Holvoet P, Davey PC, De Keyzer D., Doukoure M, Deridder E, Bochaton-Piallat ML, Gabbiani G, Beaufort E, Bishay K, Andrieux N, Benhabiles N, Marguerie G. Oxidized low-density lipoprotein correlates positively with toll-like receptor 2 and interferon regulatory factor-1 and inversely with superoxide dismutase-1 expression: studies in hypercholesterolemic swine and THP-1 cells. Arterioscler Thromb Vasc Biol 2006 July;26(7):1558-1565.
(64) Holvoet P, Davey PC, De Keyzer D., Doukoure M, Deridder E, Bochaton-Piallat ML, Gabbiani G, Beaufort E, Bishay K, Andrieux N, Benhabiles N, Marguerie G. Oxidized low-density lipoprotein correlates positively with toll-like receptor 2 and interferon regulatory factor-1 and inversely with superoxide dismutase-1 expression: studies in hypercholesterolemic swine and THP-1 cells. Arterioscler Thromb Vasc Biol 2006 July;26(7):1558-1565.
(65) Saeed AI, Sharov V, White J, Li J, Liang W, Bhagabati N, Braisted J, Klapa M, Currier T, Thiagarajan M, Sturn A, Snuffin M, Rezantsev A, Popov D, Ryltsov A, Kostukovich E, Borisovsky I, Liu Z, Vinsavich A, Trush V, Quackenbush J. TM4: a free, open-source system for microarray data management and analysis. Biotechniques 2003 February;34(2):374-378.
(66) Bar-Joseph Z, Gifford DK, Jaakkola TS. Fast optimal leaf ordering for hierarchical clustering. Bioinformatics 2001;17 Suppl 1:S22-S29.
(67) Eisen MB, Spellman PT, Brown PO, Botstein D. Cluster analysis and display of genome-wide expression patterns. Proc Natl Acad Sci U S A 1998 December 8;95(25): 14863-14868.
(68) Raychaudhuri S, Stuart JM, Altman RB. Principal components analysis to summarize microarray experiments: application to sporulation time series. Pac Symp Biocomput 2000;455-466.
(69) Bowie A, O'Neill LA. Oxidative stress and nuclear factor-kappaB activation: a reassessment of the evidence in the light of recent discoveries. Biochem Pharmacol 2000 January 1;59(1):13-23.
(70) Pathak SK, Basu S, Bhattacharyya A, Pathak S, Kundu M, Basu J. Mycobacterium tuberculosis lipoarabinomannan-mediated IRAK-M induction negatively regulates Toll-like receptor-dependent interleukin-12 p40 production in macrophages. J Biol Chem 2005 December 30;280(52):42794-42800.
(71) Heyninck K, Kreike MM, Beyaert R. Structure-function analysis of the A20-binding inhibitor of NF-kappa B activation, ABIN-1. FEBS Lett 2003 February 11;536(1-3):135-140.
(72) Wisniewski HG, Hua JC, Poppers DM, Naime D, Vilcek J, Cronstein BN. TNF/IL-1-inducible protein TSG-6 potentiates plasmin inhibition by inter-alpha-inhibitor and exerts a strong anti-inflammatory effect in vivo. J Immunol 1996 February 15;156(4):1609-1615.
(73) Evans JL, Goldfine ID, Maddux BA, Grodsky GM. Are oxidative stress-activated signaling pathways mediators of insulin resistance and beta-cell dysfunction? Diabetes 2003 January;52(1):1-8.
(74) Nunn AV, Bell J, Barter P. The integration of lipid-sensing and anti-inflammatory effects: how the PPARs play a role in metabolic balance. Nucl Recept 2007;5(1):1.
(75) Asada S, Daitoku H, Matsuzaki H, Saito T, Sudo T, Mukai H, Iwashita S, Kako K, Kishi T, Kasuya Y, Fukamizu A. Mitogen-activated protein kinases, Erk and p38, phosphorylate and regulate Foxol. Cell Signal 2007 March;19(3):519-527.
(76) O'Neill LA, Bowie AG. The family of five: TIR-domain-containing adaptors in Toll-like receptor signalling. Nat Rev Immunol 2007 May;7(5):353-364.
(77) Pennline KJ. FISHing for cytokines. Methodology combining flow cytometry and in situ hybridization. Methods Mol Biol 1998;91:197-215.
(78) Moore C, Hibbitts S, Owen N, Corden SA, Harrison G, Fox J, Gelder C, Westmoreland D. Development and evaluation of a real-time nucleic acid sequence based amplification assay for rapid detection of influenza A. J Med Virol 2004 December;74(4):619-628.
(79) Belluzo MS, Ribone ME, Lagier CM. Assembling amperometric biosensors for clinical diagnostics. Sensors 2008 March;8(3):1366-1399.
(80) Kim Y, Sohn D, Tan W. Molecular beacons in biomedical detection and clinical diagnosis. Int J Clin Exp Pathol 2008;1(2):105-116.

## Claims

1. An in vitro method of diagnosing a metabolic syndrome disorder phenotype in a subject, said method comprising: (a) analyzing the level of IRAK3 expression or activity of expression product in a biological sample isolated from said subject, and (b) compare said level of expression or activity with the IRAK3 expression or activity in a control sample; whereby a decreased level of IRAK3 expression or activity relative to a control sample is an indication of such metabolic syndrome disorder phenotype or a propensity thereto.

2. An in vitro method of diagnosing a metabolic syndrome disorder, or a propensity thereto in a subject, said method (a) obtaining an expression profile in a biological sample isolated from said subject, wherein said expression profile consists of the analysis of the level of IRAK3 expression or activity of an IRAK3 expression product in combination with the gene expression level or activity of a gene product of at least one gene selected from the group consisting of SOD2, TNFAIP36, TNFAIP3, TLR2, IRS2, and ZNF217; (b) comparing said obtained expression profile to a reference expression profile to determine whether said sample is from subject having a metabolic syndrome disorder phenotype or a propensity thereto.

3. The in vitro method of claims 1 or 2, wherein the metabolic syndrome disorder is an impaired adipose tissue accumulation or adipocyte function, an impaired glucose tolerance condition, an insulin resistance or type II diabetes disorder, a lipid homeostasis disorder or a cardiovascular disease related thereto.

4. The in vitro method of claim 4, wherein the cardiovascular disease is of the group consisting of hypertension, coronary heart disease, heart failure, congestive heart failure, atherosclerosis, arteriosclerosis, stroke, cerebrovascular disease, myocardial infarction and peripheral vascular disease.

5. The in vitro method of claims 1, 2 or 3, said method comprising analyzing the level of IRAK3 expression or activity of expression product in a sample isolated from said subject, whereby a decreased level of IRAK3 expression or activity relative to a control sample is an indication of such adipose tissue disorder, lipid homeostasis disorder and/or an impaired glucose tolerance or insulin resistance condition and/or related cardiovascular disease or a propensity thereto.

6. The in vitro method of any of the previous claims, whereby the expression product is a nucleic acid molecule selected from the group consisting of mRNA and cDNA mRNA or derived polypeptides.

7. The in vitro method of any of the previous claims, wherein the sample isolated form said subject is selected from a group consisting of a) a liquid containing cells; (b) a tissue-sample; (c) a cell-sample and (a) a cell biopsy.

8. The method according to any of the claims 1 to 7, wherein the detection comprises an immuno-cytochemical detection procedure.

9. The method according to any of the claims 1 to 7, wherein the detection reaction comprises a nucleic acid amplification reaction.

10. An vitro method for identifying or monitoring a metabolic syndrome disorder therapy in a subject said method comprising analysing the level of IRAK3 expression or activity of expression product in a sample isolated from said subject before and after treatment with said therapy, whereby an increased level of IRAK3 compared to a sample of said subject before the therapy is indicative for the efficacy of said therapy.

11. An vitro method for identifying or monitoring a metabolic syndrome disorder therapy in a subject said method comprising analyzing the expression profile in a biological sample isolated from said subject before and after treatment with said therapy, wherein said expression profile consists of the analysis of the level of IRAK3 expression or activity of an IRAK3 expression product in combination with the gene expression level or activity of a gene product of at least one gene selected from the group consisting of SOD2, TNFAIP6, TNFAIP3, TLR2, IRS2, and ZNF217; and whereby an increased level of IRAK3 and a decreased level of SOD2, TNFAIP3 and/or TNFAIP3 compared to a sample of said subject before the therapy is indicative for the efficacy of said therapy.

12. A diagnostic test kit for use in diagnosing a metabolic syndrome disorder phenotype in a subject, or for use in monitoring the effectiveness of therapy of metabolic syndrome disorder in patients receiving such therapy, or for use in predicting whether a subject is a candidate for metabolic syndrome disorder comprising,
- a) a predetermined amount of a first antibody specific for IRAK3, and a predetermined amount of an at least second antibody specific for one or more of SOD2, TNFAIP6, TNFAIP3, TLR2 or IRS2
- b) a predetermined amount of a specific binding partner to said first antibody, and a predetermined amount of a specific binding partner to said at least second antibody;
- c) buffers and other reagents necessary for monitoring detection of antibody bound to IRAK3 and said one or more of SOD2, TNFAIP6, TNFAIP3, TLR2 or IRS2; and
wherein either said antibody or said specific binding partner are detectably labeled.

13. A diagnostic test kit for use in diagnosing a metabolic syndrome disorder phenotype in a subject, or for use in monitoring the effectiveness of therapy of metabolic syndrome disorder in patients receiving such therapy, or for use in predicting whether a subject is a candidate for metabolic syndrome disorder comprising:
- a) a predetermined amount of a first antibody specific for ZNF217, and a predetermined amount of an at least second antibody specific for one or more of SOD2, TNFAIP6, TNFAIP3, TLR2, IRAK3 or IRS2;
- b) a predetermined amount of a specific binding partner to said first antibody, and a predetermined amount of a specific binding partner to said at least second antibody;
- c) buffers and other reagents necessary for monitoring detection of antibody bound to ZNF217 and said one or more of SOD2, TNFAIP6, TNFAIP3, TLR2, IRAK3, or IRS2; and
wherein either said antibody or said specific binding partner are detectably labeled.

14. A diagnostic test kit for use in diagnosing a metabolic syndrome disorder phenotype in a subject, or for use in monitoring the effectiveness of therapy of metabolic syndrome disorder in patients receiving such therapy, or for use in predicting whether a subject is a candidate for metabolic syndrome disorder comprising:
- a) one or more nucleic acids encoding one or more of the proteins selected from the group consisting of ZNF217 and IRAK3;
- b) at least one nucleic acids encoding a proteins selected from the group consisting of SOD2, TNFAIP6, TNFAIP3, TLR2, and IRS2;
- c) reagents useful for monitoring the expression level of the one or more nucleic acids or proteins encoded by the nucleic acids of steps a) - b); and
- d) instructions for use of the kit.

## Patentansprüche

1. Ein in vitro Verfahren zur Diagnose des Erscheinungsbilds einer Stoffwechselsyndromstörung bei einem Subjekt, das genannte Verfahren umfasst: (a) die Analyse der Expressionshöhe von IRAK3 (IL-Rezeptor assoziierte Kinasen) oder die Aktivität eines Expressionsprodukts in einer Probe biologischen Materials, isoliert vom genannten Subjekt, und (b) Vergleich der genannten Expressionshöhe oder Aktivität mit der IRAK3 (IL-Rezeptor assoziierte Kinasen) Expression oder Aktivität in einer Kontrollprobe; wobei eine verminderte Höhe der IRAK3 Expression oder Aktivität relativ zur Kontrollprobe eine solche Stoffwechselsyndromstörung anzeigt oder eine Neigung dazu.

2. Ein in vitro Verfahren zur Diagnose einer Stoffwechselsyndromstörung, oder einer Neigung dazu, bei einem Subjekt, das genannte Verfahren umfasst (a) die Herstellung eines Expressionsprofils in einer Probe biologischen Materials isoliert vom genannten Subjekt, worin das genannte Expressionsprofil aus der Höhe der IRAK3 Expression oder Aktivität eines IRAK3 Expressionsprodukts in Kombination mit der Genexpressionshöhe oder Aktivität eines Genprodukts von mindestens einem Gen aus einer Gruppe aus SOD2, TNF-a-induziertem Protein 6, TNF-a-induziertem Protein 3, TLR2, IRS2, und ZNF217, besteht; (b) Vergleich des genannten Expressionsprofils mit einem Referenzexpressionsprofil, um zu bestimmen, ob für die besagte Probe von dem Erscheinungsbild einer metabolischen Syndromstörung oder einer Neigung hierzu auszugehen ist.

3. Das in Vitro Verfahren der Ansprüche 1 oder 2, worin die Stoffwechselsyndromstörung eine gestörte Fettgewebsansammlung ist, oder eine adipozyte Funktion, eine gestörte Glukosetoleranz, eine Insulinresistenz oder Diabetes Typ II, eine Lipidhomöostase oder eine diesbezügliche kardiovasluläre Krankheit.

4. Das in Vitro Verfahren von Anspruch 4, worin die Gruppe aus kardiovaskulären Krankheiten besteht, Bluthochdruck, koronare Herzkrankheit, Herzversagen, Arterienverkalkung, Schlaganfall, zerebrovaskuläre Krankheit, Myokardinfarkt und periphere Gefäßkrankheit.

5. Das in vitro Verfahren von 1, 2 oder 3, das benannte Verfahren umfasst die Analyse der IRAK3 Expressionshöhe oder die Aktivität des Expressionsprodukts aus einer Probe, isoliert aus besagtem Projekt, worin eine verminderte Höhe der IRAK3 Expression oder Aktivität relativ zur Kontrollprobe auf eine solche Fettgewebsstörung, Lipidhomöostasestörung und/oder gestörte Glukosetoleranz oder Insulinresistenz und/oder begleitende kardiovaskuläre Krankheiten, oder Neigungen hierzu hinweist.

6. Das in Vitro Verfahren jeglicher der vorherigen Ansprüche, wobei das Expressionsprodukt ein Nukleinsäuremolekül ist, aus der Gruppe mRNA und cDNA mRNA oder aus abgeleiteten Polypeptiden.

7. Das in vitro Verfahren einer der vorherigen Ansprüche, worin die isolierte Probe des benannten Subjekts aus folgender Gruppe ist a) eine Flüssigkeit, die Zellen enthält; (b) eine Gewebeprobe; (c) eine Zellprobe und (a) ein Zellbiopsie.

8. Eine Verfahren zu jedem der Ansprüche 1 bis 7, worin der Nachweis ein immunzytochemisches Nachweisverfahren umfasst.

9. Das Verfahren entsprechend jedem der Ansprüche 1 bis 7, worin die Nachweisreaktion die Reaktion einer Nukleinsäureverstärkung umfasst.

10. Ein in vitro Verfahren für die Bestimmung oder Überwachung einer Stoffwechselsyndromstörungstherapie bei einem Subjekt, das genannte Verfahren umfasst die Analyse der Höhe der IRAK3 Expression oder der Aktivität des Expressionsprodukts in einer Probe des benannten Subjekts vor und nach der Behandlung mit der erwähnten Therapie, wobei ein erhöhter Wert von IRAK3, verglichen mit der Probe des benannten Subjekts vor der Therapie, ein Hinweis für die Wirkung der benannten Therapie ist.

11. Ein in vitro Verfahren für die Bestimmung oder Überwachung einer Stoffwechselsyndromstörungstherapie bei einem Subjekt, das genannte Verfahren umfasst die Analyse des Expressionsprofils in einer biologischen Probe des benannten Subjekts vor und nach der Behandlung mit der erwähnten Therapie, worin das genannte Expressionsprofil aus der Analyse der Expressionshöhe der IRAK3 Expression oder der Aktivität eines IRAK3 Expressionsprodukts in Kombination mit der Höhe der Genexpression oder Aktivität eines Genprodukts von mindestens einem Gen aus der Gruppe SOD2, TNFAIP6, TNFAIP3, TLR2, IRS2, und ZNF217 besteht; und wobei ein erhöhter Wert von IRAK3 und ein verringerter Wert von SOD2, TNFAIP3 und/oder TNFAIP3, verglichen mit der Probe des benannten Subjekts vor der Therapie ein Hinweis für die Wirkung der benannten Therapie ist.

12. Ein diagnostischer Testsatz für die Anwendung bei der Diagnose des Erscheinungsbilds einer Stoffwechselsyndromstörung bei einem Subjekt, oder für die Anwendung zur Überwachung der Therapiewirksamkeit einer Stoffwechselsyndromstörung bei Patienten, oder um festzustellen, ob ein Patient Kandidat für eine Stoffwechselsyndromstörung ist, dies umfasst
- a) eine Vorbestimmte Menge eines ersten Antikörpers spezifisch für IRAK3, und eine Vorbestimmte Menge von wenigstens einem zweiten Antikörper spezifisch für ein oder mehrere SOD2, TNFAIP6, TNFAIP3, TLR2 oder IRS2
- b) eine Vorbestimmte Menge eines spezifischen Bindungspartners zum erwähnten ersten Antikörper, und eine Vorbestimmte Menge eines spezifischen Bindungspartners mindestens zum erwähnten zweiten Antikörper;
- c) Puffer und andere Reagenzien, die notwendig sind für die Überwachung des Nachweises von Antikörpern, die an IRAK3 gebunden sind, mindestens einer der benannten SOD2, TNFAIP6, TNFAIP3, TLR2 oder IRS2; und wobei der genannte Antikörper oder spezifische Bindungspartner detektierbar markiert ist.

13. Ein diagnostischer Testsatz für die Anwendung bei der Diagnose des Erscheinungsbilds einer Stoffwechselsyndromstörung bei einem Subjekt, oder für die Anwendung bei der Überwachung der Therapiewirksamkeit einer Stoffwechselsyndromstörung bei Patienten, die eine solche Therapie erhalten, oder zur Bestimmung, ob jemand Kandidat für eine Stoffwechselsyndromstörung ist, dies umfasst:
- a) eine Vorbestimmte Menge von mindestens einem zweiten Antikörper spezifisch für ZNF217, und eine Vorbestimmte Menge von mindestens einem zweiten Antikörper spezifisch für einen oder mehrere aus SOD2, TNFAIP6, TNFAIP3, TLR2, IRAK3 oder IRS2;
- b) eine Vorbestimmte Menge eines spezifischen Bindungspartners für den erwähnten ersten Antikörper, und eine Vorbestimmte Menge eines spezifischen Bindungspartners mindestens für den erwähnten zweiten Antikörper;
- c) Puffer und andere Reagenzien, die notwendig sind für die Überwachung des Nachweises von Antikörpern, die an ZNF217 gebunden sind und benannte eine oder mehrere aus SOD2, TNFAIP6, TNFAIP3, TLR2, IRAK3, oder IRS2; und worin entweder der benannte Antikörper oder der benannte spezifische Bindungspartner detektierbar markiert ist.

14. Ein diagnostischer Testsatz für die Anwendung bei der Diagnose des Erscheinungsbilds einer Stoffwechselsyndromstörung bei einem Subjekt, oder für die Anwendung bei der Überwachung der Therapiewirksamkeit einer Stoffwechselsyndromstörung bei Patienten, die eine solche Therapie erhalten, oder um zu bestimmen, ob jemand Kandidat für eine Stoffwechselsyndromstörung ist, dies umfasst:
- a) eine oder mehrere Nukleinsäuren, die eine oder mehre Proteine aus der Gruppe ZNF217 und IRAK3 kodieren; - b) mindestens eine Nukleinsäure, die Proteine aus der Gruppe SOD2, TNFAIP6, TNFAIP3, TLR2, and IRS2 kodiert;
- c) nützliche Reagenzien für die Überwachung der Expressionshöhe von einer oder mehreren Nukleinsäuren oder Proteinen, kodiert durch die Nukleinsäuren von Schritt a) - b); und
- d) Anweisungen für die Anwendung des Satzes.

## Revendications

1. Une méthode de diagnostic in vitro du syndrome d'insulino-résistance chez un patient, ladite méthode comprenant: (a) l'analyse du niveau d'expression génique ou d'expression IRAK3, dans un prélèvement biologique isolé dudit patient, et (b) la comparaison dudit niveau d'expression ou activité avec l'expression ou l'activité IRAK3 dans un échantillon témoin; où un niveau plus faible d'expression ou d'activité IRAK3 par rapport à un échantillon témoin est un indicateur de l'existence d'un tel syndrome d'insulino-résistance ou une propension envers ce syndrome.

2. Une méthode de diagnostic in vitro d'un syndrome d'insulino-résistance, ou d'une propension envers ce syndrome chez un patient, ladite méthode (a) obtenant un profil de l'expression génique dans un échantillon biologique isolé dans ledit patient, dans lequel le profil d'expression génique consiste en une analyse du niveau d'expression ou d'activité IRAK3 combiné avec le niveau de l'expression ou de l'activité du gène d'un produit génétique d'au moins un gène, sélectionné dans un groupe de en SOD2, TNFAIP6, TNFAIP3, TLR2, IRS2, et ZNF217; (b) en comparant le profil d'expression génique avec une expression de profil de référence afin de déterminer si ledit échantillon provient du patient ayant un syndrome d'insulino-résistance ou une propension envers ce syndrome.

3. La méthode in vitro des faits 1 ou 2, où le syndrome d'insulino-résistance est une accumulation de tissus adipeux ou d'une fonction adipeuse altérée, une condition de tolérance au glucose altéré, un syndrome de résistance à l'insuline ou diabète de type II, un trouble d'homéostasie lipidique ou une maladie cardiovasculaire voisine.

4. La méthode in vitro des faits 4, dans lequel la maladie cardiovasculaire appartient à un groupe d'hypertension, d'insuffisance coronaire, d'insuffisance cardiaque, d'insuffisance cardiaque congestive, d'athéroscléroses, d'artérioscléroses, d'apoplexie, de maladie cérébro-vasculaire, d'infarctus du myocarde et de résistance vasculaire périphérique.

5. La méthode in vitro des faits 1, 2 ou 3, ladite méthode comprenant une analyse du niveau d'expression génique ou d'activité IRAK3 dans un échantillon isolé dudit patient, où un niveau plus faible d'expression ou d'activité IRAK3 en rapport avec un échantillon témoin, est un indicateur de ce syndrome de tissus adipeux, d'un trouble d'homéostasie lipidique et/ou d'une tolérance au glucose altéré ou d'une condition de résistance à l'insuline et/ou en rapport avec une maladie cardiovasculaire ou une propension envers ce syndrome.

6. La méthode in vitro d'un des faits précédents, où l'expression génique est une molécule d'acide nucléique sélectionnée d'un groupe de mRNA et cDNA mRNA ou de polypeptides dérivés.

7. La méthode in vitro d'un des faits précédents, où l'échantillon isolé dudit patient est sélectionné à partir d'un groupe de a) liquide contenant des cellules; (b) un échantillon du tissu; (c) un échantillon cellulaire et (a) une biopsie cellulaire.

8. La méthode conforme à l'une des faits 1 à 7, par lequel la détection comprend une procédure de détection immunocytochimique.

9. La méthode conforme à l'un des faits 1 à 7, où la réaction de mise en évidence comprend une réaction d'amplification d'acide nucléique.

10. Une méthode in vitro afin d'identifier ou de contrôler une thérapie du syndrome d'insulino-résistance chez un patient, ladite méthode comprenant une analyse du niveau d'expression génique ou d'activité IRAK3 dans un échantillon isolé dudit patient, avant et après le traitement, avec ladite thérapie, où un niveau plus élevé d'IRAK3 comparé à un échantillon dudit patient avant la thérapie, est un indicateur de l'efficacité de ladite thérapie.

11. Une méthode in vitro pour identifier ou contrôler une thérapie de syndrome d'insulino-résistance chez un patient, ladite méthode comprenant une analyse du profil d'expression génique dans un échantillon biologique isolé dudit patient, avant et après le traitement avec ladite thérapie, par lequel le profil d'expression consiste en une analyse du niveau d'expression génique ou d'activité IRAK3 combiné avec le niveau d'expression génique ou d'activité d'un produit génique d'au moins un des gènes sélectionnés dans groupe de SOD2, TNFAIP6, TNFAIP3, TLR2, IRS2, et ZNF217; et par lequel un niveau plus élevé d'IRAK3 et un niveau moindre de SOD2, TNFAIP3 et/ou TNFAIP3 comparé à l'échantillon dudit patient, avant la thérapie, sont un indicateur de l'efficacité de ladite thérapie.

12. Un kit de test diagnostique utilisé pour diagnostiquer un syndrome d'insulino-résistance chez un patient, ou pour contrôler l'efficacité de la thérapie du syndrome d'insulino-résistance chez des patients suivant cette thérapie, ou pour prédire si le patient est un candidat potentiel du syndrome d'insulino-résistance comprenant,
- a) une quantité prédéterminée du premier anticorps spécifique à l' IRAK3, et une quantité prédéterminée d'au moins un deuxième anticorps spécifique pour un ou plus des ou SOD2, TNFAIP6, TNFAIP3, TLR2 ou IRS2
- b) une quantité prédéterminée d'un partenaire spécifique de liaison du premier anticorps, et une quantité prédéterminée de partenaire spécifique de liaison, au moins du second anticorps;
- c) les substances tampons et autres réactifs nécessaires pour le contrôle de détection d'un anticorps lié à l'IRAK3 et pour un ou plusieurs SOD2, TNFAIP6, TNFAIP3, TLR2 ou IRS2; et
où soit ledit anticorps où ledit partenaire de liaison spécifique sont catalogués de façon intelligible.

13. Un kit de test diagnostique à utiliser pour diagnostiquer le syndrome d'insulino-résistance chez un patient, ou pour contrôler l'efficacité de la thérapie du syndrome d'insulino-résistance chez les patients suivant cette thérapie, ou pour prévoir si le patient est un candidat potentiel du syndrome d'insulino-résistance comprenant:
- a) une quantité prédéterminée d'un anticorps spécifique pour le ZNF217, et une quantité prédéterminée d'au moins un deuxième anticorps spécifique pour un ou plusieurs SOD2, TNFAIP6, TNFAIP3, TLR2, IRAK3 ou IRS2;
- b) une quantité prédéterminée de partenaires de liaison spécifique pour le premier anticorps, et une quantité prédéterminée de partenaires de liaison spécifique du deuxième anticorps;
- c) les substances tampons ou autres réactifs nécessaires pour contrôler la détection des anticorps liés au ZNF217 et un ou plusieurs SOD2, TNFAIP6, TNFAIP3, TLR2, IRAK3, ou IRS2; et
où les anticorps ou ledit partenaire de liaison spécifique sont catalogués de façon intelligible.

14. Un kit de test diagnostique utilisé pour diagnostiquer un syndrome d'insulino-résistance chez un patient, ou pour contrôler l'efficacité de la thérapie du syndrome d'insulino-résistance chez des patients suivant cette thérapie, ou pour prévoir si le patient est un candidat potentiel au syndrome d'insulino-résistance comprenant:
- a) un ou plusieurs acides nucléiques encodés, une ou plusieurs des protéines sélectionnées dans le groupe de ZNF217 et d'IRAK3;
- b) au moins un encodage d'acides nucléiques des protéines sélectionnées dans un groupe de SOD2, TNFAIP6, TNFAIP3, TLR2, et IRS2;
- c) des réactifs utiles dans le contrôle de l'expression génique niveau d'un ou plusieurs acides nucléiques ou protéines encodées par des acides nucléiques des étapes a) - b); et
- d) instructions pour le kit.
